(19) Europäisches Patentamt
European Patent Office
Office européen des brevets

(11) **EP 1 709 181 B1**

(12) **EUROPEAN PATENT SPECIFICATION**

(45) Date of publication and mention
of the grant of the patent:
**28.01.2009 Bulletin 2009/05**

(51) Int Cl.:
**C12N 15/80** (2006.01)     **C12N 15/67** (2006.01)
**C12N 5/10** (2006.01)

(21) Application number: **04806256.6**

(22) Date of filing: **23.12.2004**

(86) International application number:
**PCT/GB2004/005462**

(87) International publication number:
**WO 2005/061718 (07.07.2005 Gazette 2005/27)**

(54) **GENE EXPRESSION TECHNIQUE**

GENEXPRESSIONSMETHODE

TECHNIQUE D'EXPRESSION GENIQUE

(84) Designated Contracting States:
**AT BE BG CH CY CZ DE DK EE ES FI FR GB GR HU IE IS IT LI LT LU MC NL PL PT RO SE SI SK TR**

(30) Priority: **23.12.2003 GB 0329681**

(43) Date of publication of application:
**11.10.2006 Bulletin 2006/41**

(60) Divisional application:
**08169442.4**

(73) Proprietor: **Novozymes Biopharma DK A/S
2880 Bagsvaerd (DK)**

(72) Inventors:
• **SLEEP, Darrell
c/o Novozymes Biopharma UK Limited
Nottingham NG7 1FD (GB)**
• **SHUTTLEWORTH, Gillian
c/o Novozymes Biopharma UK Limited
Nottingham NG7 1FD (GB)**
• **FINNIS, Christopher John Arthur
c/o Novozymes Biopharma UK Limited
Nottingham NG7 1FD (GB)**

(74) Representative: **Williams, Rachel Clare
Novozymes Biopharma UK Limited
Castle Court
59 Castle Boulevard
Nottingham
NG7 1FD (GB)**

(56) References cited:
• MARTZEN MARK R ET AL: "A biochemical genomics approach for identifying genes by the activity of their products" SCIENCE (WASHINGTON D C), vol. 286, no. 5442, 5 November 1999 (1999-11-05), pages 1153-1155, XP002325596 ISSN: 0036-8075
• "pYEX4T-1 Vector Information" 1998, CLONTECH CATALOG #6196-1 , XP002325601
• CREASEY ELIZABETH A ET AL: "CesT is a bivalent enteropathogenic Escherichia coli chaperone required for translocation of both Tir and Map." MOLECULAR MICROBIOLOGY, vol. 47, no. 1, January 2003 (2003-01), pages 209-221, XP002325595 ISSN: 0950-382X
• "BD pBridge Three-Hybrid Vector" 2003, CLONTECH CATALOGUE , XP002325598 page 264; figure 2
• PAREKH RAJESH N ET AL: "Expression level tuning for optimal heterologous protein secretion in Saccharomyces cerevisiae" BIOTECHNOLOGY PROGRESS, vol. 13, no. 2, 1997, pages 117-122, XP002325597 ISSN: 8756-7938 cited in the application
• BAO W-G ET AL: "Secretion of human proteins from yeast: stimulation by duplication of polyubiquitin and protein disulfide isomerase genes in Kluyveromyces lactis" GENE: AN INTERNATIONAL JOURNAL ON GENES AND GENOMES, ELSEVIER SCIENCE PUBLISHERS, BARKING, GB, vol. 272, no. 1-2, 11 July 2001 (2001-07-11), pages 103-110, XP004274844 ISSN: 0378-1119 cited in the application

EP 1 709 181 B1

**Description**

**FIELD OF THE INVENTION**

The present application relates to gene expression techniques.

**BACKGROUND OF THE INTENTION**

The class of proteins known as chaperones have been defined by Hartl (1996, Nature, 381, 571-580) as a protein that binds to and stabilises an otherwise unstable conformer of another protein and, by controlled binding and release, facilitates its correct fate *in vivo,* be it folding, oligomeric assembly, transport to a particular subcellular compartment, or disposal by degradation.

BiP (also known as GRP78, Ig heavy chain binding protein and Kar2p in yeast) is an abundant ~70kDa chaperone of the hsp 70 family, resident in the endoplasmic reticulum (ER), which amongst other functions, serves to assist in transport in the secretory system and fold proteins.

Protein disulphide isomerase (PDI) is a chaperone protein, resident in the ER that is involved in the catalysis of disulphide bond formation during the post-translational processing of proteins.

Studies of the secretion of both native and foreign proteins have shown that transit from the ER to the Golgi is the rate-limiting step. Evidence points to a transient association of the BiP with normal proteins and a more stable interaction with mutant or misfolded forms of a protein. As a result, BiP may play a dual role in solubilising folding precursors and preventing the transport of unfolded and unassembled proteins. Robinson and Wittrup, 1995, Biotechnol. Prog. 11, 171-177, have examined the effect of foreign protein secretion on BiP (Kar2p) and PDI, protein levels in *Saccharomyces cerevisiae* and found that prolonged constitutive expression of foreign secreted proteins reduces soluble BiP and PDI to levels undetectable by Western analysis. The lowering of ER chaperone and foldase levels as a consequence of heterologous protein secretion has important implications for attempts to improve yeast expression/secretion systems.

Expression of chaperones is regulated by a number of mechanisms, including the unfolded protein response (UPR).

Using recombinant techniques, multiple PDI gene copies has been shown to increase PDI protein levels in a host cell (Farquhar et al, 1991, Gene, 108, 81-89).

Co-expression of the gene encoding PDI and a gene encoding a heterologous disulphide-bonded protein was first suggested in WO 93/25676, published on 23 December 1993, as a means of increasing the production of the heterologous protein. WO 93/25676 reports that the recombinant expression of antistasin and tick anticoagulant protein can be increased by co-expression with PDI.

This strategy has been exploited to increase the recombinant expression of other types of protein.

Robinson et al, 1994, Bio/Technology, 12, 381-384 reported that a recombinant additional PDI gene copy in *Saccharomyces cerevisiae* could be used to increase the recombinant expression of human platelet derived growth factor (PDGF) B homodimer by ten-fold and *Schizosacharomyces pombe* acid phosphatase by four-fold.

Hayano et al, 1995, FEBS Letters, 377, 505-511 described the co-expression of human lysozyme and PDI in yeast. Increases of around 30-60% in functional lysozyme production and secretion were observed.

Shusta et al, 1998, Nature Biotechnology, 16, 773-777 reported that the recombinant expression of single-chain antibody fragments (scFv) in *Saccharomyces cerevisiae* could be increased by between 2-8 fold by over-expressing PDI in the host cell.

Bao & Fukuhara, 2001, Gene, 272, 103-110 reported that the expression and secretion of recombinant human serum albumin (rHSA) in the yeast *Kluyveromyces lactis* could be increased by 15-fold or more by co-expression with an additional recombinant copy of the yeast PDI gene (*KlPDI1*).

In order to produce co-transformed yeast comprising both a PDI gene and a gene for a heterologous protein, WO 93/25676 taught that the two genes could be chromosomally integrated; one could be chromosomally integrated and one present on a plasmid; each gene could be introduced on a different plasmid; or both genes could be introduced on the same plasmid. WO 93/25676 exemplified expression of antistasin from the plasmid pKH4α2 in yeast strains having as chromosomally integrated additional copy of a PDI gene (Examples 16 and 17); expression of antistasin from the vector K991 with an additional PDI gene copy being present on a multicopy yeast shuttle vector named YEp24 (Botstein et al, 1979, Gene, 8, 17-24) (Example 20); and expression of both the antistasin and the PDI genes from the yeast shuttle vector pC1/1 (Rosenberg et al, 1984, Nature, 312, 77-80) under control of the GAL10 and GAL1 promoters, respectively. Indeed, Robinson and Wittrup, 1995, *op. cit.,* also used the GAL1-GAL10 intergenic region to express erythropoietin and concluded that production yeast strains for the secretion of heterologous proteins should be constructed using tightly repressible, inducible promoters, otherwise the negative effects of sustained secretion (i.e. lowered detectable BiP and PDI) would be dominant after the many generations of cell growth required to fill a large-scale fermenter.

Subsequent work in the field has identified chromosomal integration of transgenes as the key to maximising recombinant protein production.

Robinson *et al,* 1994, *op. cit.,* obtained the observed increases in expression of PDGF and *S. pombe* acid phosphatase using an additional chromosomally integrated PDI gene copy. *Robinson et al* reported that attempts to use the multi-copy 2μm expression vector to increase PDI protein levels had had a detrimental effect on heterologous protein secretion.

Hayano *et al,* 1995, *op. cit.* described the introduction of genes for human lysozyme and PDI into a yeast host each on a separate linearised integration vector, thereby to bring about chromosomal integration.

Shusta *et al,* 1998, *op. cit.,* reported that in yeast systems, the choice between integration of a transgene into the host chromosome versus the use of episomal expression vectors can greatly affect secretion and, with reference to Parekh & Wittrup, 1997, Biotechnol. Prog., 13, 117-122, that stable integration of the scFv gene into the host chromosome using a δ integration vector was superior to the use of a 2μm-based expression plasmid. Parekh & Wittrup, *op. cit.,* had previously taught that the expression of bovine pancreatic trypsin inhibitor (BPTI) was increased by an order of magnitude using a δ integration vector rather than a 2μm-based expression plasmid. The 2μm-based expression plasmid was said to be counter-productive for the production of heterologous secreted protein.

Bao & Fukuhara, 2001, *op. cit.,* reported that "It was first thought that the *KlPDI1* gene might be directly introduced into the multi-copy vector that carried the rHSA expression cassette. However, such constructs were found to severely affect yeast growth and plasmid stability. This confirmed our previous finding that the *KlPDI1* gene on a multi-copy vector was detrimental to growth of *K lactis* cells (Bao *et al,* 2000)". Bao et. al, 2000, Yeast, 16, 329-341, as referred to in the above-quoted passage of Bao & Fukuhara reported that the *KlPDII* gene had been introduced into *K lactis* on a multi-copy plasmid, plan707, and that the presence of the plasmid caused the strain to grow poorly. Bao *et al* concluded that over-expression of the *KlPDII* gene was toxic to *K lactis* cells. In the light of the earlier findings in *Bao et al,* Bao & Fukuhara chose to introduce a single duplication of *klPDII* on the host chromosome.

Martzen et al, 1999, Science, 286, 1153-1155 reports that a method for identifying yeast genes specifying biochemical activities, using a genomic strategy that is said to be rapid, sensitive, and widely applicable was developed with an array of 6144 individual yeast strains, each containing a different yeast open reading frame (ORF) fused to glutathione S-transferase (GST). For the identification of ORF-associated activities, strains were grown in defined pools, and GST-ORFs were purified. Then, pools were assayed for activities, and active pools were deconvoluted to identify the source strains.

Against this background, we have surprisingly demonstrated that, contrary to the suggestions in the prior art, when the genes for a chaperone protein and a heterologous protein are co-expressed on a 2μm-family multi-copy plasmid in yeast, the production of the heterologous protein is substantially increased.

## DESCRIPTION OF THE INVENTION

A first aspect of the present invention provides a method for producing heterologous protein comprising:

(a) providing a host cell comprising a 2μm-family plasmid, the plasmid comprising a gene encoding a protein comprising the sequence of a chaperone protein and a gene encoding a heterologous protein;

(b) culturing the host cell in a culture medium under conditions that allow the co-expression of the gene encoding the chaperone protein and the gene encoding a heterologous protein;

(c) purifying the thus expressed heterologous protein from the culture medium; and

(d) optionally, lyophilising the thus purified protein.

In one embodiment, step (c) purifies the thus expressed heterologous protein to a commercially acceptable level of purity or a pharmaceutically acceptable level of purity.

Preferably, the method further comprises the step of formulating the purified heterologous protein with a carrier or diluent, such as a pharmaceutically acceptable carrier or diluent and optionally presenting the thus formulated protein in a unit dosage form.

A second aspect of the present invention provides for the use of a 2μm-family plasmid as an expression vector to increase the production of a fungal (preferably yeast) or vertebrate heterologous protein by providing a gene encoding the heterologous protein and a gene encoding a protein comprising the sequence of a chaperone protein on the same 2μm-family plasmid.

A third aspect of the present invention provides a 2μm-family plasmid comprising a gene encoding a protein comprising the sequence of a chaperone protein and a gene encoding a heterologous protein, wherein if the plasmid is based on the 2μm plasmid then it is a disintegration vector.

A fourth aspect of the invention provides a host cell comprising a plasmid as defined above.

The present invention relates to recombinantly modified versions of 2μm-family plasmids.

Certain closely related species of budding yeast have been shown to contain naturally occurring circular double stranded DNA plasmids These plasmids, collectively termed 2μm-family plasmids, include pSR1, pSB3 and pSB4 from *Zygosaccharoinyces rouxii* (formerly classified as *Zygosaccharomyces bisporus*), plasmids pSB1 and pSB2 from *Zygosaccharomyces bailii,* plasmid pSM1 from *Zygosaccharomyces fermentati,* plasmid pKD1 from *Kluyveromyces drosphilarum,* an un-named plasmid from *Pichia membranaefaciens* (hereinafter "pPM1") and the 2μm plasmid and variants (such as Scp1, Scp2 and Scp3) from *Saccharomyces cerevisiae* (Volkert, et al., 1989, Microbiological Reviews, 53, 299; Murray et al., 1988, J. Mol. Biol. 200, 601; Painting, et al., 1984, J. Applied Bacteriology, 56, 331).

As a family of plasmids these molecules share a series of common features in that they typically possess two inverted repeats on opposite sides of the plasmid, have a similar size around 6-kbp (range 4757 to 6615-bp), three open reading frames, one of which encodes for a site specific recombinase (*FLP*) and an autonomously replicating sequence (*ARS*), also known as an origin of replication (*ori*)*,* located close to the end of one of the inverted repeats. (Futcher, 1988, Yeast, 4, 27; Murray *et al., op. cit.,* and Toh-e et al., 1986, Basic Life Sci. 40, 425). Despite their lack of discernible DNA sequence homology, their shared molecular architecture and the conservation of function of the three open reading frames have demonstrated a common ancestral link between the family members.

Whilst any of the above naturally occurring 2μm-family plasmids can be used in the present invention, this invention is not limited to the use of naturally occurring 2μm-family plasmids. For the purposes of this invention, a 2μm-family plasmid is as described below.

A 2μm-family plasmid is a circular, double stranded, DNA plasmid. It is typically small, such as between 3,000 to 10,000 bp, preferably between 4,500 to 7000 bp, excluding recombinantly inserted sequences.

A 2μm-family plasmid typically comprises at least three open reading frames ("ORFs") that each encodes a protein that functions in the stable maintenance of the 2μm-family plasmid as a multicopy plasmid. The proteins encoded by the three ORFs can be designated FLP, REP1 and REP2. Where a 2μm-family plasmid comprises not all three of the ORFs encoding FLP, REP1 and REP2 then ORFs encoding the missing protein(s) should be supplied in *trans,* either on another plasmid or by chromosomal integration.

A "FLP" protein is a protein capable of catalysing the site-specific recombination between inverted repeat sequences recognised by FLP. The inverted repeat sequences are termed FLP recombination target (FRT) sites and each is typically present as part of a larger inverted repeat (see below). Preferred FLP proteins comprise the sequence of the FLP proteins encoded by one of plasmids pSR1, pSB1, pSB2, pSB3, pSB4, pSM1, pKD1, pPM1 and the 2μm plasmid, for example as described in Volkert *et al, op. cit.,* Murray *et al, op. cit.,* and Painting *et al., op. cit.* Variants and fragments of these FLP proteins are also included in the present invention. "Fragments" and "variants" are those which retain the ability of the native protein to catalyse the site-specific recombination between the same FRT sequences. Such variants and fragments will usually have at least 50%, 60%, 70%, 80%, 90%, 95%, 98%, 99%, or more, homology with an FLP protein encoded by one of plasmids pSR1, pSB1, pSB2, pSB3, pSB4, pSM1, pKD1, pPM1 and the 2μm plasmid. Different FLP proteins can have different FRT sequence specificities. A typical FRT site may comprise a core nucleotide sequence flanked by inverted repeat sequences. In the 2μm plasmid, the FRT core sequence is 8 nucleotides in length and the flanking inverted repeat sequences are 13 nucleotides in length (Volkert *et al, op. cit.*). However the FRT site recognised by any given FLP protein may be different to the 2μm plasmid FRT site.

REP 1 and REP2 are proteins involved in the partitioning of plasmid copies during cell division, and may also have a role in the regulation of FLP expression. Considerable sequence divergence has been observed between REP1 proteins from different 2μm-family plasmids, whereas no sequence alignment is possible between REP2 proteins derived from different 2μm-family plasmids. Preferred REP1 and REP2 proteins comprise the sequence of the REP1 and REP2 proteins encoded by one of plasmids pSR1, pSB1, pSB2, pSB3, pSB4, pSM1, pKD1, pPM1 and the 2μm plasmid, for example as described in Volkert *et al, op. cit.,* Murray *et al, op. cit.,* and Painting *et al, op. cit.* Variants and fragments of these REP1 and REP2 proteins are also included in the present invention. "Fragments" and "variants" of REP 1 and REP2 are those which, when encoded by the plasmid in place of the native ORF, do not substantially disrupt the stable multicopy maintenance of the plasmid within a suitable yeast population. Such variants and fragments of REP1 and REP2 will usually have at least 5%, 10%, 20%, 30%, 40%, 50%, 60%, 70%, 80%, 90%, 95%, 98%, 99%, or more, homology with a REP1 and REP2 protein, respectively, as encoded by one of plasmids pSR1, pSB1, pSB2, pSB3, pSB4, pSM1, pKD1, pPM1 and the 2μm plasmid.

The REP1 and REP2 proteins encoded by the ORFs on the plasmid must be compatible. It is preferred that the REP1 and REP2 proteins have the sequences of REP1 and REP2 proteins encoded by the same naturally occurring 2μm-family plasmid, such as pSR1, pSB1, pSB2, pSB3, pSB4, pSM1, pKD1, pPM1 and the 2μm plasmid, or variant or fragments thereof.

A 2μm-family plasmid typically comprises two inverted repeat sequences. The inverted repeats may be any size, so long as they each contain an FRT site (see above). The inverted repeats are typically highly homologous. They may share greater than 50%, 60%, 70%, 80%, 90%, 95%, 96%, 97%, 98%, 99%, 99.5% or more sequence identity. In a preferred embodiment they are identical. Typically the inverted repeats are each between 200 to 1000 bp in length. Preferred inverted repeat sequences may each have a length of from 200 to 300 bp, 300 to 400 bp, 400 to 500 bp, 500 to 600 bp, 600 to 700 bp, 700 to 800 bp, 800 to 900 bp, or 900 to 1000 bp. Particularly preferred inverted repeats are those of the plasmids pSR1 (959 bp), pSB1 (675 bp), pSB2 (477 bp), pSB3 (391 bp), pSM1 (352 bp), pKD1 (346 bp), the $2\mu$m plasmid (599 bp), pSB4 or pPM1.

The sequences of the inverted repeats may be varied. However, the sequences of the FRT site in each inverted repeat should be compatible with the specificity of the FLP protein encoded by the plasmid, thereby to enable the encoded FLP protein to act to catalyse the site-specific recombination between the inverted repeat sequences of the plasmid. Recombination between inverted repeat sequences (and thus the ability of the FLP protein to recognise the FRT sites with the plasmid) can be determined by methods known in the art. For example, a plasmid in a yeast cell under conditions that favour FLP expression can be assayed for changes in the restriction profile of the plasmid which would result from a change in the orientation of a region of the plasmid relative to another region of the plasmid. The detection of changes in restriction profile indicate that the FLP protein is able to recognise the FRT sites in the plasmid and therefore that the FRT site in each inverted repeat are compatible with the specificity of the FLP protein encoded by the plasmid.

In a particularly preferred embodiment, the sequences of inverted repeats, including the FRT sites, are derived from the same $2\mu$m-family plasmid as the ORF encoding the FLP protein, such as pSR1, pSB1, pSB2, pSB3, pSB4, pSM1, pKD1, pPM1 or the $2\mu$m plasmid.

The inverted repeats are typically positioned with the $2\mu$m-family plasmid such that the two regions defined between the inverted repeats (e.g. such as defined as UL and US in the $2\mu$m plasmid) are of approximately similar size, excluding exogenously introduced sequences such as transgenes. For example, one of the two regions may have a length equivalent to at least 40%, 50%, 60%, 70%, 80%, 90%, 95% or more, up to 100%, of the length of the other region.

A $2\mu$m-family plasmid typically comprises the ORF that encodes FLP and one inverted repeat (arbitrarily termed "IR1" to distinguish it from the other inverted repeat mentioned in the next paragraph) juxtaposed in such a manner that IR1 occurs at the distal end of the FLP ORF, without any intervening coding sequence, for example as seen in the $2\mu$m plasmid. By "distal end" in this context we mean the end of the FLP ORF opposite to the end from which the promoter initiates its transcription. In a preferred embodiment, the distal end of the FLP ORF overlaps with IR1.

A $2\mu$m-family plasmid typically comprises the ORF that encodes REP2 and the other inverted repeat (arbitrarily termed "IR2" to distinguish it from IR1 mentioned in the previous paragraph) juxtaposed in such a manner that IR2 occurs at the distal end of the REP2 ORF, without any intervening coding sequence, for example as seen in the $2\mu$m plasmid. By "distal end" in this context we mean the end of the REP2 ORF opposite to the end from which the promoter initiates its transcription.

In one embodiment, the ORFs encoding REP2 and FLP may be present on the same region of the two regions defined between the inverted repeats of the $2\mu$m-family plasmid, which region may be the bigger or smaller of the regions (if there is any inequality in size between the two regions).

In one embodiment, the ORFs encoding REP2 and FLP may be transcribed from divergent promoters.

Typically, the regions defined between the inverted repeats (e.g. such as defined as UL and US in the $2\mu$m plasmid) of a $2\mu$m-family plasmid may comprise not more than two endogenous genes that encode a protein that functions in the stable maintenance of the $2\mu$m-family plasmid as a multicopy plasmid. Thus in a preferred embodiment, one region of the plasmid defined between the inverted repeats may comprise not more than the ORFs encoding FLP and REP2; FLP and REP1; or REP1 and REP2, as endogenous coding sequence.

A $2\mu$m-family plasmid typically comprises an origin of replication (also known as an "autonomously replicating sequence - "ARS"), which is typically bidirectional. Any appropriate ARS sequence can be present. Consensus sequences typical of yeast chromosomal origins of replication may be appropriate (Broach et al, 1982, Cold Spring Harbor Symp. Quant. Biol., 47, 1165-1174; Williamson, Yeast, 1985, 1, 1-14). Preferred ARSs include those isolated from pSR1, pSB1, pSB2, pSB3, pSB4, pSM1, pKD1, pPM1 and the $2\mu$m plasmid.

Thus, a preferred $2\mu$m-family plasmid may comprise ORFs encoding FLP, REP1 and REP2, two inverted repeat sequences each inverted repeat comprising an FRT site compatible with the encoded FLP protein, and an ARS sequence. Preferably the FRT sites are derived from the same $2\mu$m-family plasmid as the sequence of the encoded FLP protein. More preferably the sequences of the encoded REP1 and REP2 proteins are derived from the same $2\mu$m-family plasmid as each other. Even more preferably, the FRT sites are derived from the same $2\mu$m-family plasmid as the sequence of the encoded FLP, REP1 and REP2 proteins. Yet more preferably, the sequences of the ORFs encoding FLP, REP1 and REP2, and the sequence of the inverted repeats (including the FRT sites) are derived from the same $2\mu$m-family plasmid. Furthermore, the ARS site may be derived from the same $2\mu$m-family plasmid as one or more of the ORFs of FLP, REP1 and REP2, and the sequence of the inverted repeats (including the FRT sites).

The term "derived from" includes sequences having an identical sequence to the sequence from which they are derived. However/variants and fragments thereof, as defined above, are also included. For example, an *FLP* gene having a sequence derived from the *FLP* gene of the 2μm plasmid may have a modified promoter or other regulatory sequence compared to that of the naturally occurring gene.

Additionally or alternatively, an *FLP* gene having a sequence derived from the *FLP* gene of the 2μm plasmid may have a modified nucleotide sequence in the open reading frame which may encode the same protein as the naturally occurring gene, or may encode a modified FLP protein. The same considerations apply to other sequences on a 2μm-family plasmid having a sequence derived from a particular source.

Optionally, a 2μm-family plasmid may comprise a region derived from the *STB* region (also known as REP3) of the 2μm plasmid, as defined in Volkert *et al, op. cit.* The *STB* region in a 2μm-family plasmid of the invention may comprise two or more tandem repeat sequences, such as three, four, five or more. Alternatively, no tandem repeat sequences may be present. The tandem repeats may be any size, such as 10, 20, 30, 40, 50, 60 70, 80, 90, 100 bp or more in length. The tandem repeats in the *STB* region of the 2μm plasmid are 62 bp in length. It is not essential for the sequences of the tandem repeats to be identical. Slight sequence variation can be tolerated. It may be preferable to select an *STB* region from the same plasmid as either or both of the REP1 and REP2 ORFs. The *STB* region is thought to be a *cis*-acting element and preferably is not transcribed.

Optionally, a 2μm-family plasmid may comprise an additional ORF that encodes a protein that functions in the stable maintenance of the 2μm-family plasmid as a multicopy plasmid. The additional protein can be designated RAF or D. ORFs encoding the RAF or D gene can be seen on, for example, the 2μm plasmid and pSM1. Thus a RAF or D ORF can comprise a sequence suitable to encode the protein product of the RAF or D gene ORFs encoded by the 2μm plasmid or pSM1, or variants and fragments thereof. Thus variants and fragments of the protein products of the RAF or D genes of the 2μm plasmid or pSM1 are also included in the present invention. "Fragments" and "variants" of the protein products of the RAF or D genes of the 2μm plasmid or pSM1 are those which, when encoded by the 2μm plasmid or pSM1 in place of the native ORF, do not disrupt the stable multicopy maintenance of the plasmid within a suitable yeast population. Such variants and fragments will usually have at least 5%, 10%, 20%, 30%, 40%, 50%, 60%, 70%, 80%, 90%, 95%, 98%, 99%, or more, homology with the protein product of the RAF or D gene ORFs encoded by the 2μm plasmid or pSM1.

A naturally occurring 2μm-family plasmid may be preferred. A naturally occurring 2μm-family plasmid is any plasmid having the features defined above, which plasmid is found to naturally exist in yeast, i.e. has not been recombinantly modified to include heterologous sequence. Preferably the naturally occurring 2μm-family plasmid is selected from pSR1 (Accession No. X02398), pSB3 (Accession No. X02608) or pSB4 as obtained from *Zygosaccharomyces rouxii,* pSB1 or pSB2 (Accession No. NC_002055 or M18274) both as obtained from *Zygosaccharomyces bailli,* pSM1 (Accession No. NC_002054) as obtained from *Zygosaccharomyces fermentati,* pKD1 (Accession No. X03961) as obtained from *Kluyveromyces drosophilarum,* pPM1 from *Pichia membranaefaciens* or, most preferably, the 2μm plasmid (Accession No. NC_001398 or J01347) as obtained from *Saccharomyces cerevisiae.* Accession numbers in this paragraph refer to NCBI deposits.

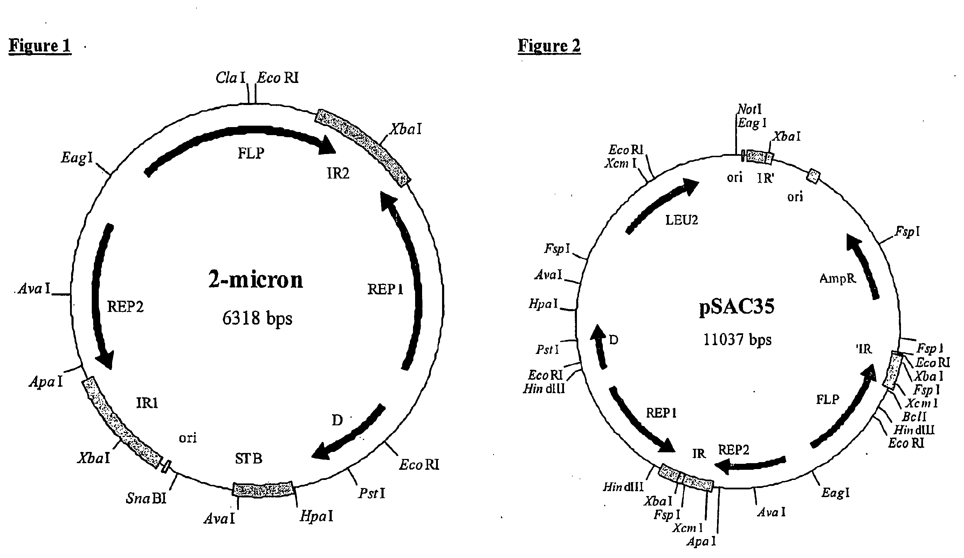

The 2μm plasmid (Figure 1) is a 6,318-bp double-stranded DNA plasmid, endogenous in most *Saccharomyces cerevisiae* strains at 60-100 copies per haploid genome. The 2μm plasmid comprises a small unique (US) region and a large unique (UL) region, separated by two 599-bp inverted repeat sequences. Site-specific recombination of the inverted repeat sequences results in interconversion between the A-form and B-form of the plasmid *in vivo* (Volkert & Broach, 1986, Cell, 46, 541). The two forms of 2μm differ only in the relative orientation of their unique regions.

While DNA sequencing of a cloned 2μm plasmid (also known as Scp1) from *Saccharomyces cerevisiae* gave a size of 6,318-bp (Hartley and Donelson, 1980, Nature, 286, 860), other slightly smaller variants of 2μm, Scp2 and Scp3, are known to exist as a result of small deletions of 125-bp and 220-bp, respectively, in a region known as *STB* (Cameron et al., 1977, Nucl. Acids Res., 4, 1429: Kikuchi, 1983, Cell, 35, 487 and Livingston & Hahne, 1979, Proc. Natl. Acad. Sci. USA, 76, 3727). In one study about 80% of natural *Saccharomyces* strains from around the world contained DNA homologous to 2μm (by Southern blot analysis) (Hollenberg, 1982, Current Topics in Microbiology and Immunobiology, 96, 119). Furthermore, variation (genetic polymorphism) occurs within the natural population of 2μm plasmids found in *S. cerevisiae* and *S. carlsbergensis,* with the NCBI sequence (accession number NC_001398) being one example.

The 2μm plasmid has a nuclear localisation and displays a high level of mitotic stability (Mead et al, 1986, Molecular & General Genetics, 205, 417). The inherent stability, of the 2μm plasmid results from a plasmid-encoded copy number amplification and partitioning mechanism, which can be compromised during the development of chimeric vectors (Futcher & Cox, 1984, J. Bacteriol., 157, 283; Bachmair & Ruis, 1984, Monatshefte für Chemie, 115, 1229). A yeast strain, which contains a 2μm plasmid is known as [cir+], while a yeast strain which does not contain a 2μm plasmid is known as [cir0].

The US-region of the 2μm plasmid contains the *REP2* and *FLP* genes, and the UL-region contains the *REP1* and *D* (also known as *RAF*) genes, the STB-locus and the origin of replication (Broach & Hicks, 1980, Cell, 21, 501; Sutton & Broach, 1985, Mol. Cell. Biol., 5, 2770). The Flp recombinase binds to FRT-sites (Flp Recognition Target) within the inverted repeats to mediate site-specific recombination, which is essential for natural plasmid amplification and control of plasmid copy number *in vivo* (Senecoff et al, 1985, Proc. Natl. Acad Sci. U.SA., 82, 7270; Jayaram, 1985, Proc. Natl. Acad. Sci. U.S.A., 82, 5875). The copy number of 2μm-family plasmids can be significantly affected by changes in Flp recombinase activity (Sleep et al, 2001, Yeast, 18, 403; Rose & Broach, 1990, Methods Enzymol., 185, 234). The Rep1 and Rep2 proteins mediate plasmid segregation, although their mode of action is unclear (Sengupta et al, 2001, J. Bacteriol., 183, 2306). They also repress transcription of the *FLP* gene (Reynolds et al, 1987, Mol. Cell. Biol., 7, 3566).

The *FLP* and *REP2* genes of the 2μm plasmid are transcribed from divergent promoters, with apparently no intervening sequence defined between them. The *FLP* and *REP2* transcripts both terminate at the same sequence motifs within the inverted repeat sequences, at 24-bp and 178-bp respectively after their translation termination codons (Sutton & Broach, 1985, Mol. Cell. Biol., 5, 2770).

In the case of *FLP,* the C-terminal coding sequence also lies within the inverted repeat sequence. Furthermore, the two inverted repeat sequences are highly conserved over 599-bp, a feature considered advantageous to efficient plasmid replication and amplification *in vivo,* although only the FRT-sites (less than 65-bp) are essential for site-specific recombination *in vitro* (Senecoff et al, 1985, Proc. Natl. Acad. Sci. U.S.A., 82; 7270; Jayaram, 1985, Proc. Natl. Acad. Sci. U.SA., 82, 5875; Meyer-Leon et al, 1984, Cold Spring Harbor Symposia On Quantitative Biology, 49, 797). The key catalytic residues of Flp are arginine-308 and tyrosine-343 (which is essential) with strand-cutting facilitated by histidine-309 and histidine 345 (Prasad et al, 1987, Proc. Natl. Acad. Sci. U.S.A., 84, 2189; Chen et al, 1992, Cell, 69, 647; Grainge et al, 2001, J. Mol. Biol., 314, 717).

Two functional domains are described in Rep2. Residues 15-58 form a Rep1-binding domain, and residues 59-296 contain a self-association and STB-binding region (Sengupta et al, 2001, J. Bacteriol., 183, 2306).

Chimeric or large deletion mutant derivatives of 2μm which lack many of the essential functional regions of the 2μm plasmid but retain the functional *cis* element *ARS* and *STB,* cannot effectively partition between mother and daughter cells at cell division. Such plasmids can do so if these functions are supplied in *trans,* by for instance the provision of a functional 2μm plasmid within the host, such as a [cir+] host.

Genes of interest have previously been inserted into the UL-region of the 2μm plasmid. For example, see plasmid pSAC3U1 in EP 0 286 424 and the plasmid shown in Figure 2, which includes a (β-lactamase gene (for ampicillin resistance), *a LEU2* selectable marker and an oligonucleotide lanker, the latter two of which are inserted into a unique *Sna*BI-site within the UL-region of the 2μm-like disintegration vector, pSAC3 (see EP 0 286 424). The *E. coli* DNA between the *Xba*I-sites that contains the ampicillin resistance gene is lost from the plasmid shown in Figure 2 after transformation into yeast. This is described in Chinery & Hinchliffe, 1989, Curr. Genet., 16, 21 and EP 0 286 424, where these types of vectors are designated "disintegration vectors". Further polynucleotide insertions can be made in a NotI-site within a linker (Sleep et al, 1991, Biotechnology (N Y), 9, 183).

Alternative insertion sites in 2μm plasmid are known in the art, including those described in-Rose & Broach (1990, Methods Enzymol., 185, 234-279), such as plasmids pCV19, pCV20, CV_{neo}, which utilise an insertion at *EcoRI* in *FLP,* plasmids pCV21, pGT41 and pYE which utilise *EcoRI* in *D* as the insertion site, plasmid pHKB52 which utilises *Pst*I in D as the insertion site, plasmid pJDB248 which utilises an insertion at *Pst*I in *D* and *EcoRI* in *D,* plasmid pJDB219 in which *PstI* in *D* and *Eco*RI in *FLP* are used as insertion sites, plasmid G18, plasmid pAB18 which utilises an insertion at *Cla*I in *FLP*, plasmids pGT39 and pA3, plasmids pYT11, pYT14 and pYT11-LEU which use *Pst*I in *D* as the insertion site, and plasmid PTY39 which uses *EcoRI* in *FLP* as the insertion site.

Other 2μm plasmids include pSAC3, pSAC3U1, pSAC3U2, pSAC300, pSAC310, pSAC3C1, pSAC3PL1, pSAC3SL4, and pSAC3SC1 are described in EP 0 286 424 and Chinery & Hinchliffe (1989, Curr. Genet., 16, 21-25) which also described *Pstl, Eagl* or SnaBI as appropriate 2μm insertion sites. Further 2μm plasmids include pAYE255, pAYE316, pAYE443, pAYE522 (Kerry-Williams et al, 1998, Yeast, 14, 161-169), pDB2244 (WO 00/44772), and pAYE329 (Sleep et al, 2001, Yeast, 18, 403-421).

In one preferred embodiment, one or more genes are inserted into a 2μm-family plasmid within an untranscribed region around the ARS sequence. For example, in the 2μm plasmid obtained from *S. cerevisiae,* the untranscribed region around the ARS sequence extends from end of the D gene to the beginning of ARS sequence. Insertion into SnaBI (near the origin of replication sequence ARS) is described in Chinery & Hinchliffe, 1989, Curr. Genet., 16, 21-25. The skilled person will appreciate that gene insertions can also be made in the untranscribed region at neighbouring positions to the *Sna*BI site described in Chinery & Hinchliffe.

In another preferred embodiment, *REP2* and *FLP* genes in a 2μm-family plasmid each have an inverted repeat adjacent to them, and one or more genes are inserted into a 2μm-family plasmid within the region between the first base after the last functional codon of either the *REP2* gene or the *FLP* gene and the last base before the FRT site in the inverted repeat adjacent to said gene. The last functional codon of either a *REP2* gene or a *FLP* gene is the codon in the open reading frame of the gene that is furthest downstream from the promoter of the gene whose replacement by a stop codon will lead to an unacceptable loss of multicopy stability of the plasmid, as defined herein. Thus, disruption of the *REP2* or *FLP* genes at any point downstream of the last functional codon in either gene, by insertion of a polynucleotide sequence insertion, deletion or substitution will not lead to an unacceptable loss of multicopy stability of the plasmid.

For example, the *REP2* gene of the 2μm plasmid can be disrupted after codon 59 and that the *FLP* gene of the 2μm plasmid can be disrupted after codon 344, each without a loss of multicopy stability of the plasmid. The last functional codon in equivalent genes in other 2μm-family plasmids can be determined routinely by making mutants of the plasmids in either the *FLP* or *REP2* genes and following the tests set out herein to determine whether the plasmid retains multicopy stability.

One can determined whether a plasmid retains multicopy stability using test such as defined in Chinery & Hinchliffe (1989, Curr. Genet., 16, 21-25). For yeast that do not grow in the non-selective media (YPD, also designated YEPD) defined in Chinery & Hinchliffe (1989, Curr. Genet., 16, 21-25) other appropriate non-selective media might be used. Plasmid stability may be defined as the percentage cells remaining prototrophic for the selectable marker after a defined number of generations. The number of generations will preferably be sufficient to show a difference between a control plasmid, such as pSAC35 or pSAC310, or to shown comparable stability to such a control plasmid. The number of generations may be 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100 or more. Higher numbers are preferred. The acceptable plasmid stability might be 1%, 2%, 3%, 4%, 5%, 10%, 15%, 20%, 25%, 30%, 40%, 50%, 60%, 70%, 75%, 80%, 85%, 90%, 95%, 96%, 97%, 98%, 99%, 99.9% or substantially 100%. Higher percentages are preferred. The skilled person will appreciate that, even though a plasmid may have a stability less than 100% when grown on non-selective media, that plasmid can still be of use when cultured in selective media. For example plasmid pDB2711 as described in the examples is only 10% stable when the stability is determined accordingly to test of Example 1, but provides a 15-fold increase in recombinant transferrin productivity in shake flask culture under selective growth conditions.

Thus one or more gene insertions may occur between the first base after the last functional codon of the *REP2* gene and the last base before the FRT site in an inverted repeat adjacent to said gene, more preferably between the first base of the inverted repeat and the last base before the FRT site, even more preferably at a position after the translation termination codon of the *REP2* gene and before the last base before the FRT site.

Additionally or alternatively one or more gene insertions may occur between the first base after the last functional codon of the *FLP* gene and the last base before the FRT site in an inverted repeat adjacent to said gene, preferably between the first base of the inverted repeat and the last base before the FRT site, more preferably between the first base after the end of the FLP coding sequence and the last base before the FRT site, such as at the first base after the end of the FLP coding sequence.

In one preferred embodiment, where the 2μm-family plasmid is based on the 2μm plasmid of *S. cerevisiae,* it is a disintegration vector as known in the art (for example, see EP 286 424). A disintegration vector may be a 2μm plasmid vector comprising a DNA sequence which is intended to be lost by recombination, three 2μm FRT sites, of which one pair of sites is in direct orientation and the other two pairs are in indirect orientation, and a DNA sequence of interest (such as an *E. coli* origin of replication and bacterial selectable marker), the said sequence to be lost being located between the said sites which are in direct orientation.

Thus, the sequence to be lost may comprise a selectable marker DNA sequence.

A preferred disintegration vector comprises a complete 2μm plasmid additionally carrying (i) a bacterial plasmid DNA sequence necessary for propagation of the vector in a bacterial host; (ii) an extra 2μm FRT site; and a selectable marker DNA sequence for yeast transformation; the said bacterial plasmid DNA sequence being present and the extra FRT site being created at a restriction site, such as *Xba*I in one of the two inverted repeat sequences of the 2μm plasmid, the said extra FRT site being in direct orientation in relation to the endogenous FRT site of the said one repeat sequence, and the bacterial plasmid DNA sequence being sandwiched between the extra FRT site and the endogenous FRT site of the said one repeat sequence. In a preferred disintegration vector, all bacterial plasmid DNA sequences are sandwiched as said. A particularly preferred 2μm plasmid vector has substantially the configuration of pSAC3 as shown in EP 286 424.

The term "disintegration vector" as used herein also includes plasmids as defined in US 6,451,559. Thus a disintegration vector may be a 2μm vector that, other than DNA sequence encoding non-yeast polypeptides, contains no bacterial (particularly *E. coli*) origin of replication, or more preferably no bacterial (particularly *E. coli*) sequence and preferably all DNA in said vector, other than DNA sequence encoding non-yeast polypeptide, is yeast-derived DNA.

The term "chaperone" as used herein refers to a protein that binds to and stabilises an otherwise unstable conformer of another protein, and by controlled binding and release, facilitates its correct fate *in vivo*, be it folding, oligomeric assembly, transport to a particular subcellular compartment, or disposal by degradation. Accordingly a chaperone is also a protein that is involved in protein folding, or which has chaperone activity or is involved in the unfolded protein response. Chaperone proteins of this type are known in the art, for example in the Stanford Genome Database (SGD), http://db.yeastgenome.org. Preferred chaperones are eukaryotic chaperones, especially preferred chaperones are yeast chaperones, including *AHA1, CCT2, CCT3, CCT4, CCT5, CCT6, CC77, CCT8, CNS1, CPR3, CPR6, EP31, ERO1, EUG1, FMO1, HCH1, HSP10, HSP12, HSP104, HSP26, HSP30, HSP42, HSP60, HSP78, HSP82, JEM1, MDJ1, MDJ2, MPD1, MPD2, PDI1, PFD1, ABC1 , APJ1, ATP11, ATP12, BTT1, CDC37, CPR7, HSC82, KAR2, LHS1,*

*MGE1, MRS11, NOB1, ECM10, SSA1, SSA2, SSA3, SSA4, SSC1, SSE2, SILI , SLS1, ORM1, ORM2, PER1, PTC2, PSE1, UB14* and *HAC1* or a truncated intronless *HAC1* (Valkonen et al. 2003, Applied Environ. Micro., 69, 2065)

A chaperone useful in the practice of the present invention may be:

● a heat shock protein, such as a protein that is a member of the hsp70 family of proteins (including Kar2p, SSA and SSB proteins, for example proteins encoded by *SSA1, SSA2, SSA3, SSA4, SSB1* and *SSB2*)*,* a protein that is a member of the HSP90-family, or a protein that is a member of the HSP40-family or proteins involved in their modulation (e.g. Sil1p), including DNA-J and DNA-J-like proteins (e.g. Jemlp, Mdj2p);

● a protein that is a member of the karyopherin/importin family of proteins, such as the alpha or beta families of karyopherin/importin proteins, for example the karyopherin beta protein PSE1;

● a protein that is a member of the ORMDL family described by Hjelmqvist et al, 2002, Genome Biology, 3(6), research0027.1-0027.16, such as Orm2p.

● a protein that is naturally located in the endoplasmic reticulum or elsewhere in the secretory pathway, such as the golgi. For example, a protein that naturally acts in the lumen of the endoplasmic reticulum (ER), particularly in secretory cells. such as PDI.

● a protein that is transmembrane protein anchored in the ER, such as a member of the ORMDL family described by Hjelmqvist *et al,* 2002*, supra,* (for example, Orm2p);

● a protein that acts in the cytosol, such as the hsp70 proteins, including SSA and SSB proteins, for example protein production *SSA1, SSA2, SSA3, SSA4, SSB1* and *SSB2;*

● a protein that acts in the nucleus, the nuclear envelope and/or the cytoplasm, such as Pse1p;

● a protein that is essential to the viability of the cell, such as PDI or an essential karyopherin protein, such as Pse1p;

● a protein that is involved in sulphydryl oxidation or disulphide bond formation, breakage or isomerization, or a protein that catalyses thiol:disulphide interchange reactions in proteins, particularly during the biosynthesis of secretory and cell surface proteins, such as protein disulphide isomerases (e.g. Pdilp, Mpdlp), homologues (e.g. Euglp) and/or related proteins (e.g. Mpd2p, Fmolp, Erolp);

● a protein that is involved in protein synthesis, assembly or folding, such as PDI and Ssa1p;

● a protein that binds preferentially or exclusively to unfolded, rather than mature protein, such as the hsp70 proteins, including SSA and SSB proteins, for example proteins encoded by *SSA1, SSA2, SSA3, SSA4, SSB1* and *SSB2;*

● a protein that prevents aggregation of precursor proteins in the cytosol, such as the hsp70 proteins, including SSA and SSB proteins, for example proteins encoded by *SSA1, SSA2, SSA3, SSA4, SSB1* and *SSB2;*

● a protein that binds to and stabilises damaged proteins, for example Ssalp;

● a protein that is involved in the unfolded protein response or provides for increased resistance to agents (such as tunicamycin and dithiothreitol) that induce the unfolded protein response, such as a member of the ORMDL family described by Hjelmqvist *et al,* 2002, *supra* (for example, Orm2p) or proteins involved in the response to stress (e.g. Ubi4p);

● a protein that is a co-chaperone and/or a protein indirectly involved in protein folding and/or the unfolded protein response (e.g. hsp104p, Mdj1p);

● a protein that is involved in the nucleocytoplasmic transport of macromolecules, such as Pse1p;

● a protein that mediates the transport of macromolecules across the nuclear membrane by recognising nuclear location sequences and nuclear export sequences and interacting with the nuclear pore complex, such as PSE1;

● a protein that is able to reactivate ribonuclease activity against RNA of scrambled ribonuclease as described in as described in EP 0 746 611 and Hillson et al, 1984, Methods Enzymol., 107, 281-292, such as PDI;

● a protein that has an acidic pI (for example, 4.0-4.5), such as PDI;

● a protein that is a member of the Hsp70 family, and preferably possesses an N-terminal ATP-binding domain and a C-terminal peptide-binding domain, such as Ssalp.

● a protein that is a peptidyl-prolyl cis-trans isomerases (e.g. Cpr3p, Cpr6p);

● a protein that is a homologue of known chaperones (e.g. Hsp10p);

● a protein that is a mitochondrial chaperone (e.g Cpr3p);

● a protein that is a cytoplasmic or nuclear chaperone (e.g Cns1p);

● a protein that is a membrane-bound chaperone (e.g. Orm2p, Fmo1p);

● a protein that has chaperone activator activity or chaperone regulatory activity (e.g. Aha1p, Hac1p, Hch1p);

● a protein that transiently binds to polypeptides in their immature form to cause proper folding transportation and/or secretion, including proteins required for efficient translocation into the endoplasmic reticulum (e.g. Lhs1p) or their site of action within the cell (e.g. PseIp);

● a protein that is a involved in protein complex assembly and/or ribosome assembly (e.g. Atp11p, PseIp, Nob1p);

● a protein of the chaperonin T-complex (e.g. Cct2p); or

● a protein of the prefoldin complex (e.g. Pfdlp).

A preferred chaperone is protein disulphide isomerase (PDI) or a fragment or variant thereof having an equivalent ability to catalyse the formation of disulphide bonds within the lumen of the endoplasmic reticulum (ER). By "PDI" we include any protein having the ability to reactivate the ribonuclease activity against RNA of scrambled ribonuclease as described in EP 0 746 611 and Hillson et al, 1984, Methods Enzymol., 107, 281-292.

PDI is an enzyme which typically catalyzes thiol:disulphide interchange reactions, and is a major resident protein component of the ER lumen in secretory cells. A body of evidence suggests that it plays a role in secretory protein biosynthesis (Freedman, 1984, Trends Biochem. Sci., 9, 438-41) and this is supported by direct cross-linking studies *in situ* (Roth and Pierce, 1987, Biochemistry, 26, 4179-82). The finding that microsomal membranes deficient in PDI show a specific defect in cotranslational protein disulphide bond formation (Bulleid and Freedman, 1988, Nature, 335, 649-51) implies that the enzyme functions as a catalyst of native disulphide bond formation during the biosynthesis of secretory and cell surface proteins. This roll is consistent with what is known of the enzyme's catalytic properties *in vitro;* it catalyzes thiol: disulphide interchange reactions leading to net protein disulphide formation, breakage or isomerization, and can typically catalyze protein folding and the formation of native disulphide bonds in a wide variety of reduced, unfolded protein substrates (Freedman et al., 1989, Biochem. Soc. Symp., 55, 167-192). PDI also functions as a chaperone since mutant PDI lacking isomerase activity accelerates protein folding (Hayano et al, 1995, FEBS Letters, 377, 505-511). Recently, sulphydryl oxidation, not disulphide isomerisation was reported to be the principal function of Protein Disulphide Isomerase in *S. cerevisiae* (Solovyov et al., 2004, J. Biol. Chem., 279 (33) 34095-34100). The DNA and amino acid sequence of the enzyme is known for several species (Scherens et al, 1991, Yeast, 7, 185-193; Farquhar et al, 1991, Gene, 108, 81-89; EP074661; EP0293793; EP0509841) and there is increasing information on the mechanism of action of the enzyme purified to homogeneity from mammalian liver (Creighton et al, 1980, J Mol. Biol., 142, 43-62; Freedman et al, 1988, Biochem. Soc. Trans., 16, 96-9; Gilbert, 1989, Biochemistry, 28, 7298-7305; Lundstrom and Holmgren, 1990, J. Biol. Chem., 265, 9114-9120; Hawkins and Freedman, 1990, Biochem. J., 275, 335-339). Of the many protein factors currently implicated as mediators of protein folding, assembly and translocation in the cell (Rothman, 1989, Cell, 59, 591-601), PDI has a well-defined catalytic activity.

The deletion or inactivation of the endogenous PDI gene in a host results in the production of an inviable host. In other words, the endogenous PDI gene is an "essential" gene.

PDI is readily isolated from mammalian tissues and the homogeneous enzyme is a homodimer (2x57 kD) with characteristically acidic pI (4.0-4.5) (Hillson *et al,* 1984, *op. cit.*). The enzyme has also been purified from wheat and from the alga *Chlamydomonas reinhardii* (Kaska et al, 1990, Biochem. J., 268, 63-68), rat (Edman et al, 1985, Nature, 317, 267-270), bovine (Yamauchi et al, 1987, Biochem. Biophys. Res. Comm., 146, 1485-1492), human (Pihlajaniemi et al, 1987, EMBO J., 6, 643-9), yeast (Scherens *et al, supra;* Farquhar *et al, op. cit.*) and chick (Parkkonen et al, 1988, Biochem. J., 256, 1005-1011). The proteins from these vertebrate species show a high degree of sequence conservation throughout and all show several overall features first noted in the rat PDI sequence (Edman *et al.,* 1985, *op. cit.*).

Preferred PDI sequences include those from humans and those from yeast species, such as *S. cerevisiae.*

A yeast protein disulphide isomerase precursor, PDI1, can be found as Genbank accession no. CAA42373 or BAA00723. It has the following sequence of 522 amino acids:

```
  1  mkfsagavls wsslllassv faqqeavape dsavvklatd sfneyiqshd lvlaeffapw
 61  cghcknmape yvkaaetlve knitlaqidc tenqdlcmeh nipgfpslki fknsdvnnsi
121  dyegprtaea ivqfmikqsq pavavvadlp aylanetfvt pvivqsgkid adfnatfysm
181  ankhfndydf vsaenadddf klsiylpsam depvvyngkk adiadadvfe kwlqvealpy
241  fgeidgsvfa qyvesglplg ylfyndeeel eeykplftel akknrglmnf vsidarkfgr
301  hagnlnmkeq fplfaihdmt edlkyglpql seeafdelsd kivleskaie slvkdflkgd
361  aspivksqei fenqdssvfq lvgknhdeiv ndpkkdvlvl yyapwcghck rlaptyqela
421  dtyanatsdv liakldhten dvrgvviegy ptivlypggk ksesvvyqgs rsldslfdfi
481  kenghfdvdg kalyeeaqek aaeeadadae ladeedaihd el
```

An alternative yeast protein disulphide isomerase sequence can be found as Genbank accession no. CAA38402. It has the following sequence of 530 amino acids

```
  1  mkfsagavls wsslllassv faqqeavape dsavvklatd sfneyiqshd lvlaeffapw
 61  cghcknmape yvkaaetlve knitlaqidc tenqdlcmeh nipgfpslki fknrdvnnsi
121  dyegprtaea ivqfmikqsq pavavvadlp aylanetfvt pvivqsgkid adfnatfysm
181  ankhfndydf vsaenadddf klsiylpsam depvvyngkk adiadadvfe kwlqvealpy
241  fgeidgsvfa qyvesglplg ylfyndeeel eeykplftel akknrglmnf vsidarkfgr
301  hagnlnmkeq fplfaihdmt edlkyglpql seeafdelsd kivleskaie slvkdflkgd
361  aspivksqei fenqdssvfq lvgknhdeiv ndpkkdvlvl yyapwcghck rlaptyqela
421  dtyanatsdv liakldhten dvrgvviegy ptivlypggk ksesvvyqgs rsldslfdfi
481  kenghfdvdg kalyeeaqek aaeeaeadae aeadadaela deedaihdel
```

The following alignment of these sequences (the sequence of Genbank accession no. CAA42373 or BAA00723 first, the sequence of Genbank accession no. CAA38402 second) shows that the differences between these two sequences are a single amino acid difference at position 114 (highlighted in bold) and that the sequence defined by Genbank: accession no. CAA38402 contains the additional amino acids EADAEAEA at positions 506-513.

```
  1   mkfsagavls wssllilassv faqqeavape dsavvklatd sfneyiqshd lvlaeffapw
  1   mkfsagavls wssillassv faqqeavape dsavvklatd sfneyiqshd lvlaeffapw


 61   cghcknmape yvkaaetlve knitlaqidc tenqdlcmeh nipgfpslki fknsdvnnsi
 61   cghcknmape yvkaaetlve knitlaqidc tenqdlcmeh nipgfpslki fknrdvnnsi


121   dyegprtaea ivqfmikqsq pavavvadlp aylanetfvt pvivqsgkid adfnatfysm
121   dyegprtaea ivqfmikqsq pavavvadlp aylanetfvt pvivqsgkid adfnatfysm


181   ankhfndydf vsaenadddf klsiylpsam depvvyngkk adiadadvfe kwlqvealpy
181   ankhfndydf vsaenadddf klsiylpsam depvvyngkk adiadadvfe kwlqvealpy


241   fgeidgsvfa qyvesglplg ylfyndeeel eeykplftel akknrglmnf vsidarkfgr
241   fgeidgsvfa qyvesglplg ylfyndeeel eeykplftel akknrglmnf vsidarkfgr


301   hagnlnmkeq fplfaihdmt edlkyglpql seeafdelsd kivleskaie slvkdflkgd
301   hagnlnmkeq fplfaihdmt edlkyglpql seeafdelsd kivleskaie slvkdflkgd


361   aspivksqei fenqdssvfq lvgknhdeiv ndpkkdvlvl yyapwcghck rlaptyqela
361   aspivksqei fenqdssvfq lvgknhdeiv ndpkkdvlvl yyapwcghck rlaptyqela


421   dtyanatsdv liakldhten dvrgvviegy ptivlypggk ksesvvyqgs rsldslfdfi
421   dtyanatsdv liakldhten dvrgvviegy ptivlypggk ksesvvyqgs rsldslfdfi


481   kenghfdvdg kalyeeaqek aaeea***** ***dadaela deedaihdel
481   kenghfdvdg kalyeeaqek aaeeaeadae aeadadaela deedaihdel
```

Variants and fragments of the above PDI sequences, and variants of other naturally occurring PDI sequences are also included in the present invention A "variant", in the context of PDI, refers to a protein wherein at one or more positions there have been amino acid insertions, deletions, or substitutions, either conservative or non-conservative, provided that such changes result in a protein whose basic properties, for example enzymatic activity (type of and specific activity), thermostability, activity in a certain pH-range (pH-stability) have not significantly been changed. "Significantly" in this context means that one skilled in the art would say that the properties of the variant may still be different but would not be unobvious over the ones of the original protein.

By "conservative substitutions" is intended combinations such as Val, Ile, Leu, Ala, Met; Asp, Glu; Asn, Gln; Ser, Thr, Gly, Ala; Lys, Arg, His; and Phe, Tyr, Trp. Preferred conservative substitutions include Gly, Ala; Val, Ile, Leu; Asp, Glu; Asn, Gln; Ser, Thr; Lys, Arg; and Phe, Tyr.

A "variant" typically has at least 25%, at least 50%, at least 60% or at least 70%, preferably at least 80%, more preferably at least 90%, even more preferably at least 95%, yet more preferably at least 99%, most preferably at least 99.5% sequence identity to the polypeptide from which it is derived.

The percent sequence identity between two polypeptides may be determined using suitable computer programs, as discussed below. Such variants may be natural or made using the methods of protein engineering and site-directed mutagenesis as are well known in the art.

A "fragment", in the context of PDI, refers to a protein wherein at one or more positions there have been deletions. Thus the fragment may comprise at most 5, 10, 20, 30, 40 or 50%, typically up to 60%, more typically up to 70%, preferably up to 80%, more preferably up to 90%, even more preferably up to 95%, yet more preferably up to 99% of the complete sequence of the full mature PDI protein. Particularly preferred fragments of PDI protein comprise one or more whole domains of the desired protein.

A fragment or variant of PDI may be a protein that, when expressed recombinantly in a host cell, can complement the deletion of the endogenously encoded PDI gene in the host cell, such as *S. cerevisiae,* and may, for example, be a naturally occurring homolog of PDI, such as a homolog encoded by another organism, such as another yeast or other fungi, or another eukaryote such as a human or other vertebrate, or animal or by a plant.

Another preferred chaperone is SSA1 or a fragment or variant thereof having an equivalent chaperone-like activity. *SSA1,* also known as YG100, is located on chromosome I of the *S. cerevisiae* genome and is 1.93-kbp in size.

One published protein sequence of SSA1 is as follows:

```
MSKAVGIDLGTTYSCVAHFANDRVDIIANDQGNRTTPSFVAFTDTERLIGDAAKNQAAMN
PSNTVFDAKRLIGRNFNDPEVQADMKHFPFKLIDVDGKPQIQVEFKGETKNFTPEQISSM
VLGKMKETAESYLGAKVNDAVVTVPAYFNDSQRQATKDAGTIAGLNVLRIINEPTAAAIA
YGLDKKGKEEHVLIFDLGGGTFDVSLLFIEDGIFEVKATAGDTHLGGEDFDNRLVNHFIQ
EFKRKNKKDLSTNQRALRRLRTACERAKRTLSSSAQTSVEIDSLFEGIDFYTSITRARFE
ELCADLFRSTLDPVEKVLRDAKLDKSQVDEIVLVGGSTRIPKVQKLVTDYFNGKEPNRSI
NPDEAVAYGAAVQAAILTGDESSKTQDLLLLDVAPLSLGIETAGGVMTKLIPRNSTISTK
KFEIFSTYADNQPGVLIQVFEGERAKTKDNNLLGKFELSGIPPAPRGVPQIEVTFDVDSN
GILNVSAVEKGTGKSNKITITNDKGRLSKEDIEKMVAEAEKFKEEDEKESQRIASKNQLE
SIAYSLKNTISEAGDKLEQADKDTVTKKAEETISWLDSNTTASKEEFDDKLKELQDIANP
IMSKLYQAGGAPGGAAGGAPGGFPGGAPPAPEAEGPTVEEVD
```

A published coding sequence for SSA1 is as follows, although it will be appreciated that the sequence can be modified by degenerate substitutions to obtain alternative nucleotide sequences which encode an identical protein product:

```
ATGTCAAAAGCTGTCGGTATTGATTTAGGTACAACATACTCGTGTGTTGCTCACTTTGCT
AATGATCGTGTGGACATTATTGCCAACGATCAAGGTAACAGAACCACTCCATCTTTTGTC
GCTTTCACTGACACTGAAAGATTGATTGGTGATGCTGCTAAGAATCAAGCTGCTATGAAT
CCTTCGAATACCGTTTTCGACGCTAAGCGTTTGATCGGTAGAAACTTCAACGACCCAGAA
GTGCAGGCTGACATGAAGCACTTCCCATTCAAGTTGATCGATGTTGACGGTAAGCCTCAA
ATTCAAGTTGAATTTAAGGGTGAAACCAAGAACTTTACCCCAGAACAAATCTCCTCCATG
GTCTTGGGTAAGATGAAGGAAACTGCCGAATCTTACTTGGGAGCCAAGGTCAATGACGCT
```

```
GTCGTCACTGTCCCAGCTTACTTCAACGATTCTCAAAGACAAGCTACCAAGGATGCTGGT
ACCATTGCTGGTTTGAATGTCTTGCGTATTATTAACGAACCTACCGCCGCTGCCATTGCT
TACGGTTTGGACAAGAAGGGTAAGGAAGAACACGTCTTGATTTTCGACTTGGGTGGTGGT
ACTTTCGATGTCTCTTTGTTGTTCATTGAAGACGGTATCTTTGAAGTTAAGGCCACCGCT
GGTGACACCCATTTGGGTGGTGAAGATTTTGACAACAGATTGGTCAACCACTTCATCCAA
GAATTCAAGAGAAAGAACAAGAAGGACTTGTCTACCAACCAAAGAGCTTTGAGAAGATTA
AGAACCGCTTGTGAAAGAGCCAAGAGAACTTTGTCTTCCTCCGCTCAAACTTCCGTTGAA
ATTGACTCTTTGTTCGAAGGTATCGATTTCTACACTTCCATCACCAGAGCCAGATTCGAA
GAATTGTGTGCTGACTTGTTCAGATCTACTTTGGACCCAGTTGAAAAGGTCTTGAGAGAT
GCTAAATTGGACAAATCTCAAGTCGATGAAATTGTCTTGGTCGGTGGTTCTACCAGAATT
CCAAAGGTCCAAAAAATTGGTCACTGACTACTTCAACGGTAAGGAACCAAACAGATCTATC
AACCCAGATGAAGCTGTTGCTTACGGTGCTGCTGTTCAAGCTGCTATTTTGACTGGTGAC
GAATCTTCCAAGACTCAAGATCTATTGTTGTTGGATGTCGCTCCATTATCCTTGGGTATT
GAAACTGCTGGTGGTGTCATGACCAAGTTGATTCCAAGAAACTCTACCATTTCAACAAAG
AAGTTCGAGATCTTTTCCACTTATGCTGATAACCAACCAGGTGTCTTGATTCAAGTCTTT
GAAGGTGAAAGAGCCAAGACTAAGGACAACAACTTGTTGGGTAAGTTCGAATTGAGTGGT
ATTCCACCAGCTCCAAGAGGTGTCCCACAAATTGAAGTCACTTTCGATGTCGACTCTAAC
GGTATTTTGAATGTTTCCGCCGTCGAAAAGGGTACTGGTAAGTCTAACAAGATCACTATT
ACCAACGACAAGGGTAGATTGTCCAAGGAAGATATCGAAAAGATGGTTGCTGAAGCCGAA
AAATTCAAGGAAGAAGATGAAAAGGAATCTCAAAGAATTGCTTCCAAGAACCAATTGGAA
TCCATTGCTTACTCTTTGAAGAACACCATTTCTGAAGCTGGTGACAAATTGGAACAAGCT
GACAAGGACACCGTCACCAAGAAGGCTGAAGAGACTATTTCTTGGTTAGACAGCAACACC
ACTGCCAGCAAGGAAGAATTCGATGACAAGTTGAAGGAGTTGCAAGACATTGCCAACCCA
ATCATGTCTAAGTTGTACCAAGCTGGTGGTGCTCCAGGTGGCGCTGCAGGTGGTGCTCCA
GGCGGTTTCCCAGGTGGTGCTCCTCCAGCTCCAGAGGCTGAAGGTCCAACCGTTGAAGAA
GTTGATTAA
```

The protein Ssalp belongs to the Hsp70 family of proteins and is resident in the cytosol. Hsp70s possess the ability to perform a number of chaperone activities; aiding protein synthesis, assembly and folding; mediating translocation of polypeptides to various intracellular locations, and resolution of protein aggregates (Becker & Craig, 1994, Eur. J. Biochem. 219, 11-23). Hsp70 genes are highly conserved, possessing an N-terminal ATP-binding domain and a C-terminal peptide-binding domain. Hsp70 proteins interact with the peptide backbone of, mainly unfolded, proteins. The binding and release of peptides by hsp70 proteins is an ATP-dependent process and accompanied by a conformational change in the hsp70 (Becker & Craig, 1994, *supra).*

Cytosolic hsp70 proteins are particularly involved in the synthesis, folding and secretion of proteins (Becker & Craig, 1994, *supra).* In *S. cerevisiae* cytosolic hsp70 proteins have been divided into two groups; SSA (SSA 1-4) and SSB (SSB 1 and 2) proteins, which are functionally distinct from each other. The SSA family is essential in that at least one protein from the group must be active to maintain cell viability (Becker & Craig, 1994, *supra).* Cytosolic hsp70 proteins bind preferentially to unfolded and not mature proteins. This suggests that they prevent the aggregation of precursor proteins, by maintaining them in an unfolded state prior to being assembled into multimolecular complexes in the cytosol and/or facilitating their translocation to various organelles (Becker & Craig, 1994, *supra).* SSA proteins are particularly involved in posttranslational biogenesis and maintenance of precursors for translocation into the endoplasmic reticulum and mitochondria (Kim et al., 1998, Proc. Natl. Acad Sci. USA. 95, 12860-12865; Ngosuwan et al., 2003, J. Biol. Chem. 278 (9), 7034-7042). Ssalp has been shown to bind damaged proteins, stabilising them in a partially unfolded form and allowing refolding or degradation to occur (Becker & Craig, 1994, *supra;* Glover & Lindquist, 1998, Cell. 94, 73-82).

Demolder et al, 1994, J. Biotechnol, 32, 179-189 reported that over-expression of SSA1 in yeast provided for increases in the expression of a recombinant chromosomally integrated gene encoding human interferon-β. There is no suggestion that increases in heterologous gene expression could be achieved if SSA1 and human interferon-β were to be encoded by recombinant genes on the same plasmid. In fact, in light of more recent developments in the field of over-expression of chaperones in yeast (e.g. Robinson *et al,* 1994, *op. cit.;* Hayano *et al,* 1995, *op. cit.;* Shusta *et al,* 1998, *op. cit;* Parekh & Wittrup, 1997, *op. cit.;* Bao & Fukuhara, 2001, *op. cit.;* and Bao *et al,* 2000, *op. cit*) the skilled person would have been disinclined to express SSA1 from a 2μm-family plasmid at all, much less to express both SSA1 and a heterologous protein from a 2μm-family plasmid in order to increase the expression levels of a heterologous protein.

Variants and fragments of SSA1 are also included in the present invention. A "variants", in the context of SSA1, refers to a protein having the sequence of native SSA1 other than at one or more positions where there have been amino acid insertions, deletions, or substitutions, either conservative or non-conservative, provided that such changes result in a protein whose basic properties, for example enzymatic activity (type of and specific activity), thermostability, activity in a certain ph-range (pH-stability) have not significantly been changed. "Significantly" in this context means that one skilled in the art would say that the properties of the variant may still be different but would not be unobvious over the ones of the original protein.

By "conservative substitutions" is intended combinations such as Val, Ile, Leu, Ala, Met; Asp, Glu; Asn, Gln; Ser, Thr, Gly, Ala; Lys, Arg, His; and Phe, Tyr, Trp. Preferred conservative substitutions include Gly, Ala; Val, Ile, Leu; Asp, Glu; Asn, Gln; Ser, Thr; Lys, Arg; and Phe, Tyr.

A "variant" of SSA1 typically has at least 25%, at least 50%, at least 60% or at least 70%, preferably at least 80%, more preferably at least 90%, even more preferably at least 95%, yet more preferably at least 99%, most preferably at least 99.5% sequence identity to the sequence of native SSA1.

The percent sequence identity between two polypeptides may be determined using suitable computer programs, as discussed below. Such variants may be natural or made using the methods of protein engineering and site-directed mutagenesis as are well known in the art.

A "fragment", in the context of SSA1, refers to a protein having the sequence of native SSA1 other than for at one or more positions where there have been deletions. Thus the fragment may comprise at most 5, 10, 20, 30, 40 or 50%, typically up to 60%, more typically up to 70%, preferably up to 80%, more preferably up to 90%, even more preferably up to 95%, yet more preferably up to 99% of the complete sequence of the full mature SSA1 protein. Particularly preferred fragments of SSA1 protein comprise one or more whole domains of the desired protein.

A fragment or variant of SSA1 may be a protein that, when expressed recombinantly in a host cell, such as *S. cerevisiae,* can complement the deletion of the endogenously encoded SSA1 gene (or homolog thereof) in the host cell and may, for example, be a naturally occurring homolog of SSA1, such as a homolog encoded by another organism, such as another yeast or other fungi, or another eukaryote such as a human or other vertebrate, or animal or by a plant.

Another preferred chaperone is PSE1 or a fragment or variant thereof having equivalent chaperone-like activity.

*PSE1,* also known as *KAP121,* is an essential gene, located on chromosome XIII.

A published protein sequence for the protein pse1p is as follows:

```
MSALPEEVNRTLLQIVQAFASPDNQIRSVAEKALSEEWITENNIEYLLTFLAEQAAFSQD
TTVAALSAVLFRKLALKAPPSSKLMIMSKNITHIRKEVLAQIRSSLLKGFLSERADSIRH
KLSDAIAECVQDDLPAWPELLQALIESLKSGNPNFRESSFRILTTVPYLITAVDINSILP
IFQSGFTDASDNVKIAAVTAFVGYFKQLPKSEWSKLGILLPSLLNSLPRFLDDGKDDALA
SVFESLIELVELAPKLFKDMFDQIIQFTDMVIKNKDLEPPARTTALELLTVFSENAPQMC
KSNQNYGQTLVMVTLIMMTEVSIDDDDAAEWIESDDTDDEEEVTYDHARQALDRVALKLG
GEYLAAPLFQYLQQMITSTEWRERFAAMMALSSAAEGCADVLIGEIPKILDMVIPLINDP
HPRVQYGCCNVLGQISTDFSPFIQRTAHDRILPALISKLTSECTSRVQTHAAAALVNFSE
FASKDILEPYLDSLLTNLLVLLQSNKLYVQEQALTTIAFIAEAAKNKFIKYYDTLMPLLL
NVLKVNNKDNSVLKGKCMECATLIGFAVGKEKFHEHSQELISILVALQNSDIDEDDALRS
```

YLEQSWSRICRILGDDFVPLLPIVIPPLLITAKATQDVGLIEEEEAANFQQYPDWDVVQV

QGKHIAIHTSVLDDKVSAMELLQSYATLLRGQFAVYVKEVMEEIALPSLDFYLHDGVRAA

GATLIPILLSCLLAATGTQNEELVLLWHKASSKLIGGLMSEPMPEITQVYHNSLVNGIKV

MGDNCLSEDQLAAFTKGVSANLTDTYERMQDRHGDGDEYNENIDEEEDFTDEDLLDEINK

SIAAVLKTTNGHYLKNLENIWPMINTFLLDNEPILVIFALVVIGDLIQYGGEQTASMKNA

FIPKVTECLISPDARIRQAASYIIGVCAQYAPSTYADVCIPTLDTLVQIVDFPGSKLEEN

RSSTENASAAIAKILYAYNSNIPNVDTYTANWFKTLPTITDKEAASFNYQFLSQLIENNS

PIVCAQSNISAVVDSVIQALNERSLTEREGQTVISSVKKLLGFLPSSDAMAIFNRYPADI

MEKVHKWFA*

A published nucleotide coding sequence of PSE1 is as follows, although it will be appreciated that the sequence can be modified by degenerate substitutions to obtain alternative nucleotide sequences which encode an identical protein product:

ATGTCTGCTTTACCGGAAGAAGTTAATAGAACATTACTTCAGATTGTCCAGGCGTTTGCT

TCCCCTGACAATCAAATACGTTCTGTAGCTGAGAAGGCTCTTAGTGAAGAATGGATTACC

GAAAACAATATTGAGTATCTTTTAACTTTTTTGGCTGAACAAGCCGCTTTCTCCCAAGAT

ACAACAGTTGCAGCATTATCTGCTGTTCTGTTTAGAAAATTAGCATTAAAAGCTCCCCCT

TCTTCGAAGCTTATGATTATGTCCAAAAATATCACACATATTAGGAAAGAAGTTCTTGCA

CAAATTCGTTCTTCATTGTTAAAAGGGTTTTTGTCGGAAAGAGCTGATTCAATTAGGCAC

AAACTATCTGATGCTATTGCTGAGTGTGTTCAAGACGACTTACCAGCATGGCCAGAATTA

CTACAAGCTTTAATAGAGTCTTTAAAAAGCGGTAACCCAAATTTTAGAGAATCCAGTTTT

AGAATTTTGACGACTGTACCTTATTTAATTACCGCTGTTGACATCAACAGTATCTTACCA

ATTTTTCAATCAGGCTTTACTGATGCAAGTGATAATGTCAAAATTGCTGCAGTTACGGCT

TTCGTGGGTTATTTTAAGCAACTACCAAAATCTGAGTGGTCCAAGTTAGGTATTTTATTA

CCAAGTCTTTTGAATAGTTTACCAAGATTTTTAGATGATGGTAAGGACGATGCCCTTGCA

TCAGTTTTTGAATCGTTAATTGAGTTGGTGGAATTGGCACCAAAACTATTCAAGGATATG

TTTGACCAAATAATACAATTCACTGATATGGTTATAAAAAATAAGGATTTAGAACCTCCA

GCAAGAACCACAGCACTCGAACTGCTAACCGTTTTCAGCGAGAACGCTCCCCAAATGTGT

AAATCGAACCAGAATTACGGGCAAACTTTAGTGATGGTTACTTTAATCATGATGACGGAG

GTATCCATAGATGATGATGATGCAGCAGAATGGATAGAATCTGACGATACCGATGATGAA

GAGGAAGTTACATATGACCACGCTCGTCAAGCTCTTGATCGTGTTGCTTTAAAGCTGGGT

GGTGAATATTTGGCTGCACCATTGTTCCAATATTTACAGCAAATGATCACATCAACCGAA

TGGAGAGAAAGATTCGCGGCCATGATGGCACTTTCCTCTGCAGCTGAGGGTTGTGCTGAT

GTTCTGATCGGCGAGATCCCAAAAATCCTGGATATGGTAATTCCCCTCATCAACGATCCT

CATCCAAGAGTACAGTATGGATGTTGTAATGTTTTGGGTCAAATATCTACTGATTTTTCA

```
CCATTCATTCAAAGAACTGCACACGATAGAATTTTGCCGGCTTTAATATCTAAACTAACG
TCAGAATGCACCTCAAGAGTTCAAACGCACGCCGCAGCGGCTCTGGTTAACTTTTCTGAA
TTCGCTTCGAAGGATATTCTTGAGCCTTACTTGGATAGTCTATTGACAAATTTATTAGTT
TTATTACAAAGCAACAAACTTTACGTACAGGAACAGGCCCTAACAACCATTGCATTTATT
GCTGAAGCTGCAAAGAATAAATTTATCAAGTATTACGATACTCTAATGCCATTATTATTA
AATGTTTTGAAGGTTAACAATAAAGATAATAGTGTTTTGAAAGGTAAATGTATGGAATGT
GCAACTCTGATTGGTTTTGCCGTTGGTAAGGAAAAATTTCATGAGCACTCTCAAGAGCTG
ATTTCTATATTGGTCGCTTTACAAAACTCAGATATCGATGAAGATGATGCGCTCAGATCA
TACTTAGAACAAAGTTGGAGCAGGATTTGCCGAATTCTGGGTGATGATTTTGTTCCGTTG
TTACCGATTGTTATACCACCCCTGCTAATTACTGCCAAAGCAACGCAAGACGTCGGTTTA
ATTGAAGAAGAAGAAGCAGCAAATTTCCAACAATATCCAGATTGGGATGTTGTTCAAGTT
CAGGGAAAACACATTGCTATTCACACATCCGTCCTTGACGATAAAGTATCAGCAATGGAG
CTATTACAAAGCTATGCGACACTTTTAAGAGGCCAATTTGCTGTATATGTTAAAGAAGTA
ATGGAAGAAATAGCTCTACCATCGCTTGACTTTTACCTACATGACGGTGTTCGTGCTGCA
GGAGCAACTTTAATTCCTATTCTATTATCTTGTTTACTTGCAGCCACCGGTACTCAAAAC
GAGGAATTGGTATTGTTGTGGCATAAAGCTTCGTCTAAACTAATCGGAGGCTTAATGTCA
GAACCAATGCCAGAAATCACGCAAGTTTATCACAACTCGTTAGTGAATGGTATTAAAGTC
ATGGGTGACAATTGCTTAAGCGAAGACCAATTAGCGGCATTTACTAAGGGTGTCTCCGCC
AACTTAACTGACACTTACGAAAGGATGCAGGATCGCCATGGTGATGGTGATGAATATAAT
GAAAATATTGATGAAGAGGAAGACTTTACTGACGAAGATCTTCTCGATGAAATCAACAAG
TCTATCGCGGCCGTTTTGAAAACCACAAATGGTCATTATCTAAAGAATTTGGAGAATATA
TGGCCTATGATAAACACATTCCTTTTAGATAATGAACCAATTTTAGTCATTTTTGCATTA
GTAGTGATTGGTGACTTGATTCAATATGGTGGCGAACAAACTGCTAGCATGAAGAACGCA
TTTATTCCAAAGGTTACCGAGTGCTTGATTTCTCCTGACGCTCGTATTCGCCAAGCTGCT
TCTTATATAATCGGTGTTTGTGCCCAATACGCTCCATCTACATATGCTGACGTTTGCATA
CCGACTTTAGATACACTTGTTCAGATTGTCGATTTTCCAGGCTCCAAACTGGAAGAAAAT
CGTTCTTCAACAGAGAATGCCAGTGCAGCCATCGCCAAAATTCTTTATGCATACAATTCC
AACATTCCTAACGTAGACACGTACACGGCTAATTGGTTCAAAACGTTACCAACAATAACT
GACAAAGAAGCTGCCTCATTCAACTATCAATTTTTGAGTCAATTGATTGAAAATAATTCG
CCAATTGTGTGTGCTCAATCTAATATCTCCGCTGTAGTTGATTCAGTCATACAAGCCTTG
AATGAGAGAAGTTTGACCGAAAGGGAAGGCCAAACGGTGATAAGTTCAGTTAAAAAGTTG
TTGGGATTTTTGCCTTCTAGTGATGCTATGGCAATTTTCAATAGATATCCAGCTGATATT
ATGGAGAAAGTACATAAATGGTTTGCATAA
```

The PSE1 gene is 3.25-kbp in size. Pselp is involved in the nucleocytoplasmic transport of macromolecules (Seedorf & Silver, 1997, Proc. Natl. Acad. Sci. USA. 94, 8590-8595). This process occurs via the nuclear pore complex (NPC) embedded in the nuclear envelope and made up of nucleoporins (Ryan & Wente, 2000, Curr. Opin. Cell Biol. 12, 361-371). Proteins possess specific sequences that contain the information required for nuclear import, nuclear localisation sequence (NLS) and export, nuclear export sequence (NES) (Pemberton et al., 1998, Curr. Opin. Cell Biol. 10, 392-399). Pselp is a karyopherin/importin, a group of proteins, which have been divided up into $\alpha$ and $\beta$ families. Karyopherins are soluble transport factors that mediate the transport of macromolecules across the nuclear membrane by recognising NLS and NES, and interact with and the NPC (Seedorf & Silver, 1997, *supra;* Pemberton *et al.,* 1998, *supra;* Ryan & Wente, 2000, *supra*). Translocation through the nuclear pore is driven by GTP hydrolysis, catalysed by the small GTP-binding protein, Ran (Seedorf & Silver, 1997, *supra*). Pselp has been identified as a karyopherin β. 14 karyopherin β proteins have been identified in *S. cerevisiae,* of which only 4 are essential. This is perhaps because multiple karyopherins may mediate the transport of a single macromolecule (Isoyama et al., 2001, J. Biol. Chem. 276 (24), 21863-21869). Pselp is localised to the nucleus, at the nuclear envelope, and to a certain extent to the cytoplasm. This suggests the protein moves in and out of the nucleus as part of its transport function (Seedorf & Silver, 1997, *supra).* Pselp is involved in the nuclear import of transcription factors (Isoyama *et* al., 2001, *supra;* Ueta et al., 2003, J. Biol. Chem. 278 (50), 50120-50127), histones (Mosammaparast et al., 2002, J. Biol. Chem. 277 (1), 862-868), and ribosomal proteins prior to their assembly into ribosomes (Pemberton *et al.,* 1998, *supra).* It also mediates the export of mRNA from the nucleus. Karyopherins recognise and bind distinct NES found on RNA-binding proteins, which coat the RNA before it is exported from the nucleus (Seedorf & Silver, 1997, Pemberton *et al.,* 1998, *supra).*

As nucleocytoplasmic transport of macromolecules is essential for proper progression through the cell cycle, nuclear transport factors, such as pselp are novel candidate targets for growth control (Seedorf & Silver, 1997, *supra).*

Overexpression of Pselp (protein secretion enhancer) in *S. cerevisiae* has also been shown to increase endogenous protein secretion levels of a repertoire of biologically active proteins (Chow et al., 1992; J. Cell. Sci. 101 (3), 709-719). There is no suggestion that increases in heterologous gene expression could be achieved if PSE1 and a heterologous protein were both to be encoded by recombinant genes on the same plasmid. In fact, in light of more recent developments in the over-expression of chaperones in yeast (e.g. Robinson *et al,* 1994, *op. cit.;* Hayano *et al,* 1995, *op. cit.;* Shusta *et al,* 1998, *op. cit;* Parekh & Wittrup, 1997, *op. cit.;* Bao & Fukuhara, 2001, *op. cit.;* and Bao *et al,* 2000, *op. cit*) the skilled person would not have attempted to over-express PSE1 from a 2μm-family plasmid at all, much less to express both PSE1 and a heterologous protein from a 2μm-family plasmid in order to increase the expression levels of a heterologous protein.

Variants and fragments of PSE1 are also included in the present invention. A "variant", in the context of PSE1, refers to a protein having the sequence of native PSE1 other than for at one or more positions where there have been amino acid insertions, deletions, or substitutions, either conservative or non-conservative, provided that such changes result in a protein whose basic properties, for example enzymatic activity (type of and specific activity), thermostability, activity in a certain pH-range (pH-stability) have not significantly been changed. "Significantly" in this context means that one skilled in the art would say that the properties of the variant may still be different but would not be unobvious over the ones of the original protein.

By "conservative substitutions" is intended combinations such as Val, Ile, Leu, Ala, Met; Asp, Glu; Asn, Gln; Ser, Thr, Gly, Ala; Lys, Arg, His; and Phe, Tyr, Trp. Preferred conservative substitutions include Gly, Ala; Val, Ile, Leu; Asp, Glu; Asn, Gin; Ser, Thr; Lys, Arg; and Phe, Tyr.

A "variant" of PSE1 typically has at least 25%, at least 50%, at least 60% or at least 70%, preferably at least 80%, more preferably at least 90%, even more preferably at least 95%, yet more preferably at least 99%, most preferably at least 99.5% sequence identity to the sequence of native PSE1.

The percent sequence identity between two polypeptides may be determined using suitable computer programs, as discussed below. Such variants may be natural or made using the methods of protein engineering and site-directed mutagenesis as are well known in the art.

A "fragment", in the context of PSE1, refers to a protein having the sequence of native PSE1 other than for at one or more positions where there have been deletions. Thus the fragment may comprise at most 5, 10, 20, 30, 40 or 50%, typically up to 60%, more typically up to 70%, preferably up to 80%, more preferably up to 90%, even more preferably up to 95%, yet more preferably up to 99% of the complete sequence of the full mature PSE1 protein. Particularly preferred fragments of PSE1 protein comprise one or more whole domains of the desired protein.

A fragment or variant of PSE1 may be a protein that, when expressed recombinantly in a host cell, such as *S. cerevisiae,* can complement the deletion of the endogenous PSE1 gene in the host cell and may, for example, be a naturally occurring homolog of PSE1, such as a homolog encoded by another organism, such as another yeast or other fungi, or another eukaryote such as a human or other vertebrate, or animal or by a plant.

Another preferred chaperone is ORM2 or a fragment or variant thereof having equivalent chaperone-like activity.

*ORM2,* also known as YLR350W, is located on chromosome XII (positions 828729 to 829379) of the *S. cerevisiae* genome and encodes an evolutionarily conserved protein with similarity to the yeast protein Ormlp. Hjelmqvist et al, 2002, Genome Biology, 3(6), research 0027.1-0027.16 reports that *ORM2* belongs to gene family comprising three human genes (ORMDL1, ORMDL2 and ORMDL3) as well as homologs in microsporidia, plants, *Drosophila,* urochordates and vertebrates. The ORMDL genes are reported to encode transmembrane proteins anchored in the proteins endoplasmic reticulum (ER).

The protein Orm2p is required for resistance to agents that induce the unfolded protein response. Hjelmqvist et al, 2002 (*supra*) reported that a double knockout of the two S. *cerevisiae* ORMDL homologs (*ORM1* and *ORM2*) leads to a decreased growth rate and greater sensitivity to tunicamycin and dithiothreitol.

One published sequence of Orm2p is as follows:

```
MIDRTKNESPAFEESPLTPNVSNLKPFPSQSNKISTPVTDHRRRRSSSVISHVEQETFED
ENDQQMLPNMNATWVDQRGAWLIHIVVIVLLRLFYSLFGSTPKWTWTLTNMTYIIGFYIM
FHLVKGTPFDFNGGAYDNLTMWEQINDETLYTPTRKFLLIVPIVLFLISNQYYRNDMTLF
LSNLAVTVLIGVVPKLGITHRLRISIPGITGRAQIS*
```

The above protein is encoded in *S. cerevisiae* by the following coding nucleotide sequence, although it will be appreciated that the sequence can be modified by degenerate substitutions to obtain alternative nucleotide sequences which encode an identical protein product:

```
ATGATTGACCGCACTAAAAACGAATCTCCAGCTTTTGAAGAGTCTCCGCTTACCCCCAAT
GTGTCTAACCTGAAACCATTCCCTTCTCAAAGCAACAAAATATCCACTCCAGTGACCGAC
CATAGGAGAAGACGGTCATCCAGCGTAATATCACATGTGGAACAGGAAACCTTCGAAGAC
```

```
GAAAATGACCAGCAGATGCTTCCCAACATGAACGCTACGTGGGTCGACCAGCGAGGCGCG
TGGTTGATTCATATCGTCGTAATAGTACTCTTGAGGCTCTTCTACTCCTTGTTCGGGTCG
ACGCCCAAATGGACGTGGACTTTAACAAACATGACCTACATCATCGGATTCTATATCATG
TTCCACCTTGTCAAAGGTACGCCCTTCGACTTTAACGGTGGTGCGTACGACAACCTGACC
ATGTGGGAGCAGATTAACGATGAGACTTTGTACACACCCACTAGAAAATTTCTGCTGATT
GTACCCATTGTGTTGTTCCTGATTAGCAACCAGTACTACCGCAACGACATGACACTATTC
CTCTCCAACCTCGCCGTGACGGTGCTTATTGGTGTCGTTCCTAAGCTGGGAATTACGCAT
AGACTAAGAATATCCATCCCTGGTATTACGGGCCGTGCTCAAATTAGTTAG
```

Variants and fragments of ORM2 are also included in the present invention. A "variant", in the context of ORM2, refers to a protein having the sequence of native ORM2 other than for at one or more positions where there have been amino acid insertions, deletions, or substitutions, either conservative or non-conservative, provided that such changes result in a protein whose basic properties, for example enzymatic activity (type of and specific activity), thermostability, activity in a certain pH-range (pH-stability) have not significantly been changed. "Significantly" in this context means that one skilled in the art would say that the properties of the variant may still be different but would not be unobvious over the ones of the original protein.

By "conservative substitutions" is intended combinations such as Val, Ile, Leu, Ala, Met; Asp, Glu; Asn, Gln; Ser, Thr, Gly, Ala; Lys, Arg, His; and Phe, Tyr, Trp. Preferred conservative substitutions include Gly, Ala; Val, Ile, Leu; Asp, Glu; Asn, Gln; Ser, Thr Lys, Arg; and Phe, Tyr.

A "variant" of ORM2 typically has at least 25%, at least 50%, at least 60% or at least 70%, preferably at least 80%, more preferably at least 90%, even more preferably at least 95%, yet more preferably at least 99%, most preferably at least 99.5% sequence identity to the sequence of native ORM2.

The percent sequence identity between two polypeptides may be determined using suitable computer programs, as discussed below. Such variants may be natural or made using the methods of protein engineering and site-directed mutagenesis as are well known in the art

A "fragment", in the context of ORM2, refers to a protein having the sequence of native ORM2 other than for at one or more positions where there have been deletions. Thus the fragment may comprise at most 5, 10, 20, 30, 40 or 50%, typically up to 60%, more typically up to 70%, preferably up to 80%, more preferably up to 90%, even more preferably up to 95%, yet more preferably up to 99% of the complete sequence of the full mature ORM2 protein. Particularly preferred fragments of ORM2 protein comprise one or more whole domains of the desired protein.

A fragment or variant of ORM2 may be a protein that, when expressed recombinantly in a host cell, such as *S. cerevisiae,* can complement the deletion of the endogenous ORM2 gene in the host cell and may, for example, be a naturally occurring homolog of ORM2, such as a homolog encoded by another organism, such as another yeast or other fungi, or another eukaryote such as a human or other vertebrate, or animal or by a plant.

A gene encoding a protein comprising the sequence of a chaperone may be formed in a like manner to that discussed below for genes encoding heterologous proteins, with particular emphasis on combinations of ORFs and regulatory regions.

The term "protein" as used herein includes all natural and non-natural proteins, polypeptides and peptides. A "heterologous protein" is a protein that is not naturally encoded by a 2$\mu$m-family plasmid and can also be described as a "non 2$\mu$m-family plasmid protein". For convenience, the terms "heterologous protein" and "non 2$\mu$m-family plasmid protein" are used synonymously throughout this application. Preferably, therefore, the heterologous protein is not a FLP, REP1, REP2, or a RAF/D protein as encoded by any one of pSR1, pSB3 or pSB4 as obtained from *Z. rouxii,* pSB1 or pSB2 both as obtained from *Z bailli,* pSM1 as obtained from *Z. fermentati,* pKD1 as obtained from *K. drosophilarum,* pPM1 as obtained from *P. membranaefaciens* or the 2$\mu$m plasmid as obtained from *S. cerevisiae.*

A gene encoding a heterologous protein comprises polynucleotide sequence encoding the heterologous protein (typically according to standard codon usage for any given organism), designated the open reading frame ("ORF"). The gene may additionally comprise some polynucleotide sequence that does not encode an open reading frame (termed "non-coding region").

Non-coding region in the gene may contain one or more regulatory sequences, operatively linked to the ORF, which allow for the transcription of the open reading frame and/or translation of the resultant transcript.

The term "regulatory sequence" refers to a sequence that modulates (i.e., promotes or reduces) the expression (i.e., the transcription and/or translation) of an ORF to which it is operably linked. Regulatory regions typically include promoters, terminators, ribosome binding sites and the like. The skilled person will appreciate that the choice of regulatory region will depend upon the intended expression system. For example, promoters may be constitutive or inducible and may be cell- or tissue-type specific or non-specific.

Suitable regulatory regions, may be 5bp, 10bp, 15bp, 20bp, 25bp, 30bp, 35bp, 40bp, 45bp, 50bp, 60bp, 70bp, 80bp, 90bp, 100bp, 120bp, 140bp, 160bp, 180bp, 200bp, 220bp, 240bp, 260bp, 280bp, 300bp, 350bp, 400bp, 450bp, 500bp, 550bp, 600bp, 650bp, 700bp, 750bp, 800bp, 850bp, 900bp, 950bp, 1000bp, 1100bp, 1200bp,1300bp, 1400bp, 1500bop or greater, in length.

Those skilled in the art will recognise that the gene encoding the chaperone, for example PDI, may additionally comprise non-coding regions and/or regulatory regions. Such non-coding regions and regulatory regions are not restricted to the native non-coding regions and/or regulatory regions normally associated with the chaperone ORF.

Where the expression system is yeast, such as *Saccharomyces cerevisiae,* suitable promoters for *S. cerevisiae* include those associated with the *PGK1* gene, *GAL1* or *GAL10* genes, *TEF1, TEF2, PYK1, PMA1, CYC1, PHO5, TRP1, ADH1, ADH2,* the genes for glyceraldehyde-3-phosphate dehydrogenase, hexokinase, pyruvate decarboaylase, phosphofructokinase, triose phosphate isomerase, phosphoglucose isomerase, glucokinase, $\alpha$-mating factor pheromone, $\alpha$-mating factor pheromone, the *PRB1* promoter, the *PRA1* promoter, the *GPD1* promoter, and hybrid promoters involving hybrids of parts of 5' regulatory regions with parts of 5' regulatory regions of other promoters or with upstream activation sites (e.g. the promoter of EP-A-258 067).

Suitable transcription termination signals are well known in the art. Where the host cell is eukaryotic, the transcription termination signal is preferably derived from the 3' flanking sequence of a eukaryotic gene, which contains proper signals for transcription termination and polyadenylation. Suitable 3' flanking sequences may, for example, be those of the gene naturally linked to the expression control sequence used, i.e. may correspond to the promoter. Alternatively, they may be different. In that case, and where the host is a yeast, preferably *S. cerevisiae,* then the termination signal of the *S. cerevisiae ADH1, ADH2, CYC1,* or *PGK1* genes are preferred.

It may be beneficial for the promoter and open reading frame of the heterologous gene, such as the those of the chaperone *PDI1,* to be flanked by transcription termination sequences so that the transcription termination sequences are located both upstream and downstream of the promoter and open reading frame, in order to prevent transcriptional read-through into neighbouring genes, such as 2$\mu$m genes, and visa versa.

In one embodiment, the favoured regulatory sequences in yeast, such as *Saccharomyces cerevisiae,* include: a yeast promoter (e.g. the *Saccharomyces cerevisiae PRB1* promoter), as taught in EP 431 880; and a transcription terminator, preferably the terminator from *Saccharomyces ADH1,* as taught in EP 60 057. Preferably, the vector incorporates at least two translation stop codons.

It may be beneficial for the non-coding region to incorporate more than one DNA sequence encoding a translational stop codon, such as UAA, UAG or UGA, in order to minimise translational read-through and thus avoid the production of elongated, non-natural fusion proteins. The translation stop codon UAA is preferred.

The term "operably linked" includes within its meaning that a regulatory sequence is positioned within any non-coding region in a gene such that it forms a relationship with an ORF that permits the regulatory region to exert an effect on the ORF in its intended manner. Thus a regulatory region "operably linked" to an ORF is positioned in such a way that the regulatory region is able to influence transcription and/or translation of the ORF in the intended manner, under conditions compatible with the regulatory sequence.

In one preferred embodiment, the heterologous protein is secreted. In that case, a sequence encoding a secretion leader sequence which, for example, comprises most of the natural HSA secretion leader, plus a small portion of the *S. cerevisiae* α-mating factor secretion leader as taught in WO 90/01063 may be included in the open reading frame.

Alternatively, the heterologous protein may be intracellular.

In another preferred embodiment, the heterologous protein comprises the sequence of a eukaryotic protein, or a fragment or variant thereof. Suitable eukaryotes include fungi, plants and animals. In one preferred embodiment the heterologous protein is a fungal protein, such as a yeast protein. In another preferred embodiment the heterologous protein is an animal protein. Exemplary animals include vertebrates and invertebrates. Exemplary vertebrates include mammals, such as humans, and non-human mammals.

Thus the heterologous protein may comprise the sequence of a yeast protein. It may, for example, comprise the sequence of a yeast protein from the same host from which the 2μm-family plasmid is derived. Those skilled in the art will recognise that a method, use or plasmid of the first, second or third aspects of the invention may comprise DNA sequences encoding more than one heterologous protein, more than one chaperone, or more than one heterologous protein and more than one chaperone.

In another preferred embodiment, the heterologous protein may comprise the sequence of albumin, a monoclonal antibody, an etoposide, a serum protein (such as a blood clotting factor), antistasin, a tick anticoagulant peptide, transferrin, lactoferrin, endostatin, angiostatin, collagens, immunoglobulins or immunoglobulin-based molecules or fragment of either (e.g. a Small Modular ImmunoPharmaceutical™ ("SMIP") or dAb, Fab' fragments, F(ab')2, scAb, scFv or scFv fragment), a Kunitz domain protein (such as those described in WO 03/066824, with or without albumin fusions), interferons, interleukins, IL10, IL11, IL2, interferon α species and sub-species, interferon β species and sub-species, interferon γ species and sub-species, leptin, CNTF, $CNTF_{Ax15}$, IL1-receptor antagonist, erythropoietin (EPO) and EPO mimics, thrombopoietin (TPO) and TPO mimics, prosaptide, cyanovirin-N, 5-helix, T20 peptide, T1249 peptide, HIV gp41, HIV gp120, urokinase, prourokinase, tPA, hirudin, platelet derived growth factor, parathyroid hormone, proinsulin, insulin, glucagon, glucagon-like peptides, insulin-like growth factor, calcitonin, growth hormone, transforming growth factor β, tumour necrosis factor, G-CSF, GM-CSF, M-CSF, FGF, coagulation factors in both pre and active forms, including but not limited to plasminogen, fibrinogen, thrombin, pre-thrombin, pro-thrombin, von Willebrand's factor, $α_1$-antitrypsin, plasminogen activators, Factor VII, Factor VIII, Factor IX, Factor X and Factor XIII, nerve growth factor, LACI, platelet-derived endothelial cell growth factor (PD-ECGF), glucose oxidase, serum cholinesterase, aprotinin, amyloid precursor protein, inter-alpha trypsin inhibitor, antithrombin III, apo-lipoprotein species, Protein C, Protein S, or a variant or fragment of any of the above.

A "variant", in the context of the above-listed proteins, refers to a protein wherein at one or more positions there have been amino acid insertions, deletions, or substitutions, either conservative or non-conservative, provided that such changes result in a protein whose basic properties, for example enzymatic activity or receptor binding (type of and specific activity), thermostability, activity in a certain pH-range (pH-stability) have not significantly been changed. "Significantly" in this context means that one skilled in the art would say that the properties of the variant may still be different but would not be unobvious over the ones of the original protein.

By "conservative substitutions" is intended combinations such as Val, Ile, Leu, Ala, Met; Asp, Glu; Asn, Gln; Ser, Thr, Gly, Ala; Lys, Arg, His; and Phe, Tyr, Trp. Preferred conservative substitutions include Gly, Ala; Val, Ile, Leu; Asp, Glu; Asn, Gln; Ser, Thr; Lys, Arg; and Phe, Tyr.

A "variant" typically has at least 25%, at least 50%, at least 60% or at least 70%, preferably at least 80%, more preferably at least 90%, even more preferably at least 95%, yet more preferably at least 99%, most preferably at least 99.5% sequence identity to the polypeptide from which it is derived.

The percent sequence identity between two polypeptides may be determined using suitable computer programs, for example the GAP program of the University of Wisconsin Genetic Computing Group and it will be appreciated that percent identity is calculated in relation to polypeptides whose sequence has been aligned optimally.

The alignment may alternatively be carried out using the Clustal W program (Thompson et al., (1994) Nucleic Acids Res., 22(22), 4673-80). The parameters used may be as follows:

- Fast pairwise alignment parameters: K-tuple(word) size; 1, window size; 5, gap penalty; 3, number of top diagonals; 5. Scoring method: x percent.
- Multiple alignment parameters: gap open penalty; 10, gap extension penalty; 0.05.
- Scoring matrix: BLOSUM.

Such variants may be natural or made using the methods of protein engineering and site-directed mutagenesis as are well known in the art.

A "fragment", in the context of the above-listed proteins, refers to a protein wherein at one or more positions there have been deletions. Thus the fragment may comprise at most 5, 10, 20, 30, 40 or 50% of the complete sequence of the full mature polypeptide. Typically a fragment comprises up to 60%, more typically up to 70%, preferably up to 80%, more preferably up to 90%, even more preferably up to 95%, yet more preferably up to 99% of the complete sequence of the full desired protein. Particularly preferred fragments of a protein comprise one or more whole domains of the protein.

In one particularly preferred embodiment the heterologous protein comprises the sequence of albumin or a variant or fragment thereof.

By "albumin" we include a protein comprising the sequence of an albumin protein obtained from any source. Typically the source is mammalian. In one preferred embodiment the serum albumin is human serum albumin ("HSA"). The term "human serum albumin" includes the meaning of a serum albumin having an amino acid sequence naturally occurring in humans, and variants thereof. Preferably the albumin has the amino acid sequence disclosed in WO 90/13653 or a variant thereof. The HSA coding sequence is obtainable by known methods for isolating cDNA corresponding to human genes, and is also disclosed in, for example, EP 73 646 and EP 286 424.

In another preferred embodiment the "albumin" comprises the sequence of bovine serum albumin. The term "bovine serum albumin" includes the meaning of a serum albumin having an amino acid sequence naturally occurring in cows, for example as taken from Swissprot accession number P02769, and variants thereof as defined below. The term "bovine serum albumin" also includes the meaning of fragments of full-length bovine serum albumin or variants thereof, as defined below.

In another preferred embodiment the albumin comprises the sequence of an albumin derived from one of serum albumin from dog (e.g. see Swissprot accession number P49822), pig (e.g. see Swissprot accession number P08835), goat (e.g. as available from Sigma as product no. A2514 or A4164), turkey (e.g. see Swissprot accession number O73860), baboon (e.g. as available from Sigma as product no. A1516), cat (e.g. see Swissprot accession number P49064), chicken (e.g. see Swissprot accession number P19121), ovalbumin (e.g. chicken ovalbumin) (e.g. see Swissprot accession number P01012), donkey (e.g. see Swissprot accession number P39090), guinea pig (e.g. as available from Sigma as product no. A3060, A2639, 05483 or A6539), hamster (e.g. as available from Sigma as product no. A5409), horse (e.g. see Swissprot accession number P35747), rhesus monkey (e.g. see Swissprot accession number Q28522), mouse (e.g. see Swissprot accession number O89020), pigeon (e.g. as defined by Khan et al, 2002, Int. J. Biol. Macromol., 30(3-4), 171-8), rabbit (e.g. see Swissprot accession number P49065), rat (e.g. see Swissprot accession number P36953) and sheep (e.g. see Swissprot accession number P14639) and includes variants and fragments thereof as defined below.

Many naturally occurring mutant forms of albumin are known. Many are described in Paters, (1996, All About Albumin: Biochemistry, Genetics and Medical Applications, Academic Press, Inc., San Diego, California, p.170-181). A variant as defined above may be one of these naturally occurring mutants.

A "variant albumin" refers to an albumin protein wherein at one or more positions there have been amino acid insertions, deletions, or substitutions, either conservative or non-conservative, provided that such changes result in an albumin protein for which at least one basic property, for example binding activity (type of and specific activity e.g. binding to bilirubin), osmolarity (oncotic pressure, colloid osmotic pressure), behaviour in a certain pH-range (pH-stability) has not significantly been changed. "Significantly" in this context means that one skilled in the art would say that the properties of the variant may still be different but would not be unobvious over the ones of the original protein.

By "conservative substitutions" is intended combinations such as Gly, Ala; Val, Ile, Leu; Asp, Glu; Asn, Gln; Ser, Thr; Lys, Arg; and Phe, Tyr. Such variants may be made by techniques well known in the art, such as by site-directed mutagenesis as disclosed in US Patent No 4,302,386 issued 24 November 1981 to Stevens.

Typically an albumin variant will have more than 40%, usually at least 50%, more typically at least 60%, preferably at least 70%, more preferably at least 80%, yet more preferably at least 90%, even more preferably at least 95%, most preferably at least 98% or more sequence identity with naturally occurring albumin. The percent sequence identity between two polypeptides may be determined using suitable computer programs, for example the GAP program of the University of Wisconsin Genetic Computing Group and it will be appreciated that percent identity is calculated in relation to polypeptides whose sequence has been aligned optimally. The alignment may alternatively be carried out using the Clustal W program (Thompson *et al.,* 1994). The parameters used may be as follows:

Fast pairwise alignment parameters: K-tuple(word) size; 1, window size; 5, gap penalty; 3, number of top diagonals; 5. Scoring method: x percent Multiple alignment parameters: gap open penalty; 10, gap extension penalty; 0.05. Scoring matrix: BLOSUM.

The term "fragment" as used above includes any fragment of full-length albumin or a variant thereof, so long as at least one basic property, for example binding activity (type of and specific activity e.g. binding to bilirubin), osmolarity (oncotic pressure, colloid osmotic pressure), behaviour in a certain pH-range (pH-stability) has not significantly been changed. "Significantly" in this context means that one skilled in the art would say that the properties of the variant may still be different but would not be unobvious over the ones of the original protein. A fragment will typically be at least 50 amino acids long. A fragment may comprise at least one whole sub-domain of albumin. Domains of HSA have been expressed as recombinant proteins (Dockal, M. et al., 1999, J. Biol. Chem., 274, 29303-29310), where domain I was defined as consisting of amino acids 1-197, domain II was defined as consisting of amino acids 189-385 and domain III was defined as consisting of amino acids 381-585. Partial overlap of the domains occurs because of the extended α-helix structure (h10-h1) which exists between domains I and II, and between domains II and III (Peters, 1996, *op. cit.,* Table 2-4). HSA also comprises six sub-domains (sub-domains IA, IB, IIA, IIB, IIIA and IIIB). Sub-domain IA comprises amino acids 6-105, sub-domain IB comprises amino acids 120-177, sub-domain IIA comprises amino acids 200-291, sub-domain IIB comprises amino acids 316-369, sub-domain IIIA comprises amino acids 392-491 and sub-domain IIIB comprises amino acids 512-583. A fragment may comprise a whole or part of one or more domains or sub-domains as defined above, or any combination of those domains and/or sub-domains.

In another particularly preferred embodiment the heterologous protein comprises the sequence of transferrin or a variant or fragment thereof. The term "transferrin" as used herein includes all members of the transferrin family (Testa, Proteins of iron metabolism, CRC Press, 2002; Harris & Aisen, Iron carriers and iron proteins, Vol. 5, Physical Bioinorganic Chemistry, VCH, 1991) and their derivatives, such as transferrin, mutant transferrins (Mason et al, 1993, Biochemistry, 32, 5472; Mason et al, 1998, Biochem. J., 330(1), 35), truncated transferrins, transferrin lobes (Mason et al, 1996, Protein Expr. Purif., 8, 119; Mason et al, 1991, Protein Expr. Purif., 2, 214), lactoferrin, mutant lactoferrins, truncated lactoferrins, lactoferrin lobes or fusions of any of the above to other peptides, polypeptides or proteins (Shin et al, 1995, Proc. Natl. Acad. Sci. USA, 92, 2820; Ali et al, 1999, J. Biol. Chem., 274, 24066; Mason et al, 2002, Biochemistry, 41, 9448).

The transferrin may be human transferrin. The term "human transferrin" is used herein to denote material which is indistinguishable from transferrin derived from a human or which is a variant or fragment thereof. A "variant" includes insertions, deletions and substitutions, either conservative or non-conservative, where such changes do not substantially alter the useful ligand-binding or immunogenic properties of transferrin.

Mutants of transferrin are included in the invention. Such mutants may have altered immunogenicity. For example, transferrin mutants may display modified (e.g. reduced) glycosylation. The N-linked glycosylation pattern of a transferrin molecule can be modified by adding/removing amino acid glycosylation consensus sequences such as N-X-S/T, at any or all of the N, X, or S/T position.

Transferrin mutants may be altered in their natural binding to metal ions and/or other proteins, such as transferrin receptor. An example of a transferrin mutant modified in this manner is exemplified below.

We also include naturally-occurring polymorphic variants of human transferrin or human transferrin analogues. Generally, variants or fragments of human transferrin will have at least 5%, 10%, 15%, 20%, 30%, 40% or 50% (preferably at least 80%, 90% or 95%) of human transferrin's ligand binding activity (for example iron-binding), weight for weight. The iron binding activity of transferrin or a test sample can be determined spectrophotometrically by 470nm:280nm absorbance ratios for the proteins in their iron-free and fully iron-loaded states. Reagents should be iron-free unless stated otherwise. Iron can be removed from transferrin or the test sample by dialysis against 0.1 M citrate, 0.1 M acetate, 10mM EDTA pH4.5. Protein should be at approximately 20mg/mL in 100mM HEPES, 10mM $NaHCO_3$ pH8.0. Measure the 470nm:280nm absorbance ratio of apo-transferrin (Calbiochem, CN Biosciences, Nottingham, UK) diluted in water so that absorbance at 280nm can be accurately determined spectrophotometrically (0% iron binding). Prepare 20mM iron-nitrilotriacetate (FeNTA) solution by dissolving 191mg nitrotriacetic acid in 2mL 1M NaOH, then add 2mL 0.5M ferric chloride. Dilute to 50mL with deionised water. Fully load apo-transferrin with iron (100% iron binding) by adding a sufficient excess of freshly prepared 20mM FeNTA, then dialyse the holo-transferrin preparation completely against 100mM HEPES, 10mM $NaHCO_3$ pH8.0 to remove remaining FeNTA before measuring the absorbance ratio at 470nm:280nm. Repeat the procedure using test sample, which should initially be free from iron, and compare final ratios to the control.

Additionally, single or multiple heterologous fusions comprising any of the above; or single or multiple heterologous fusions to albumin, transferrin or immunoglobins or a variant or fragment of any of these may be used. Such fusions include albumin N-terminal fusions, albumin C-terminal fusions and coN-terminal and C-terminal albumin fusions as exemplified by WO 01/79271, and transferrin N-terminal fusions, transferrin C-terminal fusions, and co-N-terminal and C-terminal transferrin fusions.

Examples of transferrin fusions are given in US patent applications US2003/0221201 and US2003/0226155, Shin, et al., 1995, Proc Natl Acad Sci U S A, 92, 2820, Ali, et al., 1999, J Biol Chem, 274, 24066, Mason, et al., 2002, Biochemistry, 41, 9448.

The skilled person will also appreciate that the open reading frame of any other gene or variant, or part or either, can be utilised as an open reading frame for use with the present invention. For example, the open reading frame may encode a protein comprising any sequence, be it a natural protein (including a zymogen), or a variant or a fragment (which may, for example, be a domain) of a natural protein; or a totally synthetic protein; or a single or multiple fusion of different proteins (natural or synthetic). Such proteins can be taken, but not exclusively, from the lists provided in WO 01/79258, WO 01/79271, WO 01/79442, WO 01/79443, WO 01/79444 and WO 01/79480, or a variant or fragment thereof.

Although these patent applications present the list of proteins in the context of fusion partners for albumin, the present invention is not so limited and, for the purposes of the present invention, any of the proteins listed therein may be presented alone or as fusion partners for albumin, the Fc region of immunoglobulin, transferrin, lactoferrin or any other protein or fragment or variant of any of the above, as a desired polypeptide.

The heterologous protein may be a therapeutically active protein. In other words, it may have a recognised medical effect on individuals, such as humans. Many different types of therapeutically active protein are well known in the art.

The heterologous protein may comprise a leader sequence effective to cause secretion in yeast

Numerous natural or artificial polypeptide signal sequences (also called secretion pre regions) have been used or developed for secreting proteins from host cells. The signal sequence directs the nascent protein towards the machinery of the cell that exports proteins from the cell into the surrounding medium or, in some cases, into the periplasmic space. The signal sequence is usually, although not necessarily, located at the N-terminus of the primary translation product and is generally, although not necessarily, cleaved off the protein during the secretion process, to yield the "mature" protein.

In the case of some proteins the entity that is initially secreted, after the removal of the signal sequence, includes additional amino acids at its N-terminus called a "pro" sequence, the intermediate entity being called a "pro-protein". These prop sequences may assist the final protein to fold and become functional, and are usually then cleaved off. In other instances, the pro region simply provides a cleavage site for an enzyme to cleave off the pre-pro region and is not known to have another function.

The pro sequence can be removed either during the secretion of the protein from the cell or after export from the cell into the surrounding medium or periplasmic space.

Polypeptide sequences which direct the secretion of proteins, whether they resemble signal (i.e. pre) sequences or pre-pro secretion sequences, are referred to as leader sequences. The secretion of proteins is a dynamic process involving translation, translocation and post-translational processing, and one or more of these steps may not necessarily be completed before another is either initiated or completed.

For production of proteins in eukaryotic species such as the yeasts *Saccharomyces cerevisiae, Zygosaccharomyces* species, *Kluyveromyces lactis* and *Pichia pastoris,* known leader sequences include those from the *S. cerevisiae* acid phosphatase protein (Pho5p) (see EP 366 400), the invertase protein (Suc2p) (see Smith et al. (1955) Science, 229, 1219-1224) and heat-shock protein-150 (Hsp150p) (see WO 95/33833). Additionally, leader sequences from the *S. cerevisiae* mating factor alpha-1 protein (MF$\alpha$-1) and from the human lysozyme and human serum albumin (HSA) protein have been used, the latter having been used specially, although not exclusively, for secreting human albumin. WO 90/01063 discloses a fusion of the MF$\alpha$-1 and HSA leader sequences, which advantageously reduces the production of a contaminating fragment of human albumin relative to the use of the MF$\alpha$-1 leader sequence. Modified leader sequences are also disclosed in the examples of this application and the reader will appreciate that those leader sequences can be used with proteins other than transferrin. In addition, the natural transferrin leader sequence may be used to direct secretion of transferrin and other heterologous proteins.

Where the chaperone is protein disulphide isomerase, then preferably the heterologous protein comprises disulphide bonds in its mature form. The disulphide bonds may be intramolecular and/or intermolecular.

The heterologous protein may be a commercially useful protein. Some heterologously expressed proteins are intended to interact with the cell in which they are expressed in order to bring about a beneficial effect on the cell's activities. These proteins are not, in their own right, commercially useful. Commercially useful proteins are proteins that have a utility *ex vivo* of the cell in which they are expressed. Nevertheless, the skilled reader will appreciate that a commercially useful protein may also have a biological effect on the host cell expressing it as a heterologous protein, but that that effect is not the main or sole reason for expressing the protein therein.

In one embodiment it is preferred that the heterologous protein is not β-lactamase. In another embodiment it is preferred that the heterologous protein is not antistasin. However, the reader will appreciate that neither of these provisos exclude genes encoding either β-lactamase or antistasin from being present on the 2$\mu$m-family plasmid of the invention, merely, that the gene encoding the heterologous protein encodes a protein other than β-lactamase and/or antistasin.

Plasmids can be prepared by modifying 2$\mu$m-family plasmids known in the art by inserting a gene encoding a chaperone and inserting a gene encoding a heterologous protein using techniques well known in the art such as are described in by Sambrook et al., Molecular Cloning: A Laboratory Manual, 2001, 3rd edition. For example, one such method involves ligation via cohesive ends. Compatible cohesive ends can be generated on a DNA fragment for insertion and plasmid by the action of suitable restriction enzymes. These ends will rapidly anneal through complementary base pairing and remaining nicks can be closed by the action of DNA ligase.

A further method uses synthetic double stranded oligonucleotide linkers and adaptor. DNA fragments with blunt ends are generated by bacteriophage T4 DNA polymerase or *E.coli* DNA polymerase I which remove protruding 3' termini and fill in recessed 3' ends. Synthetic linkers and pieces of blunt-ended double-stranded DNA, which contain recognition sequences for defined restriction enzymes can be ligated to blunt-ended DNA fragments by T4 DNA ligase. They are subsequently digested with appropriate restriction enzymes to create cohesive ends and ligated to an expression vector with compatible termini. Adaptors are also chemically synthesised DNA fragments which contain one blunt end used for ligation but which also possess one preformed cohesive end. Alternatively a DNA fragment or DNA fragments can be ligated together by the action of DNA ligase in the presence or absence of one or more synthetic double stranded oligonucleotides optionally containing cohesive ends.

Synthetic linkers containing a variety of restriction endonuclease sites are commercially available from a number of sources including Sigma-Genosys Ltd, London Road, Pampisford, Cambridge, United Kingdom.

Appropriate insertion sites in 2μm-family plasmids include, but are not limited to, those discussed above.

The present invention also provides a host cell comprising a plasmid as defined above. The host cell may be any type of cell. Bacterial and yeast host cells are preferred. Bacterial host cells may be useful for cloning purposes. Yeast host cells may be useful for expression of genes present in the plasmid.

In one embodiment the host cell is a yeast cell, such as a member of the *Saccharomyces, Kluyveromyces,* or *Pichia* genus, such *Saccharomyces cerevisiae, Kluyveromyces lactis, Pichia pastoris and Pichia membranaefaciens, or Zygosaccharomyces rouxii, Zygosaccharomyces bailii, Zygosaccharomyces fermentati,* or *Kluyveromyces drosphilarum* are preferred.

The host cell type may be selected for compatibility with the plasmid type being used. Plasmids obtained from one yeast type can be maintained in other yeast types (Irie et al, 1991, Gene, 108(1), 139-144; Irie et al, 1991, Mol. Gen. Genet., 225(2), 257-265). For example, pSR1 from *Zygosaccharomyces rouxii* can be maintained in *Saccharomyces cerevisiae.* Preferably, the host cell is compatible with the 2μm-family plasmid used (see below for a full description of the following plasmids). For example, where the plasmid is based on pSR1, pSB3 or pSB4 then a suitable yeast cell is *Zygosaccharomyces rouxii;* where the plasmid is based on pSB1 or pSB2 then a suitable yeast cell is *Zygosaccharomyces bailli;* where the plasmid is based on pSM1 then a suitable yeast cell is *Zygosaccharomyces fermentati*; where the plasmid is based on pKD1 then a suitable yeast cell is *Kluyveromyces drosophilarum* ; where the plasmid is based on pPM1 then a suitable yeast cell is *Pichia membranaefaciens* ; where the plasmid is based on the 2μm plasmid then a suitable yeast cell is *Saccharomyces cerevisiae* or *Saccharomyces carlsbergensis.* It is particularly preferred that the plasmid is based on the 2μm plasmid and the yeast cell is *Saccharomyces cerevisiae.*

A 2μm-family plasmid of the invention can be said to be "based on" a naturally occurring plasmid if it comprises one, two or preferably three of the genes *FLP, REP1* and *REP2* having sequences derived from that naturally occurring plasmid.

It may be particularly advantageous to use a yeast deficient in one or more protein mannosyl transferases involved in O-glycosylation of proteins, for instance by disruption of the gene coding sequence.

Recombinantly expressed proteins can be subject to undesirable post-translational modifications by the producing host cell. For example, the albumin protein sequence does not contain any sites for N-linked glycosylation and has not been reported to be modified, in nature, by O-linked glycosylation. However, it has been found that recombinant human albumin ("rHA") produced in a number of yeast species can be modified by O-linked glycosylation, generally involving mannose. The mannosylated albumin is able to bind to the lectin Concanavalin A. The amount of mannosylated albumin produced by the yeast can be reduced by using a yeast strain deficient in one or more of the *PMT* genes (WO 94/04687). The most convenient way of achieving this is to create a yeast which has a defect in its genome such that a reduced level of one of the Pmt proteins is produced. For example, there may be a deletion, insertion or transposition in the coding sequence or the regulatory regions (or in another gene regulating the expression of one of the *PMT* genes) such that little or no Pmt protein is produced. Alternatively, the yeast could be transformed to produce an anti-Pmt agent, such as an anti-Pmi antibody.

If a yeast other than *S. cerevisiae* is used, disruption of one or more of the genes equivalent to the *PMT* genes of *S. cerevisiae* is also beneficial, e.g. in *Pichia pastoris* or *Kluyveromyces lactis.* The sequence of *PMT1* (or any other *PMT* gene) isolated from *S. cerevisiae* may be used for the identification or disruption of genes encoding similar enzymatic activities in other fungal species. The cloning of the *PMT1* homologue of *Kluyveromyces lactis* is described in WO 94/04687.

The yeast will advantageously have a deletion of the *HSP150* and/or *YAP3* genes as taught respectively in WO 95/33833 and WO 95/23857.

A plasmid as defined above, may be introduced into a host through standard techniques. With regard to transformation of prokaryotic host cells, see, for example, Cohen et al (1972) Proc. Natl. Acad Sci. USA 69, 2110 and Sambrook et al (2001) Molecular Cloning, A Laboratory Manual, 3rd Ed. Cold. Spring Harbor Laboratory, Cold Spring Harbor, NY. Transformation of yeast cells is described in Sherman et al (1986) Methods In Yeast Genetic, A Laboratory Manual, Cold Spring Harbor, NY. The method of Beggs (1978) Nature 275, 104-109 is also useful. Methods for the transformation of *S. cerevisiae* are taught generally in EP 251 744, EP 258 067 and WO 90/01063. With regard to vertebrate cells, reagents useful in transfecting such cells, for example calcium phosphate and DEAE-dextran or liposome formulations, are available from Stratagene Cloning Systems or Life Technologies Inc., Gaithersburg, MD 20877, USA.

Electroporation is also useful for transforming cells and is well known in the art for transforming yeast cell, bacterial cells and vertebrate cells. Methods for transformation of yeast by electroporation are disclosed in Becker & Guarente (1990) Methods Enzymol. 194, 182.

Generally, the plasmid will transform not all of the hosts and it will therefore be necessary to select for transformed host cells. Thus, a plasmid may comprise a selectable marker, including but not limited to bacterial selectable marker and/or a yeast selectable marker. A typical bacterial selectable marker is the β-lactamase gene although many others are known in the art. Typical yeast selectable marker include *LEU2, TRP1, HIS3, HIS4, URA3, URA5, SFA1, ADE2, MET15, LYS5, LYS2, ILV2, FBA1, PSE1, PDI1* and *PGK1.* Those skilled in the art will appreciate that any gene whose chromosomal deletion or inactivation results in an inviable host, so called essential genes, can be used as a selective marker if a functional gene is provided on the plasmid, as demonstrated for *PGK1* in a *pgk1* yeast strain (Piper and Curran, 1990, Curr. Genet. 17, 119). Suitable essential genes can be found within the Stanford Genome Database (SGD), http::/db.yeastgenome.org). Any essential gene product (e.g. PDI1, PSE1, PGK1 or FBA1) which, when deleted or inactivated, does not result in an auxotrophic (biosynthetic) requirement, can be used as a selectable marker on a plasmid in a host cell that, in the absence of the plasmid, is unable to produce that gene product, to achieve increased plasmid stability without the disadvantage of requiring the cell to be cultured under specific selective conditions. By "auxotrophic (biosynthetic) requirement" we include a deficiency which can be complemented by additions or modifications to the growth medium. Therefore, preferred "essential marker genes" in the context of the present invention are those that, when deleted or inactivated in a host cell, result in a deficiency which cannot be complemented by additions or modifications to the growth medium.

Additionally, a plasmid according to any one of the first, second or third aspects of the present invention may comprise more than one selectable marker.

One selection technique involves incorporating into the expression vector a DNA sequence marker, with any necessary control elements, that codes for a selectable trait in the transformed cell. These markers include dihydrofolate reductase, G418 or neomycin resistance for eukaryotic cell culture, and tetracyclin, kanamycin or ampicillin (i.e. β-lactamase) resistance genes for culturing in *E. coli* and other bacteria. Alternatively, the gene for such selectable trait can be on another vector, which is used to co-transform the desired host cell.

Another method of identifying successfully transformed cells involves growing the cells resulting from the introduction of a plasmid of the invention, optionally to allow the expression of a recombinant polypeptide (i.e. a polypeptide which is encoded by a polynucleotide sequence on the plasmid and is heterologous to the host cell, in the sense that that polypeptide is not naturally produced by the host). Cells can be harvested and lysed and their DNA or RNA content examined for the presence of the recombinant sequence using a method such as that described by Southern (1975) J. Mol. Biol. 98, 503 or Berent et al (1985) Biotech. 3, 208 or other methods of DNA and RNA analysis common in the art. Alternatively, the presence of a polypeptide in the supernatant of a culture of a transformed cell can be detected using antibodies.

In addition to directly assaying for the presence of recombinant DNA, successful transformation can be confirmed by well known immunological methods when the recombinant DNA is capable of directing the expression of the protein. For example, cells successfully transformed with an expression vector produce proteins displaying appropriate antigenicity. Samples of cells suspected of being transformed are harvested and assayed for the protein using suitable antibodies.

Thus, in addition to the transformed host cells themselves, the present invention also contemplates a culture of those cells, preferably a monoclonal (clonally homogeneous) culture, or a culture derived from a monoclonal culture, in a nutrient medium. Alternatively, transformed cells may represent an industrially/commercially or pharmaceutically useful product and can be used without further purification or can be purified from a culture medium and optionally formulated with a carrier or diluent in a manner appropriate to their intended industrial/commercial or pharmaceutical use, and optionally packaged and presented in a manner suitable for that use. For example, whole cells could be immobilised; or used to spray a cell culture directly on to/into a process, crop or other desired target. Similarly, whole cell, such as yeast cells can be used as capsules for a huge variety of applications, such as fragrances, flavours and pharmaceuticals.

Transformed host cells may be cultured for a sufficient time and under appropriate conditions known to those skilled in the art, and in view of the teachings disclosed herein, to permit the expression of the chaperone and heterologous protein encoded by the plasmid.

The culture medium may be non-selective or place a selective pressure on the maintenance of the plasmid.

The thus produced heterologous protein may be present intracellularly or, if secreted, in the culture medium and/or periplasmic space of the host cell.

The step of "purifying the thus expressed heterologous protein from the cultured host cell or the culture medium" optionally comprises cell immobilization, cell separation and/or cell breakage, but always comprises at least one other purification step different from the step or steps of cell immobilization, separation and/or breakage.

Cell immobilization techniques, such as encasing the cells using calcium alginate bead, are well known in the art. Similarly, cell separation techniques, such as centrifugation, filtration (e.g. cross-flow filtration, expanded bed chromatography and the like are well known in the art. Likewise, methods of cell breakage, including beadmilling, sonication, enzymatic exposure and the like are well known in the art.

The at least one other purification step may be any other step suitable for protein purification known in the art. For example purification techniques for the recovery of recombinantly expressed albumin have been disclosed in: WO 92/04367, removal of matrix-derived dye; EP 464 590, removal of yeast-derived colorants; EP 319 067, alkaline precipitation and subsequent application of the albumin to a lipophilic phase; and WO 96/37515, US 5 728 553 and WO 00/44772, which describe complete purification processes.

Proteins other than albumin may be purified from the culture medium by any technique that has been found to be useful for purifying such proteins.

Suitable methods include ammonium sulphate or ethanol precipitation, acid or solvent extraction, anion or cation exchange chromatography, phosphocellulose chromatography, hydrophobic interaction chromatography, affinity chromatography, hydroxylapatite chromatography, lectin chromatography, concentration, dilution, pH adjustment, diafiltration, ultrafiltration, high performance liquid chromatography ("HPLC"), reverse phase HPLC, conductivity adjustment and the like.

In one embodiment, any one or more of the above mentioned techniques may be used to further purifying the thus isolated protein to a commercially or industrially acceptable level of purity. By commercially or industrially acceptable level of purity, we include the provision of the protein at a concentration of at least 0.01 g.L$^{-1}$, 0.02 g.L$^{-1}$, 0.03 g.L$^{-1}$, 0.04 g.L$^{-1}$, 0.05 g.L$^{-1}$, 0.06 g.L$^{-1}$, 0.07 g.L$^{-1}$, 0.08 g.L$^{-1}$, 0.09 g.L$^{-1}$, 0.1 g.L$^{-1}$, 0.2 g.L$^{-1}$, 0.3 g.L$^{-1}$, 0.4 g.L$^{-1}$, 0.5 g.L$^{-1}$, 0.6 g.L$^{-1}$, 0.7 g-L$^{-1}$, 0.8 g.L$^{-1}$, 0.9 g.L$^{-1}$, 1 g.L$^{-1}$, 2 g.L$^{-1}$, 3 g.L$^{-1}$, 4 g.L$^{-1}$, 5 g.L$^{-1}$, 6 g.L$^{-1}$, 7 g.L$^{-1}$, 8 g.L$^{-1}$, 9 g.L$^{-1}$, 10 g.L$^{-1}$, 15 g.L$^{-1}$, 20 g.L$^{-1}$, 25 g.L$^{-1}$, 30 g,L$^{-1}$, 40 g.L$^{-1}$, 50 g.L$^{-1}$, 60 g.L$^{-1}$, 70 g.L$^{-1}$, 80 g.L$^{-1}$, 90 g.L$^{-1}$, 100 g.L$^{-1}$, 150 g.L$^{-1}$, 200 g.L$^{-1}$, 250 g.L$^{-1}$, 300 g.L$^{-1}$, 350 g.L$^{-1}$, 400 g.L$^{-1}$, 500 g.L$^{-1}$, 600 g.L$^{-1}$, 700 g.L$^{-1}$, 800 g.L$^{-1}$, 900 g.L$^{-1}$, 1000 g.L$^{-1}$, or more.

It is preferred that the heterologous protein is purified to achieve a pharmaceutically acceptable level of purity. A protein has a pharmaceutically acceptable level of purity is it is essentially pyrogen free and can be administered in a pharmaceutically efficacious amount without causing medical effects not associated with the activity of the protein.

The resulting heterologous protein may be used for any of its known utilities, which, in the case of albumin, include i.v. administration to patients to treat severe bums, shock and blood loss, supplementing culture media, and as an excipient in formulations of other proteins.

Although it is possible for a therapeutically useful heterologous protein obtained by a process of the of the invention to be administered alone, it is preferable to present it as a pharmaceutical formulation, together with one or more acceptable carriers or diluents. The carrier(s) or diluent(s) must be "acceptable" in the sense of being compatible with the desired protein and not deleterious to the recipients thereof. Typically, the carriers or diluents will be water or saline which will be sterile and pyrogen free.

Optionally the thus formulated protein will be presented in a unit dosage form, such as in the form of a tablet, capsule, injectable solution or the like.

A further embodiment of the present invention provides a host cell recombinantly encoding proteins comprising the sequences of PDI and transferrin-based proteins. By "transferrin-based protein" we mean transferrin or any other member of the transferrin family (e.g. lactoferrin), a variant or fragment thereof or a fusion protein comprising transferrin, a variant or fragment thereof, including the types described above. Thus the present invention also provides for the use of a recombinant PDI gene to increase the expression of a transferrin-based protein.

The PDI gene may be provided on a plasmid, such as a 2$\mu$m-family plasmid as described above. Alternatively, the PDI gene may be chromosomally integrated. In a preferred embodiment, the PDI gene is chromosomally integrated at the locus of an endogenously encoded PDI gene, preferably without disrupting the expression of the endogenous PDI gene. In this context, "without disrupting the expression of the endogenous PDI gene" means that, although some decrease in the protein production from the endogenous PDI gene as a result of the integration may be acceptable (and preferably there is no decrease), the total level of PDI protein production in the modified host cell as a result of the combined effect of expression from the endogenous and integrated PDI genes is increased, relative to the level of PDI protein production by the host cell prior to the integration event.

The gene encoding the transferrin-based protein may be provided on a plasmid, such as a 2$\mu$m-family plasmid as described above, or may be chromosomally integrated, such as at the locus of an endogenously encoded PDI gene, preferably without disrupting the expression of the endogenous PDI gene.

In one embodiment the PDI gene is chromosomally integrated and the gene encoding the transferrin-based protein is provided on a plasmid. In another embodiment, the PDI gene is provided on a plasmid and the gene encoding the transferrin-based protein is chromosomally integrated. In another embodiment both the PDI gene and the gene encoding the transferrin-based protein are chromosomally integrated. In another embodiment both the PDI gene and the gene encoding the transferrin-based protein are provided on a plasmid.

As discussed above, Bao et al, 2000, Yeast, 16, 329-341 reported that over-expression of the *K. lactis* PDI gene *KlPDI1* was toxic to *K. lactis* cells. Against this background we have surprisingly found that, not only is it possible to over-express PDI and other chaperones without the detrimental effects reported in Bao *et al,* but that two different chaperones can be recombinantly over-expressed in the same cell and, rather than being toxic, can increase the expression of heterologous proteins to levels higher than the levels obtained by individual expression of either of the chaperones. This was not expected. On the contrary, in light of the teaching of Bao *et al*, one would think that over-expression of two chaperones would be even more toxic than the over-expression of one. Moreover, in light of the earlier findings of the present invention, it was expected that the increases in heterologous protein expression obtained by co-expression with a single chaperone would be at the maximum level possible for the cell system used. Therefore, it was particularly surprising to find that yet further increases in heterologous protein expression could be obtained by co-expression of two different chaperones with the heterologous protein.

Accordingly, as a fifth aspect of the present invention there is provided a method for producing heterologous protein comprising providing a host cell (such as defined above) comprising a first recombinant gene encoding a protein comprising the sequence of a first chaperone protein, a second recombinant gene encoding a protein comprising the sequence of a second chaperone protein and a third recombinant gene encoding a heterologous protein, wherein the first and second chaperones are different; culturing the host cell in a culture medium under conditions that allow the expression of the first, second and third genes; and optionally purifying the thus expressed heterologous protein from the cultured host cell or the culture medium; and further optionally, lyophilising the thus purified protein.

The method may further comprise the step of formulating the purified heterologous protein with a carrier or diluent and optionally presenting the thus formulated protein in a unit dosage form, in the manner discussed above.

The term "recombinant gene" includes nucleic acid sequences that operate independently as "stand alone" expressible sequences to produce an encoded protein or, in the alternative, nucleic acid sequences introduced that operate in combination with endogenous sequences (such as by integration into an endogenous sequence so as to produce a nucleic acid sequence that is different to the endogenous sequence) within the host to cause increased expression of a target protein.

The first and second chaperones may be a chaperone as discussed above, and are a combination of chaperones that, when co-expressed in the same host cell, provide an additive effect to the increase in expression of the heterologous protein. By "additive effect" we include the meaning that the level of expression of the heterologous protein in the host cell is higher when the first and second recombinant genes are simultaneously co-expressed with the third recombinant gene as compared to the same system wherein (i) the first recombinant gene is co-expressed with the third recombinant gene in the absence of the expression of the second recombinant gene and (ii) the second recombinant gene is co-expressed with the third recombinant gene in the absence of the expression of the first recombinant gene.

One preferred chaperone is protein disulphide isomerase. Another preferred chaperone is ORM2 or a fragment or variant thereof. In a particularly preferred embodiment, the first and second chaperones are protein disulphide isomerase and ORM2 or a fragment or variant thereof.

The first, second and third recombinant genes may each individually be present on a plasmid within the host cell (such as a 2$\mu$m-family plasmid, as discussed above) or be chromosomally integrated within the genome of the host cell. It will be appreciated that any combination of plasmid and chromosomally integrated first, second and third recombinant genes may be used. For example, the first, second and third recombinant genes may each individually be present on a plasmid, and this may be either the same plasmid or different plasmids. Alternatively, the first recombinant gene may be present on a plasmid, and second and third recombinant genes may be chromosomally integrated within the genome of the host cell. Alternatively, the first and second recombinant genes may be present on a plasmid and the third recombinant gene may be chromosomally integrated within the genome of the host cell. Alternatively, the first and third recombinant genes may be present on a plasmid and the second recombinant gene may be chromosomally integrated within the genome of the host cell. Alternatively, the first and second recombinant gene may be chromosomally integrated within the genome of the host cell and the third recombinant gene may be present on a plasmid. Alternatively, the first, second and third recombinant genes may each individually be chromosomally integrated within the genome of the host cell.

Particularly preferred plasmids are those defined above in respect of earlier aspects of the present invention. Accordingly, the present invention also provides a plasmid as defined above wherein the plasmid comprises two different genes (the first and second recombinant genes) encoding different chaperones. In one preferred embodiment, the plasmid may further comprise a gene encoding a heterologous protein (the third recombinant gene), such as a heterologous protein as described above.

In a sixth aspect of the present invention there is provided a method for producing a heterologous protein, such as a heterologous protein as defined above for an earlier aspect of the present invention, comprising: providing a host cell comprising a first recombinant gene encoding the protein comprising the sequence of ORM2 or a variant thereof and a second recombinant gene encoding a heterologous protein; culturing the host cell in a culture medium under conditions that allow the expression of the first and second genes; and purifying the thus expressed heterologous protein from the cultured host cell or the culture medium; and optionally, lyophilising the thus purified protein; and optionally formulating the purified heterologous protein with a carrier or diluent; and optionally presenting the thus formulated protein in a unit dosage form..

In the manner discussed above, the host cell may further comprise a further recombinant gene encoding a protein comprising the sequence of an alternative chaperone to ORM2 or a variant thereof.

Either or both of the first and second recombinant genes may be expressed from a plasmid, and preferably from the same plasmid. A further recombinant gene encoding a protein comprising the sequence of an alternative chaperone to ORM2 or a variant thereof may also be expressed from a plasmid, preferably from the same plasmid as either or both of the first and second recombinant genes. The plasmid may be a 2μm-family plasmid, such as the 2μm plasmid.

The present invention also provides, in a seventh aspect, for the use of a nucleic acid sequence encoding the protein ORM2 or a variant thereof to increase the production, in a host cell, of a heterologous protein encoded by a recombinant gene in the host cell by co-expression of the nucleic acid sequence and the recombinant gene within the host cell. Either or both of the nucleic acid sequence and the recombinant gene encoding the heterologous protein may be expressed from a plasmid within the host cell, and preferably from the same plasmid. In the manner discussed above, the host cell may further comprise a recombinant gene encoding an alternative chaperone to ORM2 or a variant thereof, which may be located on a plasmid within the host cell, preferably on the same plasmid as either or both of the nucleic acid sequence and the recombinant gene encoding the heterologous protein. Suitable plasmids include a 2μm-family plasmid, such as the 2μm plasmid, as discussed above.

In an eighth aspect of the present invention there is also provided the use of a plasmid as an expression vector to increase the production of a heterologous protein by providing a recombinant gene encoding the heterologous protein and a gene encoding ORM2 or a variant thereof on the same plasmid. The plasmid may further comprise a gene encoding an alternative chaperone to ORM2 or a variant thereof in the manner discussed above. Suitable plasmids include a 2μm-family plasmid, such as the 2μm plasmid, as discussed above.

Accordingly, in a ninth aspect, the present invention also provides a 2μm-family plasmid, preferably an expression plasmid, comprising a first gene encoding the protein ORM2 or a variant or fragment thereof and a second gene encoding a heterologous protein, as discussed above. The plasmid may further comprise a third gene encoding an alternative chaperone to ORM2 or a variant thereof. In a preferred embodiment, the third gene encodes a protein comprising the sequence of protein disulphide isomerase.

We have also demonstrated that a plasmid-borne gene encoding a protein comprising the sequence of an "essential" chaperone, such as PDI, can be used to stably maintain the plasmid in a host cell that, in the absence of the plasmid, does not produce the chaperone, and simultaneously increase the expression of a heterologous protein encoded by a recombinant gene within the host cell. This system is advantageous because it allows the user to minimise the number of recombinant genes that need to be carried by a plasmid. For example, typical prior art plasmids carry marker genes (such as those as described above) that enable the plasmid to be stably maintained during host cell culturing process. Such marker genes need to be retained on the plasmid in addition to any further genes that are required to achieve a desired effect. However, the ability of plasmids to incorporate exogenous DNA sequences is limited and it is therefore advantageous to minimise the number of sequence insertions required to achieve a desired effect. Moreover, some marker genes (such as auxotrophic marker genes) require the culturing process to be conducted under specific conditions in order to obtain the effect of the marker gene. Such specific conditions may not be optimal for cell growth or protein production, or may require inefficient or unduly expensive growth systems to be used.

For the purpose of increasing heterologous gene expression, we have found that it is possible to use a gene that recombinantly encodes a protein comprising the sequence of an "essential" chaperone for the dual purpose of increasing the production of a heterologous protein in a host cell and in the role of a selectable marker on a plasmid, where the plasmid is present within a cell that, in the absence of the plasmid, is unable to produce the chaperone. This system has the advantage that it minimises the number of recombinant genes that need to be carried by the plasmid. The system also has the advantage that the host cell can be cultured under conditions that do not have to be adapted for any particular marker gene, without loosing plasmid stability. For example, host cells produced using this system can be culture in rich media, which may be more economical than the minimal media that is commonly used to give auxotrophic marker genes their effect.

Accordingly, we also describe a host cell comprising a plasmid, the plasmid comprising a gene that encodes an essential chaperone wherein, in the absence of the plasmid, the host cell is unable to produce the chaperone. Preferably, in the absence of the plasmid, the host cell is inviable. The host cell may further comprise a recombinant gene encoding a heterologous protein, such as those described above in respect of earlier aspects of the invention.

We also describe a plasmid comprising, as the sole selectable marker, a gene encoding an essential chaperone. The plasmid may further comprise a gene encoding a heterologous protein. The plasmid may be a 2μm-family plasmid.

We also describe method for producing a heterologous protein comprising the steps of: providing a host cell comprising a plasmid, the plasmid comprising a gene that encodes an essential chaperone wherein, in the absence of the plasmid, the host cell is unable to produce the chaperone and wherein the host cell further comprises a recombinant gene encoding a heterologous protein; culturing the host cell in a culture medium under conditions that allow the expression of the essential chaperone and the heterologous protein; and optionally purifying the thus expressed heterologous protein from the cultured host cell or the culture medium; and further optionally, lyophilising the thus purified protein.

The method may further comprise the step of formulating the purified heterologous protein with a carrier or diluent and optionally presenting the thus formulated protein in a unit dosage form, in the manner discussed above. In one preferred embodiment, the method involves culturing the host cell in non-selective media, such as a rich media.

We have surprising also found that different PDI genes have the ability to increase the expression of heterologous proteins by different amounts under particular culture conditions. In particular, as discussed in Example 8, we have shown that the SKQ2n PDI1 gene provides for higher heterologous protein expression than the S288c PDI1 gene, when the host cells are cultured in minimal media.

The sole difference between the encoded proteins of the SKQ2n PDI1 and S288c PDI1 genes is that SKQ2n comprises the additional amino acids EADAEAEA at positions 506-513 (positions as defined with reference to Genbank accession no. CAA38402, as given above).

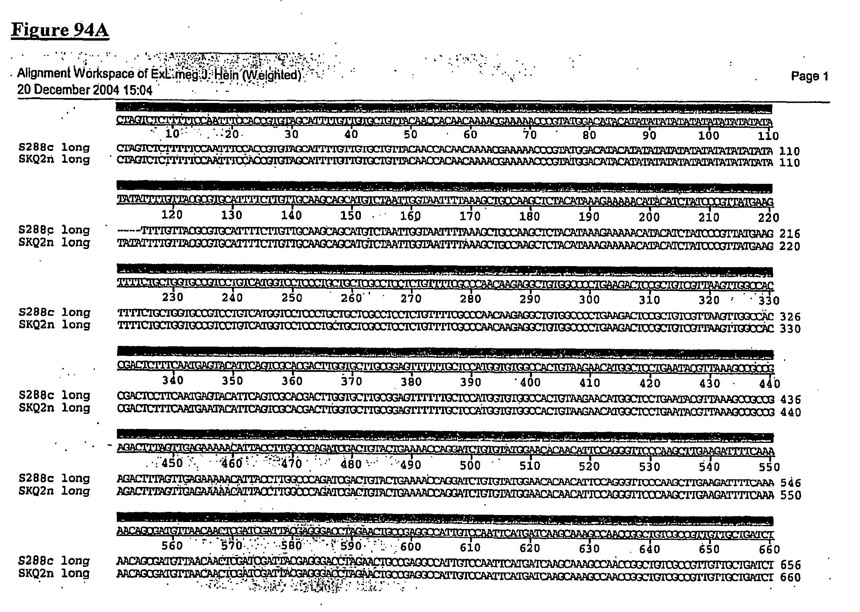

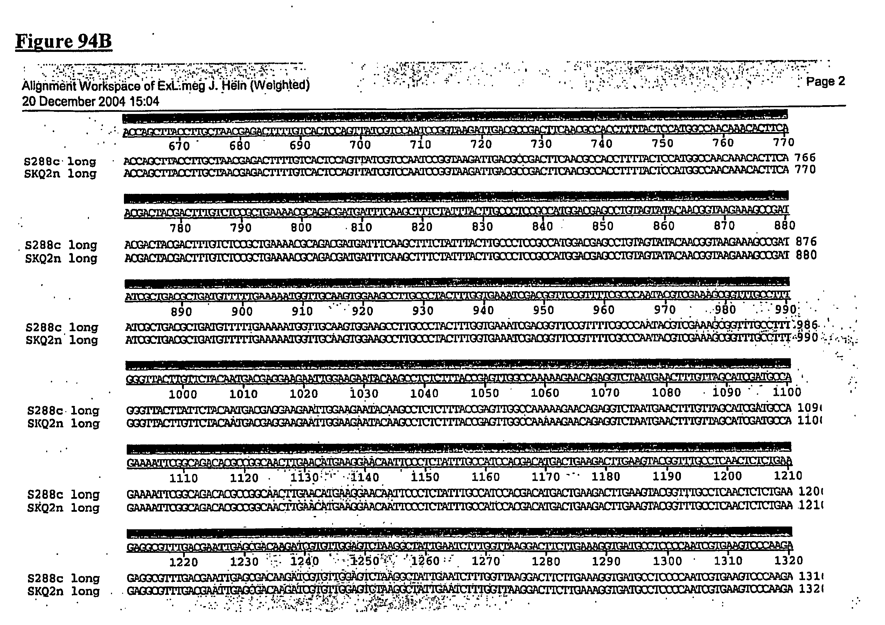

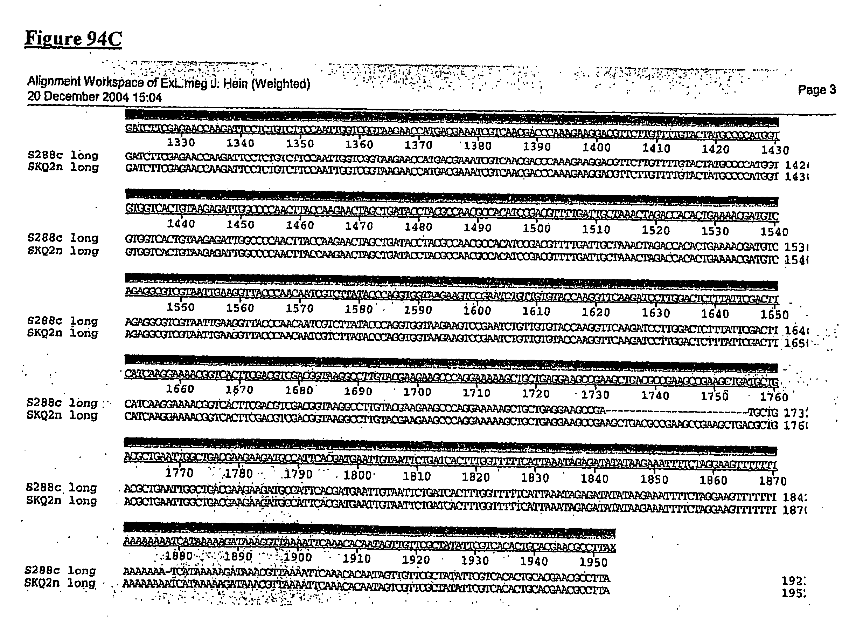

The differences between the gene sequences used are shown in the sequence alignment given in Figure 94 and can be summarised as follows -

- The promoter of SKQ2a includes a run of fourteen "TA" repeats, whereas the promoter of S288c only has twelve "TA" repeats:

- Seer41 is encoded by TCT in SKQ2n, but by TCC in S288c;

- Glu44 is encoded by GAA in SKQ2n but by GAG in S288c;

- Leu262 is encoded by TTG in SKQ2n but by TTA in S288c;

- Asp514 is encoded by GAC in SKQ2n but the homologous Asp506 is encoded by GAT in S288c;

- The terminator sequence of SKQ2n contains a run of 8 consecutive "A" bases, whereas the terminator sequence of S288c contains a run of 7 consecutive "A" bases and does not include an "A" base at the equivalent of position 1880 in the SKQ2n gene;

- The terminator sequence of SKQ2n has a '"C" at position 1919, whereas the terminator sequence of S288c has a "T" at the equivalent position.

It may be advantageous to include any or all of the above mentioned features of the SKQ2n gene in a PDI gene of choice, in order to achieve the observed increase in heterologous protein expression when the host cells are cultured in minimal media.

Accordingly, we also describe a nucleotide sequence encoding a protein disulphide isomerase, for use in increasing the expression of a heterologous protein in a host cell by expression of the nucleotide sequence within the host cell, which host cell is cultured in minimal media, wherein the nucleotide sequence encoding the protein disulphide isomerase is characterised in that it has at least one of the following characteristics -

- the nucleotide sequence comprises a promoter having the sequence of a natural PDI promoter or a functional variant thereof and comprises a run of fourteen "TA" repeats; or
- the encoded protein disulphide isomerase comprises the amino acids EADAEAEA or a conservatively substituted variant thereof, typically at positions 506-513 as defined with reference to Genbank accession no. CAA38402; or
- residue Ser41 of the encoded protein disulphide isomerase is encoded by the codon TCT; or
- residue Glu44 of the encoded protein disulphide isomerase is encoded by the codon GAA; or
- residue Leu262 of the encoded protein disulphide isomerase is encoded by codon TTG; or
- residue Asp514 of the encoded protein disulphide isomerase is encoded by codon GAC; or
- the nucleotide sequence comprises a terminator sequence having the sequence of a natural PDI terminator or a functional variant thereof and either comprises a run of 8 consecutive "A" bases and/or the base "C" at position 1919 (as defined by reference to position 1919 of the natural SKQ2n terminator sequence).

We also describe a method for producing a heterologous protein comprising the steps of: providing a host cell comprising a recombinant gene that encodes a protein disulphide isomerase and having the sequence of the above-defined nucleic acid sequence, the host cell further comprising a recombinant gene encoding a heterologous protein; culturing the host cell in a minimal culture medium under conditions that allow the expression of the protein disulphide isomerase and the heterologeous protein; and optionally purifying the thus expressed heterologous protein from the cultured host cell or the culture medium; and further optionally, lyophilising the thus purified protein; and optionally further formulating the purified heterologous protein with a carrier or diluent; and optionally presenting the thus formulated protein in a unit dosage form, in the manner discussed above.

The genes encoding the PDI and heterologous protein can be provided in the manner described above in respect of other embodiments of the present invention.

We have also found that the effects of recombinantly-provided chaperones according to the other embodiments of the present invention can be modulated by modifying the promotes that control the expression levels of the chaperone (s). Surprisingly we have found that, in some cases, shorter promoters result in increased heterologous protein expression. Without being bound by theory we believe that this is because the expression of a recombinant chaperone in host cells that already express heterologous proteins at high levels can cause the cells to overload themselves with heterologously expressed protein, thereby achieving little or no overall increase in heterologous protein production. In those cases, it may be beneficial to provide recombinant chaperone genes with truncated promoters.

Accordingly, we also describe a polynucleotide (such as a plasmid as defined above) comprising the sequence of a promoter operably connected to a coding sequence encoding a chaperone (such as those described above), for use in increasing the expression of a heterologous protein (such as those described above) in a host cell (such as those described above) by expression of the polynucleotide sequence within the host cell, wherein the promoter is characterised in that it achieves a modified, such as a higher or lower, level of expression of the chaperone than would be achieved if the coding sequence were to be operably connected to its naturally occurring promoter.

We also describe a method for producing a heterologous protein comprising the steps of: providing a host cell comprising a recombinant gene that comprising the sequence of promoter operably connected to a coding sequence encoding a chaperone, the promoter being characterised in that it achieves a lower level of expression of the chaperone than would be achieved if the coding sequence were to be operably connected to its naturally occurring promoter, and the host cell further comprising a recombinant gene encoding a heterologous protein; culturing the host cell under conditions that allow the expression of the chaperone and the heterologous protein; and optionally purifying the thus expressed heterologous protein from the cultured host cell or the culture medium; and further optionally, lyophilising the thus purified protein; and optionally further formulating the purified heterologous protein with a carrier or diluent; and optionally presenting the thus formulated protein in a unit dosage form, in the manner discussed above.

As is apparent from the examples of the present application, the combination of recombinantly expressed PDI and transferrin-based proteins provides a surprisingly high level of transferrin expression. For example, transferrin expression in a system that includes a chromosomally encoded recombinant PDI gene provided a 2-fold increase (compared to a control in which there is no chromosomally encoded recombinant PDI gene). This increase was 5-times greater than an equivalent system comprising a recombinant gene encoding human albumin in place of the recombinant transferrin gene.

The host may be any cell type, such as a prokaryotic cell (e.g. bacterial cells such as *E. coli*) or a eukaryotic cell. Preferred eukaryotic cells include fungal cells, such as yeast cells, and mammalian cells. Exemplary yeast cells are discussed above. Exemplary mammalian cells include human cells.

Host cells as described above can be cultured to produce recombinant transferrin-based proteins. The thus produced transferrin-based proteins can be isolated from the culture and purified, preferably to a pharmaceutically acceptable level of purity, for example using techniques known in the art and/or as set out above. Purified transferrin-based proteins may be formulated with a pharmaceutically acceptable carrier or diluent and may be presented in unit dosage form.

The present invention will now be exemplified with reference to the following non-limiting examples and figures.

**BRIEF DESCRIPTION OF THE FIGURES**

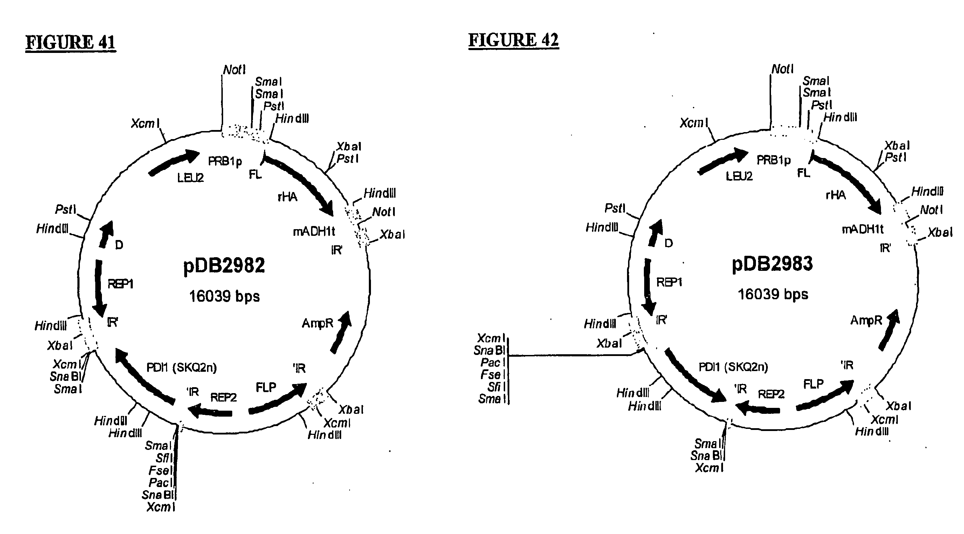

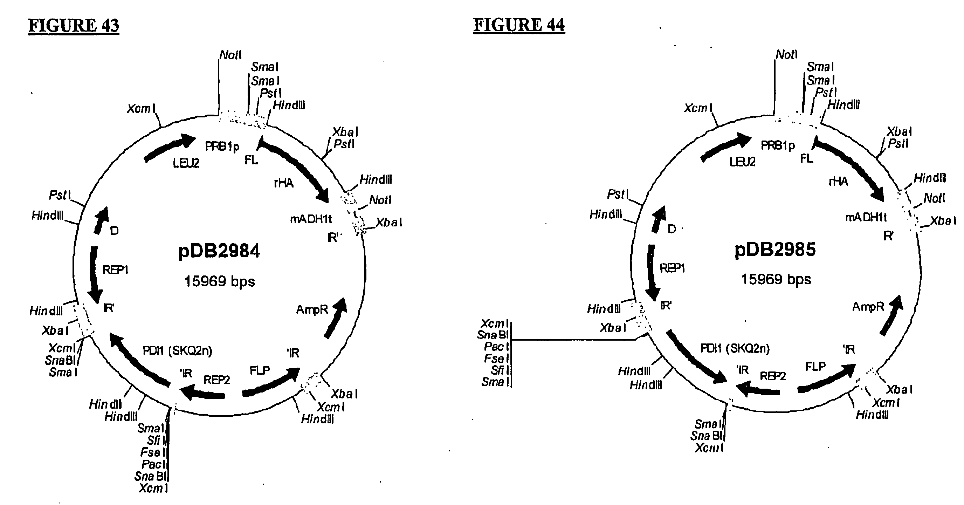

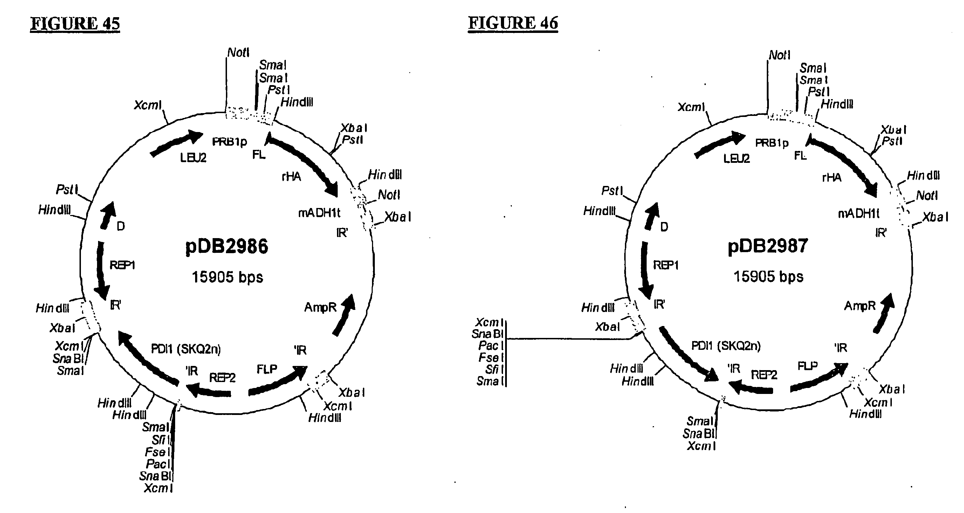

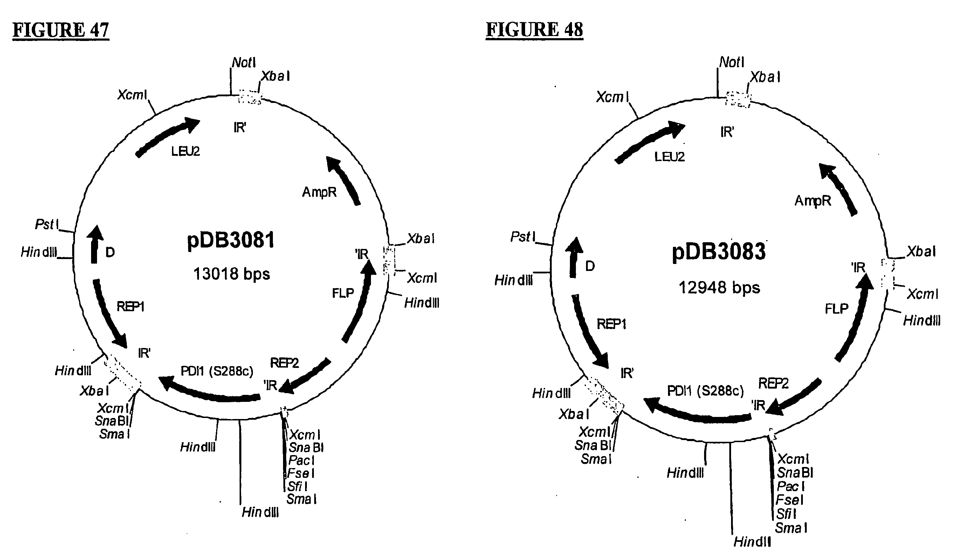

**Figures 1, 2, 4, 6 to 15, 22, 25, 27 to 52, 57 to 71, 74, 75, 77 to 79, 81 to 83, 85 to 91, 95 and 96** show various plasmid maps.

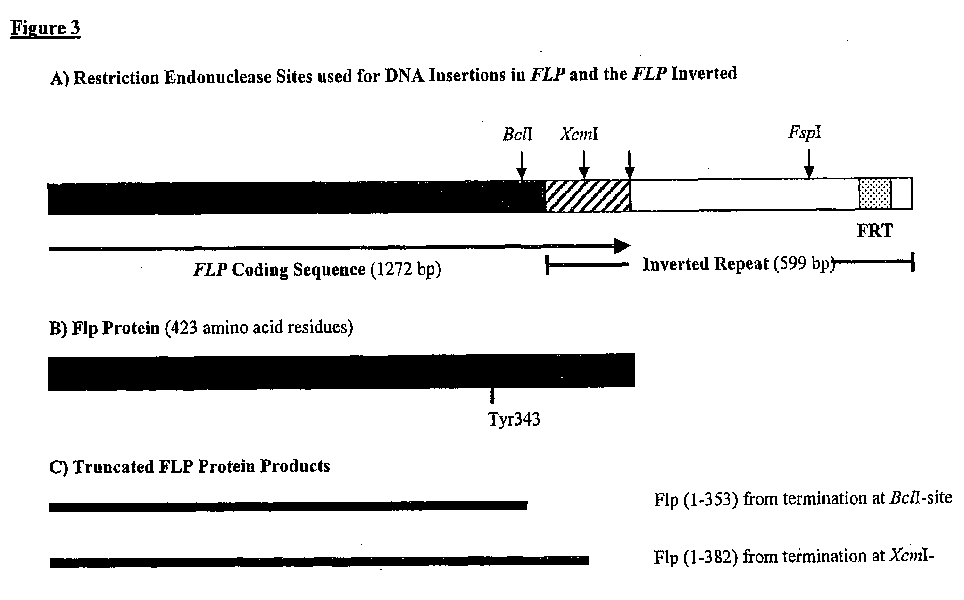

**Figure 3** shows plasmid insertion sites.

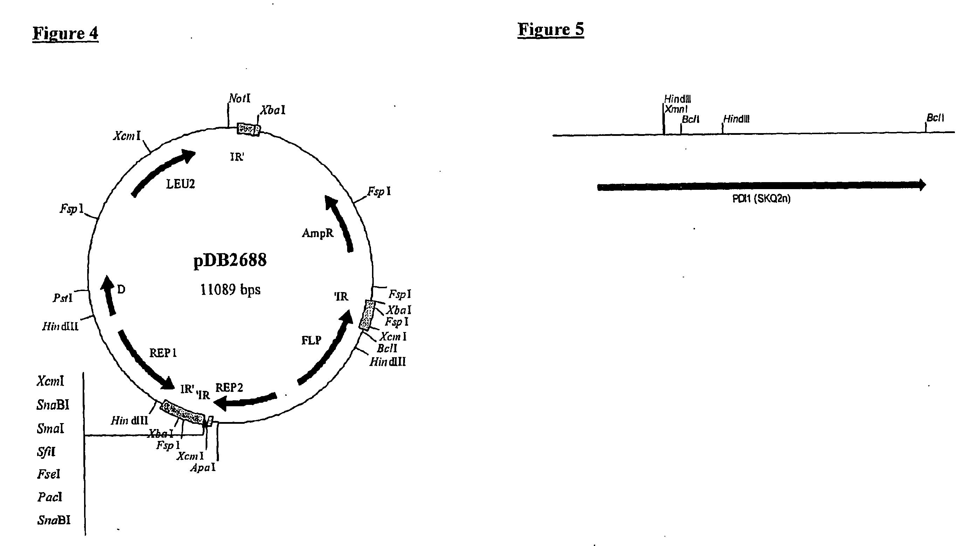

**Figure 5** shows a restriction map of a DNA fragment containing the PDI coding sequence.

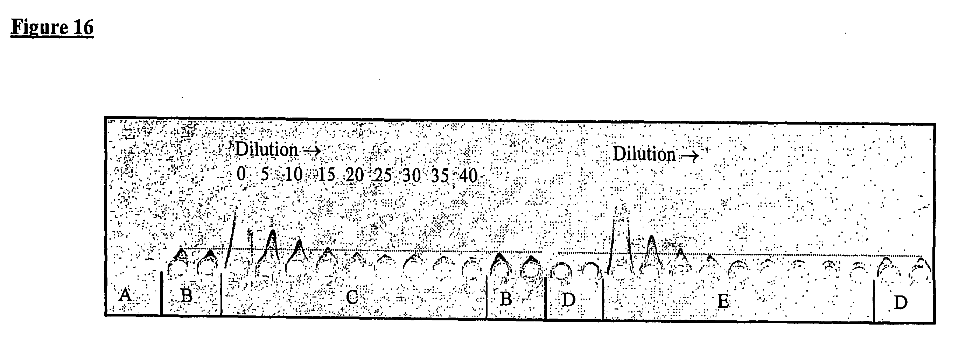

**Figure 16** shows the results of rocket immunoelectrophoresis (RIE) determination of increased recombinant transferrin (N413Q, N611Q) secretion with *PD11* over-expression. Cryopreserved yeast stocks were grown for 4-days in 10mL BMMD shake flask cultures and supernatants were loaded at 5μL per well. Goat polyclonal anti-transferrin (human) antiserum (Calbiochem) was used at 40μL per rocket immunoelectrophoresis gel (50mL). A = Control strain [pSAC35], duplicate flasks; B = Control strain [pDB2536], duplicate flasks; C = Control strain [pDB2711], neat to 40-fold aqueous dilutions; D = Control strain [pDB2931], duplicate flasks; E = Control strain [pDB2929], neat to 40-fold aqueous dilutions.

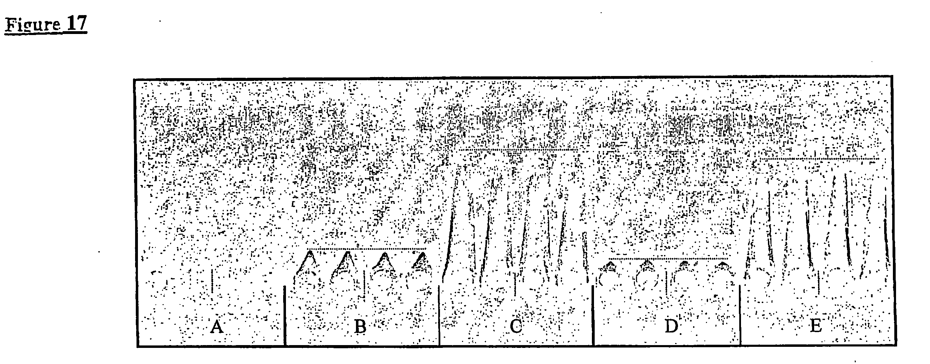

**Figure 17** shows the results of RIE analysis of recombinant transferrin (N413Q, N611Q) secretion with and without *PDI1* over-expression. Cryopreserved yeast stocks were grown for 4-days in 10mL BMMD shake flask cultures and supernatants were loaded at 5μL per well. Duplicate loadings were made of supernatants from two individual cultures of each strain. Goat polyclonal anti-transferrin (human) antiserum (Calbiochem) was used at 40μL per rocket immunoelectrophoresis gel (50mL). A = Control strain [pSAC35]; B = Control strain [pDB2536]; C = Control strain [pDB2711]; D = Control strain [pDB2931]; E = Control strain [pDB2929].

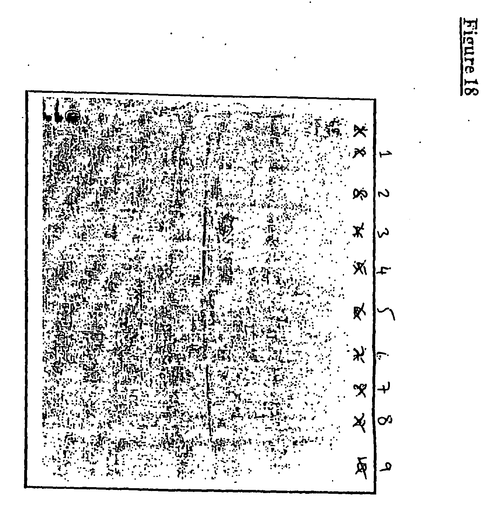

**Figure 18** shows the results of SDS-PAGE analysis of recombinant transferrin secretion with and without *PDI1* over-expression. BMMD shake flask cultures were grown for 4-days and 10μL supernatant analysed on non-reducing SDS-PAGE (4-12% NuPAGE®, MOPS buffer, In Vitrogen) with GelCode® Blue reagent (Pierce). SeeBlue Plus2 Markers (In Vitrogen). 1 = pDB2536; 2 = pDB2536; 3 = pDB2711; 4 = pDB2711; 5 = pDB2931; 6 = pDB2931; 7 = pDB2929; 8 = pDB2929; 9 = pSAC35 control.

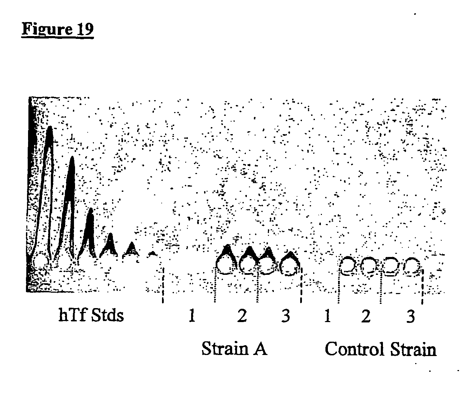

**Figure 19** shows RIE analysis of recombinant transferrin secretion from *S. cerevisiae* strains with an additional integrated copy of *PDI1.* 5-day BMMD shake flask culture supernatants were loaded at 5μL per well Strains contained: 1) pSAC35 (negative control); 2) pDB2536 (recombinant non-glycosylated transferrin (N413Q, N611Q)) or 3) pDB2506 (same as plasmid pDB2536 but the transferrin ORF encodes transferrin without the N→Q mutations at positions 413 and 611, i.e. recombinant glycosylated transferrin). Each well contained a sample derived from an individual transformant. Standards were human plasma holo-transferrin (Calbiochem) at 100, 50, 20, 10, 5 and 2mg.L$^{-1}$.

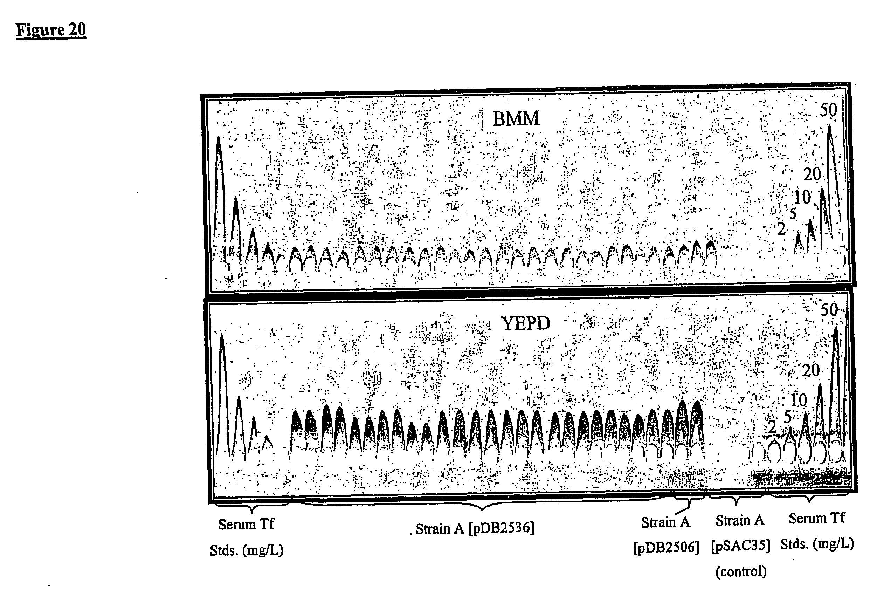

**figure 20** shows RIE analysis of recombinant transferrin secretion from Strain A [pDB2536] and Strain A [pDB2506] grown in shake flask culture. 5-day BMMD or YEPD shake flask culture supernatants were loaded in duplicate at 5μL per well.

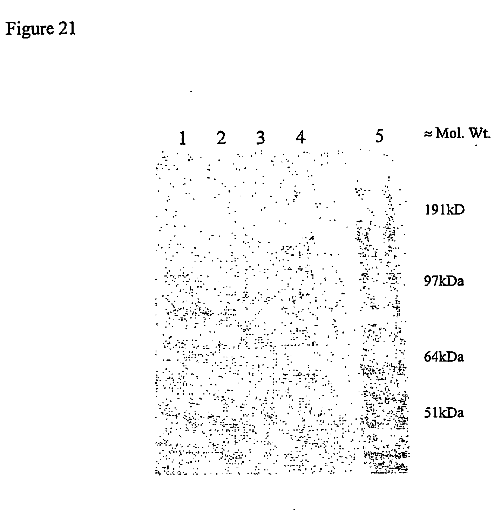

**Figure 21** shows SDS-PAGE analysis of recombinant transferrin secreted from Strain A [pDB2536] and Strain A [pDB2506] grown in shake flask culture. Cultures were grown for 5-days, in BMMD and 30μl supernatants analysed on SDS-PAGE (4-12% NuPAGE™, MOPS Buffer, InVitrogen) stained with GelCode, Blue Reagent (Pierce). 1) Strain A [pDB2536] transformant 1; 2) Strain A [pDB2536] transformant 2; 3) Strain A [pSAC35] control; 4) Strain A [pDB2506] transformant 1; 5) SeeBlue, Plus2 Protein Standards (approximate molecular weights only).

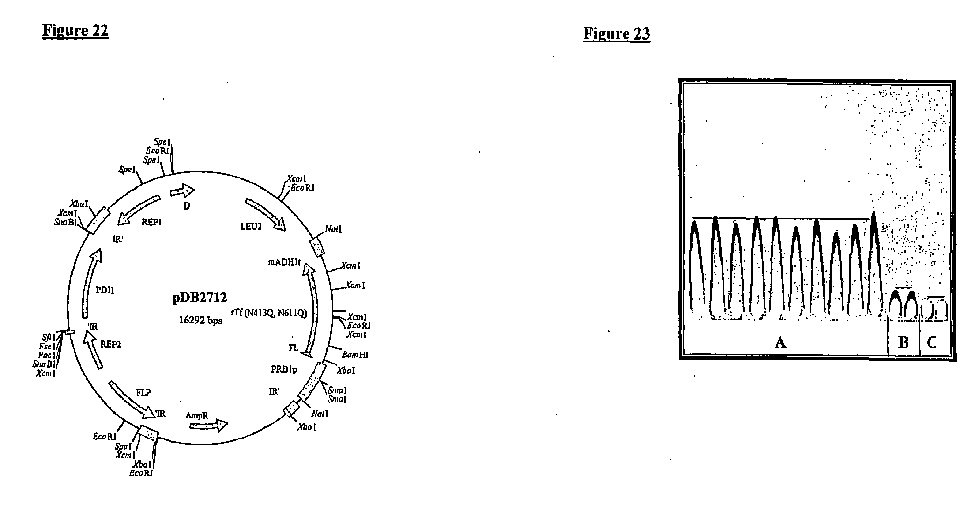

**Figure 23** shows RIE of recombinant transferrin secreted from *S. cerevisiae* strains with different PDI1 copy numbers. 3-day BMMD shake flask culture supernatants were loaded at 5μL per well. Goat polyclonal anti-transferrin (human) antiserum (Calbiochem) was used at 30μL per rocket immunoelectrophoresis gel (50mL). (A) supernatant from S *cerevisiae* control strain [pDB2711] or [pDB2712]; (B) supernatant from Strain A [pDB2536]; (C) supernatant from control strain [pDB2536].

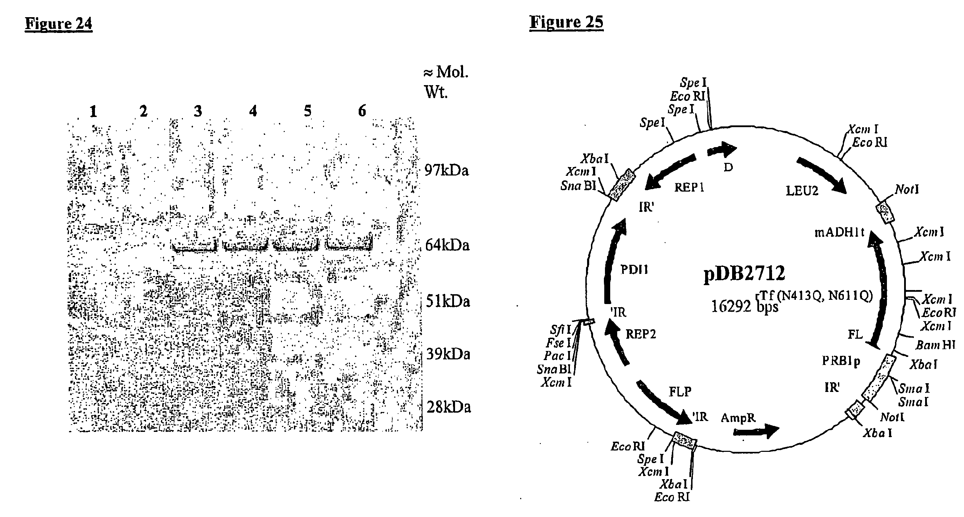

**Figure 24** shows SDS-PAGE analysis of recombinant transferrin secreted from *S. cerevisiae* strains with different PDI1 copy numbers. 4-12% NuPAGE reducing gel run with MOPS buffer (InVitrogen) after loading with 30μL of 3-day BMMD shake flask culture supernatant per lane; (lane 1) supernatant from control strain [pDB2536], (lane 2) supernatant from Strain A [pDB2536], (lanes 3-6) supernatant from control strain [pDB2711] or [pDB2712]; (lane 7) molecular weight markers (SeeBlue Plus2, InVitrogen).

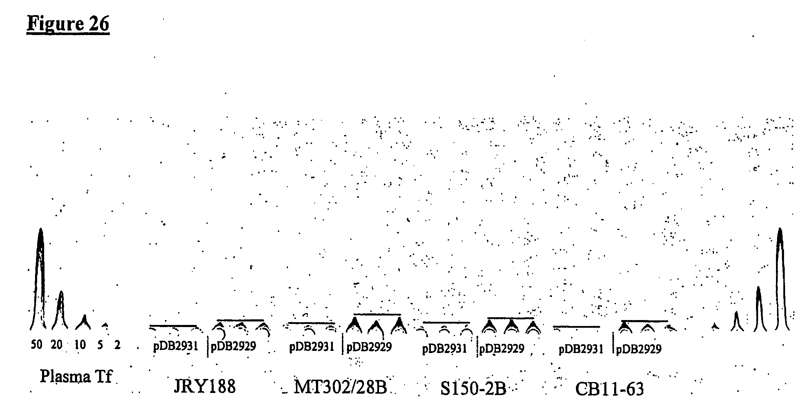

**Figure 26** shows RIE of recombinant transferrin secreted from different *S. cerevisiae* strains with and without additional PDI1 gene co-expression. 10mL YEPD shake flasks were inoculated with yeast and incubated for 4-days at 30˚C. 5μL culture supernatant loaded per well of a rocket immunoelectrophoresis gel. Plasma Tf standards concentrations are in μg/mL. 20μL goat anti-Tf / 50mL agragose. Precipin was stained with Coomassie blue.

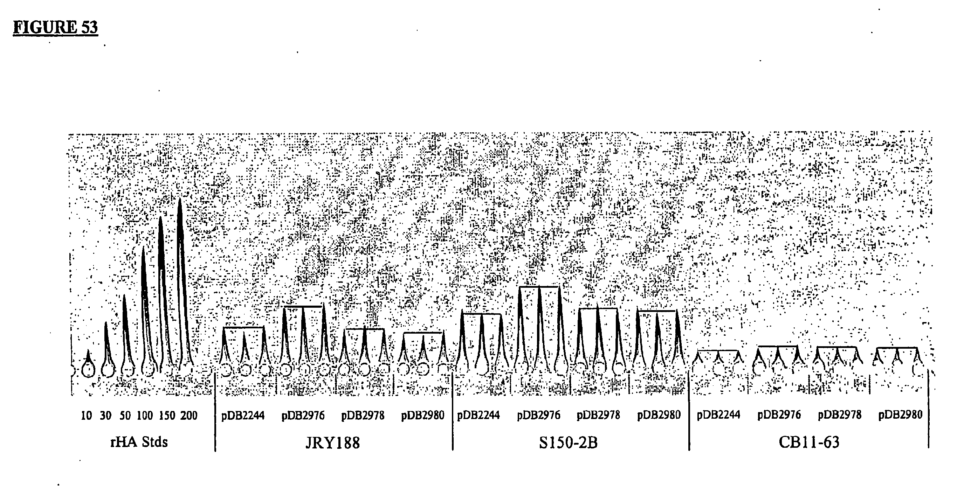

**Figure 53** shows RIE analysis of rHA expression in different *S. cerevisiae* strains when co-expressed with PDI1 genes having different length promoters. 10mL YEPD shake flasks were inoculated with yeast and incubated for 4-days at 30˚C. 4μL culture supernatant loaded per well of a rocket immunoelectrophoresis gel. rHA standards concentrations are in μg/mL. 400μL goat anti-HA (Sigma product A-1151 resuspended in 5mL water) /50mL agarose. Precipin was stained with Coomassie blue.

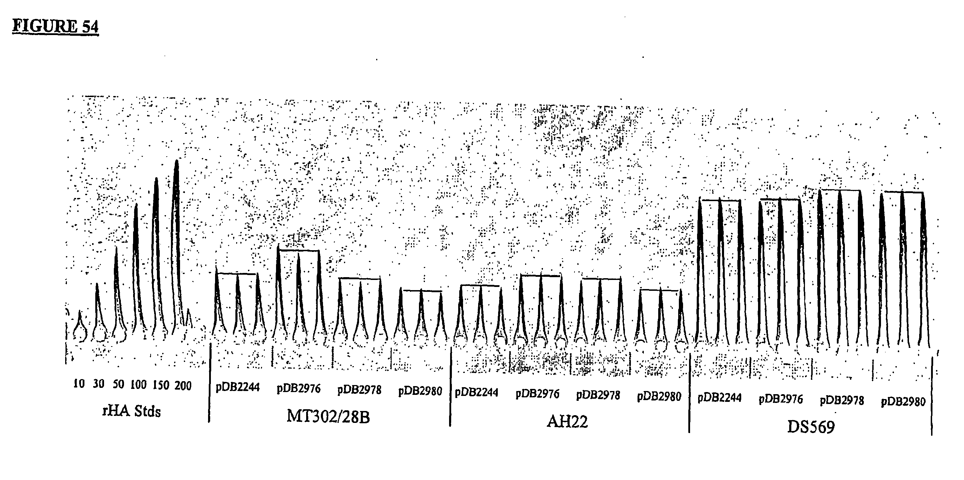

**Figure 54** shows RIE analysis of rHA expression in different *S. cerevisiae* strains when co-expressed with PDI1 genes having different length promoters. 10mL YEPD shake flasks were inoculated with yeast and incubated for 4-days at 30˚C. 4μL culture supernatant loaded per well of a rocket immunoelectrophoresis gel. rHA standards concentrations are in μg/mL. 400μL goat anti-HA (Sigma product A-1151 resuspended in 5mL water) /50mL agarose. Precipin was stained with Coomassie blue.

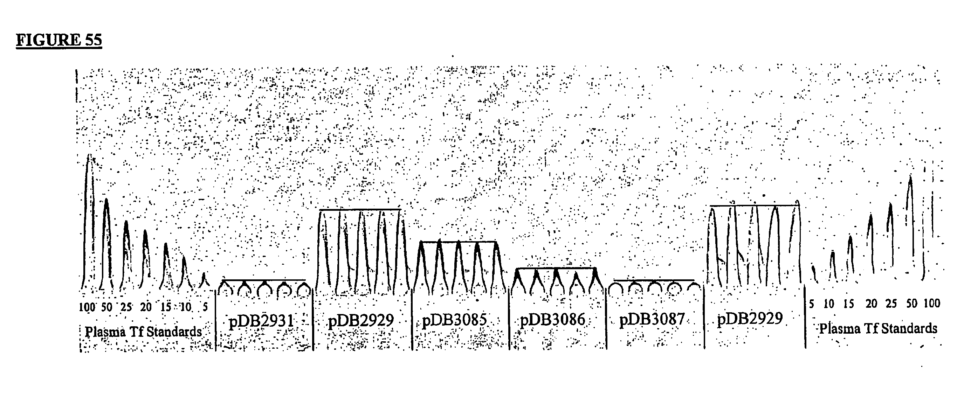

**Figure 55** shows RIE analysis of rTF expression, when co-expressed with different PDI1 constructs. 10mL BMMD shake flasks were inoculated with yeast and incubated for 4-days at 30˚C. 5μL culture supernatant was loaded per well of a rocket immunoelectrophoresis gel containing 25μL goat anti-Tf / 50mL. Plasma Tf standards concentrations are in μg/mL. Precipin was stained with Coomassie blue.

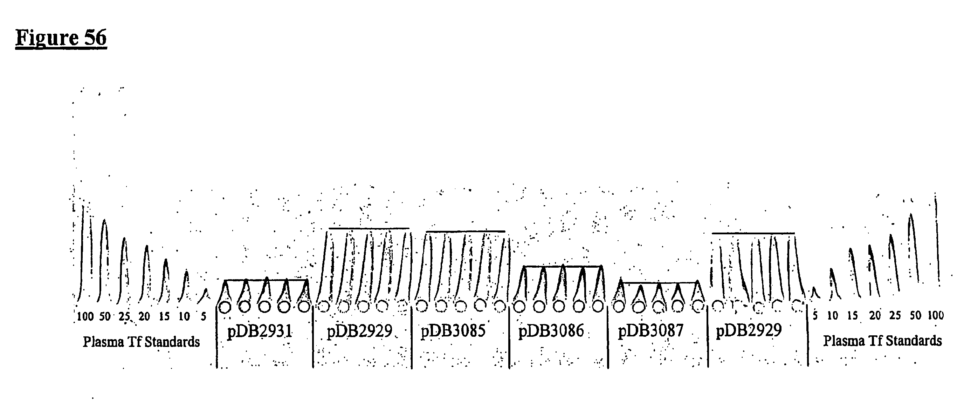

**Figure 56** shows RIE analysis of rTF expression, when co-expressed with different PDI1 constructs. 10mL YEPD shake flasks were inoculated with yeast and incubated for 4-days at 30˚C. 5μL culture supernatant was loaded per well of a rocket immunoelectrophoresis gel containing 25μL goat anti-Tf / 50mL. Plasma Tf standards concentrations are in μg/mL. Precipin was stained with Coomassie blue.

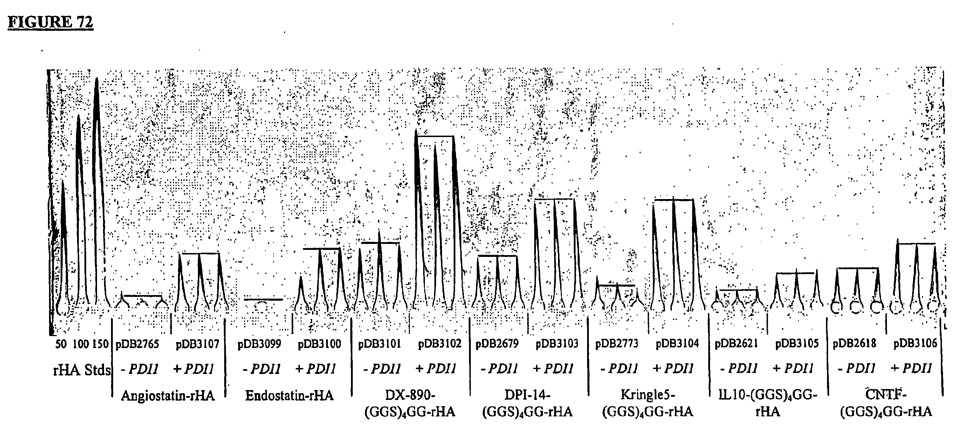

**Figure 72** shows RIE analysis of rHA fusion proteins with and without co-expressed recombinant PDI1. 10mL BMMD shake flasks were inoculated with YBX7 transformed with albumin fusion expression plasmids and incubated for 4-days at 30˚C. 4μL culture supernatant loaded per well of a rocket immunoelectrophoresis gel. rHA standards concentrations are in μg/mL. 200μL goat anti-HA (Sigma product A-1151 resuspended in 5mL water) /50mL agarose. Precipin was stained with Coomassie blue.

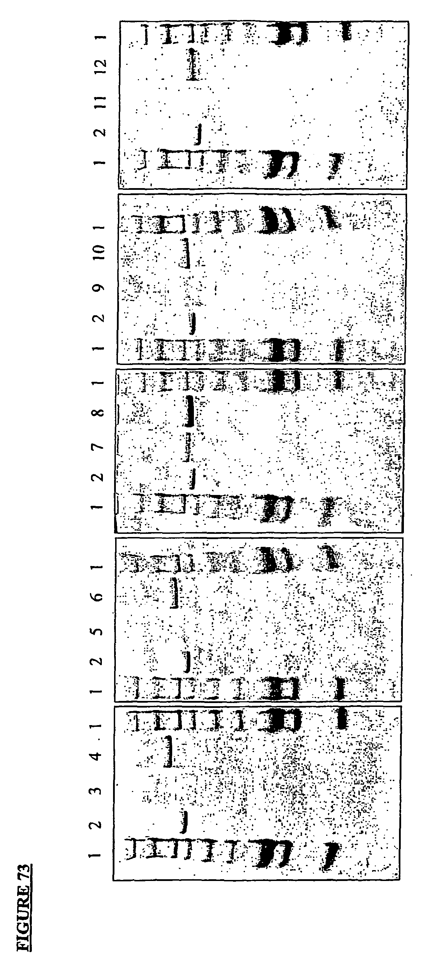

**Figure 73** shows SDS-PAGE analysis of recombinant albumin fusion secretion with and without *PDI1* present on the expression plasmid. 10mL BMMD shake flasks were inoculated with yeast and incubated for 4-days at 30˚C, 200rpm. 30μL supernatant analysed on non-reducing SDS-PAGE (4-12% NuPAGE®, MES buffer, InVitrogen) with GelCode® Blue reagent (Pierce). 1 = SeeBlue Plus2 Markers (InVitrogen); 2 = 1μg rHA; 3 = angiostatin-rHA; 4 = angiostatin-rHA + *PDI1*; 5 = endostatin-rHA; 6 = endostatin-rHA + *PDI1*; 7 = DX-890-(GGS)$_4$GG-rHA; 8 = DX-890-(GGS)$_4$GG-rHA + *PDI1*; 9 = DPI-14-(GGS)$_4$GG-rHA; 10.= DPI-14-(GGS)$_4$GG-rHA+ *PDI1;* 11 = Axokine™ (CNTF$_{Ax15}$)-(GGS)$_4$GG-rHA (Lambert et al, 2001, Proc. Natl. Acad. Sci. USA, 98, 4652-4657); 12 = Axokine™ (CNTF$_{Ax15}$) -(GGS)$_4$GG-rHA + *PDI1.*

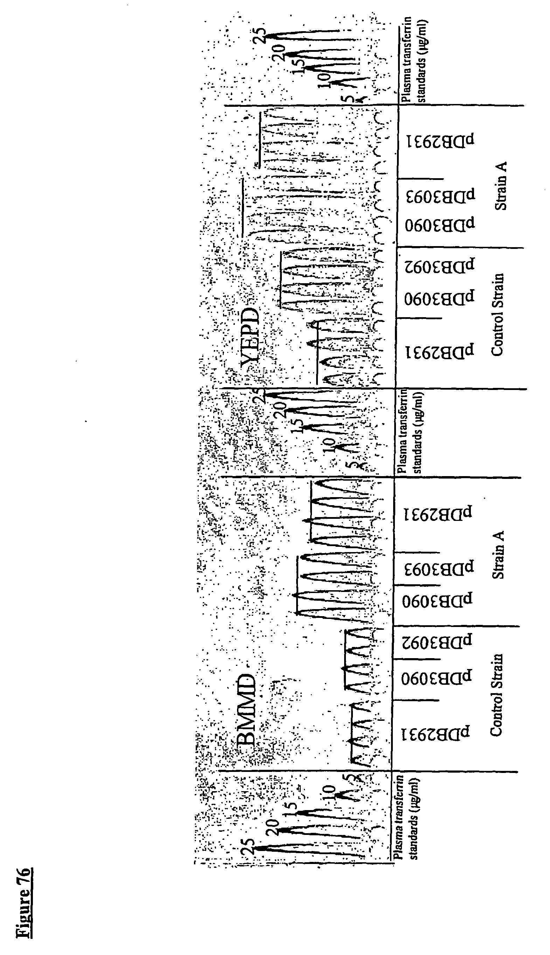

**Figure 76** shows RIE analysis demonstrating increased transferrin secretion from *S. cerevisiae* with *ORM2* co-expression from a 2μm-based plasmid. Four day shake flask culture supernatants were loaded at 5μl per well. Standards were human plasma holo-transferrin (Calbiochem), at 25, 20, 15, 10, 5 μg/ml, loaded 5μl per well. Goat polyclonal anti-transferrin (human) antiserum (Calbiochem) used at 20 μl per rocket immunoelectrophoresis gel (50 ml).

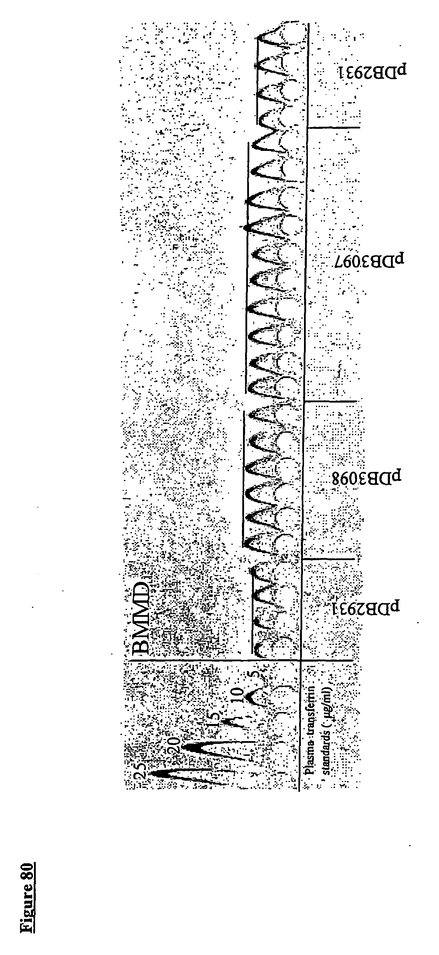

**Figure 80** shows RIE analysis demonstrating increased transferrin secretion from *S. cerevisiae* with *PSE1* co-expression from a 2μm-based plasmid. Four day shake flask culture supernatants were loaded at 5μl per well. Standards were human plasma holo-transferrin (Calbiochem), at 25, 20, 15, 10, 5 μg/ml, loaded 5μl per well. Goat polyclonal anti-transferrin (human) antiserum (Calbiochem) used at 20μl per rocket immunoelectrophoresis gel (50 ml).

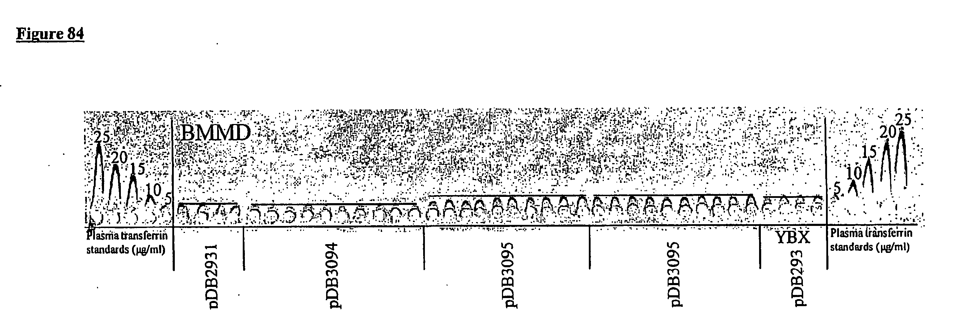

**Figure 84** shows RIE analysis demonstrating increased transferrin secretion from *S. cerevisiae* with *SSA1* co-expression from a 2μm-based plasmid. Four day shake flask culture supernatants were loaded at 5μl per well. Standards were human plasma holo-transferrin (Calbiochem), at 25, 20, 15, 10, 5 μg/ml, loaded 5μl per well. Goat polyclonal anti-transferrin (human) antiserum (Calbiochem) used at 20μl per rocket immunoelectrophoresis gel (50 ml).

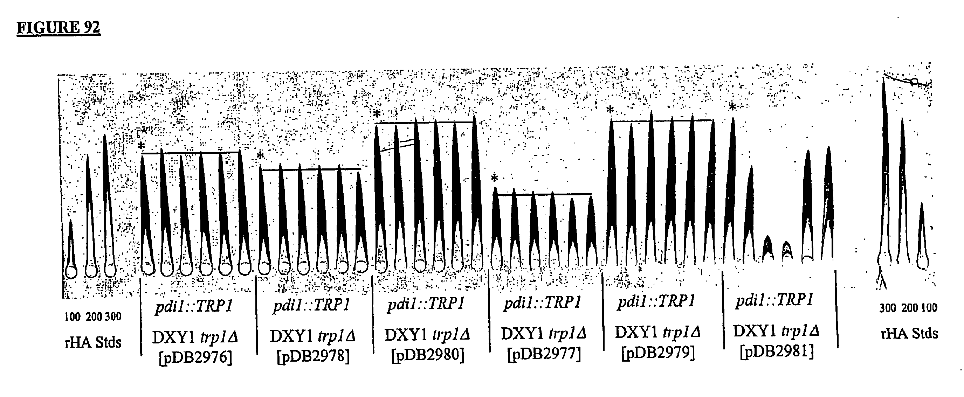

**Figure 92** shows the results of RIE. 10mL YEPD shake flasks were inoculated with DXY1 *trp1*Δ [pDB2976], DXY1 *trp1*Δ [pDB2977], DXY1 *trp1*Δ [pDB2978], DXY1 *trp1*Δ [pDB2979], DXY1 *trp1*Δ [pDB2980] or DXY1 *trp1*Δ [pDB2981] transformed to tryptophan prototrophy with a 1.41kb *Not*I/*Pst*I *pdi1::TRP1* disrupting DNA fragment was isolated from pDB3078. Transformants were grown for 4-days at 30˚C, 200rpm. 4μL culture supernatant loaded per well of a rocket immunoelectrophoresis gel. rHA standards concentrations are in μg/mL. 700μL goat anti-HA (Sigma product A-1151 resuspended in 52mL water) /50mL agarose. Precipin was stained with Coomassie blue. Isolates selected for further analysis are indicated (*).

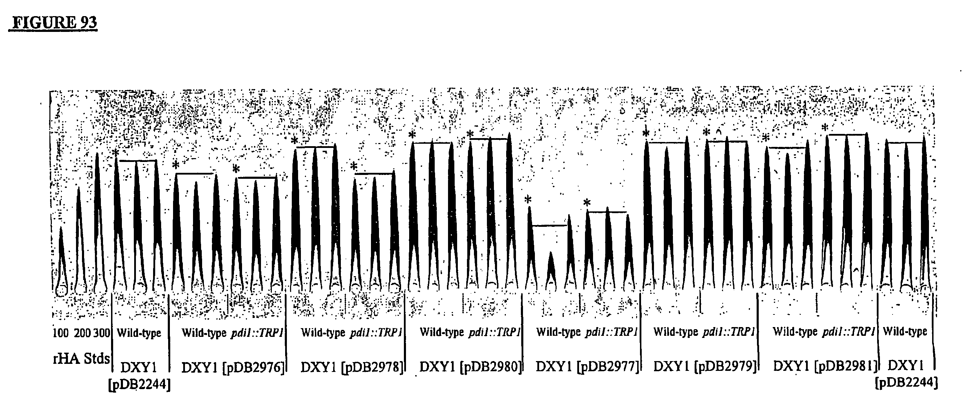

**Figure 93** shows the results of RIE. 10mL YEPD shake flasks were inoculated with DXY1 [pDB2244], DXY1 [pDB2976], DXY1 *trp1*Δ *pdi1::TRP1* [pDB2976], DXY1 [pDB2978], DXY1 *trp1*Δ *pdi1::TRP1* [pDB2978], DXY1 [pDB2980], DXY1 *trp1*Δ *pdi1::TPP1* [pDB2980], DXY1 [pDB2977], DXY1 *trp1*Δ *pdi1::TRP1* [pDB2977], DXY1 [pDB2979] DXY1 *trp1Δ pdi1::TRP1* [pDB2979], DXY1 [pDB2981] and DXY1 *trp1Δ pdi1::TRP1* [pDB2981], and were grown for 4-days at 30°C, 200rpm. 4μL culture supernatant loaded per well of a rocket immunoelectrophoresis gel. rHA standards concentrations are in μg/mL. 800μL goat anti-HA (Sigma product-A-1151 resuspended in 5mL water) /50mL agarose. Precipin was stained with Coomassie blue. Isolates selected for further analysis are indicated (*)

**Figure 94** shows a sequence alignment of the SKQ2n and S288c gene sequences with long promoters, as described in Example 6.

## EXAMPLES

Two types of expression cassette have been used to exemplify secretion of a recombinant human transferrin mutant (N413Q, N611Q) from S. *cerevisiae.* One type uses a modified HSA(pre)/MFα1(pro) leader sequence (named the "modified fusion leader" sequence). The second type of expression cassette uses only the modified HSA(pre) leader sequence.

The 24 amino acid sequence of the "modified fusion leader" is MKWVFIVSILFLFSSAYSRSLDKR.

The 18 amino acid sequence of the modified HSA(pre) leader sequence is MKWVFIVSILFLFSSAYS.

Transferrin (N413Q, N611Q) expression using these two cassettes has been studied in *S. cerevisiae* using the 2μm expression vector with and without an additional copy of the S. *cerevisiae* PDI gene, *PD11.*

## EXAMPLE 1

### *Construction of expression plasmids*

A 52-bp linker made by annealing 0.5mM solutions of oligonucleotides CF86 and CF87 (see below) was introduced into the US-region of the 2μm plasmid pSAC35 at the *Xcm*I-sites in the 599-bp inverted repeats. One *Xcm*I-site cuts 51-bp after the *REP2* translation termination codon, whereas the other *Xcm*I-site cuts 127-bp before the end of the *FLP* coding sequence, due to overlap with the inverted repeat (see Figure 3). This DNA linker contained a core region *"Sna*BI-*Pac*I-*Fse*I/*Sfi*I-*Sma*I-*Sna*BI*",* which encoded restriction sites absent from pSAC35.

## *Xcm*I Linker (CF86+CF87)

```
                                              SfiI

                                       ---------------
                   PacI                                      SnaBI

                  ----------                               -------
       SnaBI                    FseI            SmaI

      -------                  --------        ------

CF86  GGAGTGGTA CGTATTAATT AAGGCCGGCC AGGCCCGGGT ACGTACCAAT TGA
CF87  TCCTCACCAT GCATAATTAA TTCCGGCCGG TCCGGGCCCA TGCATGGTTA AC
```

Plasmid pSAC35 was partially digested *with Xcm*I, the linear 11-kb fragment was isolated from a 0.7%(w/v) agarose gel, ligated with the CF86/CF87 *Xcm*I linker (neat, $10^{-1}$ and $10^{-2}$ dilutions) and transformed into *E. coli* DH5α. Ampicillin resistant transformants were selected and screened for the presence of plasmids that could be linearised by *Sma*I digestion. Restriction enzyme analysis identified pDB2688 (Figure 4) with the linker cloned into the *Xcm*I-site after *REP2.* DNA sequencing using oligonucleotides primers CF88, CF98 and CF99 (Table 1) confirmed the insertion contained the correct linker sequence.

**Table 1**

| Oligonucleotide sequencing primers: | | |
|---|---|---|
| **Primer** | **Description** | **Sequence** |
| CF88 | *REP2* primer, 20mer | 5'-ATCACGTAATACTTCTAGGG-3' |

(continued)

| Oligonucleotide sequencing primers: | | |
|---|---|---|
| **Primer** | **Description** | **Sequence** |
| CF98 | *REP2* primer, 20mer | 5'-AGAGTGAGTTGGAAGGAAGG-3' |
| CF99 | *REP2* primer, 20mer | 5'-AGCTCGTAAGCGTCGTTACC-3' |

The yeast strain was transformed to leucine prototrophy using a modified lithium acetate method (Sigma yeast transformation kit, YEAST-1, protocol 2; (Ito et al, 1983, J. Bacteriol., 153, 163; Elble, 1992, Biotechniques, 13, 18)). Transformants were selected on BMMD-agar plates, and were subsequently patched out on BMMD-agar plates. Cryopreserved trehalose stocks were prepared from 10mL BMMD shake flask cultures (24 hrs, 30˚C, 200rpm), by addition of an equal volume of sterile 40% (w/v) trehalose

The composition of YEPD and BMMD is described by Sleep et al., 2002, Yeast, 18, 403. YEPS and BMMS are similar in composition to YEPD and BMMD accept that 2% (w/v) sucrose was substituted for the 2% (w/v) glucose as the sole initial carbon source.

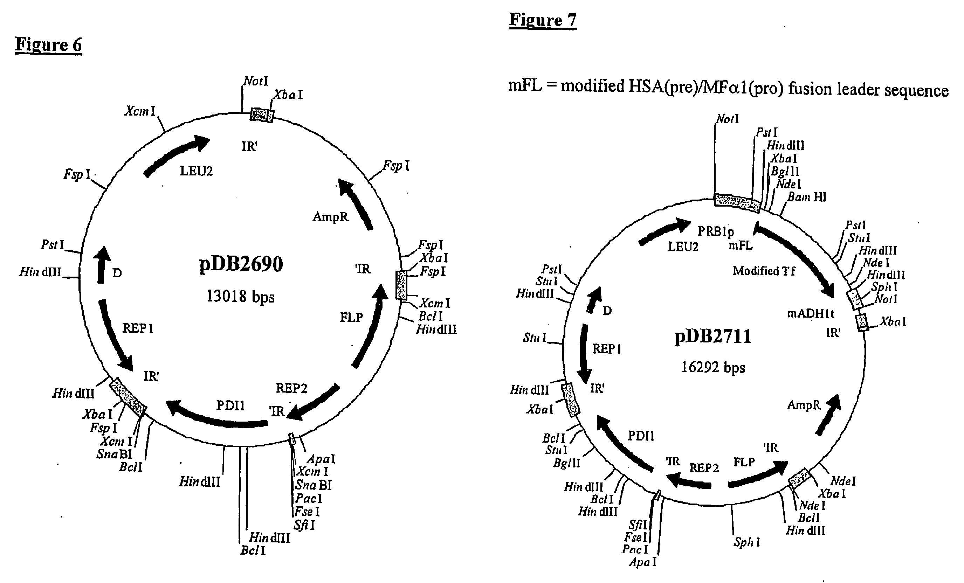

The *S. cerevisiae PDI1* gene was cloned into the XcmI-linker of pDB26988. The *PDI1* gene (Figure 5) was cloned on a 1.9-kb *Sac*I-*Spe*I fragment from a larger *S. cerevisiae* genomic SKQ2n DNA fragment containing the *PDI1* gene (as provided in the plasmid pMA3a:C7 that is described in US 6,291,205 and also described as Clone C7 in Crouzet & Tuite, 1987, Mol. Gen. Genet., 210, 581-583 and Farquhar *et al,* 1991, *supra),* which had been cloned into YIplac211 (Crietz & Sugino, 1988, Gene, 74, 527-534), and had a synthetic DNA linker containing a SacI restriction site inserted at a unique Bsu36I-site in the 3' untranslated region of the *PDI1* gene. The 1.9-kb *Sac*I-*Spe*I fragment was treated with T4 DNA polymerase to fill the *Spe*I 5'-overhang and remove the *Sac*I 3'-overhang. This *PDI1* fragment included 212-bp of the *PDI1* promoter upstream of the translation initiation codon, and 148-bp downstream of the translation termination codon. This was ligated with *Sma*I linearised/calf intestinal alkaline phosphatase treated pDB2688, to create plasmid pDB2690 (Figure 6), with the *PDI1* gene transcribed in the same direction as *REP2.* A *S. cerevisiae* strain was transformed to leucine prototrophy with pDB2690.

An expression cassette for a human transferrin mutant (N413Q, N611Q) was subsequently cloned into the NotI-site of pDB2690 to create pDB2711 (Figure 7). The expression cassette in pDB2711 contains the *S. cerevisiae PRB1* promoter, an HSA/MFα fusion leader sequence (EP 387319; Sleep et al, 1990, Biotechnology (N.Y.), 8, 42) followed by a coding sequence for the human transferrin mutant (N413Q, N611Q) and the *S. cerevisiae ADH1* terminator. Plasmid pDB2536 was constructed similarly by insertion of the same expression cassette into the *Not***I**-site of pSAC35.

The "modified fusion leader" sequence used in pDB2536 and pDB2711 comprises a modified HSA-pre sequence and a MFα1-pro sequence. An alternative leader sequence used was the modified HSA-pre sequence, which was derived from the modified fusion leader sequence by removal of the six residues of the MFα1-pro sequence.

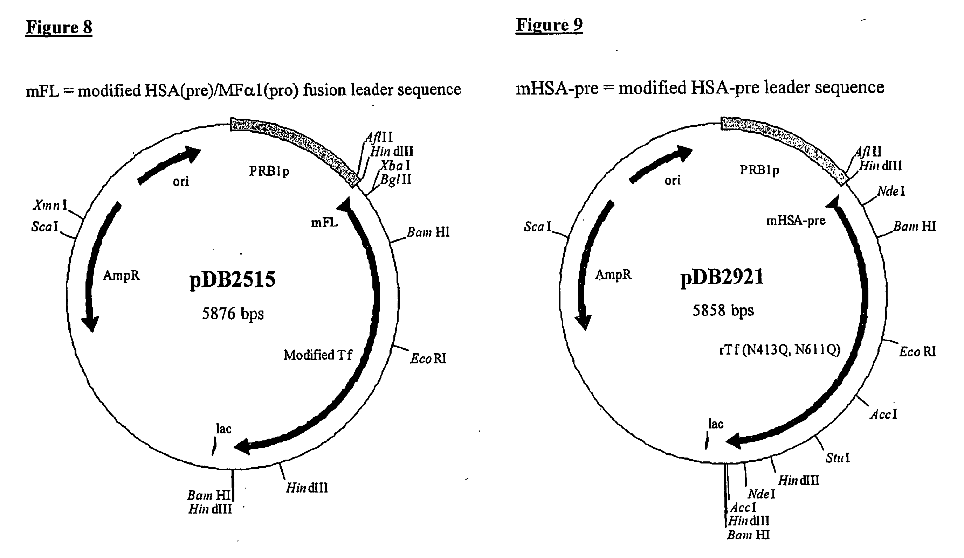

The modified fusion leader sequence in pub2515 (Figure 8) was mutated with oligonucleotides CF154 and CF155 to delete the coding sequence for the six residues (RSLDKR) of the MFα1-pro region. This was performed according to the instruction manual of the Statagene's QuickChange™ Site-Directed Mutagenesis Kit. pDB2515 is the *E. coli* cloning vector pGEM-7Z(-) (Promega) containing the 2940-bp *Not*I-*Hind*III (partial) DNA fragment of pDB2529 (see below) ligated between the *Psp*OMI and *Hind*III sites.

**CF154**

5'-GTTCTTGTTCTCCTCTGCTTACTCTGTCCCTGATAAAACTGTGAGATGG-3'

**CF155**

5'-CCATCTCACAGTTTTATCAGGGACAGAGTAAGCAGAGGAGAACAAGAAC-3'

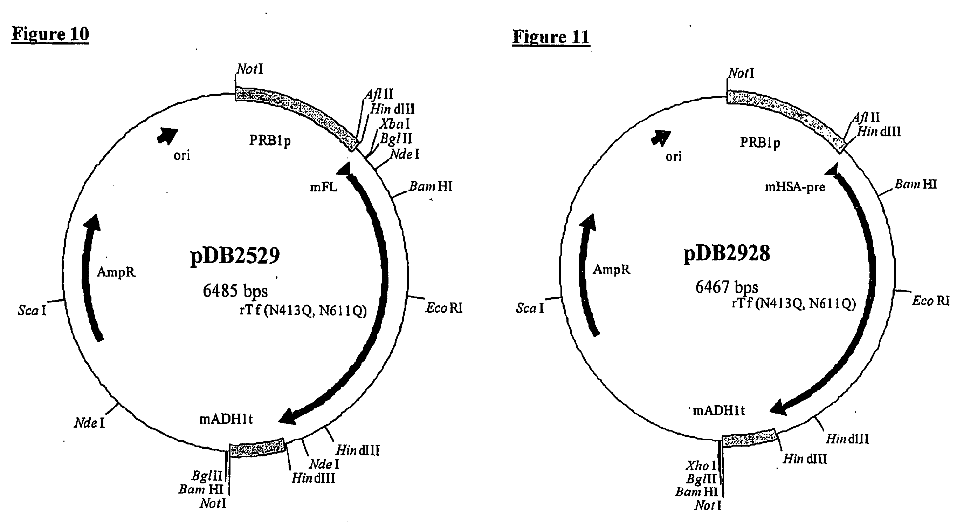

Competent *E. coli* DH5α cells were transformed with the mutated plasmids and ampicillin resistant colonies were selected. Plasmid DNA from these colonies was screened by double digestion with *Eco*RI and *Bgl*II. The correct DNA sequence for the modified HSA-pre leader was subsequently confirmed in pDB2921 (Figure 9) over a 386-bp region between the *Afl*II and *Bam*HI sites either side of the leader sequence. This 386-bp *Afl*II-*Bam*HI fragment was isolated, and ligated with a 6,081-bp *Afl*II-*Bam*HI fragment from pDB2529 (Figure 10), prepared by partial digestion with *Bam*HI and complete digestion with *Afl*II and calf intestinal alkaline phosphatase. pDB2529 is the *E. coli* cloning vector pBST(+) (Sleep et al, 2001, Yeast, 18, 403-441) containing the transferrin expression cassette of pDB2536 cloned into the unique NotI-site. This produced pDB2928 (Figure 11), which was isolated from ampicillin resistant *E. coli* DH5α cells transformed with the ligation products.

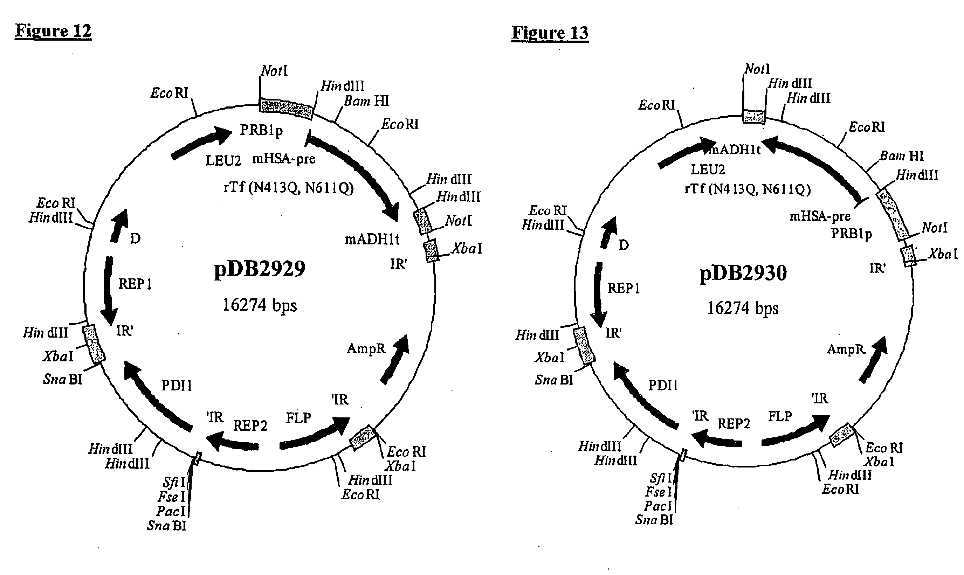

The 3,256-bp *Not*I expression cassette was isolated from pDB2928. This contained the *PRB1* promoter, the coding region for the modified HSA-pre leader sequence followed by transferrin (N413Q, N611Q), and the *ADH1* terminator. This was ligated into the *Not*I sites of the 2μm-based vectors pSAC35 and pDB2690 to generate the expression plasmids pDB2929, pDB2930, pDB2931 and pDB2932 (Figures 12-15). In pDB2929 and pDB2931 the transferrin (N413Q, N611Q) sequence is transcribed in the same direction as *LEU2,* whereas in pDB2930 and pDB2932 transcription is in the opposite direction.

## EXAMPLE 2

***Expression of transferrin.***

A *S. cerevisiae* control strain was transformed to leucine prototrophy with all the transferrin (N413Q, N611Q) expression plasmids, and cryopreserved stocks were prepared.

Strains were grown for four days at 30°C in 10mL BMMD cultures in 50mL conical flasks shaken at 200rpm. The titres of recombinant transferrin secreted into the culture supernatants were compared by rocket immunoelectrophoresis (RIE as described in Weeke, B., 1976, "Rocket immunoelectrophoresis" In N. H. Azelsen, J. Kroll, and B. Weeke [eds.], A manual of quantitative immunoelectrophoresis. Methods and applications. Universitetsforlaget, Oslo, Norway), reverse phase high performance liquid chromatography (RP-HPLC) (Table 2), and non-reducing SDS polyacrylamide electrophoresis stained with colloidal Coomassie blue stain (SDS-PAGE). The increase in recombinant transferrin secreted when *S. cerevisiae PDI1* was over-expressed was estimated to be greater than 10-fold.

**Table 2:**

| Plasmid | Secretory Leader | Additional *PDI1* | Average Transferrin Titre ($\mu$g.mL$^{-1}$) (n=2) | Estimated Increase due to Additional *PDI1* |
|---|---|---|---|---|
| pSAC35 | None | No | 0.4 | - |
| pDB2536 | Fusion Leader | No | 6.2 | - |
| pDB2711 | Fusion Leader | Yes | 112.8 | **18-fold** |
| pDB2931 | Modified HSA-pre Leader | No | 5.1 | - |
| pDB2929 | Modified HSA-pre Leader | Yes | 76.1 | **15-fold** |

RIE analysis indicated that the increased transferrin secretion in the presence of additional copies of *PDI1* was approximately 15-fold (Figure 16). By RIE analysis the increase appeared slightly larger for the modified HSA-pre leader sequence than for the modified fusion leader sequence (Figure 17).

By RP-HPLC analysis the increase in transferrin secretion was determined to be 18-fold for the modified fusion leader sequence and 15-fold for the modified HSA-pre leader sequence (Table 2).

Figure 18 shows an SDS-PAGE comparison of the recombinant transferrin secreted by S *cerevisiae* strains with and without additional *PDI1* expression.

## RP-HPLC Method for Determining Transferrin Expression

Column: 50 x 4.6mm Phenomenex Jupiter C4 300Å, 5$\mu$m
Column temperature: 45°C
Flow rate: 1mL.min$^{-1}$
Peak detection:UV absorbance at 214nm
HPLC mobile phase A:0.1% TFA, 5% Acetonitrile
HPLC mobile phase B:0.1% TFA, 95% Acetonitrile
Gradient: 0 to 3 minutes 30% B
3 to 13 minutes 30 to 55% B in a linear gradient
13 to 14 minutes 55% B
14 to 15 minutes 55 to 30% B in a linear gradient
15 to 20 minutes 30% B
Injection: Generally 100$\mu$L of sample, but any volume can be injected
Standard Curve: 0.1 to 10$\mu$g of human transferrin injected vs peak area Standard curve used for the results shown was linear up to 10$\mu$g.

$$y = 530888.x + 10526.7$$

where y = peak area, and x = amount in $\mu$g.

($r^2$): 0.999953, where Correlation Coefficient = r

## EXAMPLE 3

### *Chromosomal over-expression of PDI*

*S. cerevisiae* Strain A was selected to investigate the secretion of recombinant glycosylated transferrin expression from plasmid pDB2506 and recombinant non-glycosylated transferrin (N413Q, N611 Q) from plasmid pDB2536. Strain A has the following characteristics -

• additional chromosomally integrated *PDI1* gene integrated at the host *PDI1* chromosomal location.

• the *URA3* gene and bacterial DNA sequences containing the ampicillin resistance gene were also integrated into the S. *cerevisiae* genome at the insertion sites for the above genes.

A control strain had none of the above insertions.

Control strain [cir⁰] and Strain A [cir⁰] were transformed to leucine prototrophy with pDB2506 (recombinant transferrin), pDB2536 (recombinant non-glycosylated transferrin (N413Q, N611Q)) or pSAC35 (control). Transformants were selected on BMMD-agar.

The relative level of transferrin secretion in BMMD shake flask culture was determined for each strain/plasmid combination by rocket immunoelectrophoresis (RIE). Figure 19 shows that both strains secreted both the glycosylated and non-glycosylated recombinant transferrins into the culture supernatant.

The levels of both the glycosylated and non-glycosylated transferrins secreted from Strain A [pDB2506] and Stram A [pDB2536] respectively, appeared higher than the levels secreted from the control strain. Hence, at least in shake flask culture, *PDI1* integrated into the host genome at the *PDI1* locus in Strain A has enhanced transferrin secretion.

Furthermore, the increase in transferrin secretion observed between control strain [pDB2536] and Strain A [pDB2536] appeared to be at least a 100% increase by RIE. In contrast, the increase in rHA monomer secretion between control strain [pDB2305] and Strain A [pDB2305] was approximately 20% (data not shown). Therefore, the increase in transferrin secretion due to the additional copy of *PDI1* in Strain A was surprising large considering that transferrin has 19 disulphide bonds, compared to rHA with 17 disulphide bonds. Additional copies of the *PDI1* gene may be particularly beneficial for the secretion from *S. cerevisiae* of proteins from the transferrin family, and their derivatives.

The levels of transferrin secreted from Strain A [pDB2536] and Strain A [pDB2506] were compared by RIE for transformants grown in BMMD and YEPD (Figure 20). Results indicated that a greater than 2-fold increase in titres of both non-glycosylated recombinant transferrin (N413Q, N611Q) and glycosylated recombinant transferrin was achieved by growth in YEPD (10-20 mg.L⁻¹ serum transferrin equivalent) compared to BMMD (2-5 mg.L⁻¹ serum transferrin equivalent). The increase in both glycosylated and non-glycosylated transferrin titre observed in YEPD suggested that both transferrin expression plasmids were sufficiently stable under non-selective growth conditions to allow the expected increased biomass which usually results from growth in YEPD to be translated into increased glycosylated and non-glycosylated transferrin productivity.

SDS-PAGE analysis of non-glycosylated transferrin (N413Q, N611Q) secreted from Strain A [pDB2536] and glycosylated transferrin from Strain A [pDB2506] grown in BM1VVID shake flask culture is shown in Figure 21. Strain A [pDB2536] samples clearly showed an additional protein band compared to the Strain A [pSAC35] control. This extra band migrated at the expected position for the recombinant transferrin (N413Q, N611Q) secreted from control strain [pDB2536]. Strain A [pDB2506] culture supernatants appeared to contain a diffuse protein band at the position expected for transferrin. This suggested that the secreted recombinant transferrin was heterogeneous, possibly due to hyper-mannosylation at Asp413 and/or Asp611.

## EXAMPLE 4

### *Comparing transferrin secretion from S. cerevisiae control strain containing pDB2711 with transferrin secretion from S. cerevisiae Strain A*

Plasmid pDB2711 is as described above. Plasmid pDB2712 (Figure 22) was also produced with the *Not*I cassette in the opposite direction to pDB2711.

Control strain *S. cerevisiae* [cir⁰] was transformed to leucine prototrophy with pDB2711 and pDB2712. Transformants were selected on BMMD-agar and cryopreserved trehalose stocks of control strain [pDB2711] were prepared.

Secretion of recombinant transferrin (N413Q, N611Q) by control strain [pDB2711], control strain [pDB2712], Strain A [pDB2536], control strain [pDB2536] and an alternative control strain [pDB2536] was compared in both BMMD and YEPD shake flask culture. RIE indicated that a significant increase in recombinant transferrin secretion had been achieved from control strain [pDB2711] with multiple episomal *PDI1* copies, compared to Strain A [pDB2536] with two chromosomal copies of *PDI1,* and control strain [pDB2536] with a single chromosomal copy *of PDI1* gene (Figure 23). Control strain [pDB2711] and control strain [pDB2712] appeared to secrete similar levels of rTf (N413Q, N611Q) into the culture media. The levels of secretion were relatively consistent between control strain [pDB2711] and control strain [pub2712] transformants in both BMMD and YEPD media, suggesting that plasmid stability was sufficient for high-level transferrin secretion even under non-selective conditions. This is in contrast to the previous published data in relation to recombinant PDGF-BB and HSA where introduction of PDI1 into multicopy $2\mu$m plasmids was shown to be detrimental to the host.

**Table 3:** Recombinant transferrin titres from high cell density fermentations

| Strain | Supernatant ($g.L^{-1}$) | |
|---|---|---|
| | **GP-HPLC** | **SDS-PAGE** |
| Control [pDB2536] | 0.5/0.4 | - |
| Alternative control [pDB2536] | 1.5/1.6 | 0.6 |
| | 0.9/0.9 | 0.4/0-4/0.5 |
| Strain A [pDB2536] | 0.7 | 0.6 |
| | 0.6 | - |
| Control [pDB2711] | 3.5 | 3.6 |
| | 3.4 | 2.7/3.1 |

Reducing SDS-PAGE analysis of transferrin secreted from control strain [pDB2711], control strain [pDB2712], Strain A [pDB2536], control strain [pDB2536] and alternative control strain [pDB2536] in BMMD shake flask culture is shown in Figure 24. This shows an abundant protein band in all samples from control strain [pDB2711] and control strain [pDB2712] at the position expected for transferrin (N413Q, N611Q). The relative stain intensity of the transferrin (N413Q, N611Q) band from the different strains suggested that Strain A [pDB2536] produced more than control strain [pDB2536] and alternative control strain [pDB2536], but that there was an even more dramatic increase in secretion from control strain [pDB2711] and control strain [pDB2712]. The increased recombinant transferrin secretion observed was concomitant with the increased *PDI1* copy number in these strains. This suggested that Pdilp levels were limiting transferrin secretion in control strain, Strain A and the alternative control strain, and that elevated *PDI1* copy number was responsible for increased transferrin secretion. Elevated *PDI1* copy number could increase the steady state expression level of *PDI1* so increasing the amount of Pdi1p activity. There are a number of alternative methods by which this could be achieved without increasing the copy number of the *PDI1* gene, for example the steady state *PDI1* mRNA level could be increased by either increasing the transcription rate, say by use of a higher efficiency promoter, or by reducing the clearance rate of the *PDI1* mRNA. Alternatively, protein engineering could be used to enhance the specific activity or turnover number of the Pdi1p protein.

In high cell density fermentations control strain [pDB2711] recombinant transferrin (N413Q, N611Q) production was measured at approximately $3g.L^{-1}$ by both GP-HPLC analysis and SDS-PAGE analysis (Table 3). This level of production is several fold-higher than control strain, the alternative control strain or Strain A containing pDB2536. Furthermore, for the production of proteins for therapeutic use in humans, expression systems such as control strain [pDB2711] have advantages over those using Strain A, as they do not contain bacterial DNA sequences.

## CONCLUSIONS

Secretion of recombinant transferrin from a multicopy expression plasmid (pDB2536) was investigated in *S. cerevisiae* strains containing an additional copy of the *PDI1* gene integrated into the yeast genome. Transferrin secretion was also investigated in *S. cerevisiae* transformed with a multicopy expression plasmid, in which the *PDI1* gene has been inserted into the multicopy episomal transferrin expression plasmid (pDB2711).

A *S. cerevisiae* strain with an additional copy of the *PDI1* gene integrated into the genome at the endogenous *PDI1* locus, secreted recombinant transferrin and non-glycosylated recombinant transferrin (N413Q, N611Q) at an elevated level compared to strains containing a single copy of *PDI1.* A further increase in *PDI1* copy number was achieved by using pDB2711 In high cell density fermentation of the strain transformed with pDB2711, recombinant transferrin (N413Q, N611Q) was secreted at approximately 3g.L$^{-1}$, as measured by SDS-PAGE and GP-HPLC analysis. Therefore, increased *PDI1* gene copy number has produced a large increase in the quantity of recombinant transferrins secreted from S. *cerevisiae.*

The following conclusions are drawn -

1. In shake flask analysis of recombinant transferrin expression from pDB2536 (non-glycosylated transferrin (N413Q, N611Q) and pDB2506 (glycosylated transferrin) the *S. cerevisiae* strain Strain A secreted higher levels of both recombinant transferrins into the culture supernatant than control strains. This was attributed to the extra copy of *PDI1* integrated at the *PDI1* locus*.*

2. Control strain [pDB2711], which contained the *PDI1* gene on the multicopy expression plasmid, produced a several-fold increase in recombinant transferrin (N413Q, N611Q) secretion compared to Strain A [pDB2536] in both shake flask culture and high cell density fermentation.

3. Elevated *PDI1* copy numbers in yeast such as *S. cerevisiae* will be advantageous during the production of heterologous proteins, such as those from the transferrin family.

4. pSAC35-based plasmids containing additional copies of *PDI1* gene have advantages for the production of proteins from the transferrin family, and their derivatives, such as fusions, mutants, domains and truncated forms.

## EXAMPLE 5

### Insertion of a PDI1 gene into a 2μm-like plasmid increased secretion of recombinant transferrin from various different S cerevisiae strains

The S. *cerevisiae* strain JRY188 cir$^+$ (National Collection of Yeast Cultures) and MT302/28B cir$^+$ (Finnis et al., 1993, Eur. J. Biochem., 212, 201-210) was cured of the native 2μm plasmid by galactose induced over-expression of *FLP* from *Yep351-GAL-FLPI,* as described by Rose and Broach (1990, Meth. Enzymol., 185, 234-279) to create the *S. cerevisiae* strains JRY188 cir$^0$ and MT302/28B cir$^0$, respectively.

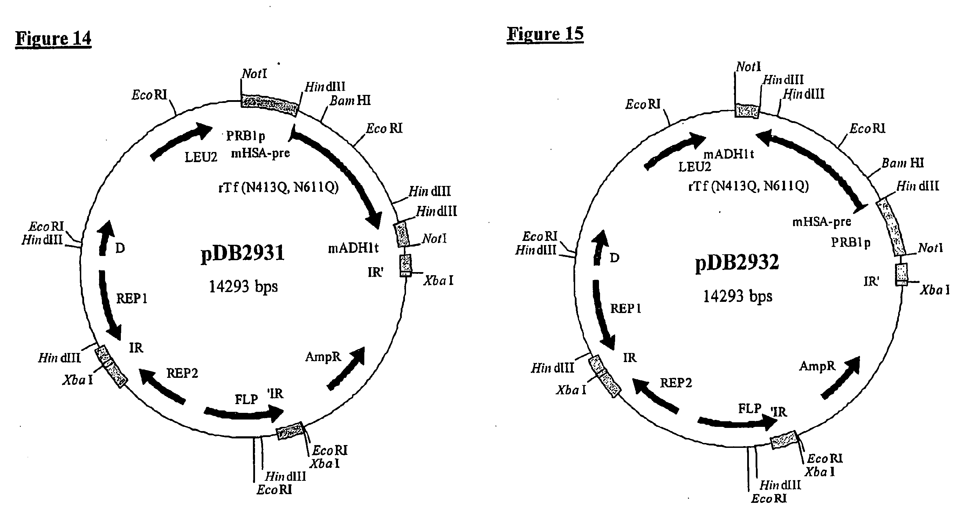

The *S. cerevisiae* strains JRY188 cir$^0$, MT302/28B cir$^0$, S150-2B cir$^0$ (Cashmore et al., 1986, Mol. Gen. Genet., 203, 154-162), CB11-63 cir$^0$ (Zealey et al., 1988, Mol. Gen. Genet., 211, 155-159) were all transformed to leucine prototrophy with pDB2931 (Figure 14) and pDB2929 (Figure 12). Transformants were selected on appropriately supplemented minimal media lacking leucine. Transformants of each strain were inoculated into 10mL YEPD in 502mL shake flasks and incubated in an orbital shaker at 30˚C, 200rpm for 4-days. Culture supernatants were harvested and the recombinant transferrin titres compared by rocket immunoelectrophoresis (Figure 26). The results indicated that the transferrin titres in supernatants from all the yeast strains were higher when *PDI1* was present in the 2μm plasmid (pDB2929) than when it was not (pDB2931)

## EXAMPLE 6

### The construction of expression vectors containing various PDII genes and the expression cassettes for various heterologous proteins on the same 2μm-like plasmid

### PCR amplification and cloning of *PDI1* genes into YIplac211:

The *PDI1* genes from S. *cerevisiae* S288c and S. *cerevisiae* SKQ2n were amplified by PCR to produce DNA fragments with different lengths of the 5'-untranslated region containing the promoter sequence. PCR, primers were designed to permit cloning of the PCR products into the *Eco*RI and *Bam*HI sites of YIplac211 (Gietz & Sugino, 1988, Gene, 74, 527-534). Additional restriction endonuclease sites were also incorporated into PCR primers to facilitate subsequent cloning. Table 4 describes the plasmids constructed and Table 5 gives the PCR primer sequences used to amplify the *PDI1* genes. Differences in the *PDI1* promoter length within these YIplac211-based plasmids are described in Table 4.

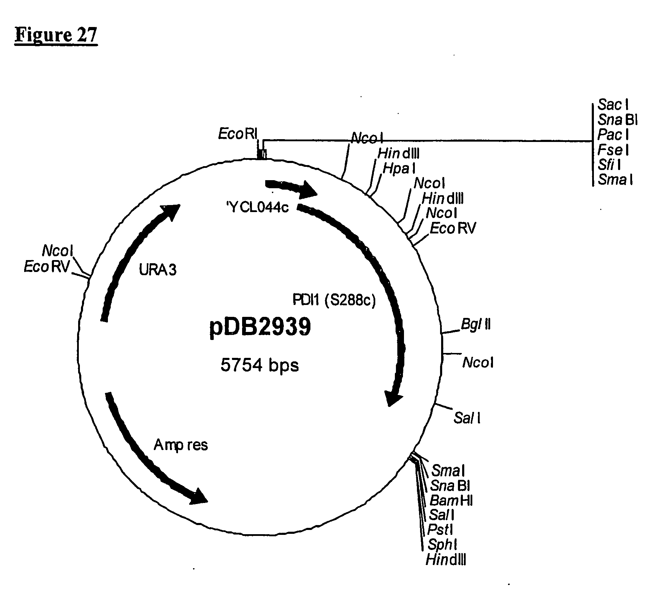

pDB2939 (Figure 27) was produced by PCR amplification of the *PDI1* gene from S. *cerevisiae* S288c genomic DNA with oligonucleotide primers DS248 and DS250 (Table 5), followed by digesting the PCR product with *Eco*RI and *Bam*HI and cloning the approximately 1.98-kb fragment into YIplac211 (Gietz & Sugino, 1988, Gene, 74, 527-534), that had been cut with *Eco*RI and *Bam*HI. DNA sequencing of pDB2939 identified a missing 'G' from within the DS248 sequence, which is marked in bold in Table 5. Oligonucleotide primers used for sequencing the *PDI1* gene are listed in Table 6, and were designed from the published S288c *PDI1* gene sequence (PDI1/YCL043C on chromosome III from coordinates 50221 to 48653 plus 1000 basepairs of upstream sequence and 1000 basepairs of downstream sequence. (http://www.yeastgenome.org/ Genebank Accession number NC001135).

**Table 4:** YIplac211-based Plasmids Containing PDI1 Genes

| Plasmid | Plasmid Base | PDI1 *Gene* | | | PCR Primers |
|---------|--------------|--------|----------|------------|-------------|
| | | *Source* | *Promoter* | *Terminator* | |
| **pDB2939** | **YIplac211** | **S288c** | **Long (~210-bp)** | **→ Bsu36I** | **DS248 +DS250** |
| **pDB2941** | **YIplac211** | **S288c** | **Medium (~140-bp)** | **→ Bsu36I** | **DS251 + DS250** |
| **pDB2942** | **Hplac211** | **S288c** | **Short (~80-bp)** | **→ Bsu36I** | **DS252 + DS250** |
| **pDB2943** | **YIplac211** | **SKQ2n** | **Long (~210-bp)** | **→ Bsu36I** | **DS248 + DS250** |
| **pDB2963** | **YIplac211** | **SKQ2n** | **Medium (~140-bp)** | **→ Bsu36I** | **DS267+DS250** |
| **pDB2945** | **YIplac211** | **SKQ2n** | **Short (~80-bp)** | **→ Bsu36I** | **DS252 + DS250** |

**Table 5**: Oligonucleotide Primers for PCR Amplification of *S. cerevisiae PDI1* Genes

| Primer | Sequence |
|--------|----------|
| DS248 | 5'-GTCAGAATTCGAGCTCTACGTATTAATTAAGGCCGGCCAGGCCCGGGCTAGT CTCTTTTTCCAATTTGCCACCGTGTAGCATTTTGTTGT-3' |
| DS249 | 5'-GTCAGGATCCTACGTACCCGGGGGATATCATTATCATCTTTGTCGTGGTCATCT TGTGTG-3' |
| DS250 | 5'-GTCAGGATCCTACGTACCCGGGTAAGGCGTTCGTGCAGTGTGACGAATAT AGCG-3' |
| DS251 | 5'-GTCAGAATTCGAGCTCTACGTATTAATTAAGGCCGGCCAGGCCCGGGCCCGT ATGGACATACATATATATATATATATATATATATATTTTGTTACGCG-3' |
| DS252 | 5'-GTCAGAATTCGAGCTCTACGTATTAATTAAGGCCGGCCAGGCCCGGGCTTGTTG CAAGCAGCATGTCTAATTGGTAATTTTAAAGCTGCC-3' |
| DS267 | 5'-GTCAGAATTCGAGCTCTACGTATTAATTAAGGCCGGCCAGGCCCGGGCCCGTA TGGACATACATATATATATATATATATATATATATTTTGTTACGCG-3' |

**Table 6:** Oligonucleotide Primers for DNA Sequencing *S cerevisiae PDI1* Genes

| Primer | Sequence |
|--------|----------|
| DS253 | 5'-CCTCCCTGCTGCTCGCC-3' |
| DS254 | 5'-CTGTAAGAACATGGCTCC-3' |
| DS255 | 5'-CTCGATCGATTACGAGGG-3' |

(continued)

| Primer | Sequence |
|--------|----------|
| DS256 | 5'-AAGAAAGCCGATATCGC-3' |
| DS257 | 5'-CAACTCTCTGAAGAGGCG-3' |
| DS258 | 5'-CAACGCCACATCCGACG-3' |
| DS259 | 5'-GTAATTCTGATCACTTTGG-3' |
| D260 | 5'-GCACTTATTATTACTACGTGG-3' |
| DS261 | 5'-GTTTTCCTTGATGAAGTCG-3' |
| DS262 | 5'-GTGACCACACCATGGGGC-3' |
| DS263 | 5'-GTTGCCGGCGTGTCTGCC-3' |
| DS264 | 5'-TTGAAATCATCGTCTGCG-3' |
| DS265 | 5'-CGGCAGTTCTAGGTCCC-3' |
| DS266 | 5'-CCACAGCCTCTTGTTGGG-3' |
| M13/pUC Primer (-40) | 5' -GTTTTCCCAGTCACGAC-3' |

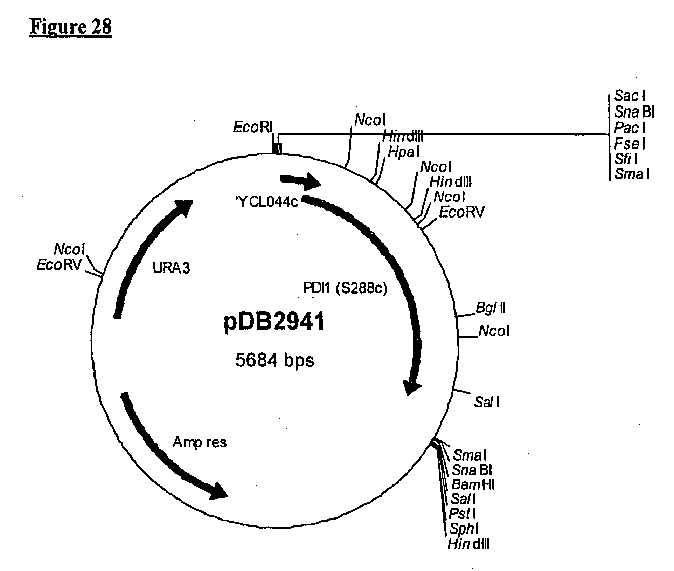

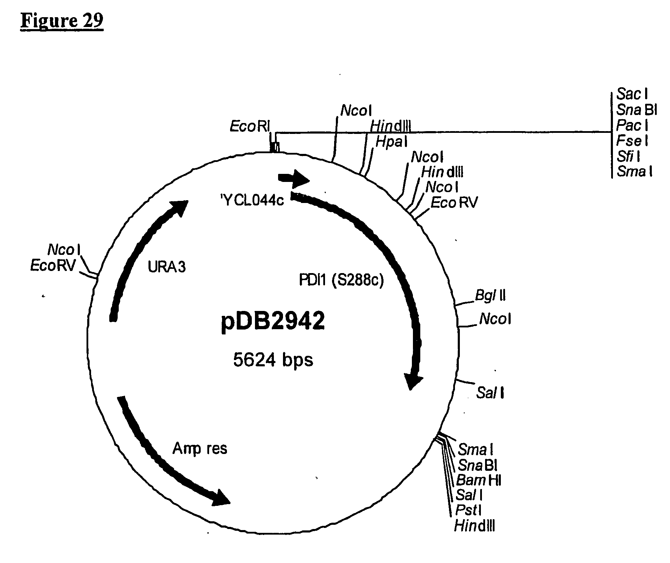

Plasmids pDB2941 (Figure 28) and pDB2942 (Figure 29) were constructed similarly using the PCR primers described in Tables 4 and 5, and by cloning the approximately 1.90-kb and 1.85-kb *Eco*RI-*Bam*HI fragments, respectively, into YIplac211. The correct DNA sequences were confirmed for the *PDI1* genes in pDB2941 and pDB2942.

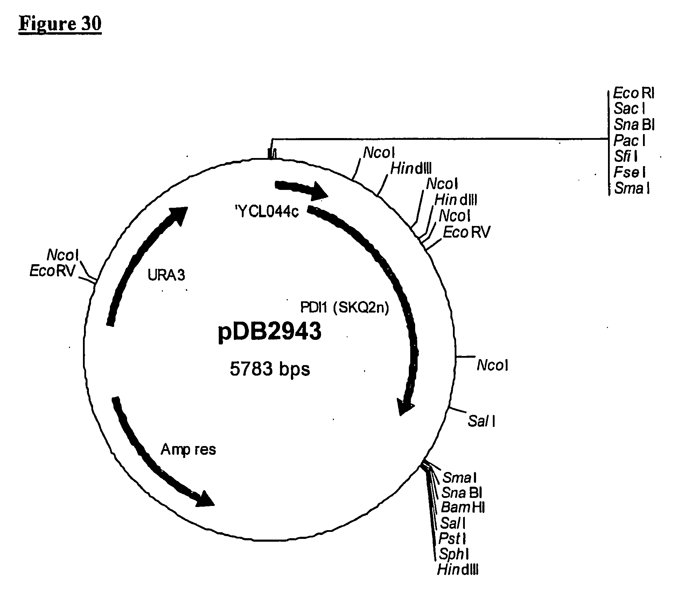

The *S. cerevisiae* SKQ2n *PDI1* gene sequence was PCR amplified from plasmid DNA containing the *PDI1* gene from pMA3a:C7 (US 6,291,205), also known as Clone C7 (Crouzet & Tuite, 1987, *supra;* Farquhar *et al,* 1991, *supra*). The SKQ2n *PDII* gene was amplified using oligonucleotide primers DS248 and DS250 (Tables 4 and 5). The approximately 2.01-kb PCR product was digested with *Eco*RI and *Bam*HI and ligated into YIplac211 (Gietz & Sugino, 1988, Gene, 74, 527-534) that has been cut with *Eco*RI and *Bam*HI, to produce plasmid pDB2943 (Figure 30). The 5' end of the SKQ2n PDI1 sequence is analogous to a blunt-ended *Spe*I-site extended to include the *Eco*RI, *Sac*I, *Sna*BI, *Pac*I, *Fse*I, *Sfi*I and *Sma*I sites, the 3' end extends up to a site analogous to a blunt-ended *Bsu*36I site, extended to include a *Sma*I, *Sna*BI and *Bam*HI sites. The *PDI1* promoter length is approximately 210bp. The entire DNA sequence was determined for the *PDII* fragment using oligonucleotide primers given in Table 6. This confirmed the presence of a coding sequence for the PDI protein of *S. cerevisiae* strain SKQ2n (NCBI accession number CAA38402), but with a serine residue at position 114 (not an arginine residue as previously published). Similarly, in the same way as in the *S. cerevisiae* S288c sequence in pDB2939, pDB2943 also had a missing 'G' from within the DS248 sequence, which is marked in bold in Table 5.

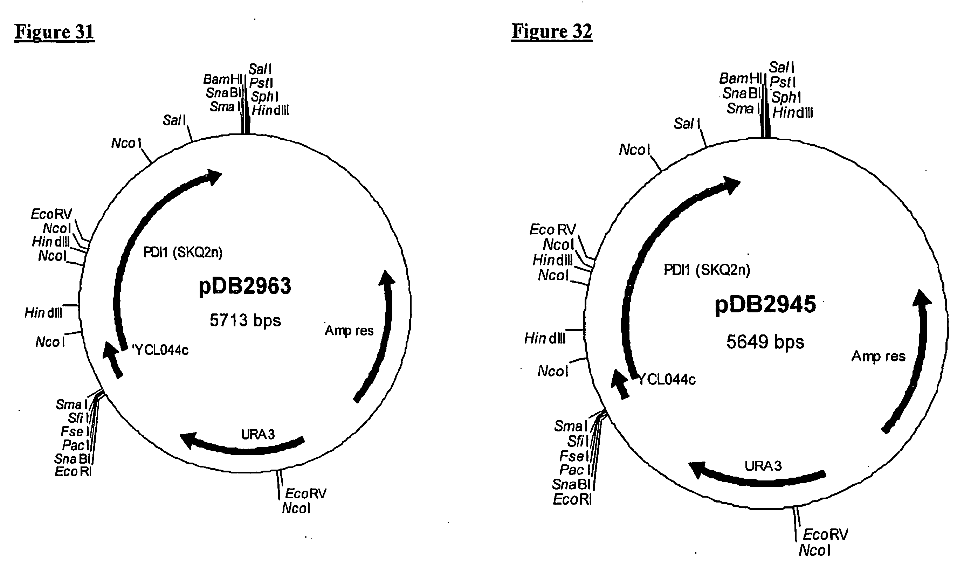

Plasmids pDB2963 (Figure 31) and pDB2945 (Figure 32) were constructed similarly using the PCR primers described in Tables 4 and 5, and by cloning the approximately 1.94-kb and 1.87-kb *Eco*RI-*Bam*HI fragments, respectively, into YIplac211. The expected DNA sequences were confirmed for the *PDII* genes in pDB2963 and pDB2945, with a serine codon at the position of amino acid 114.

**The construction of pSAC35-based rHA expression plasmids with different *PDI1* genes inserted at the *XcmI*-site after *REP2*:**

pSAC35-based plasmids were constructed for the co-expression of rHA with different *PDII* genes (Table 7).

**Table 7:** pSAC35-based plasmids for co-expression of rHA with different *PDI1* genes

| Plasmid | Plasmid Base | PDI1 *Gene at* XcmI-*site after* REP2 | | | | Heterologous Protein Expression Cassette (at NotI-site) |
|---------|--------------|--------|----------|------------|-------------|---------|
| | | Source | Promoter | Terminator | Orientation | |
| pDB2982 | pSAC35 | SKQ2n | Long | → Bsu36I | A | rHA |
| pDB2983 | pSAC35 | SKQ2n | Long | → Bsn36I | B | rHA |
| pDB2984 | pSAC35 | SKQ2n | Medium | → Bsu36I | A | rHA |
| pDB2985 | pSAC35 | SKQ2n | Medium | →Bsu36I | B | rHA |

(continued)

| Plasmid | Plasmid Base | PDI1 Gene at XcmI-site after REP2 | | | | Heterologous Protein Expression Cassette (at NotI-site) |
|---|---|---|---|---|---|---|
| | | Source | Promoter | Terminator | Orientation | |
| pDB2986 | pSAC35 | SEQ2n | Short | →Bsu36I | A | rHA |
| pDB2987 | pSAC35 | SKQ2n | Short | → Bsu36I | B | rHA |
| pDB2976 | pSAC35 | S288c | Long | → Bsu36I | A | rHA |
| pDB2977 | pSAC35 | S288c | Long | →Bsu36I | B | rHA |
| pDB2978 | pSAC35 | S288c | Medium | → Bsu36I | A | rHA |
| pDB2979 | pSAC35 | S288c | Medium | → Bsu36I | B | rHA |
| pDB2980 | pSAC35 | S288c | Short | → Bsu36I | A | rHA |
| pDB2981 | pSAC35 | S288c | Short | → Bsu36I | B | rHA |

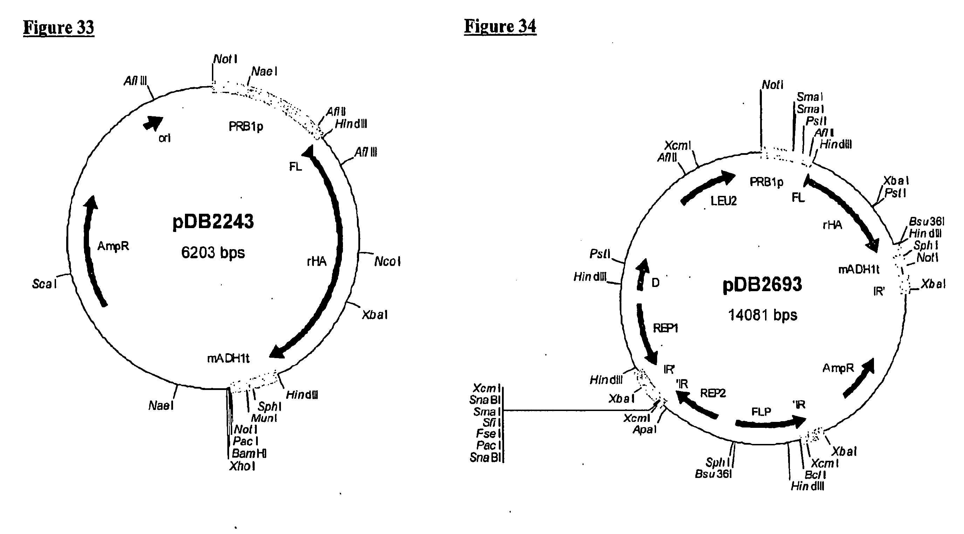

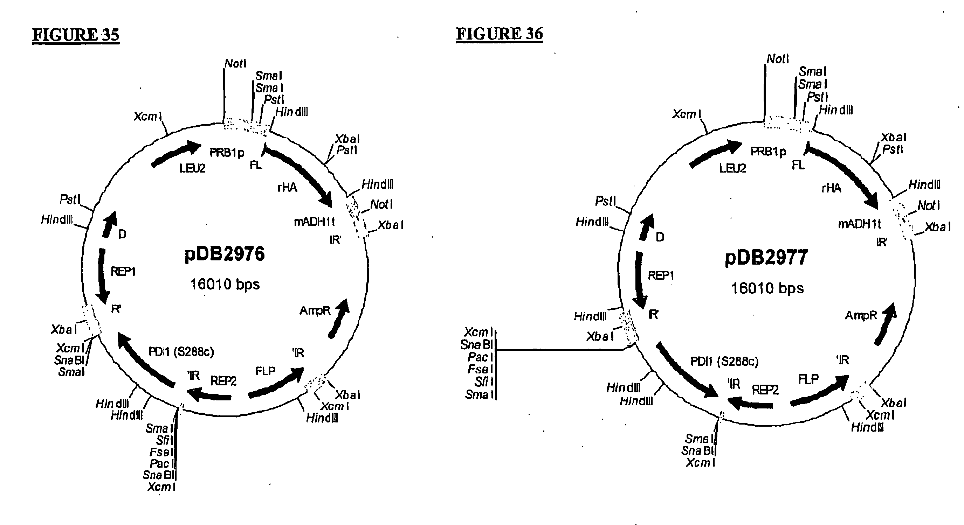

The rHA expression cassette from pDB2243 (Figure 33, as described in WO 00/44772) was first isolated on a 2,992-bp *Not*I fragment, which subsequently was cloned into the *Not*I-site of pDB2688 (Figure 4) to produce pDB2693 (Figure 34). pDB2693 was digested with *Sna*BI, treated with calf intestinal alkaline phosphatase, and ligated with SnaBI fragments containing the *PDII* genes from pDB2943, pDB2963, pDB2945, pDB2939, pDB2941 and pDB2942. This produced plasmids pDB2976 to pDB2987 (Figures 35 to 46). *PDI1* transcribed in the same orientation as *REP2* was designated "orientation A", whereas *PDI1* transcribed in opposite orientation to *REP2* was designated "orientation B" (Table 7).

***The construction of pSAC35-based transferrin expression plasmids with different PDI1 genes inserted at the XcmI-site after REP2:***

pSAC35-based plasmids were constructed for the co-expression of recombinant transferrin (N413Q, N611Q) with different *PDI1* genes (Table 8).

**Table 8:** pSAC35-based plasmids for co-expression of transferrin with different PDI1 genes

| Plasmid | Plasmid Base | PDI1 Gene at XcmI-site after REP2 | | | | Heterologous Protein Expression Cassette (at NotI-site) |
|---|---|---|---|---|---|---|
| | | Source | Promoter | Terminator | Orientation | |
| pDB2929 | pSAC35 | SKQ2n | Long | → Bsu36I | A | rTf (N413Q, N611Q) |
| pDB3085 | pSAC35 | S288c | Long | → Bsu36I | A | rTf (N413Q N611Q) |
| pDB3086 | pSAC35 | S288c | Medium | → Bsu36I | A | rTf (N413Q N611Q) |
| pDB3087 | pSAC35 | S288c | Short | → Bsu36I | A | rTf(N413Q, N611Q) |

In order to achieve this, the NotI expression cassettes for rHA expression were first deleted from pDB2976, pDB2978, and pDB2980 by NotI digestion and circularisation of the vector backbone. This produced plasmids pDB3081 (Figure 47), pDB3083 (Figure 48) and pDB3084 (Figure 49) as described in Table 9.

**Table 9:** pSAC35-based plasmids with different *PDII* genes

| Plasmid | Plasmid Base | PDI1 Gene at XcmI-site after REP2 | | | | Heterologous Protein Expression Cassette (at NotI-site) |
|---|---|---|---|---|---|---|
| | | Source | Promoter | Terminator | Orientation | |
| pDB2690 | pSAC35 | SKQ2n | Long | →Bsu36I | A | None |
| pDB3081 | pSAC35 | S288c | Long | →Bsu36I | A | None |
| pDB3083 | pSAC35 | S288c | Medium | → Bsu36I | A | None |
| pDB3084 | pSAC35 | S288c | Short | → Bsu36I | A | None |

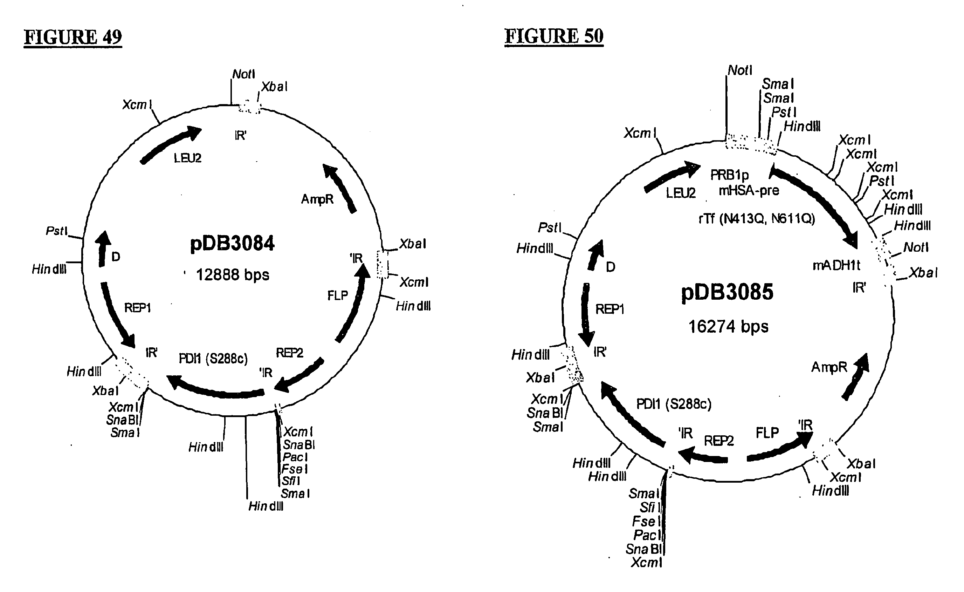

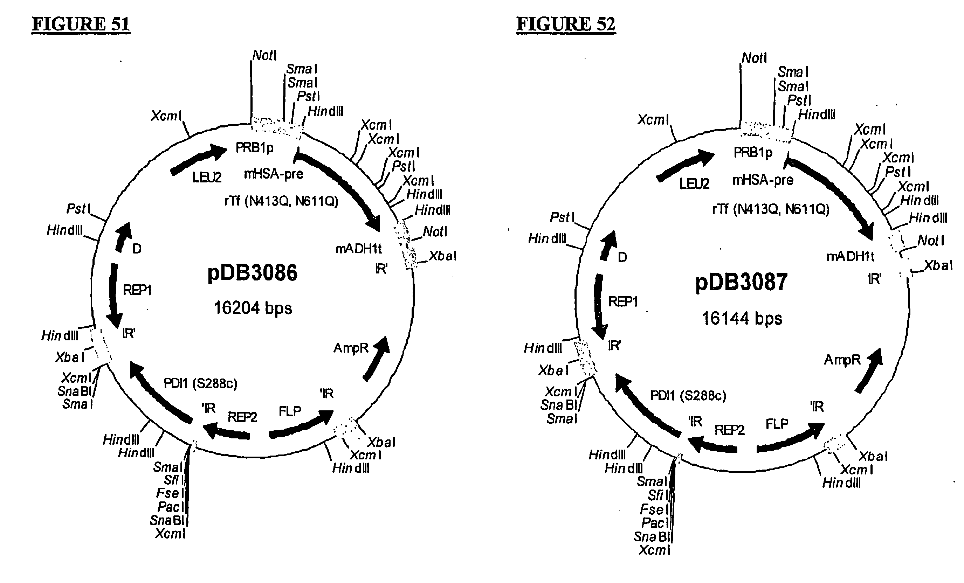

The 3,256-bp NotI fragment from pDB2928 (Figure 11) was cloned into the NotI-sites of pDB3081, pDB3083 and pDB3084, such that transcription from the transferrin gene was in the same direction as LEU2. This produced plasmids pDB3085 (Figure 50), pDB3086 (Figure 51) and pDB3087 (Figure 52) as described in Table 8.

## EXAMPLE 7

*Insertion and optimisation of a PDI1 gene in the 2μm-like plasmid increased the secretion of recombinant human serum albumin by various different S. cerevisiae strains*

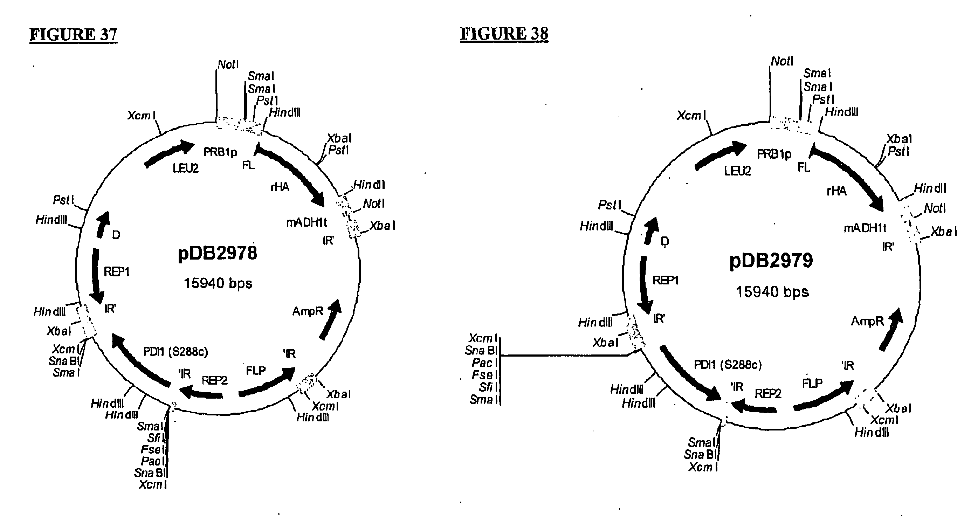

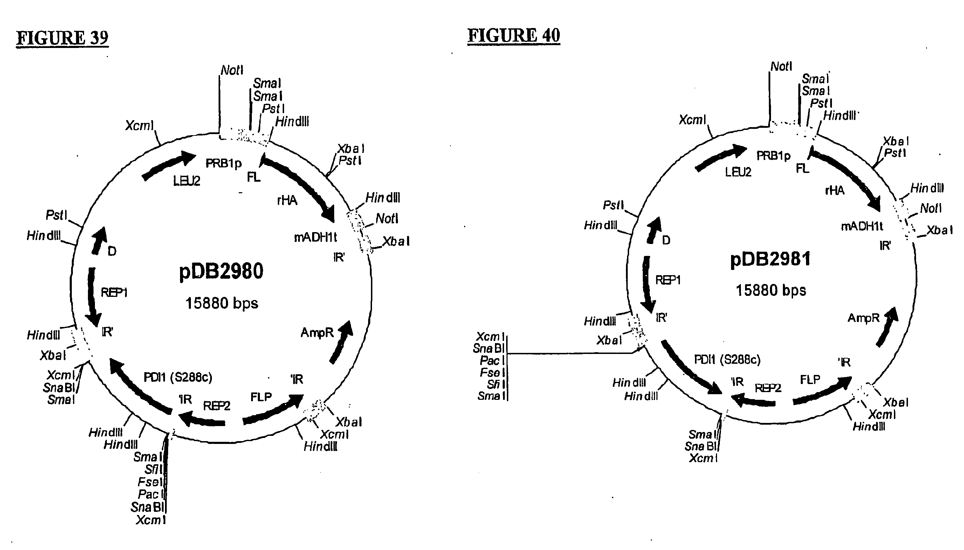

The *S. cerevisiae* strains JRY188 cir[0], MT302/28B cir[0], S150-2B cir[0], CB11-63 cir[0] (all described above), AH22 cir[0] (Mead et al., 1986, Mol. Gen. Genet., 205, 417-421) and DS569 cir[0] (Sleep et al., 1991, Bio/Technology, 9, 183-187) were transformed to leucine prototrophy with either pDB2244 (WO 00/44772), pDB2976 (Figure 35), pDB2978 (Figure 37) or pDB2980 (Figure 39) using a modified lithium acetate method (Sigma yeast transformation kit, YEAST-1, protocol 2; (Ito et al, 1983, J. Bacteriol.,153, 163; Elble, 1992, Biotechniques, 13, 18)). Transformants were selected on BMMD-agar plates with appropriate supplements, and were subsequently patched out on BMMD-agar plates with appropriate supplements.

Transformants of each strain were inoculated into 10mL YEPD in 50mL shake flasks and incubated in an orbital shaker at 30°C, 200rpm for 4-days. Culture supernatants were harvested and the recombinant albumin titres compared by rocket immunoelectrophoresis (Figures 53 and 54). The results indicated that the albumin titres in the culture supernatants from all the yeast strains were higher when *PDI1* was present in the 2μm plasmid than when it was not (pDB2244). The albumin titre in the culture supernatants in the absence of *PDI1* on the plasmid was dependant upon which yeast strain was selected as the expression host, however, in most examples tested the largest increase in expression was observed when *PDII* with the long promoter (~210-bp) was present in the 2μm plasmid (pDB2976). Modifying the *PDI1* promoter by shortening, for example to delete regulation sites, had the affect of controlling the improvement. For one yeast strain, known to be a high rHA producing strain (DS569) a shorter promoter was preferred for optimal expression.

## EXAMPLE 8

*Different PDII genes enhanced the secretion of recombinant transferrin when co-expressed on a 2μm-based plasmid.*

The secretion of recombinant transferrin (N413Q, N611Q) was investigated with co-expression of the *S. cerevisiae* SKQ2n *PDI1* gene with the long promoter (~210-bp), and the *S. cerevisiae* S288c *PDI1* with the long, medium and short promoters (~210 bp, ~140 bp and ~80 bp respectively).

The same Control Strain as used in previous examples (e.g. Example 2) was transformed to leucine prototrophy with pDB2931 (negative control plasmid without *PDI1)* and pDB2929, pDB3085, pDB3086 and pDB3087 (Table 8). Transformants were selected on BMMD-agar plates and five colonies selected for analysis. Strains were grown in 10mL BMMD and 10mL YEPD shake flask cultures for 4-days at 30°C, 200rpm and culture supernatants harvested for analysis by rocket immunoelectrophoresis (RIE).

Figure 55 shows that in minimal media (BMMD) the *S. cerevisiae* SKQ2n *PDI1* gene with the long promoter gave the highest rTF (N413Q, N611Q) titres. The *S. cerevisiae* S288c *PDII* gene gave lower rTF (N413Q, N611Q) titres, which decreased further as the *PDII* promoter length was shortened.

Figure 56 shows that in rich media (YEPD) the *S. cerevisiae* SKQ2n *PDII* and S. *cerevisiae* S288c *PDII* genes with the long promoters gave similar rTF (N413Q, N611 Q) production levels. Also, the shorter the promoter length of the *S. cerevisiae* S288c *PDII* gene the lower was the rTF (N413Q, N611Q) production level.

## EXAMPLE 9

*PDI1 on the 2μm-based plasmid enhanced the secretion of recombinant albumin fusions.*

The affect of co-expression of the *S. cerevisiae* SKQ2n *PDII* gene with the long promoter (~210-bp) upon the expression of recombinant albumin fusions was investigated.

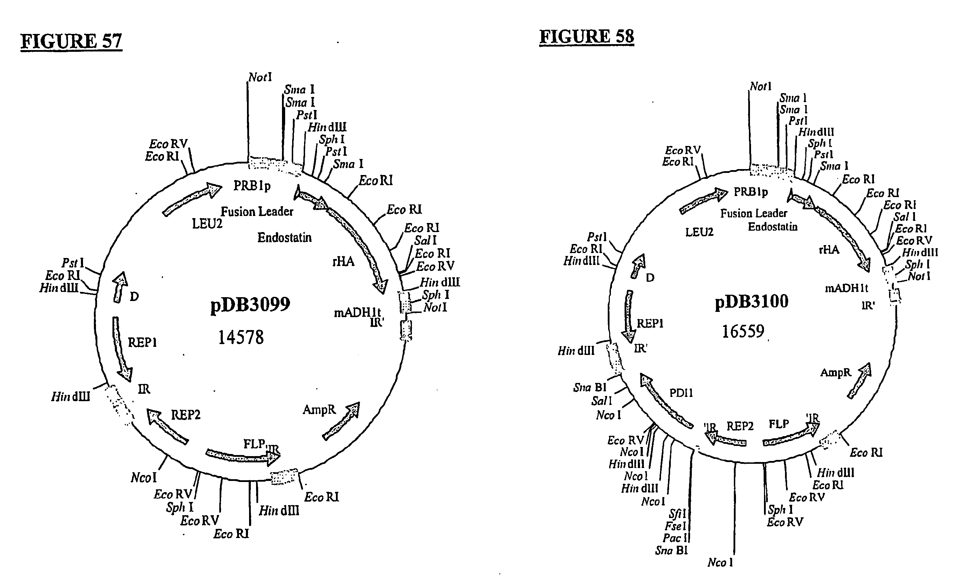

The construction of a *Not*I N-terminal endostatin-albumin expression cassette (pDB2556) has been previously described (WO 03/066085). Appropriate yeast vector sequences were provide by a "disintegration" plasmid pSAC35 generally disclosed in EP-A-286 424 and described by Sleep, D., et al., 1991, Bio/Technology, 9, 183-187. The 3.54kb *Not*I N-terminal endostatin-albumin expression cassette was isolated from pDB2556, purified and ligated into *Not*I digested pSAC35, which had been treated with calf intestinal phosphatase, creating plasmid pDB3099 containing the *Not*I expression cassette in the same orientation to the *LEU2* selection marker (Figure 57). An appropriate yeast *PDII* vector sequences were provide by a "disintegration" plasmid pDB2690 (Figure 6). The 3.54kb *Not*I N-terminal endostatin-albumin expression cassette was isolated from pDB2556, purified and ligated into *Not*I digested pDB2690, which had been treated with calf intestinal phosphatase, creating plasmid pDB3100 containing the *Not*I expression cassette in the same orientation to the, *LEU2* selection marker (Figure 58).

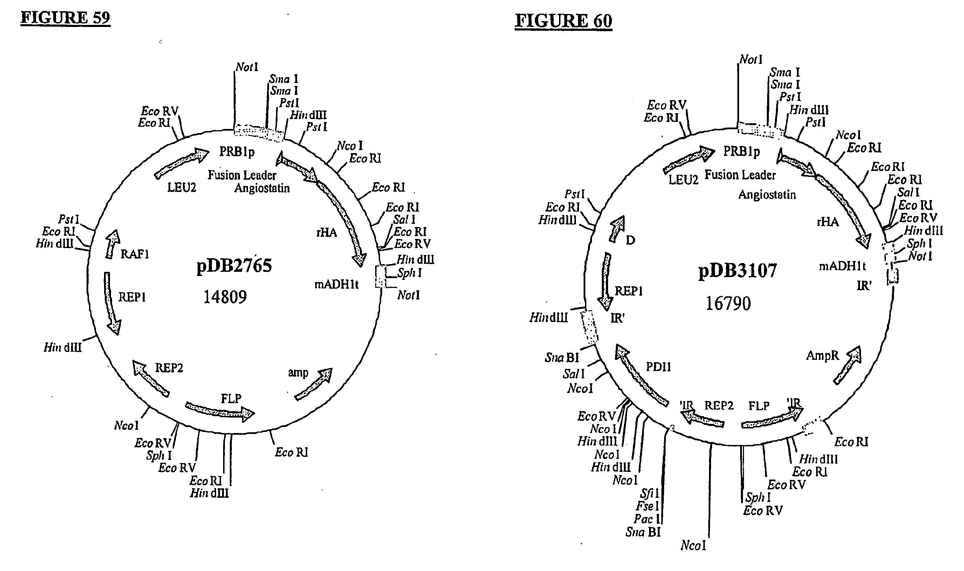

The construction of an *Not*I N-terminal angiostatin-albumin expression cassette (pDB2556) has been previously described (WO 03/066085), as has the construction of a pSAC35-based yeast expression vector, pDB2765 (Figure 59). The 3.77kb *Not*I N-terminal angiostatin-albumin expression cassette was isolated from pDB2556, purified and ligated into *Not*I digested pDB2690, an appropriate yeast *PDII* expression vector, which had been treated with calf intestinal phosphatase, creating plasmid pDB3107 containing the *Not*I expression cassette in the same orientation to the *LEU2* selection marker (Figure 60).

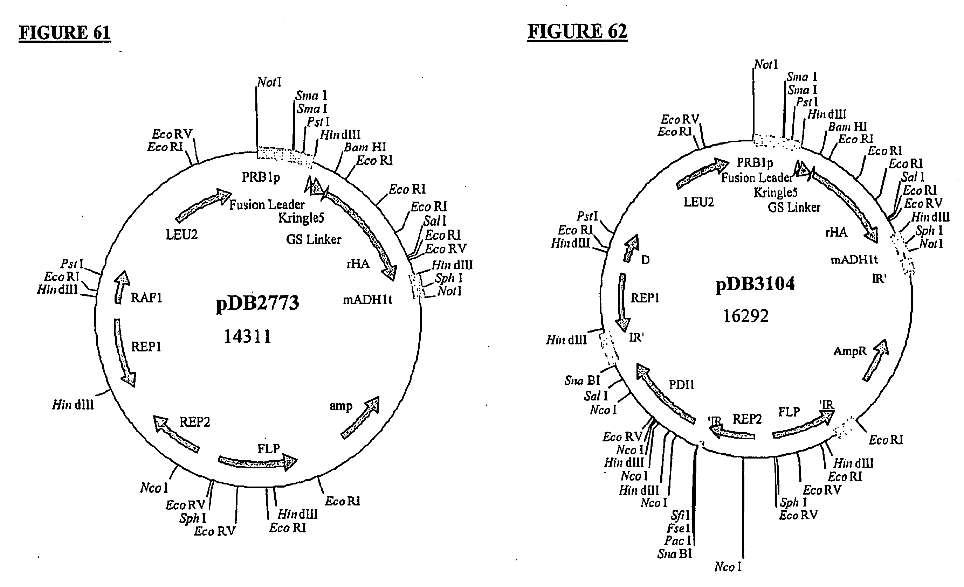

The construction of an *Not*I N-terminal Kringle5-(OGS)$_4$GG-albumin expression cassette (pDB2771) has been previously described (WO 03/066085), as has the construction of a pSAC35-based yeast expression vector, pDB2773 (Figure 61). The 3.27kb *Not*I N-terminal Kringle5-(GGS)$_4$GG-albumin expression cassette was isolated from pDB2771, purified and ligated into *Not*I digested pDB2690, an appropriate yeast *PDII* expression vector, which had been treated with calf intestinal phosphatase, creating plasmid pDB3104 containing the *Not*I expression cassette in the same orientation to the *LEU2* selection marker (Figure 62).

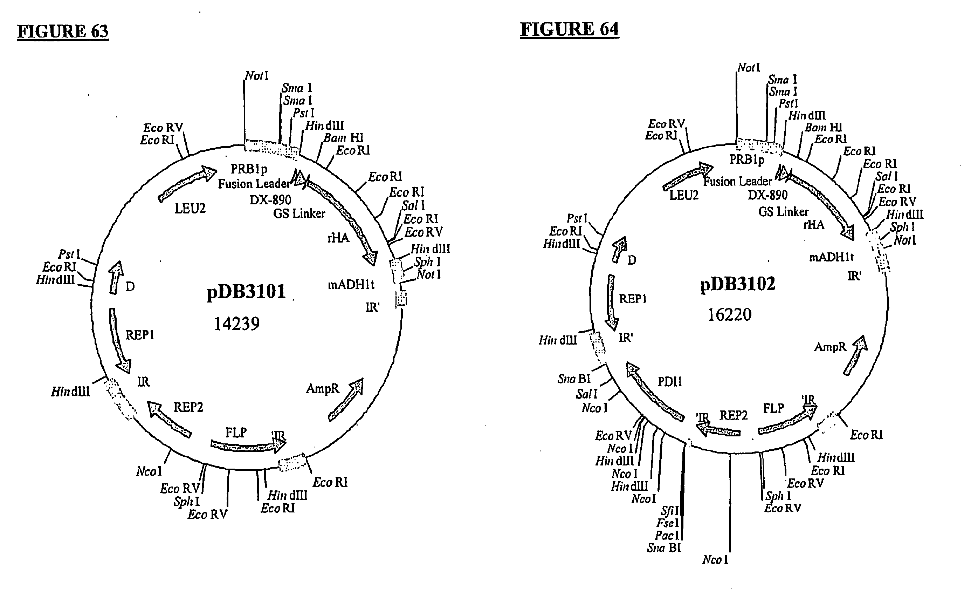

The construction of an *Not*I N-terminal DX-890-(GGS)$_4$GG-albumin expression cassette (pDB2683) has been previously described (WO 03/066824). Appropriate yeast vector sequences were provide by the "disintegration" plasmid pSAC35.. The 3.20kb *Not*I N-terminal DX-890-(GGS)$_4$GG-albumin expression cassette was isolated from pDB2683, purified and ligated into *Not*I digested pSAC35, which had been treated with calf intestinal phosphatase, creating plasmid pDB3101 containing the *Not*I expression cassette in the same orientation to the *LEU2* selection marker (Figure 63). An appropriate yeast *PDII* vector sequences were provide by a "disintegration" plasmid pDB2690 (Figure 6). The 3.20kb *Not*I N-terminal DX-890-(GGS)$_4$GG-albumin expression cassette was isolated from pDB2683, purified and ligated into *Not*I digested pDB2690, which had been treated with calf intestinal phosphatase, creating plasmid pDB3102 containing the *Not*I expression cassette in the same orientation to the *LEU2* selection marker (Figure 64).

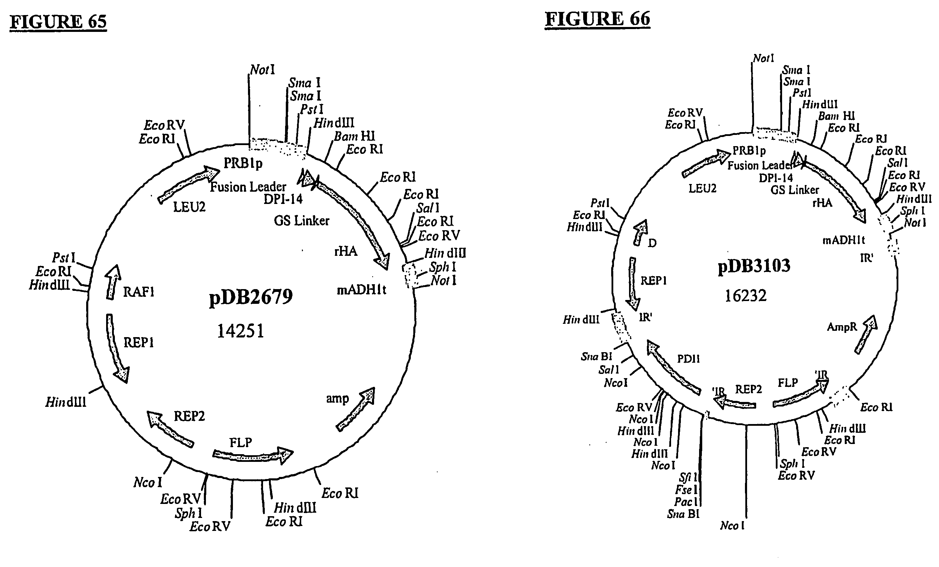

The construction of an *Not*I N-terminal DPI-14-(GGS)$_4$GG-albumin expression cassette (pDB2666) has been previously described (WO 03/066824), as has the construction of a pSAC35-based yeast expression vector, pDB2679 (Figure 65). The 3.21kb *Not*I N-terminal DPI-14-(GGS)$_4$GG-albumin expression cassette was isolated from pDB2666, purified and ligated into *Not*I digested pDB2690, an appropriate yeast *PDI1* expression vector, which had been treated with calf intestinal phosphatase, creating plasmid pDB3103 containing the *Not*I expression cassette in the same orientation to the *LEU2* selection marker (Figure 66).

CNTF was cloned from human genomic DNA by amplification of the two exons using the following primers for exon 1 and exon 2, respectively, using standard conditions.

**Exon 1 primers:**

    5'-CTCGGTACCCAGCTGACTTGTTTCCTGG-3';and
    5'-ATAGGATTCCGTAAGAGCAGTCAG-3'

**Exon 2 primers:**

    5'-GTGAAGCATCAGGGCCTGAAC-3;' and
    5'-CTCTCTAGAAGCAAGGAAGAGAGAAGGGAC-3'

Both fragments were ligated under standard conditions, before being re-amplified by PCR using primers 5'-CTCGGTACCCAGCTGACTTGTTTCCTGG-3' and 5'-CTCTCTAGAAGCAAGGAAGAGAGAAGGGAC-3' and cloned into vector pCR4 (Invitrogen). To generate Axokine™ (as disclosed in Lambert et al, 2001, PNAS, 98, 4652-4657) site-directed mutagenesis was employed to introduce C17A (TGT→GCT) and Q63R (CAG→AGA) mutations. DNA sequencing also revealed the presence of a silent T→C substitution V85V (GTT→GTC) as described in WO 2004/015113.

The Axokine™ cDNA was amplified by PCR using single stranded oligonucleotides MH33 and MH36 to create an approximate 0.58kbp PCR fragment.

MH33

5'-ATGCAGATCTTTGGATAAGAGAGCTTTCACAGAGCATTCACCGCTGACCCC-3'

MH36

5'-CACCGGATCCACCCCCAGTCTGATGAGAAGAAATGAAACGAAGGTCATGG-3'

This was achieved with FastStart Taq DNA polymerase (Roche) in a 50µl reaction, which was initiated by a 4-minute incubation at 95°C and followed by 25 cycles of PCR (95°C for 30secs, 55°C for 30secs, 72°C for 60sec). A PCR product of the expected size was observed in a 10µl sample following electrophoresis in an ethidium bromide stained 1% agarose geL The remaining PCR product was purified using a QIAquick PCR purification kit (Qiagen) and digested to completion with BamHI and BglII. DNA of approximately the expected size was excised from an ethidium bromide stained 1% (w/v) agarose gel and purified.

Plasmid pDB2573X provided a suitable transcription promoter and terminator, along with a suitable secretory leader sequence and DNA sequences encoding part of a (GGS)$_4$GG peptide linker fused to the N-terminus of human albumin. The construction of pDB25733X has been previously described (WO 03/066824).

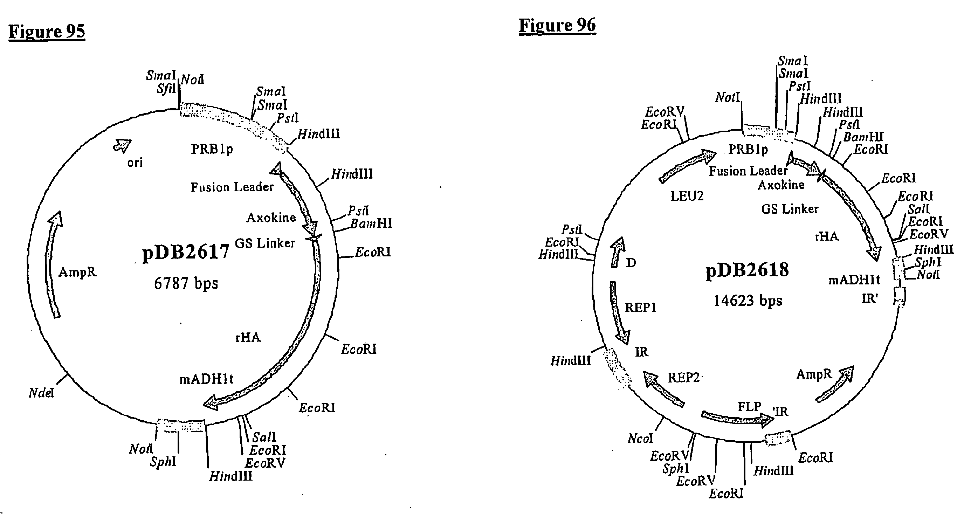

The 0.57kb BamHI and BglII digested PCR product was ligated with pDB2573X, which had been digested with BamHI, BglII and calf intestinal alkaline phosphatase to create plasmid pDB2617 (Figure 95) and the correct DNA sequence confirmed for the PCR generated fragment and adjacent sequences using oligonucleotide primers CF84, CF85. PRB and DS229.

CF84

5'-CCTATGTGAAGCATCAGGGC-3'

**CF85**

5'-CCAACATTAATAGGCATCCC-3'

**PRB**

5'-CGTCCCGTTATATTGGAG-3'

**DS229**

5'-CTTGTCACAGTTTTCAGCAGATTCGTCAG-3'

Plasmid pDB2617 was digested with NdeI and NotI, and the 3.586-kb NotI expression cassette for Axokine™-(GGS)$_4$GG-albumin secretion was purified from an agarose gel.

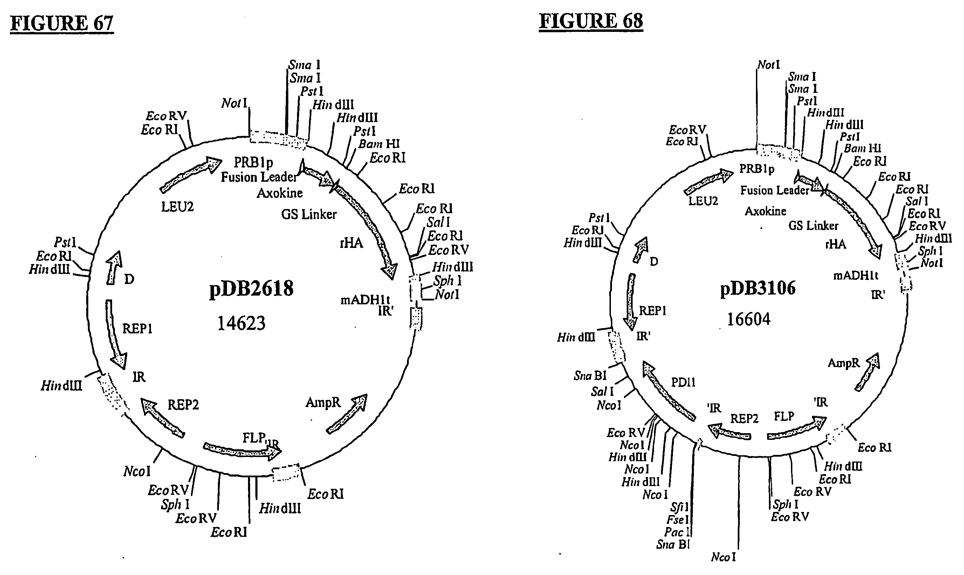

Appropriate yeast vector sequences were provided by the "disintegration" plasmid pSAC35. The 3.586kb NotI N-terminal Axokine™-(GGS)$_4$GG-albumin expression cassette was isolated from pDB2617, purified and ligated into NotI digested pSAC35, which had been treated with calf intestinal phosphatase, creating plasmid pDB2618 containing the NotI expression cassette in the same orientation to the LEU2 selection marker (Figure 96). Appropriate yeast PDI1 vector sequences were provide by a "disintegration" plasmid pDB2690 (Figure 6). The 3.586kb NotI N-terminal Axokine™-(GGS)4GG-albumin expression cassette was isolated from pDB2617, purified and ligated into NotI digested pDB2690, which had been treated with calf intestinal phosphatase, creating plasmid pDB3106 containing the *Not*I expression cassette in the same orientation to the LEU2 selection marker (Figure 68).

A human IL10 cDNA (NCBI accession number (NM_000572) was amplified by PCR using single stranded oligonucleotides CF68 and CF69.

**CF68**

5'-GCGCAGATCTTTGGATAAGAGAAGCCCAGGCCAGGGCACCCAGTCTGAGAACAGCTGCAC-3'

**CF69**

5'-GCTTGGATCCACCGTTTCGTATCTTCATTGTCATGTAGGCTTCTATGTAG-3'

The 0.43kb DNA fragment was digested to completion with *Bam*HI and partially digested with *Bgl*II and the 0.42kb *Bgl*II-*Bam*HI DNA fragment isolated.

Plasmid pDB2573X provided a suitable transcription promoter and terminator, along with a suitable secretory leader sequence and DNA sequences encoding part of a (GGS)$_4$GG peptide linker fused to the N-terminus of human albumin. The construction of pDB2573X has been previously described (WO 03/066824).

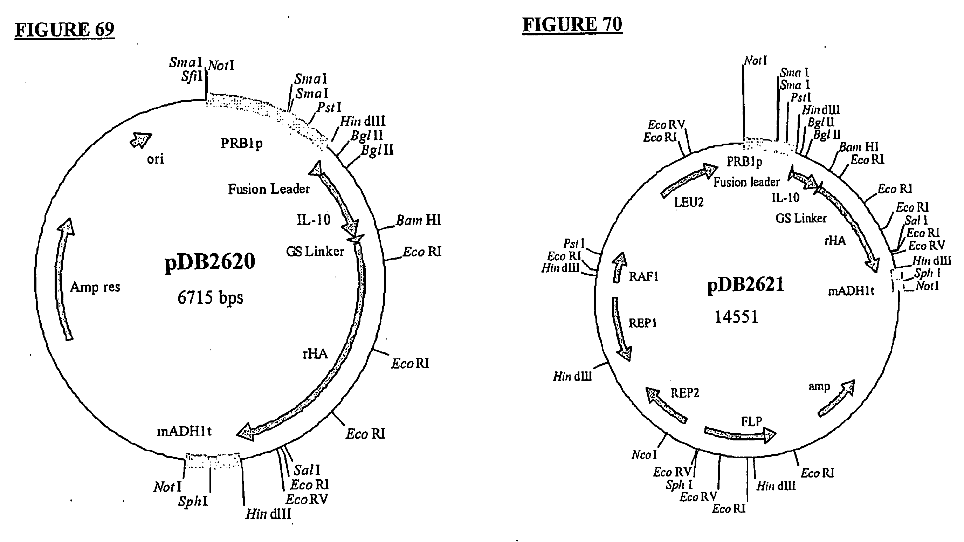

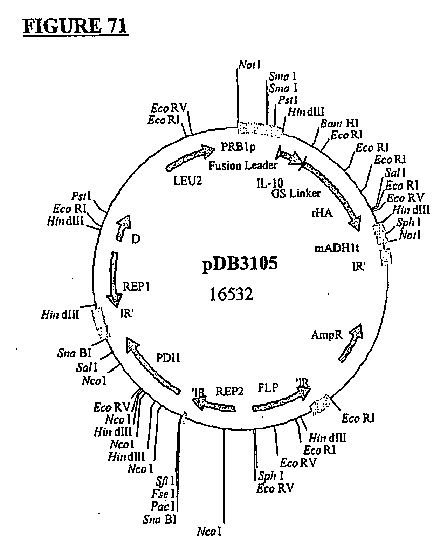

Plasmid pDB2573X was digested to completion with *Bgl*II and *Bam*HI, the 6.21kb DNA fragment was isolated and treated with calf intestinal phosphatase and then ligated with the 0.42kb *Bgl*II/*Bam*HI N-terminal IL10 cDNA to create pDB2620 (Figure 69). Appropriate yeast vector sequences were provided by the "disintegration" plasmid pSAC35. The 3.51kb *Not*I N-terminal IL10-(GGS)$_4$GG-albumin expression cassette was isolated from pDB2620, purified and ligated into *Not*I digested pSAC35, which had been treated with calf intestinal phosphatase, creating plasmid pDB2621 containing the *Not*I expression cassette in the same orientation to the *LEU2* selection marker (Figure 70). An appropriate yeast *PDI1* vector sequences were provide by a "disintegration" plasmid pDB2690 (Figure 6). The 3.51kb *Not*I N-terminal IL10-(GGS)$_4$GG-albumin expression cassette was isolated from pDB2620, purified and ligated into *Not*I digested pDB2690, which had been treated with calf intestinal phosphatase, creating plasmid pDB3105 containing the *Not*I expression cassette in the same orientation to the *LEU2* selection marker (Figure 71).

The same control yeast strain as used in previous examples was transformed to leucine prototrophy using a modified lithium acetate method (Sigma yeast transformation kit, YEAST-1, protocol 2; (Ito et al, 1983, J. Bacteriol., 153, 163; Elble, 1992, Biotechniques, 13, 18)). Transformants were selected on BMMD-agar plates, and were subsequently patched out on BMMD-agar plates. Cryopreserved trehalose stocks were prepared from 10mL BMMD shake flask cultures (24 hrs, 30˚C, 200rpm).

Transformants of each strain were inoculated into 10mL BMMD in 50mL shake flasks and incubated in an orbital shaker at 30˚C, 200rpm for 4-days. Culture supernatants were harvested and the recombinant albumin fusion titres compared by rocket immunoelectrophoresis (Figure 72). The results indicated that the albumin fusion titre in the culture supernatants from yeast strain was higher when *PDII* was present in the 2μm plasmid than when it was not.

The increase in expression of the albumin fusions detected by rocket immunoelectrophoresis was further studied by SDS-PAGE analysis. BMMD shake flask cultures of YBX7 expressing various albumin-fusions were grown for 4-days in an orbital shaker at 30˚C, 200rpm. A sample of the culture supernatant was analysed by SDS-PAGE (Figure 73). A protein band of the expected size for the albumin fusion under study was observed increase in abundance.

## EXAMPLE 10

### Co-expression of *S. cerevisiae ORM2* and recombinant transferrin on a 2μm-based plasmid

The ORM2 gene from *S. cerevisiae* S288c was cloned into the *Xcm*I-site after *REP2* on a pSAC35-based plasmid containing an expression cassette for rTf (N413Q, N611 Q) at the *Not*I-site in the UL-region.

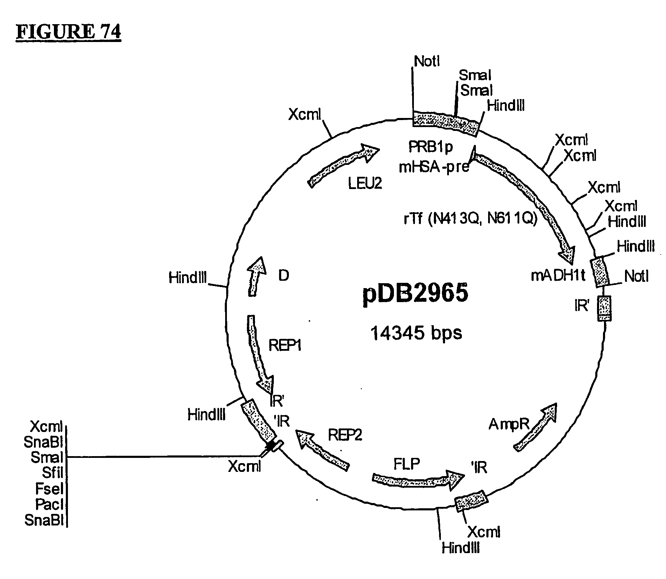

Plasmid pDB2965 (Figure 74) was constructed by insertion of the 3,256-bp *Not*I fragment containing the rTf (N413Q, N611Q) expression cassette from pDB2928 (Figure 11) into the *Not*I-site of pDB2688 (Figure 4). pDB2688 was linearised by *Not*I digestion and was treated with alkaline phosphatase. The rTf expression cassette from pDB2928 was cloned into the *Not*I site of pDB2688 to produce pDB2965, with the transferrin gene transcribed in the same direction as *LEU2.*

The *ORM2* gene was amplified from *S. cerevisiae* S288c genomic DNA by PCR with oligonucleotide primers GS11 and GS12 (Table 10) using the Expand High Fidelity PLUS PCR System (Roche).

**Table 10:** Oligonucleotide Primers for PCR Amplification of S. *cerevisiae* Chaperones

| Primer | Description | Oligonucleotide Sequence |
|---|---|---|
| GS11 | *ORM2* primer, 54mer | 5'–GCGC**TACGTATTAATTAA**ATTGCTCATATATA GTGGGGGGGAATACTCATGCTG–3' |
| GS12 | *ORM2* primer, 49mer | 5'–GCGC**TACGTAGGCCGGCC**AGAGAATATAAAGAA AGATGATGATGTAAGG–3' |
| CED037 | *SSA1* primer, 70mer | 5'–ATACGC**GCATGC**GAATAATTTTTTTTTGCCTATC TATAAAATTAAAGTAGCAGTACTTCAACCATTAGTG–3' |
| CED038 | *SSA1* primer, 50mer | 5'–ATACGC**GCATGC**CGACAAATTGTTACGTTGTGCTTTG ATTTCTAAAGCGC–3' |
| CED009 | *PSE1* primer, 50mer | 5'–ATAGCG**GGATCC**AAGCTTCGACACATACATAATAACT CGATAAGGTATGG–3' |
| CED010 | *PSE1* primer, 39mer | 5'–TATCGC**GGATCC**CGTCTTCACTGTACATTACACAT AAGC–3' |

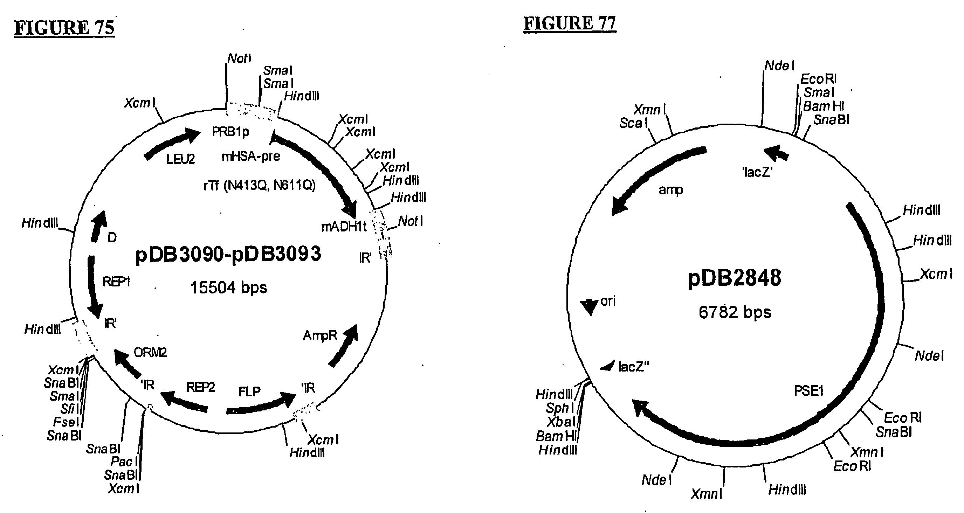

Primers were designed to incorporate *Sna*BI and *Pac*I restriction recognition sites at the 5' end of the forward primer and *Sna*BI and *Fse*I restriction recognition sites at the 5' end of the reverse primer for cloning into the linker at the *Xcm*I-site of the vector, pDB2965. PCR was carried out under the following conditions: 200 μM dNTP mix, 2.5 U of Expand HiFi enzyme blend, 1 x Expand HiFi reaction buffer, 0.8 μg genomic DNA; 1 cycle of 94˚C for 2 minutes, 30 cycles of 94˚C for 30 seconds, 55˚C for 30 seconds, 72˚C for 3 minutes, and 1 cycle 72˚C for 7 minutes. 0.4 μM of each primer was used. The required 1,195-bp PCR product and the pDB2965 vector were digested with *Pac*I and *Fse*I, ligated together and transformed into competent *E. coli* DH5α cells. Ampicillin resistant transformants were selected. *ORM2*-containing constructs were identified by restriction enzyme analysis of plasmid DNA isolated from the ampicillin resistant clones. Four plasmid clones were prepared pDB3090, pDB3091, pDB3092, and pBD3093, all of which had the same expected DNA fragment pattern during restriction analysis (Figure 75).

The *S. cerevisiae* Control Strain and Strain A (as described in Example 3) were selected to investigate the effect on transferrin secretion when the transferrin and *ORM2* genes were co-expressed from the 2μm-based plasmids. The Control Strain and Strain A were transformed to leucine prototrophy by plasmids pDB3090, pDB3092 and pBD3093, as well as a control plasmid pDB2931 (Figure 14), containing the rTf (N413Q, N611Q) expression cassette without *ORM2*. Transformants were selected on BMMD agar and patched out on BMMD agar for subsequent analysis.

To investigate the effect of *ORM2* co-expression on transferrin secretion, 10mL selective (BMMD) and non-selective (YEPD) liquid media were inoculated with strains containing the ORM2/transferrin co-expression plasmids. The shake flask culture was then incubated at 30˚C with shaking (200 rpm) for 4 days. The relative level of transferrin secretion was determined by rocket gel immunoeletrophoresis (RIE) (Figure 76).

Levels of transferrin secreted from Control Strain [pDB3090] and Control Strain [pDB3092] were greater than the levels from Control Strain [pDB2931] in both BMMD and YEPS media. Similarly, the levels of transferrin secreted from both Strain A [pDB3090] and Strain A [pDB3093] were greater than the levels from Strain A [pDB2931] in both BMMD and YEPS media. Transferrin secretion from all Strain A transformants was higher than the Control Strain transformants grown in the same media. Strain A contains an additional copy of *PDI1* in the genome, which enhanced transferrin secretion. Therefore in Strain A, the increased expression of *ORM2* and *PDI1* had a cumulative effect on the secretion of transferrin.

## EXAMPLE 11

### Co-expression: of S. cerevisiae PSE1 and recombinant transferrin on a 2μm-based plasmid

The *PSE1* gene from *S. cerevisiae* S288c was cloned into the *Xcm*I-site after *REP2* on a pSAC35-based plasmid containing an expression cassette for rTf (N413 Q, N611Q) at the *Not*I-site in the UL-region.

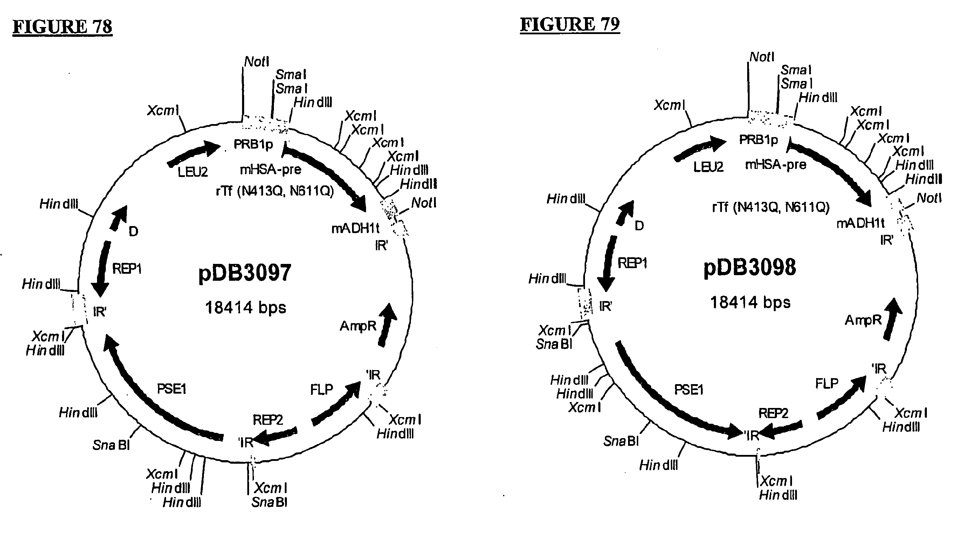

The 3.25-kp wild-type *PSE1* gene was amplified from S. *cerevisiae* S288c genomic DNA by PCR with oligonucleotide primers CED009 and CED010 (Table 10) using the Expand High Fidelity PCR Kit (Roche). Primers were designed to incorporate *Bam*HI restriction recognition sites at the 5' end to facilitate cloning into the vector, pUC19. PCR was carried out under the following conditions: 1 cycle of 94°C for 2 minutes; 10 cycles of 94°C for 15 seconds, 45 °C for 30 seconds, 68°C for 4 minutes and 30 seconds; 20 cycles of 94°C for 15 seconds, 45°C for 30 seconds, 68°C for 4 minutes and 30 seconds (increasing 5 seconds per cycle); and 1 cycle of 68°C for 10 minutes. The required PCR product was digested with *Bam*HI then ligated into pUC19, which had been digested with *Bam*HI and treated with alkaline phosphatase, producing construct pDB2848 (Figure 77). Sequencing of pDB2848 confirmed that amplified sequences were as expected for *S. cererisiae* S288c *PSE1*, when compared to the sequence from *PSE1*/YMR308C on chromosome XIII from coordinates 892220 to 888951 plus 1000 basepairs of upstream sequence and 1000 basepairs of downstream sequence (*Saccharomyces* Genome Database at http://www.yeastgenome.org/). The *PSE1* gene was then excised from pDB2848 by *Bam*HI digestion, and the resulting 4,096-bp fragment phenol:chloroform extracted, ethanol precipitated and treated with DNA polymerase Klenow fragment to fill in the 5'-overhang. Plasmid pDB2965 (Figure 74) was linearised by *Sna*BI digestion, and alkaline phosphatase treated. The linearised pDB2965 vector and the *PSE1* insert were ligated, and transformed into competent *E. coli* DH5α cells. Ampicillin resistant transformants were selected. Plasmids pDB3097 (Figure 78) and pDB3098 (Figure 79) were identified to contain the *PSE1* gene by restriction enzyme analysis of plasmid DNA isolated from the ampicillin resistant clones. In pDB3097 the *PSE1* gene is transcribed in the same orientation as *REP2*, whereas in pDB3098 the *PNE1* gene is transcribed in the opposite orientation to *REP2*.

The *S. cerevisiae* Control Strain was transformed to leucine prototrophy by plasmids, pDB3097 and pBD3098, as well as a control plasmid pDB2931 (Figure 14), containing the rTf (N413Q, N611Q) expression cassette without *PSE1.* Transformants were selected on BMMD agar and patched out on BMMD agar for subsequent analysis.

To investigate the effect of *PSE1* expression on transferrin secretion, flasks containing 10mL selective (BMMD) liquid media were inoculated with strains containing the *PSE1*/transferin co-expression plasmids. The shake flask culture was then incubated at 30°C with shaking (200 rpm) for 4 days. The relative level of transferrin secretion was determined by rocket gel immunoeletrophoresis, (RIE) (Figure 80).

Levels of transferrin secreted from Control Strain [pDB3097] and Control Strain [pDB3098] were greater than the levels from Control Strain [pDB2931] in BMMD media. Therefore, expression of *PSE1* from the 2μm-based plasmids had enhanced transferrin secretion from *S. cerevisiae.* Transferrin secretion was improved with the *PSE1* gene transcribed in either direction relative to the *REP2* gene in pDB3097 and pDB3098.

## EXAMPLE 12

### Co-expression of S. cerevisiae SSA1 and recombinant transferrin: on a 2 μm-based plasmid

The *SSA1* gene from *S. cerevisiae* S288c was cloned into the *Xcm*I-site after *REP2* on a pSAC35-based plasmid containing an expression cassette for rTf (N413Q, N611Q) at the *Not*I-site in the UL-region.

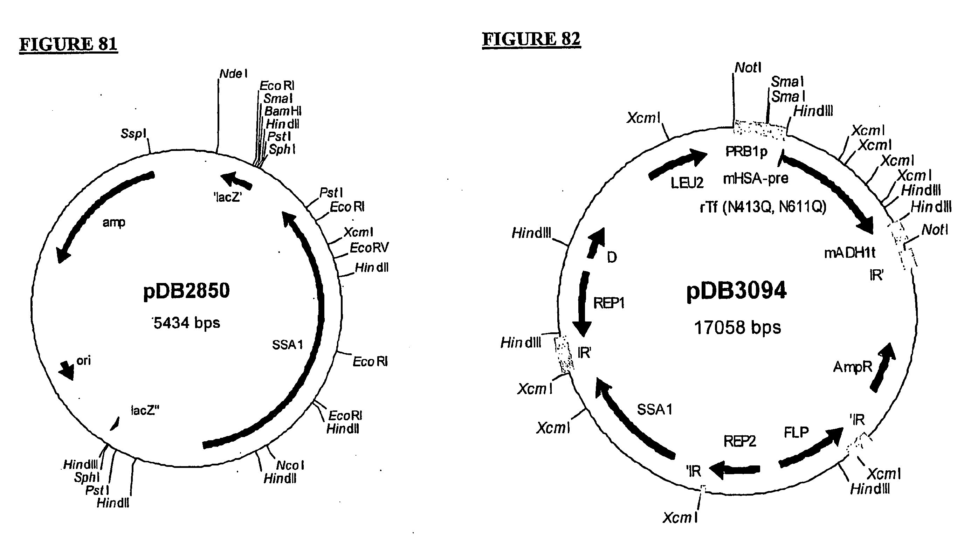

The 1.93-kb *SSA1* gene was amplified from *S. cerevisiae* S288c genomic DNA by PCR with oligonucleotide primers CED037 and CED038 (Table 10) using the Expand High Fidelity PCR Kit (Roche). Primers were designed to incorporate *Sph*I restriction recognition sites at their 5' ends to facilitate cloning into the vector, pUC19. PCR was carried out under the following conditions: 1 cycle of 94°C for 10 minutes, 35 cycles of 94°C for 1 minute, 55°C for 1 minute, 72°C for 5 minutes, and 1 cycle of 72°C for 10 minutes. The required PCR product was digested with *Sph*I then ligated into pUC19, which had been digested with *Sph*I and treated with alkaline phosphatase, producing construct pDB2850 (Figure 81). Sequencing of pDB2850 confirmed the expected sequence of *S. cerevisiae* S288c *SSA1*/YAL005C on chromosome I from coordinates 141433 to 139505 plus 1000 basepairs of upstream sequence and 1000 basepairs of downstream published in the *Saccharomyces* Genome Database (http://www.yeastgenome.org/).

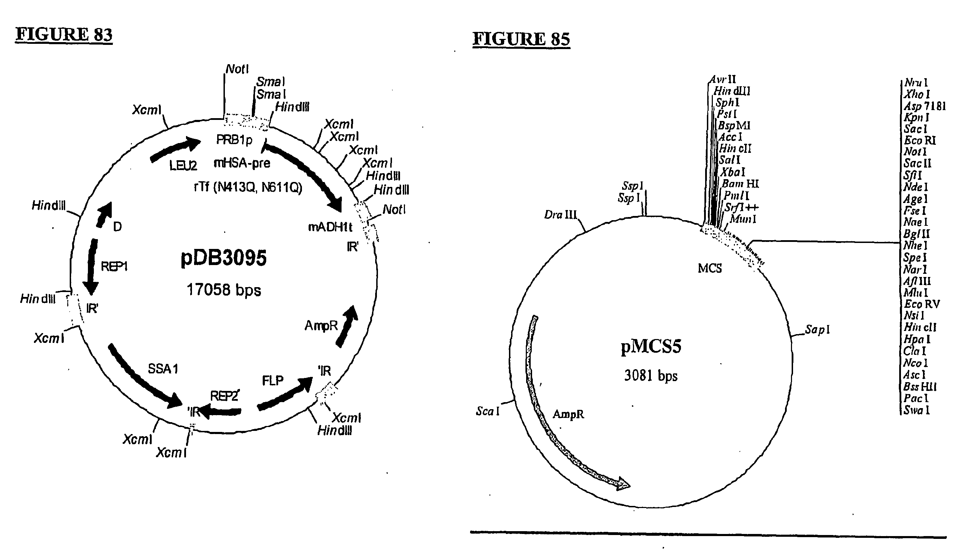

The *SSA1* gene was excised from pDB2850 by *Sph*I-digestion, and the resulting 2,748-bp fragment phenol: chloroform extracted, ethanol precipitated and treated with T4 DNA polymerase to remove the 3'-overhang. Plasmid pDB2965 was linearised by *Sna*BI digestion and treated with calf alkaline phosphatase. The linearised pDB2965 vector and the *SSA1* insert were ligated and transformed into competent *E. coli* DH5α cells. Ampicillin resistant transformants were selected. *SSA1* constructs pDB3094 (Figure 82), and pDB3095 (Figure 83) were identified by restriction enzyme analysis of plasmid DNA isolated from the ampicillin resistant clones. In pDB3094, the *SSA1* gene is transcribed in the same direction as *REP2*, whereas in pDB3095 the *SSA1* gene is transcribed in the opposite direction to *REP2.*

The *S. cerevisiae* Control Strain was transformed to leucine prototrophy by plasmids, pDB3094 and pBD3095, as well as a control plasmid pDB2931 (Figure 14), containing the rTf (N413Q, N611Q) expression cassette without *SSA1.* Transformants were selected on BMMD agar and patched out on BMMD agar for subsequent analysis.

To investigate the effect of *SSA1* expression on transferrin secretion, flasks containing 10mL selective (BMMD) liquid media were inoculated with strains containing the SSA*1*/transferrin co-expression plasmids. The shake flask cultures were incubated at 30˚C with shaking (200 rpm) for 4 days. The relative level of transferrin secretion was determined by rocket gel immunoeletrophoresis (RIE) (Figure 84).

Levels of transferrin secreted from Control Strain [pDB3095] were greater than the levels from Control Strain [pDB2931] and Control Strain [pDB3094] in BMMD media. Therefore, expression of *SSA1* from the 2 $\mu$m-based plasmids had enhanced transferrin secretion from *S. cerevisiae.* Transferrin secretion was improved with the *SSA1* gene transcribed in the opposite direction relative to the *REP2* gene in pDB3094.

## EXAMPLE 13

***PDI1 gene disruption, combined with a PDI1 gene on the 2*$\mu$*m-based plasmid enhanced the secretion of recombinant albumin and plasmid stability.***

Single stranded oligonucleotide DNA primers listed in Table 11 were designed to amplify a region upstream of the yeast *PDI1* coding region and another a region downstream of the yeast *PDII* coding region.

**Table 11:** Oligonucleotide primers

| Primer | Description | Sequence |
|--------|-------------|----------|
| DS299 | 5' *PDII* primer, 38mer | 5'- CGTA<u>GCGGCCGC</u>CTGAAAGGGGTTGACCGTCCGT CGGC -3' |
| DS300 | 5' *PDII* primer, 40mer | 5'-CGTA<u>AAGCTT</u>CGCCGCCCGACAGGGTAACATATTAT CAC -3' |
| DS301 | 3' *PDII* primer, 38mer | 5'-CGTA<u>AAGCTT</u>GACCACGTAGTAATAATAAGTGCAT GGC-3' |
| DS302 | 3'*PDII* primer, 41mer | 5'-CGTA<u>CTGCAG</u>ATTGGATAGTGATTAGAGTGTATAGTCC CGG-3' |
| DS303 | 18mer | 5'-GGAGCGACAAACCTTTCG-3' |
| DS304 | 20mer | 5'-ACCGTAATAAAAGATGGCTG-3' |
| DS305 | 24mer | 5'-CATCTTGTGTGTGAGTATGGTCGG-3' |
| DS306 | 14mer | 5'-CCCAGGATAATTTTCAGG-3' |

Primers DS299 and DS300 amplified the 5' region of *PDI1* by PCR, while primers DS301 and DS302 amplified a region 3' of *PDI1,* using genomic DNA derived S288c as a template. The PCR conditions were as follows: 1$\mu$L S288c template DNA (at 0.01ng/uL, 0.1ng/$\mu$L, 1ng/$\mu$L, 10ng/$\mu$L and 100ng/$\mu$L), 5$\mu$L 10XBuffer (Fast Start Taq+Mg, (Roche)), 1$\mu$L 10mM dNTP's, 5$\mu$L each primer (2$\mu$M), 0.4$\mu$L Fast Start Taq, made up to 50$\mu$L with H$_2$O. PCRs were performed using a Perkin-Elmer Thermal Cycler 9700. The conditions were: denature at 95˚C for 4min [HOLD], then [CYCLE] denature at 95˚C for 30 seconds, anneal at 45˚C for 30 seconds, extend at 72˚C for 45 seconds for 20 cycles, then [HOLD] 72˚C for 10min and then [HOLD] 4˚C. The 0.22kbp *PDI1* 5' PCR product was cut with *Not*I and *Hind*III, while the 0.34kbp *PDII* 3' PCR product was cut with *Hind*III and *Pst*I.

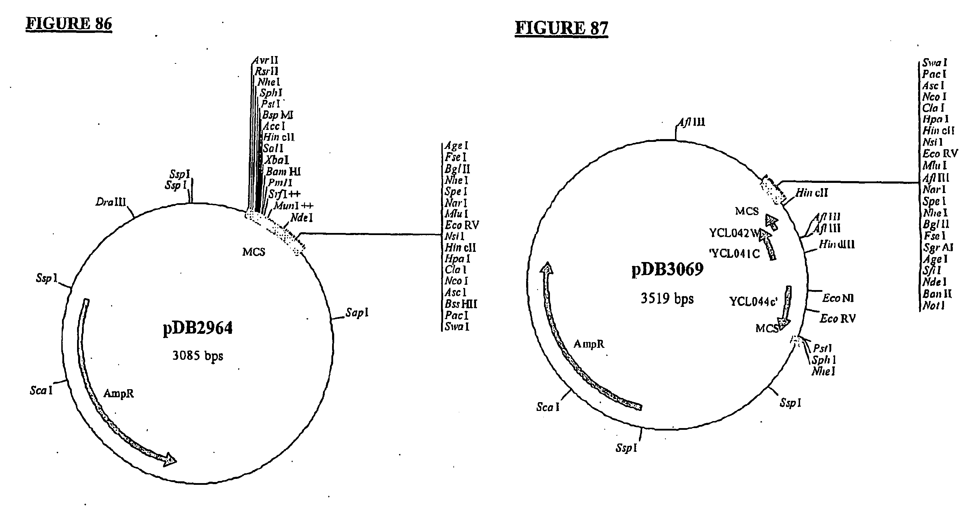

Plasmid pMCS5 (Hoheisel, 1994, Biotechniques 17, 456-460) (Figure 85) was digested to completion with *Hind*III, blunt ended with T4 DNA polymerase plus dNTPs and religated to create pDB2964 (Figure 86).

Plasmid pDB2964 was *Hind*III digested, treated with calf intestinal phosphatase, and ligated with the 0.22kbp *PDI1* 5' PCR product digested with *Not*I and *Hind*III and the 0.34kbp *PDI1* 3' PCR product digested with *Hind*III and *Pst*I to create pDB3069 (Figure 87) which was sequenced with forward and reverse universal primers and the DNA sequencing primers DS303, DS304, DS305 and DS306 (Table 11).

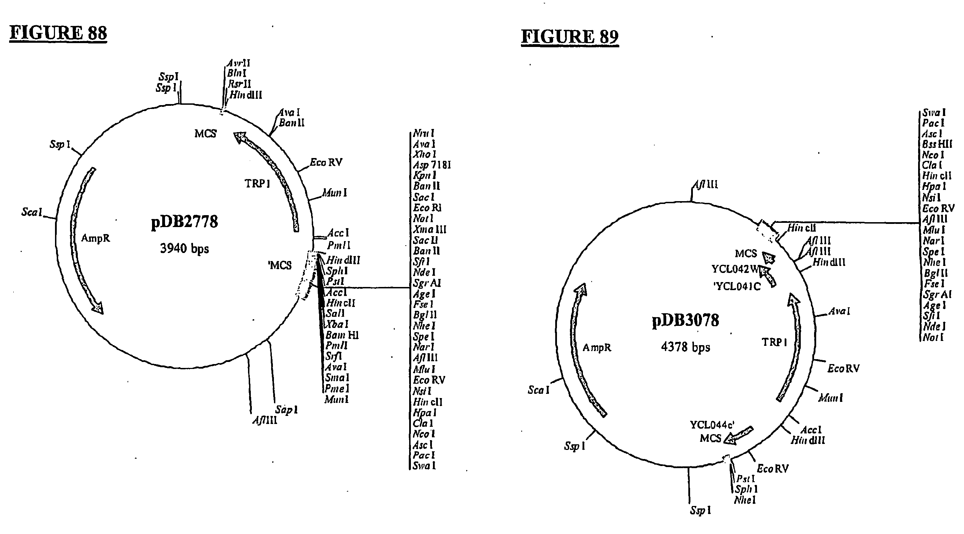

Primers DS234 and DS235 (Table 12) were used to amplify the modified *TRP1* marker gene from YIplac204 (Gietz & Sugino, 1988, Gene, 74, 527-534), incorporating *Hind*III restriction sites at either end of the PCR product. The PCR conditions were as follows: 1$\mu$L template YIplac204 (at 0.01ngl/$\mu$L, 0.1ng/$\mu$L, Ing/$\mu$L, 10ng/$\mu$L and 100ng/$\mu$L), 5$\mu$L 10XBuffer (Fast Start Taq+Mg, (Roche)), 1$\mu$L 10mM dNTP's, 5$\mu$pL each primer (2$\mu$M), 0.4$\mu$L Fast Start Taq, made up to 50$\mu$L with $H_2O$. PCRs were performed using a Perkin-Elmer Thermal Cycler 9600. The conditions were: denature at 95˚C for 4min [HOLD], then [CYCLE] denature at 95˚C for 30 seconds, anneal for 45 seconds at 45˚C, extend at 72˚C for 90sec for 20 cycles, then [HOLD] 72˚C for 10min and then [HOLD] 4˚C. The 0.86kbp PCR product was digested with *Hind*III and cloned into the *Hind*III site of pMCS5 to create pDB2778 (Figure 88). Restriction enzyme digestions and sequencing with universal forward and reverse primers as well as DS236, DS237, DS238 and DS239 (Table 12) confirmed that the sequence of the modified *TRP1* gene was correct.

**Table 12**: Oligonucleotide primers

| Primer | Description | Sequence |
|---|---|---|
| DS230 | *TRP1* 5' UTR | 5'-TAGCGAATTCAATCAGTAAAAATCAACGG-3' |
| DS231 | *TRP1* 5' UTR | 5'-GTCAAAGCTTCAAAAAAAGAAAAGCTCCGG-3' |
| DS232 | *TRP1* 3'UTR | 5'-TAGCGGATCCGAATTCGGCGGTTGTTTGCAAGACC GAG-3' |
| DS233 | *TRP1* 3'UTR | 5'-GTCAAAGCTTTAAAGATAATGCTAAATCATTTGG-3' |
| DS234 DS235 | *TRP1* | 5'-TGACAAGCTTTCGGTCGAAAAAAGAAAAGG AG AGG-3' |
| | *TRP1* | 5'-TGACAAGCTTGATCTTTTATGCTTGCTTTTC-3' |
| DS236 | *TRP1* | 5'-AATAGTTCAGGCACTCCG-3' |
| DS237 | *TRP1* | 5'-TGGAAGGCAAGAGAGCC-3' |
| DS238 | *TRP1* | 5'-TAAAATGTAAGCTCTCGG-3' |
| DS239 | *TRP1* | 5'-CCAACCAAGTATTTCGG-3' |
| CED005 | $\Delta$*TRP1* | 5'-GAGCTGACAGGGAAATGGTC-3' |
| CED006 | $\Delta$*TRP1* | 5'-TACGAGGATACGGAGAGAGG-3' |

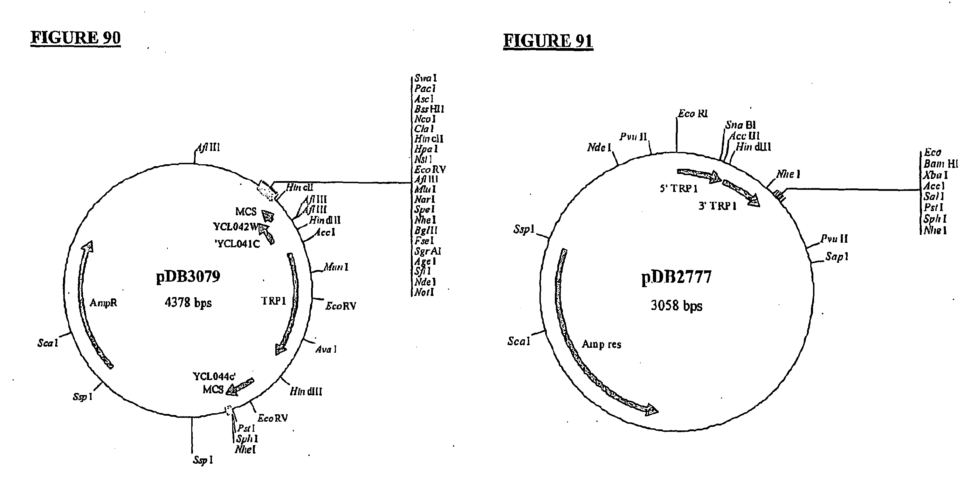

The 0.86kbp *TRP1* gene was isolated from pDB2778 by digestion with *Hind*III and cloned into the *Hind*III site of pDB3069 to create pDB3078 (Figure 89) and pDB3079 (Figure 90). A 1.41kb *pdil::TRP1* disrupting DNA fragment was isolated from pDB3078 or pDB3079 by digestion with *Not*I/*Pst*I.

Yeast strains incorporating a *TAP1* deletion (*trp1*$\Delta$) were to be constructed in such a way that no homology to the *TRP1* marker gene (pDB2778) should left in the genome once the *trp1*$\Delta$ had been created, so preventing homologous recombination between future *TRP1* containing constructs and the *TRP1* locus. In order to achieve the total removal of the native TRP1 sequence from the genome of the chosen host strains, oligonucleotides were designed to amplify areas of the 5' UTR and 3' UTR of the *TRP1* gene outside of *TRP1* marker gene present on integrating vector YIplac204 (Gietz:& Sugino, 1988, Gene, 74, 527-534). The YIplac204 *TRP1* marker gene differs from the native/chromosomal *TRP1* gene in that internal *Hind*III, *Pst*I and *Xba*I sites were removed by site directed mutagenesis (Gietz & Sugino, 1988, Gene, 74, 527-534). The YIplac204 modified *TRP1* marker gene was constructed from a 1.453kbp blunt-ended genomic fragment *Eco*RI fragment, which contained the *TRP1* gene and only 102bp of the *TRP1* promoter (Gietz & Sugino, 1988, Gene, 74, 527-534). Although this was a relatively short promoter sequence it was clearly sufficient to complement *trp1* auxotrophic mutations (Gietz & Sugino, 1988, Gene, 74, 527-534). Only DNA sequences upstream of the *Eco*RI site, positioned 102bp 5' to the start of the *TRP1* ORF were used to create the 5' *TRP1* UTR. The selection of the 3' UTR was less critical as long as it was outside the 3' end of the functional modified *TRP1* marker, which was chosen to be 85bp downstream of the translation stop codon.

Single stranded oligonucleotide DNA primers were designed and constructed to amplify the 5' UTR and 3' UTR regions of the *TRP1* gene so that during the PCR amplification restriction enzyme sites would be added to the ends of the PCR products to be used in later cloning steps. Primers DS230 and DS231 (Table 12) amplified the 5' region of *TRP1* by PCR, while primers DS232 and DS233 (Table 12) amplified a region 3' of *TRP1,* using S288c genomic DNA as a template. The PCR conditions were as follows: $\mu$L template S288c genomic DNA (at 0.01ng/$\mu$L, 0.1ng/$\mu$L, 1ng/ $\mu$L, 10ng/$\mu$L and 100ng/$\mu$L), 5$\mu$L 10XBuffer (Fast Start Taq+Mg, (Roche)), 1$\mu$L, 10mM dNTP's, 5$\mu$L each primer (2$\mu$M),

0.4µL Fast Start Taq, made up to 50µL with $H_2O$. PCRs were performed using a Perkin-Elmer Thermal Cycler 9600. The conditions were: denature at 95˚C for 4min [HOLD], then [CYCLE] denature at 95˚C for 30 seconds, anneal for 45 seconds at 45˚C, extend at 72˚C for 90sec for 20 cycles, then [HOLD] 72˚C for 10min and then [HOLD] 4˚C.

The 0.19kbp *TRP1* 5' UTR PCR product was cut with *Eco*RI and *Hind*III, while the 0.2kbp *TRP1* 3' UTR PCR product was cut with *Bam*HI and *Hind*III and ligated into pAYE505 linearised with *Bam*HI/*Eco*RI to create plasmid pDB2777 (Figure 91). The construction of pAYE505 is described in WO 95/33833. DNA sequencing using forward and reverse primers, designed to prime from the plasmid backbone and sequence the cloned inserts, confirmed that in both cases the cloned 5' and 3' UTR sequences of the *TRP1* gene had the expected DNA sequence. Plasmid pDB2777 contained a *TRP1* disrupting fragment that comprised a fusion of sequences derived from the 5' and 3' UTRs of *TRP1*. This 0.383kbp *TRP1* disrupting fragment was excised from pDB2777 by complete digestion with *Eco*RI.

Yeast strain DXY1 (Kerry-Williams et al., 1998, Yeast, 14, 161-169) was transformed to leucine prototrophy with the albumin expression plasmid pDB2244 using a modified lithium acetate method (Sigma yeast transformation kit, YEAST-1, protocol 2; (Ito et al, 1983, J. Bacteriol., 153, 163; Elble, 1992, Biotechniques, 13, 18)) to create yeast strain DXY1 [pDB2244]. The construction of the albumin expression plasmid pDB2244 is described in WO 00/44772. Transformants were selected on BMMD-agar plates, and were subsequently patched out on BMMD-agar plates. Cryopreserved trehalose stocks were prepared from 10mL BMMD shake flask cultures (24 hrs, 30˚C, 200rpm).

DXY1 [pDB2244] was transformed to tryptophan autotrophy with the 0.383kbp *Eco*RI *TRP1* disrupting DNA fragment from pDB2777 using a nutrient agar incorporating the counter selective tryptophan analogue, 5-fluoroanthranilic acid (5-FAA), as described by Toyn et al., (2000 Yeast 16, 553-560). Colonies resistant to the toxic effects of 5-FAA were picked and streaked onto a second round of 5-FAA plates to confirm that they really were resistant to 5-FAA and to select away from any background growth. Those colonies which grew were then were re-patched onto BMMD and BMMD plus tryptophan to identify which were tryptophan auxotrophs.

Subsequently colonies that had been shown to be tryptophan auxotrophs were selected for further analysis by transformation with YCplac22 (Gietz & Sugino, 1988, Gene, 74, 527-534) to ascertain which isolates were *trp1*.

PCR amplification across the *TRP1* locus was used to confirm that the trp⁻ phenotype was due to a deletion in this region. Genomic DNA was prepared from isolates identified as resistant to 5-FAA and unable to grow on minimal media without the addition of tryptophan. PCR amplification of the genomic *TRP1* locus with primers CED005 and CED006 (Table 12) was achieved as follows: 1µL template genomic DNA, 5µL 10XBuffer (Fast Start Taq+Mg, (Roche)), 1µL 10mM dNTP's, 5µL each primer (2µM), 0.4µL Fast Start Taq, made up to 50µL with $H_2O$. PCRs were performed using a Perkin-Elmer Thermal Cycler 9600. The conditions were: denature at 94˚C for 10min [HOLD], then [CYCLE] denature at 94˚C for 30 seconds, anneal for 30 seconds at 55˚C, extend at 72˚C for 120sec for 40 cycles, then [HOLD] 72˚C for 10min and then [HOLD] 4˚C. PCR amplification of the wild type *TRP1* locus resulted in a PCR product of 1.34kbp in size, whereas amplification across the deleted *TRP1* region resulted in a PCR product 0.84kbp smaller at 0.50kbp. PCR analysis identified a DXY1 derived trp⁻ strain (DXY1 *trp1*Δ [pDB2244]) as having the expected deletion event.

The yeast strain DXY1 *trp1*Δ [pDB2244] was cured of the expression plasmid pDB2244 as described by Sleep et al., (1991, Bio/Technology, 9, 183-187). DXY1 *trp1*Δ cir⁰ was re-transformed the leucine prototrophy with either pDB2244, pDB2976, pDB2977, pDB2978, pDB2979, pDB2980 or pDB2981 using a modified lithium acetate method (Sigma yeast transformation kit, YEAST-1, protocol 2; (Ito et al, 1983, J. Bacteriol., 153, 163; Elble, 1992, Biotechniques, 13, 18)). Transformants were selected on BMMD-agar plates supplemented with tryptophan, and were subsequently patched out on BMMD-agar plates supplemented with tryptophan. Cryopreserved trehalose stocks were prepared from 10mL BMMD shake flask cultures supplemented with tryptophan (24 hrs, 30˚C, 200rpm).

The yeast strains DXY1 *trp1*Δ [pDB2976], DXY1 *trp1*Δ [pDB2977], DXY1 *trp1*Δ [pDB2978], DXY1 *trp1*Δ [pDB2979], DXY1 *trp1*Δ [pDB2980] or DXY1 *trp1*Δ [pDB2981] was transformed to tryptophan prototrophy using the modified lithium acetate method (Sigma yeast transformation kit, YEAST-1, protocol 2; (Ito et al, 1983, J. Bacteriol., 153,163; Elble, 1992, Biotechniques, 13, 18)) with a 1.41kb *pdi1::TRP1* disrupting DNA fragment was isolated from pDB3078 by digestion with *Not*I/*Pst*I. Transformants were selected on BMMD-agar plates and were subsequently patched out on BMMD-agar plates.

Six transformants of each strain were inoculated into 10mL YEPD in 50mL shake flasks and incubated in an orbital shaker at 30˚C, 200rpm for 4-days. Culture supernatants and cell biomass were harvested. Genomic DNA was prepared (Lee, 1992, Biotechniques, 12, 677) from the tryptophan prototrophs and DXY1 [pDB2244]. The genomic *PDI1* locus amplified by PCR of with primers DS236 and DS303 (Table 11 and 12) was achieved as follows: 1µL template genomic DNA, 5µL 10XBuffer (Fast Start Taq+Mg, (Roche)), 1µL 10mM dNTP's, 5µL each primer (2µM), 0.4µL Fast Start Taq, made up to 50µL with $H_2O$. PCRs were performed using a Perkin-Elmer Thermal Cycler 9700. The conditions were: denture at 94˚C for 4min [HOLD], then [CYCLE] denature at 94˚C for 30 seconds, anneal for 30 seconds at 50˚C, extend at 72˚C for 60sec for 30 cycles, then [HOLD] 72˚C for 10min and then [HOLD] 4˚C. PCR amplification of the wild type *PDI1* locus resulted in no PCR product, whereas amplification across the deleted *PDI1* region resulted in a PCR product 0.65kbp. PCR analysis identified that all 36 potential *pdi1::TRP1* strains tested had the expected *pdi1::TRP1* deletion.

The recombinant albumin titres were compared by rocket immunoelectrophoresis (Figure 92). Within each group, all six *pdi1::TRP1* disruptants of DXY1 *trp1Δ* [pDB2976], DXY1 *trp1Δ* [pDB2978], DXY1 *trp1Δ* [pDB2980], DXY1 *trp1Δ* [pDB2977] and DXY1 *trp1Δ* [pDB2979] had very similar rHA productivities. Only the six *pdi1::TRP1* disruptants of DXY1 *trp1Δ1* [pDB2981] showed variation in rHA expression titre. The six *pdil::TRP1* disruptants indicated in Figure 92 were spread onto YEPD agar to isolate single colonies and then re-patched onto BMMD agar.

Three single celled isolates of DXY1 *trp1Δ pdi1::TRP1* [pDB2976], DXY1 *trp1Δ pdil::TRP1* [pDB2978], DXY1 *trp1Δ pdi1::TRP1* [pDB2980], DXY1 *trp1Δ pdi1::TRP1* [pDB2977], DXY1 *trp1Δ pdi1::TRP1* [pDB2979] and DXY1 *trp1Δ pdi1::TRP1* [pDB2981] along with DXY1 [pDB2244], DXY1 [pDB2976], DXY1 [pDB2978], DXY1 [pDB2980], DXY1 [pDB2977], DXY1 [pDB2979] and DXY1 [pDB2981] were inoculated into 10mL YEPD in 502mL shake flasks and incubated in an orbital shaker at 30°C, 200rpm for 4-days. Culture supernatants were harvested and the recombinant albumin titres were compared by rocket immunoelectrophoresis (Figure 93). The thirteen wild type *PDI1* and *pdi1::TRP1* disruptants indicated in Figure 93 were spread onto YEPD agar to isolate single colonies. One hundred single celled colonies from each strain were then re-patched onto BMMD agar or YEPD agar containing a goat anti-HSA antibody to detect expression of recombinant albumin (Sleep et al., 1991, Bio/Technology, 9, 183-187) and the Leu+/rHA+, Leu+/rHA-, Leu-/rHA+ or Leu-/rHA- phenotype of each colony scored (Table 13).

## Table 13:

| | PDI1 | | | | *pdi1::TRP1* | | | |
|---|---|---|---|---|---|---|---|---|
| | Leu+ rHA+ | Leu- rHA+ | Leu+ rHA- | Leu- rHA- | Leu+ rHA+ | Leu- rHA+ | Leu+ rHA- | Leu- rHA- |
| pDB2244 | 100 | 0 | 0 | 0 | | | | |
| pDB2976 | 7 | 0 | 47 | 46 | 97 | 0 | 3 | 0 |
| pDB2978 | 86 | 0 | 0 | 14 | 100 | 0 | 0 | 0 |
| pDB2980 | 98 | 0 | 0 | 2 | 100 | 0 | 0 | 0 |
| pDB2977 | 0 | 0 | 4 | 96 | 100 | 0 | 0 | 0 |
| pDB2979 | 69 | 0 | 6 | 25 | 100 | 0 | 0 | 0 |
| pDB2981 | 85 | 0 | 0 | 15 | 92 | 0 | 0 | 8 |

These data indicate plasmid retention is increased when the *PDI1* gene is used as a selectable marker on a plasmid in a host strain having no chromosomally encoded PDI, even in a non-selective medium such as the exemplified rich medium.

## Claims

1. A method for producing non-2μm-family plasmid protein comprising:

 (a) providing a host cell comprising a 2μm-family plasmid, the plasmid comprising a gene encoding a protein comprising the sequence of a chaperone protein and a gene encoding a non-2μm-family plasmid protein;

(b) culturing the host cell in a culture medium under conditions that allow the co-expression of the gene encoding protein comprising the sequence of the chaperone protein and the gene encoding a non-2μm-family plasmid protein; and

(c) purifying the thus expressed non-2μm-family plasmid protein from the cultured host cell or the culture medium.

2. The method of Claim 1 further comprising the step of formulating the purified non-2μm-family plasmid protein with a carrier or diluent and optionally presenting the thus formulated protein in a unit dosage form.

3. Use of a 2μm-famiiy plasmid as an expression vector to increase the production of a fungal or vertebrate non-2μm-family plasmid protein by providing a gene encoding the non-2μm-family plasmid protein and a gene encoding a chaperone protein on the same 2μm-family plasmid.

4. The use of Claim 3 wherein the fungal non-2μm-family plasmid protein is a yeast non-2μm-family plasmid protein.

5. A 2μm-family plasmid comprising a gene encoding a protein comprising the sequence of a chaperone protein and a gene encoding a non-2μm-131 family plasmid protein, wherein if the plasmid is based on the 2μm plasmid then it is a disintegration vector.

6. A method, use or plasmid according to any preceding claim wherein the chaperone has a sequence of a fungal chaperone or a mammalian chaperone.

7. A method, use or plasmid according to Claim 6 wherein the chaperone has a sequence of a yeast chaperone.

8. A method, use or plasmid according to Claim 6 wherein the chaperone has a sequence of a human chaperone.

9. A method, use or plasmid according to any preceding claim wherein the chaperone comprises the sequence of a protein encoded by any one of *AHA1, CCT2, CCT3, CCT4, CCT5, CCT6, CCT7, CCT8, CNS1, CPR3, CPR6, EPS1, ENRO1, EUG1, FMO1, HCH1, HSP10, HSP12, HSP104, HSP26, HSP30, HSP42, HSP60, HSP78, HSP82, JEM1, MDJ1, MDJ2, MPD1, MPD2, PDI1, PFD1, ABC1, APJ1, ATP11, ATP12, BTT1, CDC37, CPR7, HSC82, KAR2, LHS1, MGE1, MRS11, NOB1, ECM10, SSA1, SSA2, SSA3, SSA4, SSC1, SSE2, SIL1, SLS1, UBI4, ORM1, ORM2, PER1, PTC2; PSE1* and *HAC1* or truncated intronless *HAC1*.

10. A method, use or plasmid according to any preceding claim wherein the chaperone is protein disulphide isomerase, or comprises the sequence of a protein encoded by *POSE1, ORM2* or *SSA1* or a variant or fragment thereof, which variant or fragment possesses the same enzymatic activity.

11. A method according to any one of Claims 1, 2, or 5 to 10 wherein the host cell also expresses a second recombinant gene encoding a chaperone that is different to the first chaperone encoded by the plasmid.

12. A method according to Claim 11 wherein the second recombinant gene encoding a chaperone is chromosomally integrated.

13. A method, use or plasmid according to any one of Claims 1 to 11 wherein the plasmid comprises two different genes encoding different chaperones, one of which gene is the second recombinant gene encoding a chaperone as defined by Claim 11.

14. A method, use or plasmid according to any one of Claims 11 to 13 wherein one of the chaperones is protein disulphide isomerase.

15. A method, use or plasmid according to any one of Claims 11 to 14 wherein one of the chaperones is ORM2.

16. A method, use or plasmid according to Claim 11 to 15 wherein the two chaperones are protein disulphide isomerase and ORM2.

17. A method, use or plasmid according to any preceding claim wherein the non-2μm-family plasmid protein comprises a leader sequence effective to cause secretion in yeast.

**18.** A method, use or plasmid according to any preceding claim wherein the non-2μm-family plasmid protein is a eukaryotic protein, or a fragment or variant thereof, preferably a vertebrate or a fungal protein.

**19.** A method, use or plasmid according to Claim 18 wherein the non-2μm-family plasmid protein is a yeast protein.

**20.** A method, use or plasmid according to any preceding claim wherein the non-2μm-family plasmid protein is a commercially useful protein.

**21.** A method, use or plasmid according to any preceding claim wherein the non-2μm-family plasmid protein comprises a sequence selected from albumin, a monoclonal antibody, an etoposide, a serum protein, a blood clotting factor, antistasin, a tick anticoagulant peptide, transferrin, lactoferrin, endostatin, angiostatin, collagen, an immunoglobulin or fragment thereof, an Immunoglobulin-based molecule or fragment thereof, a Kunitz domain protein, interferons, interleukins, IL10, IL11, IL2, interferon α species and sub-species, interferon β species and sub-species, interferon γ species and sub-species, leptin, CNTF, CNTF$_{Ax15}$, IL1-receptor antagonist, erythropoietin and erythropoietin mimics, thrombopoietin and thrombopoietin mimics, prosaptide, cyanovirin-N, 5-helix, T20 peptide, T1249 peptide, HIV gp41, HIV gp120, urokinase, prourokinase, tPA, hirudin, platelet derived growth factor, parathyroid hormone, proinsulin, insulin, glucagon, glucagon-like peptides, insulin-like growth factor, calcitonin, growth hormone, transforming growth factor β, tumour necrosis factor, G-CSF, GM-CSF, M-CSF, FGF, coagulation factors in both pre and active forms, including but not limited to plasminogen, fibrinogen, thrombin, pre-thrombin, pro-thrombin, von Willebrand's factor, α$_1$-antitrypsin, plasminogen activators, Factor VII, Factor VIII, Factor IX, Factor X and Factor XIII, nerve growth factor, LACI, platelet-derived endothelial cell growth factor, glucose oxidase, serum cholinesterase, aprotinin, amyloid precursor protein, inter-alpha trypsin inhibitor, antithrombin III, apo-lipoprotein species, Protein C, Protein S, or a variant or fragment of any of the above.

**22.** A method, use or plasmid according to any preceding claim wherein the non-2μm-family plasmid protein comprises the sequence of an Immunoglobulin-based molecule or fragment thereof, wherein the Immunoglobulin-based molecule is selected from a dAb, a Fab' fragment, F(ab')$_2$, scAb, scFv or a scFv fragment.

**23.** A method, use or plasmid according to any preceding claim wherein the non-2μm-family plasmid protein comprises the sequence of albumin or a variant or fragment thereof.

**24.** A method, use or plasmid according to any preceding claim wherein the non-2μm-family plasmid protein comprises the sequence of a transferrin family member, or a variant or fragment thereof.

**25.** A method, use or plasmid according to Claim 24 wherein the non-2μm-family plasmid protein comprises the sequence of transferrin or lactoferrin, or a variant or fragment thereof.

**26.** A method, use or plasmid according to any preceding claim wherein the non-2μm-family plasmid protein comprises a fusion protein.

**27.** A method, use or plasmid according to Claim 26 wherein the fusion protein is a fusion protein of albumin or a transferrin family member or a variant or fragment of either, fused directly or indirectly to the sequence of another protein.

**28.** A host cell comprising a plasmid as defined by any preceding claim.

**29.** A host cell according to Claim 28 wherein a chaperone encoded by the plasmid is an essential gene.

**30.** A host cell according to Claim 29 wherein, in the absence of the plasmid, the host cell does not produce the chaperone.

**31.** A host cell according to any one of Claims 28 to 30 which is a yeast cell.

**32.** A host cell according to Claim 31 in which the plasmid is based on pSR1, pSB3 or pSB4 and the yeast cell is *Zygosaccharomyces rouxii,* the plasmid is based on pSB1 or pSB2 and the yeast cell is *Zygosaccharomyces bailli,* the plasmid is based on pSM1 and the yeast cell is *Zygosaccharomyces fermentati,* the plasmid is based on pKD1 and the yeast cell is *Kluyveromyces drosophilarum,* or the plasmid is based on the 2μm plasmid and the yeast cell is *Saccharomyces cerevisiae* or *Saccharomyces carlsbergensis.*

33. A host cell according to Claim 32 in which the plasmid is based on the 2μm plasmid and the yeast cell is *Saccharomyces cerevisiae* or *Saccharomyces carlsbergensis.*

34. A method according to Claim 1 wherein the host cell is a host cell as defined by any one of Claims 28 to 33.

35. A method according to Claim 34 wherein the host cell is a host cell as defined by Claim 30, or any other claim dependent thereon.

36. A method according to Claim 34 wherein the step (b) involves culturing the host cell in non-selective media, such as a rich media.

37. A method for producing non-2μm-family plasmid protein comprising:

    (a) providing a host cell comprising a first recombinant gene encoding a protein comprising the sequence of a first chaperone protein, a second recombinant gene encoding a protein comprising the sequence of a second chaperone protein and a third recombinant gene encoding a non-2μm-family plasmid protein, wherein the first and second chaperones are different;
    (b) culturing the host cell in a culture medium under conditions that allow the expression of the first, second and third genes; and
    (c) optionally purifying the thus expressed non-2μm-family plasmid protein from the cultured host cell or the culture medium; and
    (d) optionally, lyophilising the thus purified protein.

38. The method of Claim 37 further comprising the step of formulating the purified non-2μm-faniily plasmid protein with a carrier or diluent and optionally presenting the thus formulated protein in a unit dosage form.

39. A method according to Claim 37 or 38 wherein the first and second chaperones comprise the sequence of a protein encoded by any one of *AHA1. CCT2, CCT3, CCT4, CCT5, CCT6, CCT7, CCT8, CNS1, CPR3, CPR6, EPS1, ERO1, EUG1, FMO1, HCH1, HSP10, HSP12, HSP104, HSP26, HSP30, HSP42, HSP60, HSP78, HSP82, JEM1, MDJ1, MDJ2, MPD1, MPD2, PDI1, PFD1, ABC1, APJ1, ATP11, ATP12, BTT1, CDC37, CPR7, HSC82, KAR2, LHS1, MGE1, MRS11, NOBI, ECM10, SSA1, SSA2, SSA3, SSA4, SSC1, SSE2, SIL1, SLS1, UBI4, ORM1, ORM2, PER1, PTC2, PSE1* and *HAC1* or truncated intronless *HAC1.*

40. A method according to any one of Claims 37 to 39 wherein the first chaperone is protein disulphide isomerase.

41. A method according to any one of Claims 38 to 40 wherein the second chaperone is ORM2.

42. A method according to any one of Claims 37 to 41 wherein at least one of the first or second chaperones is encoded by a chromosomally integrated recombinant gene.

43. A method according to any one of Claims 37 to 42 wherein at least one of the first or second chaperones is encoded by a gene on a plasmid.

44. A method according to Claim 43 wherein the plasmid is a plasmid as defined by any one of Claims 1 to 33.

45. A host cell comprising a first recombinant gene encoding a protein comprising the sequence of protein disulphide isomerase (PDI) and a second recombinant gene encoding a protein comprising the sequence of a transferrin-based protein.

46. Use of a recombinant gene encoding a protein comprising the sequence of protein disulphide isomerase (PDI) to increase the expression of a transferrin-based protein.

47. A host cell according to Claim 45 or use according to Claim 46 wherein the transferrin-based protein comprises the sequence of transferrin or any other member of the transferrin family, a variant or fragment thereof or a fusion protein comprising transferrin, a variant or fragment thereof.

48. A host cell or use according to Claim 47 wherein the transferrin-based protein comprises the sequence of lactoferrin, or a variant or fragment thereof.

**49.** A host cell or use according to any one of Claims 45 to 48 wherein the first recombinant gene encoding a protein comprising the sequence of protein disulphide isomerase (PDI) is provided on a plasmid.

**50.** A host cell or use according to Claim 49 wherein the plasmid is a 2$\mu$m-family plasmid.

**51.** A host cell or use according to any one of Claims 45 to 48 wherein the first recombinant gene encoding a protein comprising the sequence of protein disulphide isomerase (PDI) is chromosomally integrated.

**52.** A host cell or use according to Claim 51 wherein the first recombinant gene encoding a protein comprising the sequence of protein disulphide isomerase (PDI) is chromosomally integrated at the locus of an endogenously encoded PDI gene.

**53.** A host cell or use according to Claim 52 wherein the first recombinant gene encoding a protein comprising the sequence of protein disulphide isomerase (PDI) is chromosomally integrated at the locus of an endogenously encoded PDI gene without disrupting the expression of the endogenous PDI gene.

**54.** A host cell or use according to any one of Claims 45 to 53 wherein the second recombinant gene encoding a protein comprising the sequence of a transferrin-based protein is provided on a plasmid.

**55.** A host cell or use according to Claim 54 wherein the plasmid is a 2$\mu$m-family plasmid.

**56.** A host cell or use according to any one of Claims 45 to 52 wherein the second recombinant gene encoding a protein comprising the sequence of a transferrin-based protein is chromosomally integrated.

**57.** A host cell or use according to Claim 55 wherein the second recombinant gene encoding a protein comprising the sequence of a transferrin-based protein is chromosomally integrated at the locus of an endogenously encoded PDI gene.

**58.** A host cell or use according to Claim 57 wherein the second recombinant gene encoding a protein comprising the sequence of a transferrin-based protein is chromosomally integrated at the locus of an endogenously encoded PDI gene without disrupting the expression of the endogenous PDI gene.

**59.** A method for producing non-2$\mu$m-family plasmid protein comprising:

(a) providing a host cell comprising a first recombinant gene encoding a protein comprising the sequence of ORM2, or a variant or fragment thereof which possesses the same enzymatic activity as ORM2, and a second recombinant gene encoding a non-2$\mu$m-family plasmid protein; and
(b) culturing the host cell in a culture medium under conditions that allow the expression of the first and second genes.

**60.** The method of Claim 59 further comprising the step of formulating the purified non-2$\mu$m-family plasmid protein with a carrier or diluent and , optionally presenting the thus formulated protein in a unit dosage form.

**61.** A method according to Claim 59 or 60 wherein the first recombinant gene encoding a protein comprising the sequence of ORM2, or a variant or fragment thereof which possesses the same enzymatic activity as ORM2, is integrated into the chromosome of the host cell.

**62.** A method according to Claim 59 or 60 wherein the first recombinant gene encoding a protein comprising the sequence of ORM2, or a variant or fragment thereof which possesses the same enzymatic activity as ORM2, is located on a plasmid.

**63.** A host cell comprising first recombinant gene encoding a protein comprising the sequence of ORM2, or a variant or fragment thereof which possesses the same enzymatic activity as ORM2, and a second recombinant gene encoding a non-2$\mu$m-family plasmid protein.

**64.** Use of a recombinant gene encoding a protein comprising the sequence of ORM2, or a variant or fragment thereof which possesses the same enzymatic activity as ORM2, to increase the expression of non-2$\mu$m-family plasmid protein in a host cell.

65. A 2μm-family plasmid comprising a first recombinant gene encoding a protein comprising the sequence of ORM2, or a variant or fragment thereof which possesses the same enzymatic activity as ORM2; and a second recombinant gene encoding a non-2μm-family plasmid protein.

66. A method, use, host cell or plasmid according to any one of Claims 59 to 65 wherein the non-2μm-family plasmid protein is as defined in any one of Claims 17 to 27.

67. The method of Claim 1 further comprising the step of lyophilising the thus purified protein.

68. The method of Claim 59 further comprising the step of purifying the thus expressed non-2μm-family plasmid protein from the cultured host cell or the culture medium.

69. The method of Claim 68 further comprising the step of lyophilising the thus purified protein.

70. The method of Claim 68 or 69 further comprising the step of formulating the purified or lyophilised non-2μm-family plasmid protein with a carrier or diluent.

71. The method of Claim 70 further comprising the step of presenting the thus formulated protein in a unit dosage form.

**Patentansprüche**

1. Verfahren zum Herstellen von Nicht-2-μm-Familien-Plasmidprotein, wobei das Verfahren umfasst:

   a) Bereitstellen einer Wirtszelle, umfassend ein 2-μm-Familien-Plasmid, das Plasmid umfassend ein Gen, das ein Protein kodiert, das die Sequenz eines Chaperonproteins umfasst, und ein Gen, das ein Nicht-2-μm-Familien-Plasmidprotein kodiert;
   b) Kultivieren der Wirtszelle in einem Kulturmedium unter Bedingungen, die die Koexpression des Gens, das das Protein kodiert, das die Sequenz des Chaperonproteins umfasst, und des Gens, das ein Nicht-2-μm-Familien-Plasmidprotein kodiert, erlaubt;
   c) Reinigen des auf diese Weise exprimierten Nicht-2-μm-Familien-Plasmid-proteins aus der kultivierten Wirtszelle oder dem Kulturmedium.

2. Verfahren nach Anspruch 1, weiterhin umfassend den Schritt des Formulierens des gereinigten Nicht-2-μm-Famil-len-Plasmidproteins mit einem Träger oder Verdünnungsmittel und gegebenenfalls das Darbieten des auf diese Weise formulierten Proteins in einer Einheitsdosierungsform.

3. Verwendung eines 2-μm-Familien-Plasmids als ein Expressionsvektor, um die Herstellung eines Pilz- oder Verte-braten-Nicht-2-μm-Familien-Plasmidproteins durch Bereitstellen eines Gens, das das Nicht-2-μm-Familien-Plas-midprotein kodiert, und einem Gen, das ein Chaperonprotein auf dem gleichen 2-μm-Familien-Plasmid kodiert, zu erhöhen.

4. Verwendung nach Anspruch 3, wobei das Pilz-Nicht-2-μm-Familien-Plasmidprotein ein Hefe-Nicht-2-μm-Familien-Plasmidprotein ist.

5. Ein 2-μm-Familien-Plasmid, umfassend ein Gen, das ein Protein kodiert, umfassend die Sequenz eines Chaperonproteins und ein Gen, das ein Nicht-2-μm-Familien-Plasmidprotein kodiert, wobei das Plasmid, wenn es auf dem 2-μm-Plasmid basiert, ein Disintegrationsvektor ist.

6. Verfahren, Verwendung oder Plasmid nach einem beliebigen vorhergehenden Anspruch, wobei das Chaperon eine Sequenz eines Pilzchaperons oder eines Säugerchaperons hat.

7. Verfahren, Verwendung oder Plasmid nach Anspruch 6, wobei das Chaperon eine Sequenz eines Hefechaperons hat.

8. Verfahren, Verwendung oder Plasmid nach Anspruch 6, wobei das Chaperon eine Sequenz eines humanen Chaperons hat.

9. Verfahren, Verwendung oder Plasmid nach einem beliebigen vorhergehenden Anspruch, wobei das Chaperon die Sequenz eines Proteins umfasst, das kodiert ist durch ein beliebiges von *AHA1, CCT2, CCT3, CCT4, CCT5, CCT6, CCT7, CCT8, CNS1, CPR3, CPR6, EPS1, ERO1, EUG1, FMO1, HCH1, HSP10, HSP12, HSP104, HSP26, HSP30, HSP42, HSP60, HSP78, HSP82, JEM1, MDJ1, MDJ2, MPD1, MPD2, PDI1, PFD1, ABC1, APJ1, ATP11, ATP12, BTT1, CDC37, CPR7, HSC82, KAR2, LHS1, MGE1, MRS11, NOB1, ECM10, SSA1, SSA2, SSA3, SSA4, SSC1, SSE2, SIL1, SLS1, UBI4, ORM1, ORM2, PER1, PTC2, PSE1* und *HAC1* oder verkürztes intronfreies *HAC1*.

10. Verfahren, Verwendung oder Plasmid nach einem beliebigen vorhergehenden Anspruch, wobei das Chaperon Proteindisulphidisomerase ist oder die Sequenz eines Proteins umfasst, das durch *PSE1, ORM2* oder *SSA1,* oder einer Variante oder eines Fragments davon, kodiert ist, wobei die Variante oder das Fragment die gleiche enzymatische Aktivität besitzt.

11. Verfahren nach einem beliebigen der Ansprüche 1, 2 oder 5 bis 10, wobei die Wirtszelle auch ein zweites rekombinantes Gen exprimiert, das ein Chaperon kodiert, das sich von dem ersten Chaperon, das durch das Plasmid kodiert ist, unterscheidet.

12. Verfahren nach Anspruch 11, wobei das zweite rekombinante Gen, das ein Chaperon kodiert, chromosomal integriert ist.

13. Verfahren, Verwendung oder Plasmid nach einem beliebigen der Ansprüche 1 bis 11, wobei das Plasmid zwei unterschiedliche Gene umfasst, die unterschiedliche Chaperone kodieren, von denen eines der Gene das zweite rekombinante Gen ist, das ein wie durch Anspruch 11 definiertes Chaperon kodiert.

14. Verfahren, Verwendung oder Plasmid nach einem beliebigen der Ansprüche 11 bis 13, wobei eines der Chaperone Proteindisulphidisomerase ist.

15. Verfahren, Verwendung oder Plasmid nach einem beliebigen der Ansprüche 11 bis 14, wobei eines der Chaperone ORM2 ist.

16. Verfahren, Verwendung oder Plasmid nach Anspruch 11 bis 15, wobei die zwei Chaperone Proteindisulphidisomerase und ORM2 sind.

17. Verfahren, Verwendung oder Plasmid nach einem beliebigen vorhergehenden Anspruch, wobei das Nicht-2-$\mu$m-Familien-Plasmidprotein eine Leadersequenz umfasst, die wirksam die Sekretion in Hefe herbeiführt.

18. Verfahren, Verwendung oder Plasmid nach einem beliebigen vorhergehenden Anspruch, wobei das Nicht-2-$\mu$m-Familien-Plasmidprotein ein eukaryotisches Protein oder ein Fragment oder eine Variante davon ist, bevorzugt ein Vertebraten- oder ein Pilzprotein ist.

19. Verfahren, Verwendung oder Plasmid nach Anspruch 18, wobei das Nicht-2-$\mu$m-Familien-Plasmidprotein ein Hefeprotein ist.

20. Verfahren, Verwendung oder Plasmid nach einem beliebigen vorhergehenden Anspruch, wobei das Nicht-2-$\mu$m-Familien-Plasmidprotein ein kommerziell verwendbares Protein ist.

21. Verfahren, Verwendung oder Plasmid nach einem beliebigen vorhergehenden Anspruch, wobei das Nicht-2-$\mu$m-Familien-Plasmidprotein eine Sequenz umfasst, ausgewählt aus Albumin, einem monoklonalen Antikörper, einem Etoposid, einem Serumprotein, einem Blutgerinnungsfaktor, Antistasin, einem Tick-Antikoagulantpeptid, Transferrin, Lactoferrin, Endostatin, Angiostatin, Kollagen, einem Immunglobulin oder Fragment davon, einem Molekül auf Immunglobulin-Basis oder einem Fragment davon, einem Kunitz-Domänen-Protein, Interferone, Interleukine, IL10, IL11, IL2, Interferon-$\alpha$-Arten und -Unterarten, Interferon-$\beta$-Arten und - Unterarten, Interferon-$\gamma$-Arten und -Unterarten, Leptin, CNTF, CNTF$_{Ax15}$, IL1-Rezeptorantagonist, Erythropoietin und Erythropoietinimitatoren, Thrombopoietin und Thrombopoietinimitatoren, Prosaptid, Cyanovirin-N, 5-Helix, T20-Peptid, T1249-Peptid, HIV gp41, HIV gp120, Urokinase, Pro-Urokinase, tPA, Hirudin, Platelet-derived-Growth-Factor, Parathormon, Pro-Insulin, Insulin, Glucagon, glucagonartige Peptide, Insulinähnlicher Wachstumsfaktor (Insulin-like-Growth-Faktor), Calcitonin, Wachstumshormon, Transformierender Wachstumsfaktor $\beta$ (Transforming-Growth-Factor $\beta$), Tumornekrosefaktor, G-CSF, GM-CSF, M-CSF, FGF, Gerinnungsfaktoren in sowohl Prä- als auch aktiven Formen, einschließlich aber nicht limitiert auf Plasminogen, Fibrinogen, Thrombin, Präthrombin, Prothrombin, Von-Willebrand-Faktor, $\alpha_1$-Antitrypsin, Plasmi-

nogenaktivatoren, Faktor VII, Faktor VIII, Faktor IX, Faktor X und Faktor XIII, Nervenwachstumsafktor (Nerve-Growth-Factor), LACI, Platelet-derived-endothelial-Cell-Growth-Factor, Glukoseoxidase, Serumcholinesterase, Aprotinin, amyloides Vorläuferprotein, Inter-alpha-Trypsininhibitor, Antithrombin III, Apolipoproteinarten, Protein C, Protein S, oder eine Variante oder ein Fragment eines beliebigen der Vorstehenden.

22. Verfahren, Verwendung oder Plasmid nach einem beliebigen vorhergehenden Anspruch, wobei das Nicht-2-$\mu$m-Familien-Plasmidprotein die Sequenz eines Moleküls auf Immunglobulin-Basis oder eines Fragments davon umfasst, wobei das Molekül auf Immunglobulin-Basis ausgewählt ist aus einem dAb, einem Fab'-Fragment, F(ab')$_2$, scAb, scFv oder einem scFv-Fragment.

23. Verfahren, Verwendung oder Plasmid nach einem beliebigen vorhergehenden Anspruch, wobei das Nicht-2-$\mu$m-Familien-Plasmidprotein die Sequenz von Albumin oder einer Variante oder eines Fragments davon umfasst.

24. Verfahren, Verwendung oder Plasmid nach einem beliebigen vorhergehenden Anspruch, wobei das Nicht-2-$\mu$m-Familien-Plasmidprotein die Sequenz von einem Transferrin-Familienmitglied oder einer Variante oder eines Fragments davon umfasst.

25. Verfahren, Verwendung oder Plasmid nach Anspruch 24, wobei das Nicht-2-$\mu$m-Familien-Plasmidprotein die Sequenz von Transferrin oder Lactoferrin oder einer Variante oder eines Fragments davon umfasst.

26. Verfahren, Verwendung oder Plasmid nach einem beliebigen vorhergehenden Anspruch, wobei das Nicht- 2-$\mu$m-Familien-Plasmidprotein ein Fusionsprotein umfasst.

27. Verfahren, Verwendung oder Plasmid nach Anspruch 26, wobei das Fusionsprotein ein Fusionsprotein von Albumin oder ein Trasferrin-Familienmitglied oder eine Variante oder ein Fragment von beiden ist, das direkt oder indirekt mit der Sequenz eines anderen Proteins fusioniert ist.

28. Wirtszelle, umfassend ein wie nach einem beliebigen vorhergehenden Anspruch definiertes Plasmid.

29. Wirtszelle nach Anspruch 28, wobei ein Chaperon, das durch das Plasmid kodiert ist, ein essentielles Gen ist.

30. Wirtszelle nach Anspruch 29, wobei in Abwesenheit des Plasmids die Wirtszelle nicht das Chaperon erzeugt.

31. Wirtszelle nach einem beliebigen der Ansprüche 28 bis 30, welche eine Hefezelle ist.

32. Wirtszelle nach Anspruch 31, in welcher das Plasmid auf pSR1, pSB3 oder pSB4 basiert und die Hefezelle *Zygosaccharomyces rouxii* ist, das Plasmid auf pSB1 oder pSB2 basiert und die Hefezelle *Zygosaccharomyces bailli* ist, das Plasmid auf pSM1 basiert und die Hefezelle *Zygosaccharomyces fermentati* ist, das Plasmid auf pKD1 basiert und die Hefezelle *Kluyveromyces drosophilarum* ist, oder das Plasmid auf dem 2-$\mu$m-Plasmid basiert und die Hefezelle *Saccharomyces cerevisiae* oder *Saccharomyces carlsbergensis* ist.

33. Wirtszelle nach Anspruch 32, in welcher das Plasmid auf dem 2-$\mu$m-Plasmid basiert und die Wirtszelle *Saccharomyces cerevisiae* oder *Saccharomyces carlsbergensis* ist.

34. Verfahren nach Anspruch 1, wobei die Wirtzelle eine wie nach einem beliebigen der Ansprüche 28 bis 33 definierte Wirtszelle ist.

35. Verfahren nach Anspruch 34, wobei die Wirtszelle eine wie nach Anspruch 30 oder einem beliebigen anderen Anspruch, der von diesem abhängt, definierte Wirtszelle ist.

36. Verfahren nach Anspruch 34, wobei der Schritt b) das Kultivieren der Wirtszelle in nichtselektivem Medium, wie einem reichhaltigen Medium, einbezieht.

37. Verfahren zum Herstellen von Nicht-2-$\mu$m-Familien-Plasmidprotein, wobei das Verfahren umfasst:

(a) Bereitstellen einer Wirtszelle, umfassend ein erstes rekombinantes Gen, das ein Protein kodiert, das die Sequenz eines ersten Chaperonproteins umfasst, ein zweites rekombinantes Gen, das ein Protein kodiert, das die Sequenz eines zweiten Chaperonproteins umfasst, und ein ein drittes rekombinantes Gen, das ein Nicht-

2-$\mu$m-Familien-Plasmidprotein kodiert, wobei das erste und zweite Chaperon sich unterscheiden;

(b) Kultivieren der Wirtszelle in einem Kulturmedium unter Bedingungen, die die Expression des ersten, zweiten und dritten Genes erlauben; und

(c) gegebenenfalls Reinigen des auf diese Weise exprimierten Nicht-2-$\mu$m-Familien-Plasmidproteins aus der kultivierten Wirtszelle oder dem Kulturmedium; und

(d) gegebenenfalls Lyophilisieren des auf diese Weise gereinigten Proteins.

38. Verfahren nach Anspruch 37, weiterhin umfassend den Schritt des Formulierens des gereinigten Nicht-2-$\mu$m-Familien-Plasmidprotiens mit einem Träger oder einem Verdünnungsmittel und gegebenenfalls Darbieten des auf diese Weise formulierten Proteins in einer Einheitsdosierungsform.

39. Verfahren nach Anspruch 37 oder 38, wobei das erste und zweite Chaperon die Sequenz eines Proteins umfassen, kodiert durch ein beliebiges von *AHA1, CCT2, CCT3, CCT4, CCT5, CCT6, CCT7, CCT8, CNS1, CPR3, CPR6, EPS1, ERO1, EUG1, FMO1, HCH1, HSP10, HSP12, HSP104, HSP26, HSP30, HSP42, HSP60, HSP78, HSP82, JEM1, MDJ1, MDJ2, MPD1, MPD2, PDI1, PFD1, ABC1, APJ1, ATP11, ATP12, BTT1, CDC37, CPR7, HSC82, KAR2, LHS1, MGE1, MRS11, NOB1, ECM10, SSA1, SSA2, SSA3, SSA4, SSC1, SSE2, SIL1, SLS1, UBI4, ORM1, ORM2, PER1, PTC2, PSE1* und *HAC1* oder verkürztes intronfreies *HAC1*.

40. Verfahren nach einem beliebigen der Ansprüche 37 bis 39, wobei das erste Chaperon Proteindisulphidisomerase ist.

41. Verfahren nach einem beliebigen der Ansprüche 38 bis 40, wobei das zweite Chaperon ORM2 ist.

42. Verfahren nach einem beliebigen der Ansprüche 37 bis 41, wobei mindestens eines des ersten oder zweiten Chaperons durch ein chromosomal integriertes, rekombinantes Gen kodiert ist.

43. Verfahren nach einem beliebigen der Ansprüche 37 bis 42, wobei mindestens eines des ersten oder zweiten Chaperons durch ein Gen auf einem Plasmid kodiert wird.

44. Verfahren nach Anspruch 43, wobei das Plasmid ein wie nach einem beliebigen der Ansprüche 1 bis 33 definiertes Plasmid ist.

45. Wirtszelle, umfassend ein erstes rekombinantes Gen, kodierend ein Protein, das die Sequenz von Proteindisulphidisomerase (PDI) umfasst, und ein zweites rekombinantes Gen, kodierend ein Protein, das die Sequenz eines Proteins auf Transferrin-Basis umfasst.

46. Verwendung eines rekombinanten Gens, kodierend ein Protein, das die Sequenz von Proteindisulphidisomerase (PDI) umfasst, um die Expression eines Proteins auf Transferrin-Basis zu erhöhen.

47. Wirtszelle nach Anspruch 45 oder Verwendung nach Anspruch 46, wobei das Protein auf Transferrin-Basis die Sequenz von Transferrin oder eines beliebigen anderen Mitglieds der Transferrinfamilie, einer Variante oder eines Fragments davon oder eines Fusionsproteins, umfassend Transferrin, eine Variante oder ein Fragment davon, umfasst.

48. Wirtszelle oder Verwendung nach Anspruch 47, wobei das Protein auf Transferrin-Basis die Sequenz von Lactoferrin oder einer Variante oder eines Fragments davon umfasst.

49. Wirtszelle oder Verwendung nach einem beliebigen der Ansprüche 45 bis 48, wobei das erste rekombinante Gen, kodierend ein Protein, das die Sequenz von Proteindisulphidisomerase (PDI) umfasst, auf einem Plasmid bereitgestellt ist.

50. Wirtszelle oder Verwendung nach Anspruch 49, wobei das Plasmid ein 2-$\mu$m-Familien-Plasmid ist.

51. Wirtszelle oder Verwendung nach einem beliebigen der Ansprüche 45 bis 48, wobei das erste rekombinante Gen, kodierend ein Protein, das die Sequenz von Proteindisulphidisomerase (PDI) umfasst, chromosomal integriert ist.

52. Wirtszelle oder Verwendung nach Anspruch 51, wobei das erste rekombinante Gen, kodierend ein Protein, das die Sequenz von Proteindisulphidisomerase (PDI) umfasst, chromosomal in dem Locus eines endogen kodierten PDI-Gens integriert ist.

**53.** Wirtszelle oder Verwendung nach Anspruch 52, wobei das erste rekombinante Gen, kodierend ein Protein, das die Sequenz von Proteindisulphidisomerase (PDI) umfasst, chromosomal in dem Locus eines endogen kodierten PDI-Gens integriert ist, ohne die Expression des endogenen PDI-Gens zu unterbrechen.

**54.** Wirtszelle oder Verwendung nach einem beliebigen der Ansprüche 45 bis 53, wobei das zweite rekombinante Gen, kodierend ein Protein, das die Sequenz eines Proteins auf Transferrin-Basis umfasst, auf einem Plasmid bereitgestellt ist.

**55.** Wirtszelle oder Verwendung nach Anspruch 54, wobei das Plasmid ein 2-$\mu$m-Familien-Plasmid ist.

**56.** Wirtszelle oder Verwendung nach einem beliebigen der Ansprüche 45 bis 52, wobei das zweite rekombinante Gen, kodierend ein Protein, das die Sequenz eines Proteins auf Transferrin-Basis umfasst, chromosomal integriert ist.

**57.** Wirtszelle oder Verwendung nach Anspruch 55, wobei das zweite rekombinante Gen, kodierend ein Protein, das die Sequenz eines Proteins auf Transferrin-Basis umfasst, chromosomal in dem Locus eines endogen kodierten PDI-Gens integriert ist.

**58.** Wirtszelle oder Verwendung nach Anspruch 57, wobei das zweite rekombinante Gen, kodierend ein Protein, das die Sequenz eines Proteins auf Transferrin-Basis umfasst, chromosomal in dem Locus eines endogen kodierten PDI-Gens integriert ist, ohne die Expression des endogenen PDI-Gens zu unterbrechen.

**59.** Verfahren zum Herstellen von Nicht-2-$\mu$m-Familien-Plasmidprotein, wobei das Verfahren umfasst:

(a) Bereitstellen einer Wirtszelle, umfassend ein erstes rekombinantes Gen, kodierend ein Protein, das die Sequenz von ORM2 oder einer Variante oder eines Fragments davon, welches die gleiche enzymatische Aktivität wie ORM2 besitzt, umfasst, und ein zweites rekombinantes Gen, kodierend ein Nicht-2-$\mu$m-Familien-Plasmidprotein; und
(b) Kultivieren der Wirtszelle in einem Kulturmedium unter Bedingungen, die die Expression des ersten und zweiten Gens erlauben.

**60.** Verfahren nach Anspruch 59, weiterhin umfassend den Schritt des Formulierens des gereinigten Nicht-2-$\mu$m-Familien-Plasmidproteins mit einem Träger oder Verdünnungsmittel und gegebenenfalls Darbieten des auf diese Weise formulierten Proteins in einer Einheitsdosierungsform.

**61.** Verfahren nach Anspruch 59 oder 60, wobei das erste rekombinante Gen, kodierend ein Protein, das die Sequenz von ORM2 oder einer Variante oder eines Fragments davon, welche(s) die gleiche enzymatische Aktivität wie ORM2 besitzt, umfasst, in das Chromosom der Wirtszelle integriert ist.

**62.** Verfahren nach Anspruch 59 oder 60, wobei das erste rekombinante Gen, kodierend ein Protein, das die Sequenz von ORM2 oder einer Variante oder eines Fragments davon, welche(s) die gleiche enzymatische Aktivität wie ORM2 besitzt, umfasst, auf einem Plasmid vorliegt.

**63.** Wirtszelle umfassend ein erstes rekombinantes Gen, kodierend ein Protein, das die Sequenz von ORM2 oder einer Variante oder eines Fragments davon, welche(s) die gleiche enzymatische Aktivität wie ORM2 besitzt, umfasst, und ein zweites rekombinantes Gen, kodierend ein Nicht-2-$\mu$m-Familien-Plasmidprotein.

**64.** Verwendung eines rekombinanten Gens, kodierend ein Protein, das die Sequenz von ORM2 oder einer Variante oder eines Fragments davon, welche(s) die gleiche enzymatische Aktivität wie ORM2 besitzt, umfasst, um die Expression von Nicht-2-$\mu$m-Familien-Plasmidprotein in einer Wirtszelle zu erhöhen.

**65.** 2-$\mu$m-Familien-Plasmid, umfassend ein erstes rekombinantes Gen, kodierend ein Protein, das die Sequenz von ORM2 oder einer Variante oder eines Fragments davon, welche(s) die gleiche enzymatische Aktivität wie ORM2 besitzt, umfasst, und ein zweites rekombinantes Gen, kodierend ein Nicht-2-$\mu$m-Familien-Plasmidprotein.

**66.** Verfahren, Verwendung, Wirtszelle oder Plasmid nach einem beliebigen der Ansprüche 59 bis 65, wobei das Nicht-2-$\mu$m-Familien-Plasmidprotein wie in einem beliebigen der Ansprüche 17 bis 27 definiert ist.

**67.** Verfahren nach Anspruch 1, weiterhin umfassend den Schritt des Lyophilisierens des auf diese Weise gereinigten Proteins.

**68.** Verfahren nach Anspruch 59, weiterhin umfassend den Schritt des Reinigens des auf diese Weise exprimierten Nicht-2-$\mu$m-Familien-Plasmidproteins aus der kultivierten Wirtszelle oder dem Kulturmedium.

**69.** Verfahren nach Anspruch 68, weiterhin umfassend den Schritt des Lyophilisierens des auf diese Weise gereinigten Proteins.

**70.** Verfahren nach Anspruch 68 oder 69, weiterhin umfassend den Schritt des Formulierens des gereinigten oder lyophilisierten Nicht-2-$\mu$m-Familien-Plasmidproteins mit einem Träger oder Verdünnungsmittel.

**71.** Verfahren nach Anspruch 70, weiterhin umfassend den Schritt des Darbietens des auf diese Weise formulierten Proteins in einer Einheitsdosierungsform.

**Revendications**

**1.** Procédé de production d'une protéine de plasmide n'appartenant pas à la famille de 2 $\mu$m comprenant les étapes consistant à :

> (a) fournir une cellule hôte comprenant un plasmide de la famille de 2 $\mu$m, le plasmide comprenant un gène codant pour une protéine comprenant la séquence d'une protéine chaperon et un gène codant pour une protéine de plasmide n'appartenant pas à la famille de 2 $\mu$m ;
> (b) cultiver la cellule hôte dans un milieu de culture dans des conditions qui permettent la co-expression du gène codant pour une protéine comprenant la séquence de la protéine chaperon et le gène codant pour une protéine de plasmide n'appartenant pas à la famille de 2 $\mu$m ; et
> (c) purifier la protéine de plasmide n'appartenant pas à la famille de 2 $\mu$m ainsi exprimée à partir de la cellule hôte cultivée ou du milieu de culture.

**2.** Procédé selon la revendication 1, comprenant en outre l'étape consistant à formuler la protéine de plasmide n'appartenant pas à la famille de 2 $\mu$m purifiée avec un support ou un diluant, et éventuellement à présenter la protéine ainsi formulée sous la forme d'un dosage unitaire.

**3.** Utilisation d'un plasmide de la famille de 2 $\mu$m en tant que vecteur d'expression pour augmenter la production d'une protéine de plasmide n'appartenant pas à la famille de 2 $\mu$m fongique ou de vertébré en fournissant un gène codant pour la protéine de plasmide n'appartenant pas à la famille de 2 $\mu$m et un gène codant pour une protéine chaperon sur le même plasmide de la famille de 2 $\mu$m.

**4.** Utilisation selon la revendication 3, dans laquelle la protéine fongique de plasmide n'appartenant pas à la famille de 2 $\mu$m est une protéine de levure de plasmide n'appartenant pas à la famille de 2 $\mu$m.

**5.** Plasmide de la famille de 2 $\mu$m comprenant un gène codant pour une protéine comprenant la séquence d'une protéine chaperon et un gène codant pour une protéine de plasmide n'appartenant pas à la famille de 2 $\mu$m, dans lequel si le plasmide est basé sur le plasmide de 2 $\mu$m alors c'est un vecteur de désintégration.

**6.** Procédé, utilisation ou plasmide selon l'une des revendications précédentes, dans lequel/laquelle le chaperon a une séquence d'un chaperon fongique ou d'un chaperon de mammifère.

**7.** Procédé, utilisation ou plasmide selon la revendication 6, dans lequel/laquelle le chaperon a une séquence d'un chaperon de levure.

**8.** Procédé, utilisation ou plasmide selon la revendication 6, dans lequel/laquelle le chaperon a une séquence d'un chaperon humain.

**9.** Procédé, utilisation ou plasmide selon l'une des revendications précédentes, dans lequel/laquelle le chaperon comprend la séquence d'une protéine codée par l'un quelconque de *AHA1, CCT2, CCT3, CCT4, CCT5, CCT6, CCT7, CCT8, CNS1, CPR3, CPR6, EPS1, ERO1, EUG1, FMO1, HCH1, HSP10, HSP12, HSP104, HSP26, HSP30, HSP42, HSP60, HSP78, HSP82, JEMI, MDJ1, MDJ2, MPD1, MPD2, PDI1, PFD1, ABC1, APJ1, ATP11, ATP12,*

EP 1 709 181 B1

*BTT1, CDC37, CPR7, HSC82, KAR2, LHS1, MGE1, MRS11, NOB1, ECM10, SSA1, SSA2, SSA3, SSA4, SSC1, SSE2, SIL1, SLS1, UBI4, ORM1, ORM2, PER1, PTC2, PSEI* et *HAC1* ou *HAC1* tronqué sans intron.

10. Procédé, utilisation ou plasmide selon l'une des revendications précédentes, dans lequel/laquelle le chaperon est la disulfure-isomérase protéique, ou comprend la séquence d'une protéine codée par *PSE1, ORM2* ou *SSA1* ou un variant ou un fragment de ceux-ci, lequel variant ou fragment possède la même activité enzymatique.

11. Procédé selon l'une quelconque des revendications 1, 2 ou 5 à 10 dans lequel la cellule hôte exprime également un second gène recombinant codant pour un chaperon qui est différent du premier chaperon codé par le plasmide.

12. Procédé selon la revendication 11, dans lequel le second gène recombinant codant pour un chaperon est intégré dans des chromosomes.

13. Procédé, utilisation ou plasmide selon l'une quelconque des revendications 1 à 11, dans lequel/laquelle le plasmide comprend deux gènes différents codant pour des chaperons différents, l'un desdits gènes est le second gène recombinant codant pour un chaperon tel que défini dans la revendication 11.

14. Procédé, utilisation ou plasmide selon l'une quelconque des revendications 11 à 13, dans lequel/laquelle l'un des chaperons est la disulfure-isomérase protéique.

15. Procédé, utilisation ou plasmide selon l'une quelconque des revendications 11 à 14, dans lequel/laquelle l'un des chaperons est *ORM2.*

16. Procédé, utilisation ou plasmide selon la revendication 11 à 15, dans lequel/laquelle les deux chaperons sont la disulfure-isomérase protéique et *ORM2.*

17. Procédé, utilisation ou plasmide selon l'une des revendications précédentes, dans lequel/laquelle la protéine de plasmide n'appartenant pas à la famille de 2 $\mu$m comprend une séquence de tête efficace pour causer une sécrétion dans la levure.

18. Procédé, utilisation ou plasmide selon l'une des revendications précédentes, dans lequel/laquelle la protéine de plasmide n'appartenant pas à la famille de 2 $\mu$m est une protéine eucaryote, ou un fragment ou une variante de celle-ci, de préférence une protéine de vertébré ou fongique.

19. Procédé, utilisation ou plasmide selon la revendication 18, dans lequel/laquelle la protéine de plasmide n'appartenant pas à la famille de 2 $\mu$m est une protéine de levure.

20. Procédé, utilisation ou plasmide selon l'une des revendications précédentes, dans lequel/laquelle la protéine de plasmide n'appartenant pas à la famille de 2 $\mu$m est une protéine utilisable commercialement

21. Procédé, utilisation ou plasmide selon l'une des revendications précédentes, dans lequel/laquelle la protéine de plasmide n'appartenant pas à la famille de 2 $\mu$m comprend une séquence choisie parmi l'albumine, un anticorps monoclonal, un étoposide, une protéine de sérum, un facteur de coagulation du sang, l'antistasine, un peptide anticoagulant de la tique, la transferrine, la lactoferrine, l'endostatine, l'angiostatine, le collagène, une immunoglobuline ou un fragment de celle-ci, une molécule à base d'immunoglobuline ou une fragment de celle-ci, une protéine contenant un domaine Kunitz, des intérferons, des interleukines, IL10, IL11, IL2, les espèces et sous-espèces d'interféron $\alpha$, les espèces et sous-espèces d'interféron $\beta$, les espèces et sous-espèces d'interféron $\gamma$, la leptine, CNTF, CNTF $_{Ax15}$', l'antagoniste du récepteur de l'IL1, l'érythropoïétine et les mimétiques de l'érythropoïétine, la thrombopoïétine et les mimétiques de la thrombopoïétine, la Prosaptide, la cyanovirine-N, la 5-Hélix, le peptide T20, le peptide T1249, la gp41 du VIH, la gp120 du VIH, l'urokinase, la prourokinase, le tPA, l'hirudine, le facteur de croissance dérivé des plaquettes, l'hormone parathyroïdienne, la pro-insuline, l'insuline, le glucagon, les peptides de type glucagon, le facteur de croissance de type insuline, la calcitonine, l'hormone de croissance, le facteur de croissance transformant $\beta$, un facteur onconécrosant, le G-CSF, le GM-CSF, le M-CSF, le FGF, les facteurs de coagulation sous forme préactive et active, comprenant, mais de façon non exhaustive, le plasminogène, le fibrinogène, la thrombine, la pré-thrombine, la pro-thrombine, le facteur de von Willebrand, l'$\alpha_1$-antitrypsine, les activateurs du plasminogène, le Facteur VIL le Facteur VIII, le Facteur IX, le Facteur X et le Facteur XIII, le facteur de croissance des nerfs, le LACI, le facteur de croissance des cellules endothéliales dérivé des plaquettes, la glucose oxydase, la cholinestérase du sérum, l'aprotinine, le précurseur du peptide amyloïde, l'inhibiteur de l'inter-alpha trypsine, l'antithrombine III, les espèces d'apo-lipoprotéines, la Protéine C, la Protéine S, ou un variant ou fragment de l'un quelconque de ceux-ci.

22. Procédé, utilisation ou plasmide selon l'une des revendications précédentes, dans lequel/laquelle la protéine de plasmide n'appartenant pas à la famille de 2 μm comprend la séquence d'une molécule à base d'immunoglobuline ou un fragment de celle-ci, dans lequel/laquelle la molécule à base d'immunoglobuline est choisie parmi dAb, un fragment Fab', F(ab')$_2$, scAb, scFv ou un fragment scFv.

23. Procédé, utilisation ou plasmide selon l'une des revendications précédentes, dans lequel/laquelle la protéine de plasmide n'appartenant pas à la famille de 2 μm comprend la séquence d'albumine ou un variant ou fragment de celle-ci.

24. Procédé, utilisation ou plasmide selon l'une des revendications précédentes, dans lequel/laquelle la protéine de plasmide n'appartenant pas à la famille de 2 μm comprend la séquence d'un membre de la famille des transferrines, ou un variant ou fragment de celui-ci.

25. Procédé, utilisation ou plasmide selon la revendication 24, dans lequel/laquelle la protéine de plasmide n'appartenant pas à la famille de 2 μm comprend la séquence de transferrine ou de lactoferrine, ou un variant ou fragment de celles-ci.

26. Procédé, utilisation ou plasmide selon l'une des revendications précédentes, dans lequel/laquelle la protéine de plasmide n'appartenant pas à la famille de 2 μm comprend une protéine de fusion.

27. Procédé, utilisation ou plasmide selon la revendication 26, dans lequel/laquelle la protéine de fusion est une protéine de fusion d'albumine ou d'un membre de la famille des transferrines, ou un variante ou fragment de celle-ci/celui-ci, fusionné(e) directement ou indirectement à la séquence d'une autre protéine.

28. Cellule hôte comprenant un plasmide tel que défini dans l'une des revendications précédentes.

29. Cellule hôte selon la revendication 28, dans laquelle un chaperon codé par le plasmide est un gène essentiel.

30. Cellule hôte selon la revendication 29, dans laquelle, en l'absence du plasmide, la cellule hôte ne produit pas le chaperon.

31. Cellule hôte selon l'une quelconque des revendications 28 à 30, qui est une cellule de levure.

32. Cellule hôte selon la revendication 31, dans laquelle le plasmide est basé sur pSR1, pSB3 ou pSB4 et la cellule de levure est *Zygosaccharomyces rouxii,* le plasmide est basé sur pSB1 ou pSB2 et la cellule de levure est *Zygosaccharomyces bailli,* le plasmide est basé sur pSM1 et la cellule de levure est *Zygosaccharomyces fermentati,* le plasmide est basé sur pKD1 et la cellule de levure est *Kluyveromyces drosophilarum,* ou le plasmide est basé sur le plasmide de 2 μm et la cellule de levure est *Saccharomyces cerevisiae* ou *Saccharomyces carlsbergensis.*

33. Cellule hôte selon la revendication 32, dans laquelle le plasmide est basé sur le plasmide de 2 μm et la cellule de levure est *Saccharomyces cerevisiae* ou *Saccharomyces carlsbergensis.*

34. Procédé selon la revendication 1, dans lequel la cellule hôte est une cellule hôte telle que définie dans l'une quelconque des revendications 28 à 33.

35. Procédé selon la revendication 34, dans lequel la cellule hôte est une cellule hôte telle que définie dans la revendication 30, ou toute autre revendication dépendant de celle-ci.

36. Procédé selon la revendication 34, dans lequel l'étape (b) consiste à cultiver la cellule hôte dans des milieux non sélectifs, tels que des milieux riches.

37. Procédé de production d'une protéine de plasmide n'appartenant pas à la famille de 2 μm comprenant les étapes consistant à :

    (a) fournir une cellule hôte comprenant un premier gène recombinant codant pour une protéine comprenant la séquence d'une première protéine chaperon, un second gène recombinant codant pour une protéine comprenant la séquence d'une seconde protéine chaperon, et un troisième gène recombinant codant pour une protéine de plasmide n'appartenant pas à la famille de 2 $\mu$m, dans lequel les premier et second chaperons sont différents ;

(b) cultiver la cellule hôte dans un milieu de culture dans des conditions qui permettent l'expression des premier, second et troisième gènes ; et

(c) éventuellement purifier la protéine de plasmide n'appartenant pas à la famille de 2 $\mu$m ainsi exprimée à partir de la cellule hôte cultivée ou du milieu de culture; et

(d) éventuellement, lyophiliser la protéine ainsi purifiée.

**38.** Procédé selon la revendication 37, comprenant en outre l'étape consistant à formuler la protéine de plasmide n'appartenant pas à la famille de 2 $\mu$m purifiée avec $\mu$m support ou un diluant, et éventuellement à présenter la protéine ainsi formulée sous forme de dosage unitaire.

**39.** Procédé selon la revendication 37 ou 38, dans lequel les premier et second chaperons comprennent la séquence d'une protéine codée par l'un quelconque de *AHA1*, *CCT2*, *CCT3*, *CCT4*, *CCT5*, *CCT6*, *CCT7*, *CCT8*, *CNS1*, *CPR3*, *CPR6*, *EPS1*, *ERO1*, *EUG1*, *FMO1*, *HCH1*, *HSP10*, *HSP12*, *HSP104*, *HSP26*, *HSP30*, *HSP42*. *HSP60*, *HSP78*, *HSP82*, *JEM1*, *MDJ1*, *MDJ2*, *MPD1*, *MPD2*, *PD11*, *PFD1*, *ABC1*, *APJ1*, *ATP11*. *ATP12*, *BTT1*, *CDC37*, *CPR7*, *HSC82*, *KAR2*, *LHS1*, *MGE1*, *MRS11*, *NOB1*, *ECM10*, *SSA1*, *SSA2*, *SSA3*, *SSA4*, *SSC1*, *SSE2*, *SIL1*, *SLS1*, *UB14*, *ORM1*, *ORM2*, *PER1*, *PTC2*, *PSE1* et *HAC1* ou *HAC1* tronqué sans intron.

**40.** Procédé selon l'une quelconque des revendications 37 à 39, dans lequel le premier chaperon est la disulfure-isomérase protéique.

**41.** Procédé selon l'une quelconque des revendications 38 à 40, dans lequel le second chaperon est ORM2.

**42.** Procédé selon l'une quelconque des revendications 37 à 41, dans lequel au moins un des premier et second chaperons est codé par un gène recombinant intégré dans des chromosomes.

**43.** Procédé selon l'une quelconque des revendications 37 à 42, dans lequel au moins un des premier et second chaperons est codé par un gène sur un plasmide.

**44.** Procédé selon la revendication 43, dans lequel le plasmide est un plasmide tel que défini dans l'une quelconque des revendications 1 à 33.

**45.** Cellule hôte comprenant un premier gène recombinant codant pour une protéine comprenant la séquence de disulfure-isomérase protéique (PDI) et un second gène recombinant codant pour une protéine comprenant la séquence d'une protéine à base de transferrine.

**46.** Utilisation d'un gène recombinant codant pour une protéine comprenant la séquence de disulfure-isomérase protéique (PDI) pour augmenter l'expression d'une protéine à base de transferrine.

**47.** Cellule hôte selon la revendication 45 ou utilisation selon la revendication 46, dans laquelle la protéine à base de transferrine comprend la séquence de transferrine ou de tout autre élément de la famille des transferrines, un variant ou fragment de celle-ci/celui-ci ou une protéine de fusion comprenant de la transferrine, un variant ou fragment de celle-ci.

**48.** Cellule hôte ou utilisation selon la revendication 47, dans laquelle la protéine à base de transferrine comprend la séquence de lactoferrine, ou un variant ou fragment de celle-ci.

**49.** Cellule hôte ou Utilisation selon l'une quelconque des revendications 45 à 48, dans laquelle le premier gène recombinant codant pour une protéine comprenant la séquence de disulfure-isomérase protéique (PDI) est fourni sur un plasmide,

**50.** Cellule hôte ou utilisation selon la revendication 49, dans laquelle le plasmide est un plasmide de la famille de 2 $\mu$m.

**51.** Cellule hôte ou utilisation selon l'une quelconque des revendications 45 à 48, dans laquelle le premier gène recombinant codant pour une protéine comprenant la séquence de disulfure-isomérase protéique (PDI) est intégré dans des chromosomes.

**52.** Cellule hôte ou utilisation selon la revendication 51, dans laquelle le premier gène recombinant codant pour une protéine comprenant la séquence de disulfure-isomérase protéique (PDI) est intégré dans des chromosomes au locus d'un gène de PDI codé de façon endogène.

**53.** Cellule hôte ou utilisation selon la revendication 52, dans laquelle le premier gène recombinant codant pour une protéine comprenant la séquence de disulfure-isomérase protéique (PDI) est intégré dans des chromosomes au locus d'un gène de PDI codé de façon endogène sans interrompre l'expression du gène de PDI endogène.

**54.** Cellule hôte ou utilisation selon l'une quelconque des revendications 45 à 53, dans laquelle le second gène recombinant codant pour une protéine comprenant la séquence d'une protéine à base de transferrine est fourni sur un plasmide.

**55.** Cellule hôte ou utilisation selon la revendication 54, dans laquelle le plasmide est un plasmide de la famille de 2 $\mu$m.

**56.** Cellule hôte ou utilisation selon l'une quelconque des revendications 45 à 52, dans laquelle le second gène recombinant codant pour une protéine comprenant la séquence d'une protéine à base de transferrine est intégré dans des chromosomes.

**57.** Cellule hôte ou utilisation selon la revendication 55, dans laquelle le second gène recombinant codant pour une protéine comprenant la séquence d'une protéine à base de transferrine est intégré dans des chromosomes au locus d'un gène de PDI codé de façon endogène.

**58.** Cellule hôte ou utilisation selon la revendication 57, dans laquelle le second gène recombinant codant pour une protéine comprenant la séquence d'une protéine à base de transferrine est intégré dans des chromosomes au locus d'un gène de PDI codé de façon endogène sans interrompre l'expression du gène de PDI endogène.

**59.** Procédé de production d'une protéine de plasmide n'appartenant pas à la famille de 2 $\mu$m comprenant les étapes consistant à :

   (a) fournir une cellule hôte comprenant un premier gène recombinant codant pour une protéine comprenant la séquence de ORM2, ou un variant ou fragment de celui-ci qui possède la même activité enzymatique que ORM2, et un second gène recombinant codant pour une protéine de plasmide n'appartenant pas à la famille de 2 $\mu$m ; et
   (b) cultiver la cellule hôte dans un milieu de culture dans des conditions qui permettent l'expression des premier et second gènes.

**60.** Procédé selon la revendication 59, comprenant en outre l'étape consistant à formuler la protéine de plasmide n'appartenant pas à la famille de 2 $\mu$m purifiée avec un support ou un diluant, et éventuellement à présenter la protéine ainsi formulée sous la forme d'un dosage unitaire.

**61.** Procédé selon la revendication 59 ou 60, dans lequel le premier gène recombinant codant pour une protéine comprenant la séquence de ORM2, ou un variant ou fragment de celui-ci qui possède la même activité enzymatique que ORM2, est intégré dans le chromosome de la cellule hôte.

**62.** Procédé selon la revendication 59 ou 60, dans lequel le premier gène recombinant codant pour une protéine comprenant la séquence de ORM2, ou un variant ou fragment de celui-ci qui possède la même activité enzymatique que ORM2, est situé sur un plasmide.

**63.** Cellule hôte comprenant un premier gène recombinant codant pour une protéine comprenant la séquence de ORM2, ou un variant ou un fragment de celui-ci qui possède la même activité enzymatique que ORM2, et une second gène recombinant codant pour une protéine de plasmide n'appartenant pas à la famille de 2 $\mu$m.

**64.** Utilisation d'un gène recombinant codant pour une protéine comprenant la séquence de ORM2, ou un variant ou fragment de celui-ci qui possède la même activité enzymatique que ORM2, pour augmenter l'expression d'une protéine de plasmide n'appartenant pas à la famille de 2 $\mu$m, dans une cellule hôte.

**65.** Plasmide de la famille de 2 $\mu$m comprenant un premier gène recombinant codant pour une protéine comprenant la séquence de ORM2, ou un variant ou fragment de celui-ci qui possède la même activité enzymatique que ORM2, et un second gène recombinant codant pour une protéine de plasmide n'appartenant pas à la famille de 2 $\mu$m.

66. Procédé, utilisation, cellule hôte ou plasmide selon l'une quelconque des revendications 59 à 65, dans lequel/laquelle la protéine de plasmide n'appartenant pas à la famille de 2 $\mu$m est telle que définie dans l'une quelconque des revendications 17 à 27.

67. Procédé selon la revendication 1, comprenant en outre l'étape consistant à lyophiliser la protéine ainsi purifiée.

68. Procédé selon la revendication 59, comprenant en outre l'étape consistant à purifier la protéine de plasmide n'appartenant pas à la famille de 2 $\mu$m ainsi exprimée à partir de la cellule hôte cultivée ou du milieu de culture.

69. Procédé selon la revendication 68, comprenant en outre l'étape consistant à lyophiliser la protéine ainsi purifiée.

70. Procédé selon la revendication 68 ou 69, comprenant en outre l'étape consistant à formuler la protéine de plasmide n'appartenant pas à la famille de 2 $\mu$m purifiée ou lyophilisée avec un support ou un diluant.

71. Procédé selon la revendication 70, comprenant en outre l'étape consistant à présenter la protéine ainsi formulée sous forme de dosage unitaire.

EP 1 709 181 B1

Figure 2

Figure 1

68

## Figure 3

**A) Restriction Endonuclease Sites used for DNA Insertions in *FLP* and the *FLP* Inverted**

*Bcl*I  *Xcm*I  *Fsp*I

FRT

*FLP* Coding Sequence (1272 bp)  Inverted Repeat (599 bp)

**B) Flp Protein (423 amino acid residues)**

Tyr343

**C) Truncated FLP Protein Products**

Flp (1-353) from termination at *Bcl*I-site

Flp (1-382) from termination at *Xcm*I-

Figure 4

Figure 5

## Figure 6

pDB2690
13018 bps

NotI
XbaI
XcmI
IR'
LEU2
FspI
FspI
AmpR
FspI
XbaI
FspI
PstI
D
'IR
XcmI
BclI
HindIII
HindIII
FLP
REP1
REP2
IR'
'IR
PDI1
HindIII
XbaI
FspI
XcmI
SnaBI
BclI
HindIII
ApaI
XcmI
SnaBI
PacI
FseI
SfiI
HindIII
BclI

## Figure 7

mFL = modified HSA(pre)/MFα1(pro) fusion leader sequence

pDB2711
16292 bps

NotI
PstI
HindIII
XbaI
BglII
NdeI
BamHI
PstI
HindIII
XbaI
BglII
NdeI
PRBIp
LEU2
mFL
Modified Tf
PstI
StuI
HindIII
NdeI
HindIII
SphI
NotI
PstI
StuI
HindIII
mADHIt
IR'
XbaI
StuI
D
REP1
IR'
HindIII
XbaI
AmpR
BclI
StuI
BglII
PDI1
'IR
REP2
FLP
'IR
NdeI
XbaI
NdeI
BclI
HindIII
HindIII
BclI
HindIII
SfiI
FseI
PacI
ApaI
SphI

EP 1 709 181 B1

**Figure 9**

mHSA-pre = modified HSA-pre leader sequence

**Figure 8**

mFL = modified HSA(pre)/MFα1(pro) fusion leader sequence

Figure 10

Figure 11

EP 1 709 181 B1

73

Figure 13

pDB2930
16274 bps

Figure 12

pDB2929
16274 bps

Figure 14

Figure 15

Figure 14

Figure 15

EP 1 709 181 B1

**Figure 16**

Figure 17

## Figure 18

Figure 19

hTf Stds          1     2     3     1     2     3

                  Strain A          Control Strain

Figure 20

Figure 21

**Figure 22**

**Figure 23**

**Figure 24**

**Figure 25**

EP 1 709 181 B1

≈ Mol. Wt.

1  2  3  4  5  6

97kDa

64kDa

51kDa

39kDa

28kDa

pDB2712
16292 bps
Tf (N413Q, N611Q)

EP 1 709 181 B1

**Figure 26**

50 20 10 5 2    pDB2931 pDB2929    pDB2931 pDB2929    pDB2931 pDB2929    pDB2931 pDB2929

Plasma Tf    JRY188    MT302/28B    S150-2B    CB11-63

Figure 27

EP 1 709 181 B1

Figure 28

Figure 29

EP 1 709 181 B1

Figure 30

EP 1 709 181 B1

Figure 31

Figure 32

EP 1 709 181 B1

Figure 33

Figure 34

EP 1 709 181 B1

FIGURE 36

FIGURE 35

91

FIGURE 38

FIGURE 37

FIGURE 40

FIGURE 39

FIGURE 42

FIGURE 41

**FIGURE 44**

**FIGURE 43**

EP 1 709 181 B1

FIGURE 46

FIGURE 45

96

FIGURE 47

pDB3081
13018 bps

FIGURE 48

pDB3083
12948 bps

**FIGURE 50**

**FIGURE 49**

FIGURE 51

FIGURE 52

pDB3086
16204 bps

pDB3087
16144 bps

EP 1 709 181 B1

**FIGURE 53**

FIGURE 54

**FIGURE 55**

EP 1 709 181 B1

EP 1 709 181 B1

**Figure 56**

| 100 50 25 20 15 10 5 | pDB2931 | pDB2929 | pDB3085 | pDB3086 | pDB3087 | pDB2929 | 5 10 15 20 25 50 100 |

Plasma Tf Standards    pDB2931    pDB2929    pDB3085    pDB3086    pDB3087    pDB2929    Plasma Tf Standards

**FIGURE 58**

**FIGURE 57**

FIGURE 59

FIGURE 60

pDB2765
14809

pDB3107
16790

EP 1 709 181 B1

**FIGURE 62**

pDB3104
16292

**FIGURE 61**

pDB2773
14311

FIGURE 64

FIGURE 63

FIGURE 65

FIGURE 66

FIGURE 65

pDB2679
14251

Not I
Sma I
Sma I
Pst I
Hin dIII
Bam HI
Eco RI
Eco RV
Eco RI
PRB1p
Fusion Leader
DPI-14
GS Linker
LEU2
rHA
Eco RI
Eco RI
Sal 1
Eco RI
Eco RV
Hin dIII
Sph I
Not I
mADH1t
Pst I
Eco RI
Hin dIII
RAF1
REP1
HindIII
REP2
FLP
amp
Nco I
Eco RV
Sph I
Eco RV
Hin dIII
Eco RI
Eco RI

FIGURE 66

pDB3103
16232

Not I
Sma I
Sma I
Pst I
HindIII
Bam HI
Eco RI
Eco RV
Eco RI
Eco RI
Eco RI
PRB1p
Fusion Leader
DPI-14
GS Linker
LEU2
rHA
Sal 1
Eco RI
Eco RV
HindIII
Sph I
Not I
Pst I
Eco RI
HindIII
D
REP1
mADH1t
IR'
Hin dIII
IR'
AmpR
Sna BI
Sal I
Nco I
PDI1
'IR
REP2
FLP
'IR
Eco RI
Eco RV
Nco I
Hin dIII
Nco I
Hin dIII
Nco I
Sfi I
Fse I
Pac I
Sna BI
Nco I
Hin dIII
Eco RI
Eco RV
Sph I
Eco RV

EP 1 709 181 B1

**FIGURE 68**

pDB3106
16604

**FIGURE 67**

pDB2618
14623

FIGURE 69

FIGURE 70

pDB2620
6715 bps

pDB2621
14551

EP 1 709 181 B1

FIGURE 71

FIGURE 72

FIGURE 73

**FIGURE 74**

FIGURE 75

FIGURE 77

pDB3090-pDB3093
15504 bps

pDB2848
6782 bps

EP 1 709 181 B1

Figure 76

**FIGURE 79**

pDB3098
18414 bps

**FIGURE 78**

pDB3097
18414 bps

Figure 80

FIGURE 82

FIGURE 81

**FIGURE 85**

pMCS5
3081 bps

MCS

Avr II
Hin dIII
Sph I
Pst I
Bsp MI
Acc I
Hin cII
Sal I
Xba I
Bam HI
Pml I
Srf I++
Mun I

Nru I
Xho I
Asp 718I
Kpn I
Sac I
Eco RI
Not I
Sac II
Sfi I
Nde I
Age I
Fse I
Nae I
Bgl II
Nhe I
Spe I
Nar I
Afl III
Eco RV
Nsi I
Hin cII
Hpa I
Cla I
Nco I
Asc I
Bss HII
Pac I
Swa I

Ssp I
Ssp I
Dra III
Sca I
AmpR
Sap I

**FIGURE 83**

pDB3095
17058 bps

Figure 84

**FIGURE 87**

pDB3069
3519 bps

Swa I, Pac I, Asc I, Nco I, Cla I, Hpa I, Hin cII, Nsi I, Eco RV, Mlu I, Afl III, Nar I, Spe I, Nhe I, Bgl II, Fse I, Sgr AI, Age I, Sfi I, Nde I, Ban II, Not I

Hin cII, Afl III, Afl III, Hin dIII, Eco NI, Eco RV, Pst I, Sph I, Nhe I

MCS, YCL042W, YCL041C, YCL044c', MCS

Afl III, Ssp I, Ssp I, AmpR, Sca I

**FIGURE 86**

pDB2964
3085 bps

Age I, Fse I, Bgl II, Nhe I, Spe I, Nar I, Mlu I, Eco RV, Nsi I, Hin cII, Hpa I, Cla I, Nco I, Asc I, Bss HII, Pac I, Swa I

Avr II, Rsr II, Nhe I, Sph I, Pst I, Bsp MI, Acc I, Hin cII, Sal I, Xba I, Bam HI, Pml I, Srf I ++, Mun I ++, Nde I

MCS

Sap I, Ssp I, Ssp I, Dra III, Ssp I, AmpR, Sca I

EP 1 709 181 B1

**FIGURE 88**

**FIGURE 89**

pDB2778
3940 bps

AmpR

TRP1

MCS

'MCS

Ssp I
Ssp I
Ssp I
Sca I
Afl III
Sap I

Avr II
Bln I
Rsr II
Hin dIII
Ava I
Ban II
Eco RV
Mun I
Acc I
Pml I
Hin dIII
Sph I
Pst I
Acc I
Hin cII
Sal I
Xba I
Bam HI
Pml I
Srf I
Ava I
Sma I
Pme I
Mun I

Nru I
Ava I
Xho I
Asp 718I
Kpn I
Ban II
Sac I
Eco RI
Not I
Xma III
Sac II
Ban II
Sfi I
Nde I
Sgr AI
Age I
Fse I
Bgl II
Nhe I
Spe I
Nar I
Afl III
Mlu I
Eco RV
Nsi I
Hin cII
Hpa I
Cla I
Nco I
Asc I
Pac I
Swa I

pDB3078
4378 bps

AmpR

TRP1

MCS
YCL042W
'YCL041C

YCL044c'
MCS

Afl III
Hin cII
Afl III
Afl III
Hin dIII
Ava I
Eco RV
Mun I
Acc I
Hin dIII
Eco RV
Pst I
Sph I
Nhe I
Sca I
Ssp I
Ssp I

Swa I
Pac I
Asc I
Bss HII
Nco I
Cla I
Hin cII
Hpa I
Nsi I
Eco RV
Afl III
Mlu I
Nar I
Spe I
Nhe I
Bgl II
Fse I
Sgr AI
Age I
Sfi I
Nde I
Not I

FIGURE 91

FIGURE 90

**FIGURE 92**

100 200 300

rHA Stds

*pdi1::TRP1*

DXY1 *trp1Δ*
[pDB2976]

*pdi1::TRP1*

DXY1 *trp1Δ*
[pDB2978]

*pdi1::TRP1*

DXY1 *trp1Δ*
[pDB2980]

*pdi1::TRP1*

DXY1 *trp1Δ*
[pDB2977]

*pdi1::TRP1*

DXY1 *trp1Δ*
[pDB2979]

*pdi1::TRP1*

DXY1 *trp1Δ*
[pDB2981]

300 200 100

rHA Stds

**FIGURE 93**

# Figure 94A

Alignment Workspace of ExL.meg J. Hein (Weighted)
20 December 2004 15:04

CTAGTCTCTTTTTCCAATTTCCACCGTGTAGCATTTTGTTGTGCTGTTACAACCACAACAAAACGAAAAACCCGTATGGACATACATATATATATATATATATATATATATA

| | | | | | | | | | | |
10 20 30 40 50 60 70 80 90 100 110

S288c long  CTAGTCTCTTTTTCCAATTTCCACCGTGTAGCATTTTGTTGTGCTGTTACAACCACAACAAAACGAAAAACCCGTATGGACATACATATATATATATATATATATATATATATATA 110
SKQ2n long  CTAGTCTCTTTTTCCAATTTCCACCGTGTAGCATTTTGTTGTGCTGTTACAACCACAACAAAACGAAAAACCCGTATGGACATACATATATATATATATATATATATATATATATA 110

TATATTTTGTTACGCGTGCATTTTCTTGTTGCAAGCAGCATGTCTAATTGGTAATTTTAAAGCTGCCAAGCTCTACATAAAGAAAAACATACATCTATCCCGTTATGAAG

| | | | | | | | | | |
120 130 140 150 160 170 180 190 200 210 220

S288c long  -----TTTTGTTACGCGTGCATTTTCTTGTTGCAAGCAGCATGTCTAATTGGTAATTTTAAAGCTGCCAAGCTCTACATAAAGAAAAACATACATCTATCCCGTTATGAAG 216
SKQ2n long  TATATTTTGTTACGCGTGCATTTTCTTGTTGCAAGCAGCATGTCTAATTGGTAATTTTAAAGCTGCCAAGCTCTACATAAAGAAAAACATACATCTATCCCGTTATGAAG 220

TTTTCTGCTGGTGCCGTCCTGTCATGGTCCTCCCTGCTGCTCGCCTCCTCTCGTTTTCGCCCAACAAGAGGCTGTGGCCCCTGAAGACTCCGCTGTCGTTAAGTTGGCCAC

| | | | | | | | | | |
230 240 250 260 270 280 290 300 310 320 330

S288c long  TTTTCTGCTGGTGCCGTCCTGTCATGGTCCTCCCTGCTGCTCGCCTCCTCTCGTTTTCGCCCAACAAGAGGCTGTGGCCCCTGAAGACTCCGCTGTCGTTAAGTTGGCCAC 326
SKQ2n long  TTTTCTGCTGGTGCCGTCCTGTCATGGTCCTCCCTGCTGCTCGCCTCCTCTCGTTTTCGCCCAACAAGAGGCTGTGGCCCCTGAAGACTCCGCTGTCGTTAAGTTGGCCAC 330

CGACTCTTTCAATGAGTACATTCAGTCGCACGACTTGGTGCTTGCGGAGTTTTTTGCTCCATGGTGTGGCCACTGTAAGAACATGGCTCCTGAATACGTTAAAGCCGCCG

| | | | | | | | | | |
340 350 360 370 380 390 400 410 420 430 440

S288c long  CGACTCCTTCAATGAGTACATTCAGTCGCACGACTTGGTGCTTGCGGAGTTTTTTGCTCCATGGTGTGGCCACTGTAAGAACATGGCTCCTGAATACGTTAAAGCCGCCG 436
SKQ2n long  CGACTCTTTCAATGAATACATTCAGTCGCACGACTTGGTGCTTGCGGAGTTTTTTGCTCCATGGTGTGGCCACTGTAAGAACATGGCTCCTGAATACGTTAAAGCCGCCG 440

AGACTTTAGTTGAGAAAAACATTACCTTGGCCCAGATCGACTGTACTGAAAACCAGGATCTGTGTATGGAACACAACATTCCAGGGTTCCCAAGCTTGAAGATTTTCAAA

| | | | | | | | | | |
450 460 470 480 490 500 510 520 530 540 550

S288c long  AGACTTTAGTTGAGAAAAACATTACCTTGGCCCAGATCGACTGTACTGAAAACCAGGATCTGTGTATGGAACACAACATTCCAGGGTTCCCAAGCTTGAAGATTTTCAAA 546
SKQ2n long  AGACTTTAGTTGAGAAAAACATTACCTTGGCCCAGATCGACTGTACTGAAAACCAGGATCTGTGTATGGAACACAACATTCCAGGGTTCCCAAGCTTGAAGATTTTCAAA 550

AACAGCGGATGTTAACAACTCGATCGATTACGAGGGACCTAGAACTGCCGAGGCCATTGTCCAATTCATGATCAAGCAAAGCCAACCGGCTGTCGCCCGTTGTTGCTGATCT

| | | | | | | | | | |
560 570 580 590 600 610 620 630 640 650 660

S288c long  AACAGCGGATGTTAACAACTCGATCGATTACGAGGGACCTAGAACTGCCGAGGCCATTGTCCAATTCATGATCAAGCAAAGCCAACCGGCTGTCGCCCGTTGTTGCTGATCT 656
SKQ2n long  AACAGCGGATGTTAACAACTCGATCGATTACGAGGGACCTAGAACTGCCGAGGCCATTGTCCAATTCATGATCAAGCAAAGCCAACCGGCTGTCGCCCGTTGTTGCTGATCT 660

EP 1 709 181 B1

# Figure 94B

Alignment Workspace of Ext_meg J. Hein (Weighted)
20 December 2004 15:04

Page 2

s288c long
SKQ2n long

s288c long
SKQ2n long

s288c long
SKQ2n long

s288c long
SKQ2n long

s288c long
SKQ2n long

s288c long
SKQ2n long

s288c long
SKQ2n long

# Figure 94C



GATCTTCGAGAACCAAGATTCCTCTGTCTTCCAATTGGTCGGTAAGAACCATGACGAAATCGTCAACGACCCAAAGAAGGACGTTCTTGTTTTGTACTATCCCCCATGGT

1330 1340 1350 1360 1370 1380 1390 1400 1410 1420 1430

S288c long GATCTTCGAGAACCAAGATTCCTCTGTCTTCCAATTGGTCGGTAAGAACCATGACGAAATCGTCAACGACCCAAAGAAGGACGTTCTTGTTTTGTACTATCCCCCATGGT

SKQ2n long GATCTTCGAGAACCAAGATTCCTCTGTCTTCCAATTGGTCGGTAAGAACCATGACGAAATCGTCAACGACCCAAAGAAGGACGTTCTTGTTTTGTACTATCCCCCATGGT 142(

1431(

GTGGTCACTGTAAGAGATTGGCCCCAACTTACCAAGAACTAGCTGATACCTACGCCAACGCCACATCCGACGTTTTGATTGCTAAACTAGACCACACTGAAAACGATGTC

1440 1450 1460 1470 1480 1490 1500 1510 1520 1530 1540

S288c long GTGGTCACTGTAAGAGATTGGCCCCAACTTACCAAGAACTAGCTGATACCTACGCCAACGCCACATCCGACGTTTTGATTGCTAAACTAGACCACACTGAAAACGATGTC 153(

SKQ2n long GTGGTCACTGTAAGAGATTGGCCCCAACTTACCAAGAACTAGCTGATACCTACGCCAACGCCACATCCGACGTTTTGATTGCTAAACTAGACCACACTGAAAACGATGTC 154(

AGAGGCGTCGTAATTGAAGGTTACCCAACAATCGTCTTATACCCAGGTGGTAAGAAGTCCGAATCTGTTGTGTACCAAGGTTCAAGATCCTTGGACTCTTTATTCGACTT

1550 1560 1570 1580 1590 1600 1610 1620 1630 1640 1650

S288c long AGAGGCGTCGTAATTGAAGGTTACCCAACAATCGTCTTATACCCAGGTGGTAAGAAGTCCGAATCTGTTGTGTACCAAGGTTCAAGATCCTTGGACTCTTTATTCGACTT 164(

SKQ2n long AGAGGCGTCGTAATTGAAGGTTACCCAACAATCGTCTTATACCCAGGTGGTAAGAAGTCCGAATCTGTTGTGTACCAAGGTTCAAGATCCTTGGACTCTTTATTCGACTT 165(

CATCAAGGAAAACGGTCACTTCGACGTCGACGGTAAGGCCTTGTACGAAGAAGCCCAGGAAAAAGCTGCTGAGGAAGCCGAACCTGACGCCGAAGCCGAAGCTGATGCTG

1660 1670 1680 1690 1700 1710 1720 1730 1740 1750 1760

S288c long CATCAAGGAAAACGGTCACTTCGACGTCGACGGTAAGGCCTTGTACGAAGAAGCCCAGGAAAAAGCTGCTGAGGAAGCCGA--------------------TGCTG 173:

SKQ2n long CATCAAGGAAAACGGTCACTTCGACGTCGACGGTAAGGCCTTGTACGAAGAAGCCCAGGAAAAAGCTGCTGAGGAAGCCGAAGCTGACGCCGAAGCCGAAGCTGACGCTG 176(

ACGCTGAATTGGCTGACGAAGAAGATGCCATTCACGATGAATTGTAATTCTGATCACTTTGGTTTTTCATTAAATAGAGATATATAAGAAATTTTCTAGGAAGTTTTTTT

1770 1780 1790 1800 1810 1820 1830 1840 1850 1860 1870

S288c long ACGCTGAATTGGCTGACGAAGAAGATGCCATTCACGATGAATTGTAATTCTGATCACTTTGGTTTTTCATTAAATAGAGATATATAAGAAATTTTCTAGGAAGTTTTTTT 184:

SKQ2n long ACGCTGAATTGGCTGACGAAGAAGATGCCATTCACGATGAATTGTAATTCTGATCACTTTGGTTTTTCATTAAATAGAGATATATAAGAAATTTTCTAGGAAGTTTTTTT 187(

AAAAAAAATCATAAAAAGATAAACGTTAAAATTCAAACACAATAGTTGTTCGCTATATTCGTCACACTGCACGAACGCCTTAX

1880 1890 1900 1910 1920 1930 1940 1950

S288c long AAAAAAA-TCATAAAAAGATAAACGTTAAAATTCAAACACAATAGTTGTTCGCTATATTCGTCACACTGCACGAACGCCTTA

SKQ2n long AAAAAAAATCATAAAAAGATAAACGTTAAAATTCAAACACAATAGTTCGTTCGCTATATTCGTCACACTGCACGAACGCCTTA 192:
195:

EP 1 709 181 B1

129

Figure 96

Figure 95

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9325676 A
- EP 0286424 A
- WO 0044772 A
- EP 286424 A
- US 6451559 B
- EP 0746611 A
- EP 074661 A
- EP 0293793 A
- EP 0509841 A
- EP 258067 A
- EP 431880 A
- EP 60057 A
- WO 9001063 A
- WO 03066824 A
- WO 9013653 A
- EP 73646 A
- US 4302386 A, Stevens
- WO 0179271 A
- US 20030221201 A
- US 20030226155 A

- WO 0179258 A
- WO 0179442 A
- WO 0179443 A
- WO 0179444 A
- WO 0179480 A
- EP 366400 A
- WO 9533833 A
- WO 9404687 A
- WO 9523857 A
- EP 251744 A
- WO 9204367 A
- EP 464590 A
- EP 319067 A
- WO 9637515 A
- US 5728553 A
- US 6291205 B
- EP 387319 A
- WO 03066085 A
- WO 2004015113 A

**Non-patent literature cited in the description**

- **HARTL.** *Nature,* 1996, vol. 381, 571-580
- **ROBINSON ; WITTRUP.** *Biotechnol. Prog.,* 1995, vol. 11, 171-177
- **FARQUHAR et al.** *Gene,* 1991, vol. 108, 81-89
- **ROBINSON et al.** *Bio/Technology,* 1994, vol. 12, 381-384
- **HAYANO et al.** *FEBS Letters,* 1995, vol. 377, 505-511
- **SHUSTA et al.** *Nature Biotechnology,* 1998, vol. 16, 773-777
- **BAO ; FUKUHARA.** *Gene,* 2001, vol. 272, 103-110
- **BOTSTEIN et al.** *Gene,* 1979, vol. 8, 17-24
- **ROSENBERG et al.** *Nature,* 1984, vol. 312, 77-80
- **WITTRUP.** *Biotechnol. Prog.,* 1997, vol. 13, 117-122
- **BAO.** *Yeast,* 2000, vol. 16, 329-341
- **MARTZEN et al.** *Science,* 1999, vol. 286, 1153-1155
- **VOLKERT et al.** *Microbiological Reviews,* 1989, vol. 53, 299
- **MURRAY et al.** *J. Mol. Biol.,* 1988, vol. 200, 601

- **PAINTING et al.** *J. Applied Bacteriology,* 1984, vol. 56, 331
- **FUTCHER.** *Yeast,* 1988, vol. 4, 27
- **TOH-E et al.** *Basic Life Sci.,* 1986, vol. 40, 425
- **BROACH et al.** *Cold Spring Harbor Symp. Quant. Biol.,* 1982, vol. 47, 1165-1174
- **WILLIAMSON.** *Yeast,* 1985, vol. 1, 1-14
- **VOLKERT ; BROACH.** *Cell,* 1986, vol. 46, 541
- **HARTLEY ; DONELSON.** *Nature,* 1980, vol. 286, 860
- **CAMERON et al.** *Nucl. Acids Res.,* 1977, vol. 4, 1429
- **KIKUCHI.** *Cell,* 1983, vol. 35, 487
- **LIVINGSTON ; HAHNE.** *Proc. Natl. Acad. Sci. USA,* 1979, vol. 76, 3727
- **HOLLENBERG.** *Current Topics in Microbiology and Immunobiology,* 1982, vol. 96, 119
- **MEAD et al.** *Molecular & General Genetics,* 1986, vol. 205, 417
- **FUTCHER ; COX.** *J. Bacteriol.,* 1984, vol. 157, 283
- **BACHMAIR ; RUIS.** *Monatshefte für Chemie,* 1984, vol. 115, 1229
- **BROACH ; HICKS.** *Cell,* 1980, vol. 21, 501

- **SUTTON ; BROACH.** *Mol. Cell. Biol.,* 1985, vol. 5, 2770
- **SENECOFF et al.** *Proc. Natl. Acad Sci. U.SA.,* 1985, vol. 82, 7270
- **JAYARAM.** *Proc. Natl. Acad. Sci. U.S.A.,* 1985, vol. 82, 5875
- **SLEEP et al.** *Yeast,* 2001, vol. 18, 403
- **ROSE ; BROACH.** *Methods Enzymol.,* 1990, vol. 185, 234
- **SENGUPTA et al.** *J. Bacteriol.,* 2001, vol. 183, 2306
- **REYNOLDS et al.** *Mol. Cell. Biol.,* 1987, vol. 7, 3566
- **SENECOFF et al.** *Proc. Natl. Acad. Sci. U.S.A.,* 1985, vol. 82, 7270
- **JAYARAM.** *Proc. Natl. Acad. Sci. U.SA.,* 1985, vol. 82, 5875
- **MEYER-LEON et al.** *Cold Spring Harbor Symposia On Quantitative Biology,* 1984, vol. 49, 797
- **PRASAD et al.** *Proc. Natl. Acad. Sci. U.S.A.,* 1987, vol. 84, 2189
- **CHEN et al.** *Cell,* 1992, vol. 69, 647
- **GRAINGE et al.** *J. Mol. Biol.,* 2001, vol. 314, 717
- **CHINERY ; HINCHLIFFE.** *Curr. Genet.,* 1989, vol. 16, 21
- **SLEEP et al.** *Biotechnology (N Y),* 1991, vol. 9, 183
- **ROSE ; BROACH.** *Methods Enzymol.,* 1990, vol. 185, 234-279
- **CHINERY ; HINCHLIFFE.** *Curr. Genet.,* 1989, vol. 16, 21-25
- **KERRY-WILLIAMS et al.** *Yeast,* 1998, vol. 14, 161-169
- **SLEEP et al.** *Yeast,* 2001, vol. 18, 403-421
- **VALKONEN et al.** *Applied Environ. Micro.,* 2003, vol. 69, 2065
- **HJELMQVIST et al.** *Genome Biology,* 2002, vol. 3 (6
- **HILLSON et al.** *Methods Enzymol.,* 1984, vol. 107, 281-292
- **FREEDMAN.** *Trends Biochem. Sci.,* 1984, vol. 9, 438-41
- **ROTH ; PIERCE.** *Biochemistry,* 1987, vol. 26, 4179-82
- **BULLEID ; FREEDMAN.** *Nature,* 1988, vol. 335, 649-51
- **FREEDMAN et al.** *Biochem. Soc. Symp.,* 1989, vol. 55, 167-192
- **SOLOVYOV et al.** *J. Biol. Chem.,* 2004, vol. 279 (33), 34095-34100
- **SCHERENS et al.** *Yeast,* 1991, vol. 7, 185-193
- **CREIGHTON et al.** *J Mol. Biol.,* 1980, vol. 142, 43-62
- **FREEDMAN et al.** *Biochem. Soc. Trans.,* 1998, vol. 16, 96-9
- **GILBERT.** *Biochemistry,* 1989, vol. 28, 7298-7305
- **LUNDSTROM ; HOLMGREN.** *J. Biol. Chem.,* 1990, vol. 265, 9114-9120
- **HAWKINS ; FREEDMAN.** *Biochem. J.,* 1990, vol. 275, 335-339
- **ROTHMAN.** *Cell,* 1989, vol. 59, 591-601
- **KASKA et al.** *Biochem. J,* 1990, vol. 268, 63-68
- **EDMAN et al.** *Nature,* 1985, vol. 317, 267-270
- **YAMAUCHI et al.** *Biochem. Biophys. Res. Comm.,* 1987, vol. 146, 1485-1492
- **PIHLAJANIEMI et al.** *EMBO J.,* 1987, vol. 6, 643-9
- **PARKKONEN et al.** *Biochem. J.,* 1988, vol. 256, 1005-1011
- **BECKER ; CRAIG.** *Eur. J. Biochem.,* 1994, vol. 219, 11-23
- **KIM et al.** *Proc. Natl. Acad Sci. USA.,* 1998, vol. 95, 12860-12865
- **NGOSUWAN et al.** *J. Biol. Chem.,* 2003, vol. 278 (9), 7034-7042
- **GLOVER ; LINDQUIST.** *Cell,* 1998, vol. 94, 73-82
- **DEMOLDER et al.** *J. Biotechnol,* 1994, vol. 32, 179-189
- **SEEDORF ; SILVER.** *Proc. Natl. Acad. Sci. USA.,* 1997, vol. 94, 8590-8595
- **RYAN ; WENTE.** *Curr. Opin. Cell Biol.,* 2000, vol. 12, 361-371
- **PEMBERTON et al.** *Curr. Opin. Cell Biol.,* 1998, vol. 10, 392-399
- **ISOYAMA et al.** *J. Biol. Chem.,* 2001, vol. 276 (24), 21863-21869
- **UETA et al.** *J. Biol. Chem.,* 2003, vol. 278 (50), 50120-50127
- **MOSAMMAPARAST et al.** *J. Biol. Chem.,* 2002, vol. 277 (1), 862-868
- **CHOW et al.** *J. Cell. Sci.,* 1992, vol. 101 (3), 709-719
- **THOMPSON et al.** *Nucleic Acids Res.,* 1994, vol. 22 (22), 4673-80
- **KHAN et al.** *Int. J. Biol. Macromol.,* 2002, vol. 30 (3-4), 171-8
- **PATERS.** All About Albumin: Biochemistry, Genetics and Medical Applications. Academic Press, Inc, 1996, 170-181
- **DOCKAL, M. et al.** *J. Biol. Chem.,* 1999, vol. 274, 29303-29310
- **TESTA.** Proteins of iron metabolism. CRC Press, 2002
- Iron carriers and iron proteins. **HARRIS ; AISEN.** Physical Bioinorganic Chemistry. VCH, 1991, vol. 5
- **MASON et al.** *Biochemistry,* 1993, vol. 32, 5472
- **MASON et al.** *Biochem. J.,* 1998, vol. 330 (1), 35

- **MASON et al.** *Protein Expr. Purif.,* 1996, vol. 8, 119
- **MASON et al.** *Protein Expr. Purif.,* 1991, vol. 2, 214
- **SHIN et al.** *Proc. Natl. Acad. Sci. USA,* 1995, vol. 92, 2820
- **ALI et al.** *J. Biol. Chem.,* 1999, vol. 274, 24066
- **MASON et al.** *Biochemistry,* 2002, vol. 41, 9448
- **SHIN et al.** *Proc Natl Acad Sci U S A,* 1995, vol. 92, 2820
- **ALI et al.** *J Biol Chem,* 1999, vol. 274, 24066
- **SMITH et al.** *Science,* 1955, vol. 229, 1219-1224
- **SAMBROOK et al.** Molecular Cloning: A Laboratory Manual. 2001
- **IRIE et al.** *Gene,* 1991, vol. 108 (1), 139-144
- **IRIE et al.** *Mol. Gen. Genet.,* 1991, vol. 225 (2), 257-265
- **COHEN et al.** *Proc. Natl. Acad Sci. USA,* 1972, vol. 69, 2110
- **SAMBROOK et al.** Molecular Cloning, A Laboratory Manual. Cold. Spring Harbor Laboratory, 2001
- **SHERMAN et al.** Methods In Yeast Genetic, A Laboratory Manual. Cold Spring Harbor, 1986
- **BEGGS.** *Nature,* 1978, vol. 275, 104-109
- **BECKER ; GUARENTE.** *Methods Enzymol.,* 1990, vol. 194, 182
- **PIPER ; CURRAN.** *Curr. Genet.,* 1990, vol. 17, 119
- **SOUTHERN.** *J. Mol. Biol.,* 1975, vol. 98, 503
- **BERENT et al.** *Biotech.,* 1985, vol. 3, 208
- **BAO et al.** *Yeast,* 2000, vol. 16, 329-341
- **LAMBERT et al.** *Proc. Natl. Acad. Sci. USA,* 2001, vol. 98, 4652-4657
- **ITO et al.** *J. Bacteriol.,* 1983, vol. 153, 163
- **ELBLE.** *Biotechniques,* 1992, vol. 13, 18
- **SLEEP et al.** *Yeast,* 2002, vol. 18, 403
- **CROUZET ; TUITE.** *Mol. Gen. Genet.,* 1987, vol. 210, 581-583
- **CRIETZ ; SUGINO.** *Gene,* 1988, vol. 74, 527-534
- **SLEEP et al.** *Biotechnology (N.Y.),* 1990, vol. 8, 42
- **SLEEP et al.** *Yeast,* 2001, vol. 18, 403-441
- Rocket immunoelectrophoresis. **WEEKE, B.** A manual of quantitative immunoelectrophoresis. Methods and applications. Universitetsforlaget, 1976
- **FINNIS et al.** *Eur. J. Biochem.,* 1993, vol. 212, 201-210
- **ROSE ; BROACH.** *Meth. Enzymol.,* 1990, vol. 185, 234-279
- **CASHMORE et al.** *Mol. Gen. Genet.,* 1986, vol. 203, 154-162
- **ZEALEY et al.** *Mol. Gen. Genet.,* 1988, vol. 211, 155-159
- **GIETZ ; SUGINO.** *Gene,* 1988, vol. 74, 527-534
- **MEAD et al.** *Mol. Gen. Genet.,* 1986, vol. 205, 417-421
- **SLEEP et al.** *Bio/Technology,* 1991, vol. 9, 183-187
- **SLEEP, D. et al.** *Bio/Technology,* 1991, vol. 9, 183-187
- **LAMBERT et al.** *PNAS,* 2001, vol. 98, 4652-4657
- **HOHEISEL.** *Biotechniques,* 1994, vol. 17, 456-460
- **TOYN et al.** *Yeast,* 2000, vol. 16, 553-560
- **LEE.** *Biotechniques,* 1992, vol. 12, 677